# EUROPÄISCHE PATENTSCHRIFT

(11) **EP 0 325 130 B1**
(45) Veröffentlichungstag und Bekanntmachung des Hinweises auf die Patenterteilung: **05.11.2003**
(21) Anmeldenummer: 89100250.3
(22) Anmeldetag: 09.01.1989
(51) Int. Cl.: C07D 213/65, A61K 31/44, C07D 213/80, C07D 213/82, C07D 213/85, C07D 213/89, C07D 401/06, C07D 401/12, C07D 405/04, C07D 405/06, C07D 405/12

(54) **Substituierte Pyridine**
Substituted pyridines
Pyridines substituées

(30) Priorität: 20.01.1988 DE 3801406; 11.07.1988 IT 2131788
(43) Veröffentlichungstag der Anmeldung: 26.07.1989
(60) Teilanmeldung: 01109309.3
(73) Patentinhaber: BAYER AG, 51368 Leverkusen (DE)
(72) Erfinder: Angerbauer, Rolf, Dr., D-5600 Wuppertal 1 (DE); Fey, Peter, Dr., D-5600 Wuppertal 1 (DE); Hübsch, Walter, Dr., D-5600 Wuppertal 1 (DE); Philipps, Thomas, Dr., D-5000 Koeln 80 (DE); Bischoff, Hilmar, Dr., D-5600 Wuppertal 2 (DE); Petzinna, Dieter, Dr. Dr., D-4000 Duesseldorf 21 (DE); Schmidt, Delf, Dr., D-5600 Wuppertal 1 (DE); Thomas, Günter, Dr., I-20020 Arese (Mi) (IT)

(56) Entgegenhaltungen:
- EP-A- 0 306 929
- EP-A- 0 307 342
- EP-A- 0 319 847
- US-A- 4 681 893

## Beschreibung

Die Erfindung betrifft substituierte Pyridine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP-A 22 478; US 4 231 938]. Darüberhinaus sind auch bestimmte Indolderivate bzw. Pyrazolderivate Inhibitoren der HMG-CoA-Reduktase [EP-A 1 114 027; US-Patent 4 613 610].

Es wurden nun substituierte Pyridine der allgemeinen Formel (Ia) und (Ib) 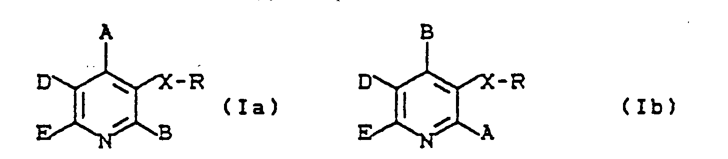 in welcher
A
   - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
   - für Phenyl oder Naphthyl steht, das bis zu 4-fach gleich oder verschieden substituiert sein kann durch C₁-C₆-Alkyl, das gegebenenfalls durch Hydroxy oder C₁-C₆-Alkoxy substituiert sein kann, C₁-C₆-Alkoxy, C₁-C₆- Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom oder Cyano,
   - für Methyl, Ethyl, Propyl, Isopropyl, Butyl oder tert. Butyl steht,
B
   - für Cyclopropyl, Cyclopentyl oder Cyclohexyl steht oder
   - für C₁-C₆-Alkyl steht, das substituiert sein kann durch Fluor, Chlor, Brom, Cyano, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆- Alkoxycarbonyl, Benzoyl, C₁-C₆-Alkylcarbonyl, durch eine Gruppe der Formel -NR¹R²,
      worin
      R¹ und R² gleich oder verschieden sind und C₁-C₆- Alkyl, Phenyl, Benzyl, Acetyl, Benzoyl, Phenylsulfonyl oder C₁-C₆-Alkylsulfonyl bedeuten,
   oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
D,E
   - gleich oder verschieden sind und
   - für Cyclopropyl, Cyclopentyl oder Cyclohexyl stehen, oder
   - für geradkettiges oder verzweigtes C₁-C₆-Alkyl stehen, das im Fall des Substituenten D substituiert ist und im Fall des Substituenten E substituiert sein kann
      durch Azido, Fluor, Chlor, Brom, Cyano, Hydroxy, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR¹R²,
   worin R¹ und R² die oben angegebene Bedeutung haben,
   oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, C₁-C₆- Alkyl, C₁-C₆-Alkoxy, Trifluormethyl, oder Trifluormethoxy substituiert sein können,
   - für Thienyl, Furyl, Thiazolyl, Tetrazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, das bis zu 2-fach gleich oder verschieden substituiert sein kann durch Fluor, Chlor, Brom, C₁-C₆-Alkyl, C₁-C₆- Alkoxy, Phenyl, Phenoxy, Trifluormethyl, Trifluormethoxy oder C₁-C₆-Alkoxycarbonyl, oder
   - für Naphthyl steht, das bis zu 4-fach gleich oder verschieden substituiert sein kann durch C₁-C₆-Alkyl, das gegebenenfalls durch Hydroxy oder C₁-C₆-Alkoxy substituiert sein kann, C₁-C₆-Alkoxy, C₁-C₆-Alkyl-thio, C₁-C₆- Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR¹R²,
   wobei
   R¹ und R² die oben angegebene Bedeutung haben,
   oder
   - für eine Gruppe der Formel -CR¹¹R¹²-Y steht,
   R¹¹ und R¹² gleich oder verschieden sein können und
   - für Wasserstoff, oder
   - für C₁-C₆- Alkyl stehen, das gegebenenfalls durch Hydroxy, Fluor, Chlor, C₁-C₆-Alkoxy oder C₁-C₆-Alkoxycarbonyl substituiert sein kann, oder
   - für Cyclopropyl, Cyclopentyl oder Cyclohexyl stehen, oder
   R¹¹ und R¹² gemeinsam einen gesättigten oder ungesättigten carbocyclischen oder heterocyclischen Ring mit bis zu 6 Kohlenstoffatomen bilden,
   - Y -: eine Gruppe der Formel -NR¹³R¹⁴, -COR¹⁵, -S-R¹⁶, SO-R¹⁶, -SO₂R¹⁶, -OR¹⁷ oder -N₃ bedeutet,
   wobei
   R¹³ und R¹⁴ gleich oder verschieden sind und
   - für Wasserstoff, C₁-C₆-Alkyl, Phenyl oder Benzyl stehen, wobei die genannten Reste durch Fluor, Chlor, Brom, C₁-C₆-Alkyl, C₁-C₆-Alkoxy oder Trifluormethyl substituiert sein können, oder
   - für eine Gruppe der Formel -COR¹⁵, -SO₂R¹⁶ stehen,
   oder R¹³ und R¹⁴ gemeinsam eine Alkylenkette bilden, die durch O, N, S, N-C₁-C₆-Alkyl, N-Benzyl, N-Phenyl, N-Carbamoyl oder N-C₁-C₆-Alkoxycarbonyl unterbrochen sein kann,
   R¹⁵
   - eine Gruppe -NR¹⁸R¹⁹ bedeutet, oder
   - C₁-C₆-Alkyl oder C₁-C₆-Alkoxy bedeutet, oder
   - gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
   R⁶
   - Cyclopropyl, Cyclopentyl, Cyclohexyl, oder
   - gegebenenfalls durch Cyano, Fluor, Chlor, Brom, Trifluormethyl oder C₁-C₆-Alkoxycarbonyl substituiertes geradkettiges oder verzweigtes C₁-C₆-Alkyl bedeutet, oder
   - gegebenenfalls ein- oder mehrfach gleich oder verschieden durch C₁-C₆-Alkyl, C₁-C₆-Alkoxy, Fluor, Chlor, Brom, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
   - Trimethylsilyl oder Dimethylethylsilyl bedeutet, oder
   - eine Gruppe -NR⁹R¹⁰ bedeutet,
      wobei
      R⁹ und R¹⁰ die oben angegebene Bedeutung haben,
   R¹⁷
   - für Wasserstoff, oder
   - für Cyclopropyl, Cyclopentyl oder Cyclohexyl steht, oder
   - für C₁-C₆-Alkyl steht, das substituiert sein kann durch Fluor, Chlor, Brom, Cyano, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-A.lkoxycarbonyl, Benzoyl, C₁-C₆-A.lkylcarbonyl, durch eine Gruppe der Formel -NR¹R²,
      worin
      R¹ und R² die oben angegebene Bedeutung haben,
      oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl oder Trifluormethoxy substituiert sein kann,
   - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
   - für Benzyl, Phenyl oder Naphthyl steht, die bis zu 4-fach gleich oder verschieden substituiert sein können durch C₁-C₆-Alkyl, C₁-C₆-Alkoxy, C₁-C₆-Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR¹R²,
      wobei
      R¹ und R² die oben angegebene Bedeutung haben,
      oder
   - für 2,5-Dioxo-tetrahydropyrryl,
   - für Tetrahydropyranyl, oder
   - für Dimethyl-tert.butylsilyl, Tripropylsilyl oder Tributylsilyl steht, oder
   - eine Gruppe der Formel COR¹⁶ bedeutet,
   wobei
   R¹⁶ die oben angegebene Bedeutung hat,
   und
   R¹⁸ und R¹⁹ gleich oder verschieden sind und
   - Wasserstoff bedeuten, oder
   - gegebenenfalls durch Cyano, Fluor, Chlor oder Brom substituiertes C₁-C₆-Alkyl bedeuten, oder
   - gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeuten,
   oder
   D, E gemeinsam einen Ring der Formel 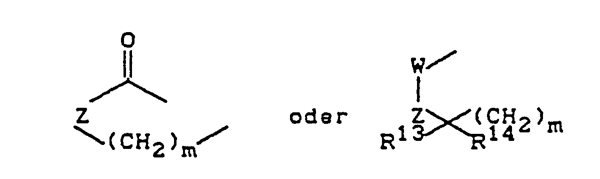 bilden,
   worin
   W - für eine Gruppe der Formel C=O oder für CH-OH steht,
   m - für eine Zahl 1 oder 2 steht,
   Z - für O, CH₂ oder NHR²⁰ steht, R¹³ und R¹⁴ die oben angegebene Bedeutung haben,
   und
   R²⁰ - für Wasserstoff, C₁-C₆-Alkyl, Phenyl, Benzyl, Carbamoyl oder C₁-C₆-Alkoxy-carbonyl steht,
   X - für eine Gruppe der Formel -CH=CH- steht,
   R - für eine Gruppe der Formel 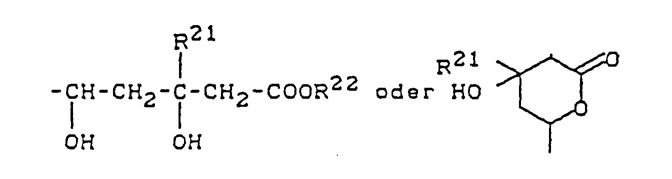 steht,
   worin
   R²¹ - Wasserstoff oder C₁-C₆-Alkyl bedeutet, und
   R²² - Wasserstoff C₁-C₆-Alkyl, Phenyl oder Benzyl bedeutet, oder ein Kation bedeutet,
sowie deren Oxidationsprodukte.

Unter den Oxidationsprodukten der erfindungsgemäßen Verbindungen der allgemeinen Formel (Ia) und (Ib) versteht man die entsprechenden Verbindungen des Pyridin-N-oxids.

Überraschenderweise zeigen die erfindungsgemäßen substituierten Pyridine eine überlegene inhibitorische Wirkung auf die HMG-CoA Reduktase (3-Hydroxy-3-methyl-glutaryl Coenzym A Reduktase).

Steht R²² für einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester (C₁ bis C₄) und Aralkylester (C₇ bis C₁₀), bevorzugt Niederalkylester sowie Benzylester. Darüber hinaus seien die folgenden Esterreste genannt: Methylester, Ethylester, Propylester, Benzylester.

Steht R²² für ein Kation so, ist bevorzugt ein physiologisch verträgliches Metall-oder Ammoniumkation gemeint. Bevorzugt sind hierbei Alkali-bzw. Erdalkalikationen wie beispielsweise Natrium-, Kalium-, Magnesium- oder Calciumkationen, sowie Aluminium-oder Ammoniumkationen, sowie nicht-toxische substituierte Ammoniumkationen aus Aminen wie Diniederalkylamine, Triniederalkylamine, Prokain, Dibenzylamin, N,N'-Dibenzylethylendiamin, N-Benzyl-β-phenylethylamin, N-Methylmorpholin oder N-Ethylmorpholin, 1-Ephenamin, Dihydroabiethylamin, N,N'-Bis-dihydroabiethylethylendiamin, N-Niederalkylpiperidin und andere Amine, die zur Bildung von Salzen verwendet werden können.

Bevorzugt sind solche Verbindungen der allgemeinen Formeln (Ia) und (Ib)
in welchen
A
   - für Thienyl, Furyl, Pyridyl, Pyrimidyl, Chinolyl oder Isochinolyl steht, das durch Fluor, Chlor, Methyl, Methoxy oder Trifluormethyl substituiert sein kann, oder
   - für Phenyl steht, das bis zu 3-fach gleich oder verschieden substituiert sein kann durch Methyl, Hydroxymethyl, Ethyl, Propyl, Isopropyl, Hydroxyethyl, Hydroxypropyl, Butyl, Isobutyl, Methoxymethyl, Ethoxymethyl, Propoxymethyl, tert.-Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
   - für Methyl, Ethyl, Propyl, Isopropyl, Butyl oder tert. Butyl steht,
B
   - für Cyclopropyl, Cyclopentyl oder Cyclohexyl steht, oder
   - für Methyl, Ethyl, Propyl, Isopropyl, Butyl, sec.-Butyl oder tert.Butyl steht, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl Butoxycarbonyl Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
D,E
   - gleich oder verschieden sind und
   - für Cyclopropyl, Cyclopentyl oder Cyclohexyl stehen, oder
   - für Methyl, Ethyl, Propyl, Isopropyl, Butyl, Isobutyl, tert.Butyl, Pentyl, Isopentyl, Hexyl oder Isohexyl stehen, die im Fall des Substituenten D substituiert sind und im Fall des Substituenten E substituiert sein können
      durch Azido, Fluor, Chlor, Brom, Iod, Cyano, Hydroxy, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR¹R²,
      wobei
      R¹ und R² gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten, oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
   - für Thienyl, Furyl, Pyridyl, Pyrimidyl, Pyrazinyl, Tetrazolyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl stehen, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.-Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
   - für eine Gruppe der Formel -CR¹¹R¹²-Y stehen,
      worin
      R¹¹ und R¹² gleich oder verschieden sind und
      - für Wasserstoff, oder
      - für Methyl, Ethyl, Propyl, Isopropyl stehen, das gegebenenfalls durch Hydroxy, Fluor, Chlor, Methoxy, Ethoxy, Methoxycarbonyl, Ethoxycarbonyl substituiert sein kann, oder
      - für Cyclopropyl, Cyclopentyl oder Cyclohexyl stehen,
         oder R¹¹ und R¹² gemeinsam für Cyclopropyl, Cyclopentyl oder Cyclohexyl stehen,
         und
         - Y -: eine Gruppe der Formel -NR¹³R¹⁴, -COR¹⁵, -SR¹⁶, -SO-R¹⁶, -SO₂R¹⁶, -OR¹⁷ oder -N₃ bedeutet,
      wobei
      R¹³ und R¹⁴ gleich oder verschieden sind, und
      - für Wasserstoff, Methyl, Ethyl, Propyl, Isopropyl, Butyl, Isobutyl, oder
      - für gegebenenfalls durch Fluor, Chlor, Methyl oder Methoxy subsituiertes Phenyl, oder
      - für eine Gruppe -COR¹⁵ oder -SO₂R¹⁶ stehen, oder
      R¹³ und R¹⁴ gemeinsam mit dem Stickstoffatom einen Ring der Reihe Piperidin, Piperazin, Morpholin, Morpholin-N-oxid, N-Niederalkylpiperazin, Benzylpiperazin oder Phenylpiperazin bilden,
      R¹⁵
      - Wasserstoff bedeutet, oder
      - eine Gruppe -NR¹⁸R¹⁹ bedeutet, oder
      - Methyl, Ethyl, Propyl, Isopropyl, Methoxy, Ethoxy, Propoxy, Isopropoxy, oder
      - gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,
      R¹⁶
      - gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl, Isobutyl oder Isopentyl bedeutet, oder
      - gegebenenfalls ein- oder mehrfach gleich oder verschieden durch Methyl, Ethyl, Propyl, Isopropyl, Methoxy, Fluor oder Chlor substituiertes
      Benzyl, Phenyl, Naphthyl, Thienyl, Furyl, Pyridyl, Pyrimidyl, Chinolyl oder Isochinolyl bedeutet, oder
   - Trimethylsilyl oder Dimethylethylsilyl bedeutet,
   - eine Gruppe -NR⁹R¹⁰ bedeutet,
      wobei
      R⁹ und R¹⁰ die oben angegebene Bedeutung haben,
      R¹⁷
      - für Wasserstoff, Cyclopropyl, Cyclopentyl oder Cyclohexyl steht, oder
      - für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR¹R²,
         wobei
         R¹ und R² gleich oder verschieden sind und Methyl, Ethyl, Propyl, Isopropyl, Butyl, Isobutyl, tert.Butyl, Phenyl, Benzyl, Acetyl, Methyl-sulfonyl, Ethylsulfonyl, Propylsulfonyl, Isopropyl-sulfonyl oder Phenylsulfonyl bedeuten,
         oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroarylund Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein kann, oder
      - für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.-Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.-Butoxycarbonyl substituiert sein können, oder
      - für Benzyl oder Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe -NR¹R² substituiert sein kann,
         wobei
         R¹ und R² die oben angegebene Bedeutung haben,
         oder
      - für 2,5-Dioxo-tetrahydropyrryl,
      - für Tetrahydropyranyl steht, oder
      - für Dimethyl-tert,butylsilyl oder Trimethylsilyl steht, oder
      - eine Gruppe -COR¹⁶ bedeutet,
         wobei
         R¹⁶ die oben angegebene Bedeutung hat,
      und
      R¹⁸ und R¹⁹ gleich oder verschieden sind und
   - Wasserstoff bedeuten, oder - gegebenenfalls durch Fluor oder Chlor substituiertes Methyl, Ethyl, Propyl, Isopropyl, Butyl, Isobutyl, Pentyl, Isopentyl, Hexyl oder Isohexyl bedeuten, oder
   - Phenyl bedeuten, das durch Fluor, Chlor, Methyl oder Methoxy substituiert sein kann,
oder
D und E gemeinsam einen Ring der Formel 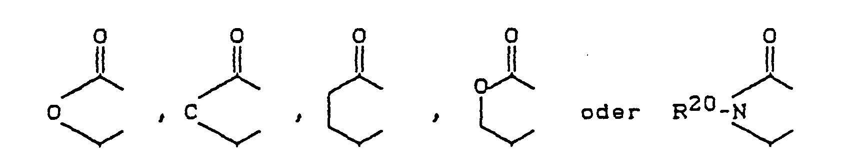 bilden,
worin
- R²⁰ -: für Wasserstoff, Methyl, Ethyl, Propyl, Isopropyl, Carbamoyl, Methoxy-carbonyl oder Ethoxycarbonyl steht,
- X -: für eine Gruppe der Formel -CH=CH- steht,
- R -: für eine Gruppe der Formel 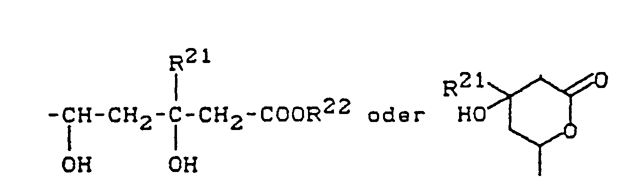
steht,
worin
R²¹ - Wasserstoff, Methyl, Ethyl, Propyl, Isopropyl, Butyl, Isobutyl oder tert.Butyl bedeutet,
und
R²² - Wasserstoff, Methyl, Ethyl, Propyl, Isopropyl, Butyl, Isobutyl, tert.Butyl oder Benzyl bedeutet, oder ein Natrium-, Kalium-, Calcium -, oder Magnesium- oder Ammoniumion bedeutet,
sowie deren Oxidationsprodukte,

Besonders bevorzugt sind Verbindungen de allgemeiren Formeln (Ia) und (Ib)
in welchen
A
   - für Thienyl oder Furyl steht, oder
   - für Phenyl steht, das bis zu 2-fach gleich oder verschieden durch Methyl, Hydroxymethyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Trifluormethyl substituiert sein kann,
   - für Methyl, Ethyl, Propyl oder Isopropyl steht,
B
   - für Cyclopropyl, Methyl, Ethyl, Propyl, Isopropyl, Butyl, Isobutyl oder tert.Butyl steht, das durch Fluor, Chlor, Methoxy, Phenyl oder Phenoxy substituiert sein kann,
D,E gleich oder verschieden sind und
   - für Cyclopropyl, Cyclopentyl oder Cyclohexyl stehen, oder
   - für Methyl, Ethyl, Propyl, Isopropyl, Butyl, Isobutyl, tert.Butyl, Pentyl, Isopentyl, Hexyl oder Isohexyl stehen, die im Fall des Substituenten D substituiert sind und im Fall des Substituenten E substituiert sein können durch Azido, Fluor, Chlor, Iod, Methoxy, Ethoxy, Propoxy, Isopropoxy, Methylthio, Ethylthio, Methylsulfonyl, Ethylsulfonyl, Ethoxycarbonyl, Propoxycarbonyl, Isopropoxycarbonyl oder durch eine Gruppe der Formel NR¹R²,
wobei
R¹ und R² gleich oder verschieden sind und
- für Wasserstoff, Methyl, Ethyl, Propyl, Isopropyl, Phenyl oder Benzyl stehen, oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy, oder
- für gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Tetrazolyl, Benzthiazolyl oder Benzimidazolyl stehen, oder
- für eine Gruppe der Formel -CR¹¹R¹²-Y stehen
   worin
   R¹¹ und R¹² Wasserstoff, Methyl oder Ethyl bedeuten,
   und
   - Y -: eine Gruppe der Formel -NR¹³R¹⁴, -COR¹⁵, -S-R¹⁶, -SO-R¹⁶, -SO₂R¹⁶ oder -OR¹⁷ bedeutet,
   wobei
   R¹³ und R¹⁴ gleich oder verschieden sind, und
   - für Wasserstoff, Methyl, Ethyl, Propyl, oder
   - für eine Gruppe -COR¹⁵ oder -SO₂R¹⁶ stehen
      oder R¹³ und R¹⁴ gemeinsam mit dem Stickstoffatom einen Ring der Reihe Morpholin oder Morpholin-N-oxid bilden,
      und
   R¹⁵
   - Wasserstoff oder Methyl bedeutet, oder
   - eine Gruppe -NR¹⁸R¹⁹ bedeutet, oder
   - Methyl, Ethyl, Propyl, Methoxy oder Ethoxy bedeutet,
   R¹⁶
   - Trifluormethyl, Methyl, Ethyl, Propyl, Isopropyl, Butyl, Isobutyl, Isopentyl oder Benzyl bedeutet, oder
   - gegebenenfalls durch ein- oder mehrere Methyl oder Chlor substituiertes Phenyl, oder Naphthyl bedeutet,
   - Trimethylsilyl oder Dimethylethylsilyl, oder
   - eine Gruppe -NR⁹R¹⁰ bedeutet,
      wobei
      R⁹ und R¹⁰ die oben angegebene Bedeutung haben,
   R¹⁷
   - für Wasserstoff, Cyclopropyl, Cyclopentyl oder Cyclohexyl steht, oder
   - für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Methoxy, Ethoxy, Propoxy, Isopropoxy, Methylthio, Ethylthio, Methylsulfonyl, Ethylsulfonyl, Ethoxycarbonyl, Propoxycarbonyl, Isopropoxycarbonyl oder durch eine Gruppe der Formel NR¹R²,
      wobei
      R¹ und R² gleich oder verschieden sind und
      - für Wasserstoff, Methyl, Ethyl, Propyl, Isopropyl, Phenyl oder Benzyl stehen,
      oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy, oder
   - für gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Benzthiazolyl oder Benzimidazolyl steht, oder
   - für Benzyl oder Phenyl steht, das bis zu 2-fach gleich oder verschieden durch Methyl, Ethyl, Propyl, Isopropyl, Butyl, Isobutyl, tert, Butoxy, Methoxy, Ethoxy, Propoxy, Isopropoxy, Methylthio, Ethylthio, Methylsulfonyl, Ethylsulfonyl, Phenyl, Phenoxy, Phenylsulfonyl, Benzyloxy, Fluor, Chlor, Brom, Cyano, Trifluormethyl, Trifluormethoxy, Methoxycarbonyl, Ethoxycarbonyl oder eine Gruppe der Formel -NR¹R² substituiert sein kann,
      wobei
      R¹ und R² die oben angegebene Bedeutung haben,

   - für 2,5-Dioxo-tetrahydropyrryl oder
   - für Tetrahydropyranyl, oder
   - für Dimethyl-tert.butylsilyl oder Trimethylsilyl steht, oder
   - eine Gruppe -COR¹⁶ bedeutet,
      wobei
      R¹⁶ die oben angegebene Bedeutung hat,
   und
   R¹⁸ und R¹⁹ gleich oder verschieden sind und
   - Wasserstoff bedeuten, oder
   - Methyl, Ethyl, Propyl, Isopropyl, Butyl oder Isobutyl bedeuten, oder
   - Phenyl bedeuten,
oder
D und E gemeinsam einen Ring der Formel 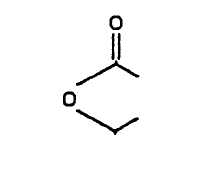 bilden,
- X -: für eine Gruppe der Formel 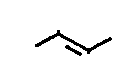
(E-konfiguriert) steht , und
- R -: für eine Gruppe der Formel 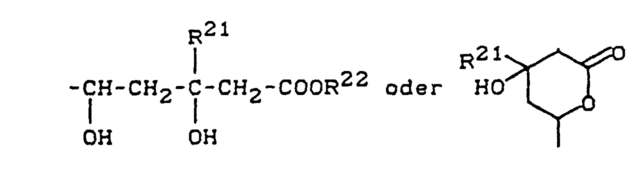 steht,
worin
- R²¹ -: Wasserstoff bedeutet
und
- R²2 -: Wasserstoff, Methyl oder Ethyl bedeutet, oder ein Natrium- oder Kaliumion bedeutet
und deren Oxidationsprodukte.

Die erfindungsgemäßen substituierten Pyridine der allgemeinen Formel (Ia, b) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes R ergeben sich unterschiedliche Stereoisomere, die im folgenden näher erläutert werden sollten:
a) Steht die Gruppe -X- für eine Gruppe der Formel -CH=CH- , so können die erfindungsgemäßen Verbindun-gen in zwei stereoisomeren Formen existieren, die an der Doppelbindung E-konfiguriert (II) oder Z-konfiguriert (III) sein können: 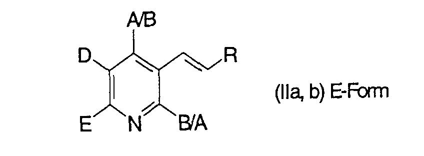 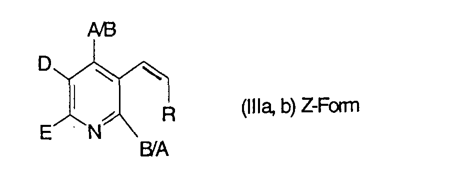 (A, B, D, E und R haben die oben angegebene Bedeutung).
   Bevorzugt sind diejenigen Verbindungen der allgemeinen Formel (I) die E-konfiguriert sind (II).
b) Steht der Rest -R- für eine Gruppe der Formel 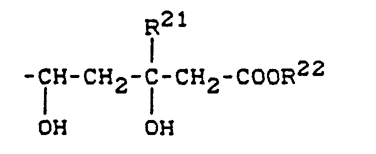
   so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen. 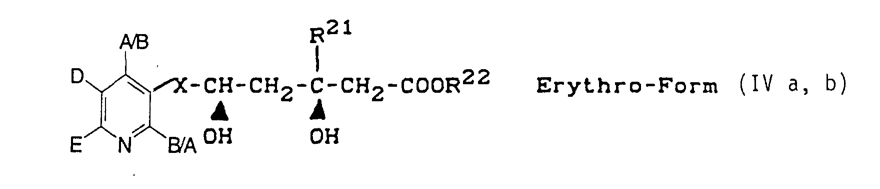 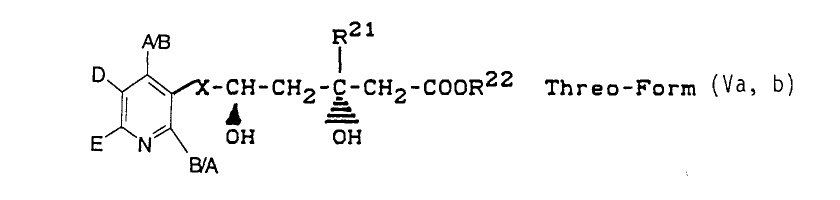 Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).
   Bevorzugt sind hierbei die Erythro-konfigurierten Isomeren, besonders bevorzugt das 3R,5S-Isomere sowie das 3R,5S-3S,5R-Racemat.
c) Steht der Rest -R- für eine Gruppe der Formel 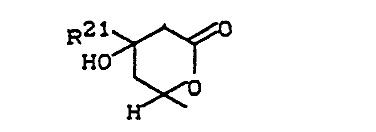
   so besitzen die substituierten Pyridine mindestens zwei asymmetrische Kohlenstoffatome, nämlich das Kohlenstoffatom an dem die Hydroxygruppe gebunden ist, und das Kohlenstoffatom an welchem der Rest der Formel 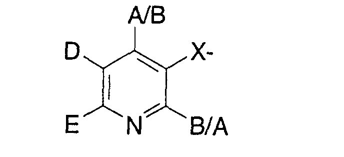 gebunden ist. Je nach der Stellung der Hydroxygruppe zur freien Valenz am Lactonring können die substituierten Pyridine als cis-Lactone (VI) oder als trans-Lactone (VII) vorliegen. 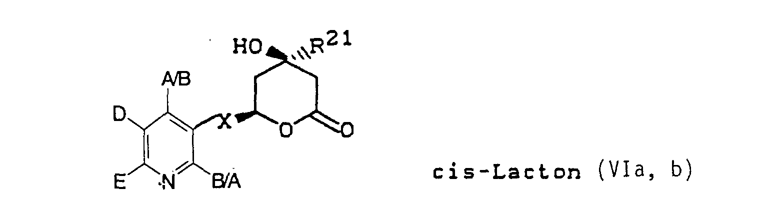 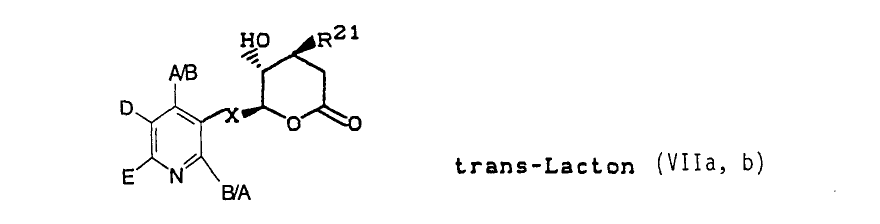
   Sowohl vom cis-Lacton aus als auch vom trans-Lacton existieren wiederum jeweils zwei Isomeren nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomeren sind die trans-Lactone. Besonders bevorzugt ist hierbei das 4R,6S-Isomere (trans) sowie das 4R,6S-4S,6R-Racemat.

Außerdem wurde ein Verfahren zur Herstellung der substituierten Pyridine der allgemeinen Formel (Ia, b)
gefunden,
das dadurch gekennzeichnet ist, daß man
Ketone der allgemeinen Formel (VIIIa, b) 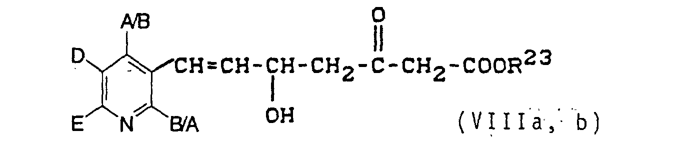 in welcher
A, B, D und E die oben angegebene Bedeutung haben,
und
R²³ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = -CH₂-CH₂-) die Ethenverbindungen (X = -CH=CH-) nach üblichen Methoden hydriert,
und gegebenenfalls Isomeren trennt.

Das erfindungsgemäße Verfahren kann durch das folgendes Formelschema erläutert werden:

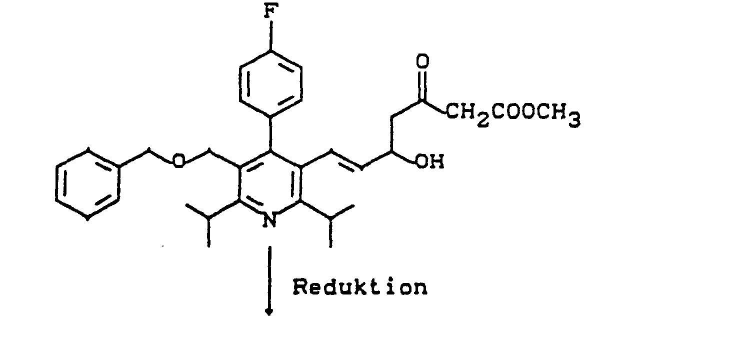

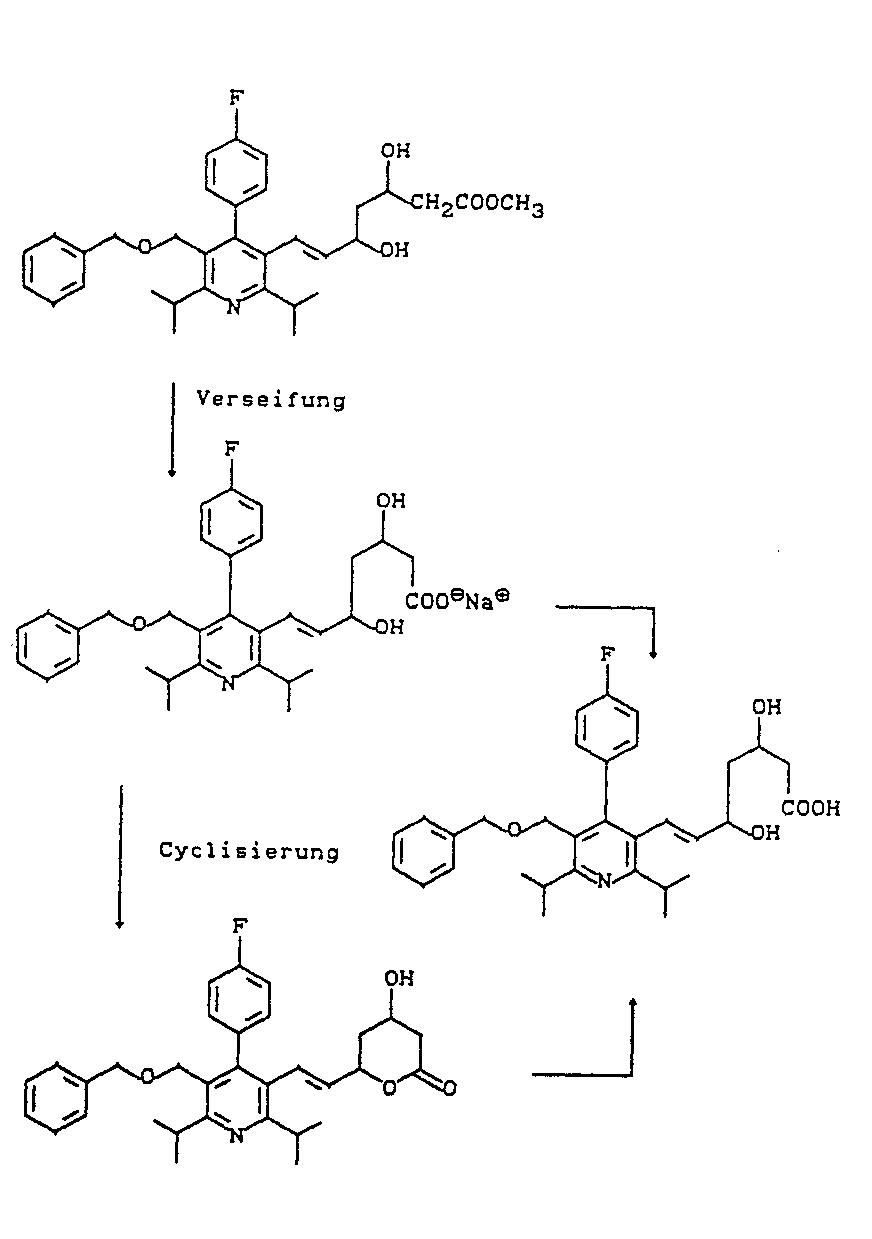

Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels-Die Reduktion erfolgt im allgemeinen in einem Temperaturbereich von -80°C bis + 30°C. Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic) 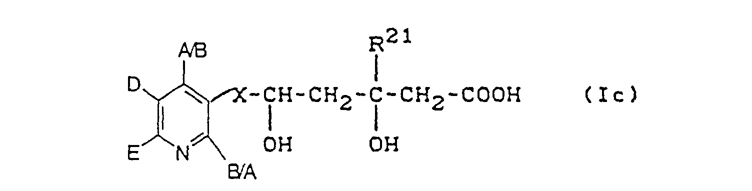 in welcher A, B, D, E und R²⁰ die oben angegebene Bedeutung haben.

Die Carbonsäureester im Rahmen der allgemeinen Formel (I) entsprechen der Formel (Id) 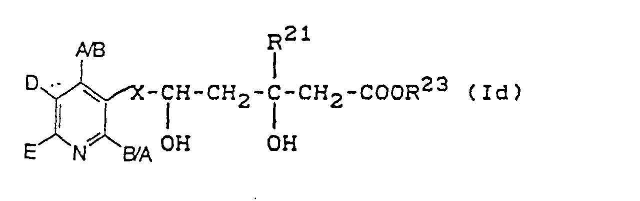 in welcher
A, B, D, E, und R²¹ die oben angegebene Bedeutung haben,
und
R²³ - für Alkyl steht.

Die Salze der erfindungsgemäßen Verbindungen im Rahmen der allgemeinen Formel (I) entsprechen der Formel (Ie) 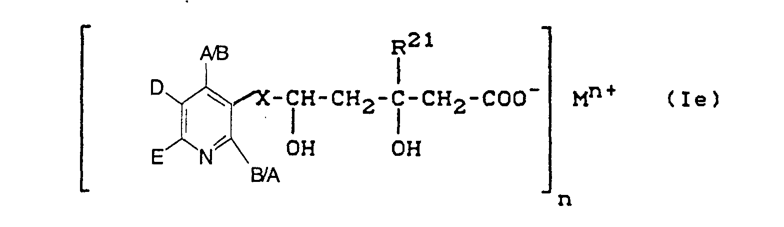 in welcher
A, B, D, E und R²¹ die oben angegebene Bedeutung haben,
und
Mⁿ⁺ für ein Kation steht, wobei n die Wertigkeit angibt.

Die Lactone im Rahmen der allgemeinen Formel (I) entsprechen der Formel (If) 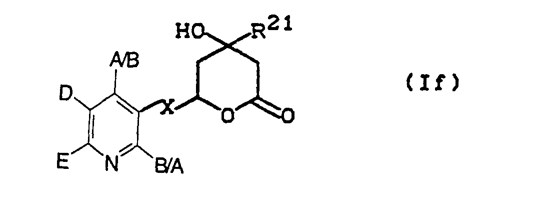 in welcher
A, B, D, E und R²¹ die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäueren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cylisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N'-Dicyclohexylcarbodiimid-Paratoluolsulfonat, N-Cyclohexyl-N'-[2-(N''-methylmorpholinium)ethyl]carbodiimid oder N-(3-Dimethylaminopropyl)-N'-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben werden. Bevorzugt wird hierbei die Trennung der Isomeren auf der Stufe der racemischen Ester. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig)

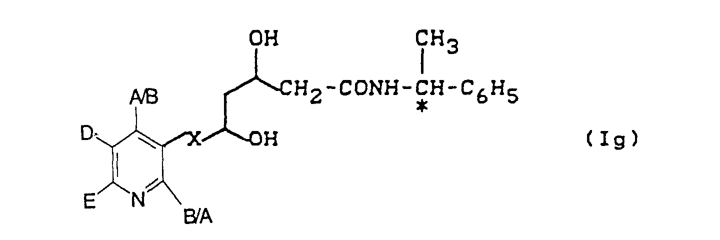

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

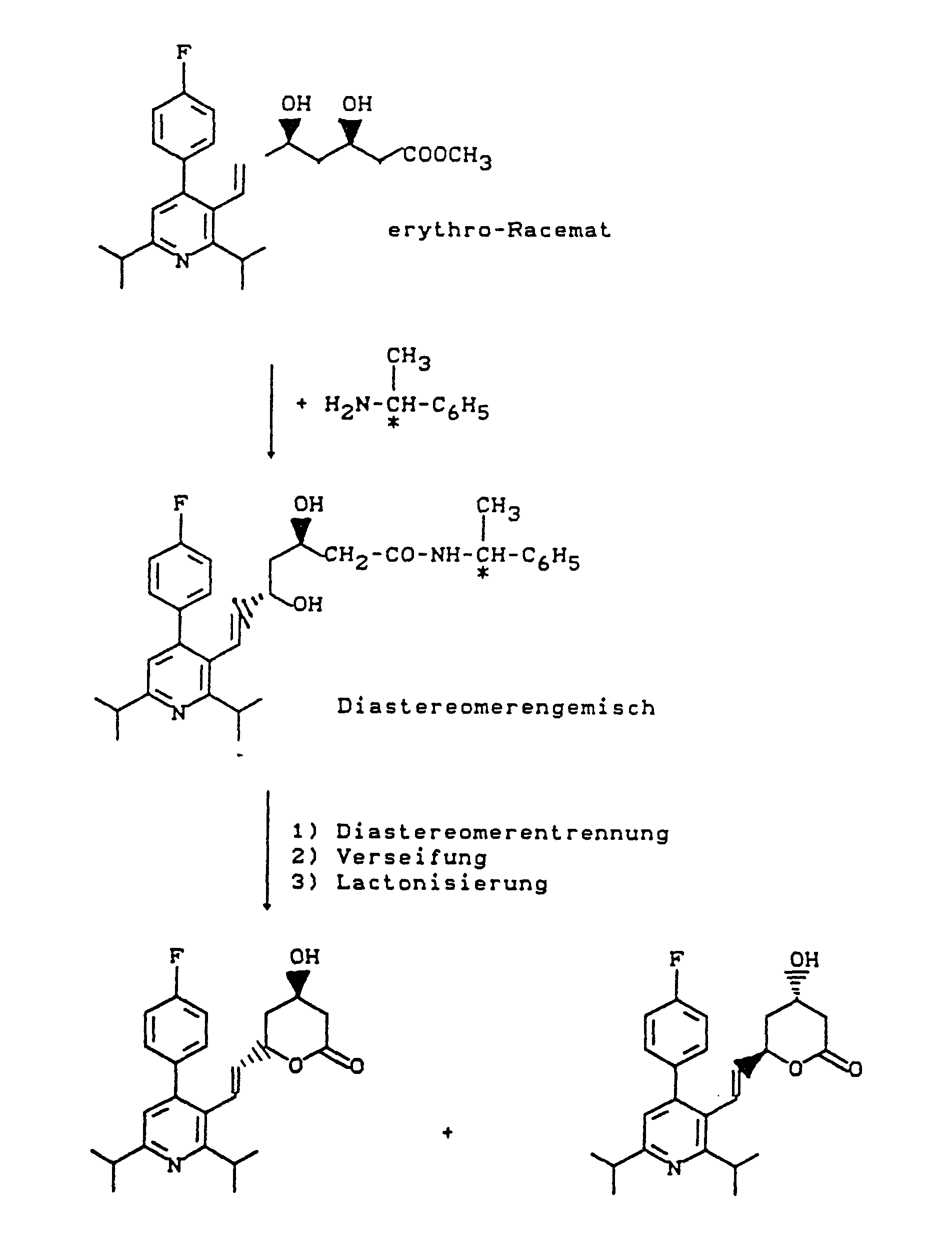

Die als Ausgangsstoffe eingesetzten Ketone (VIII) sind neu.

Es wurde ein Verfahren zur Herstellung der erfindungsgemäßen Ketone der allgemeinen Formel (VIIIa, b) 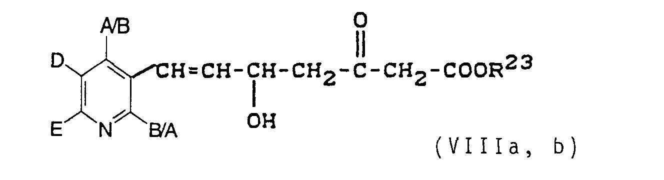 in welcher
A, B, D, E und R²³ die oben angegebene Bedeutung haben,
gefunden, das dadurch gekennzeichnet ist, daß man
Aldehyde der allgemeinen Formel (IXa, b) 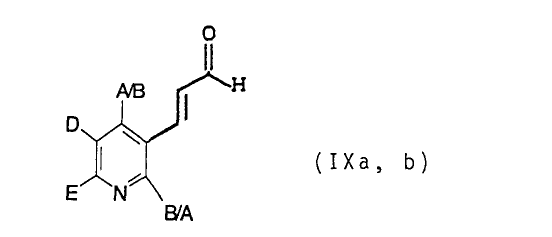 in welcher
A, B, D und E die oben angegebene Bedeutung haben,
in inerten Lösemitteln mit Acetessigester der allgemeinen Formel (X) 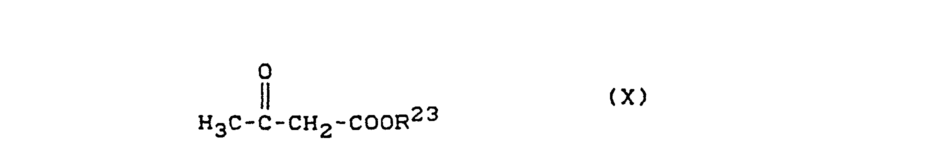 in welcher
R²³ die oben angegebene Bedeutung hat,
in Anwesenheit von Basen umsetzt.

Das erfindungsgemäße Verfahren kann beispielsweise durch folgendes Formelschema erläutert werden:

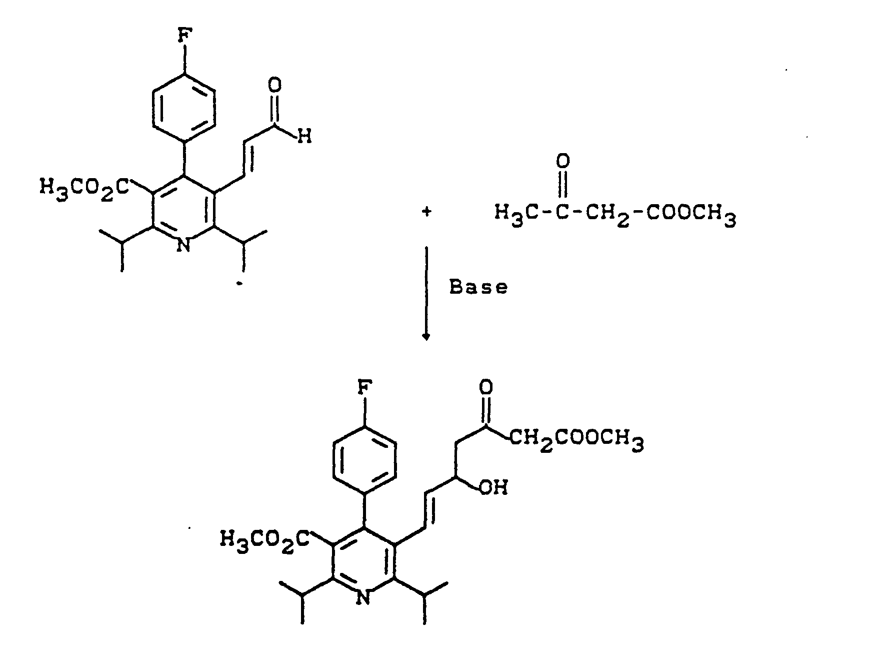

Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt: Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für die Verbindungen des Typs (Ia) erläutert werden.

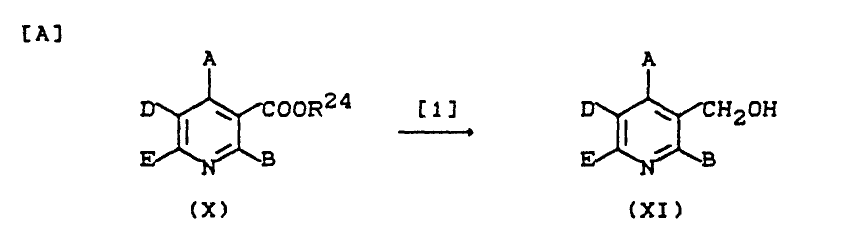

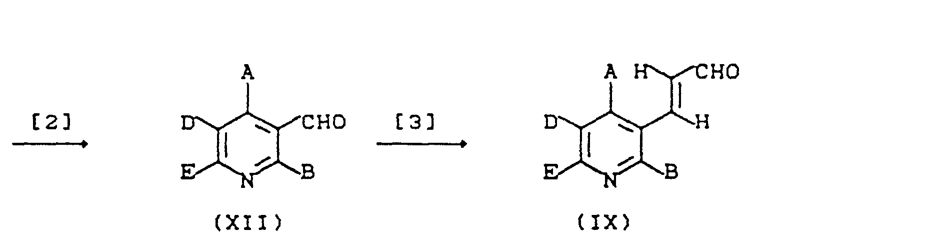

Hierbei werden gemäß Schema A Pyridine der Formel (X),in welchen R²⁴ für einen Alkylrest mit bis zu 4 Kohlenstoffatomen steht, im ersten Schritt [1] in inerten Lösemitteln wie Ethern, beispielsweise Diethylether, Tetrahydrofuran oder Dioxan, vorzugsweise Tetrahydrofuran, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat, in Temperaturbereichen von -70°C bis +100°C, vorzugsweise von -70°C bis Raumtemperatur, bzw. von Raumtemperatur bis 70°C je nach verwendetem Reduktionsmittel zu den Hydroxymethylverbindungen (XI) reduziert. Vorzugsweise wird die Reduktion mit Lithiumaluminiumhydrid in Tetrahydrofuran in einem Temperaturbereich von Raumtemperatur bis 80°C durchgeführt. Die Hydroxymethylverbindungen (XI) werden im zweiten Schritt [2] nach üblichen Methoden zu den Aldehyden (VII) oxidiert. Die Oxidation kann beispielsweise mit Pryidiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Trifluoressigsäure/Dimethylsulfoxid nach den üblichen Methoden der Swern Oxidation durchgeführt werden. Die Aldehyde (XII) werden im dritten Schritt [3] mit Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IX) umgesetzt.

Die hierbei als Ausgangsstoffe eingesetzten Pyridine der Formel (X) erhält man hierbei im allgemeinen gemäß Schema B durch Oxidation von Dihydropyridinen (XIII), die wiederum je nach der Bedeutung des Restes D durch Variation der entsprechenden funktionellen Gruppen erhalten wurden. Die hierbei als Ausgangsstoffe eingesetzten Dihydropyridine sind bekannt oder können nach bekannten Methoden hergestellt werden [EP-A 88 276, DE-A 28 47 236]. Die Oxidation der Dihydropyridine (XIII) zu den Pyridinen (X) kann beispielsweise mit Chromoxid in Eisessig in einem Temperaturbereich von -20°C bis +150°C, vorzugsweise bei Rückflußtemperatur, oder mit 2,3-Dichlor-5,6-dicyan-p-benzochinon als Oxidationsmittel in inerten Lösemitteln wie Chlorkohlenwasserstoffe, vorzugsweise Methylenchlorid in einem Temperaturbereich von 0°C bis +100°C, vorzugsweise bei Raumtemperatur durchgeführt werden.

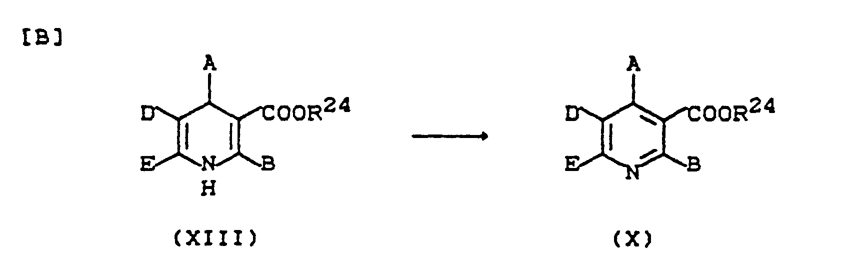

Die Variation des Restes D soll in den folgenden Reaktionsgleichungen an einigen Beispielen erläutert werden:

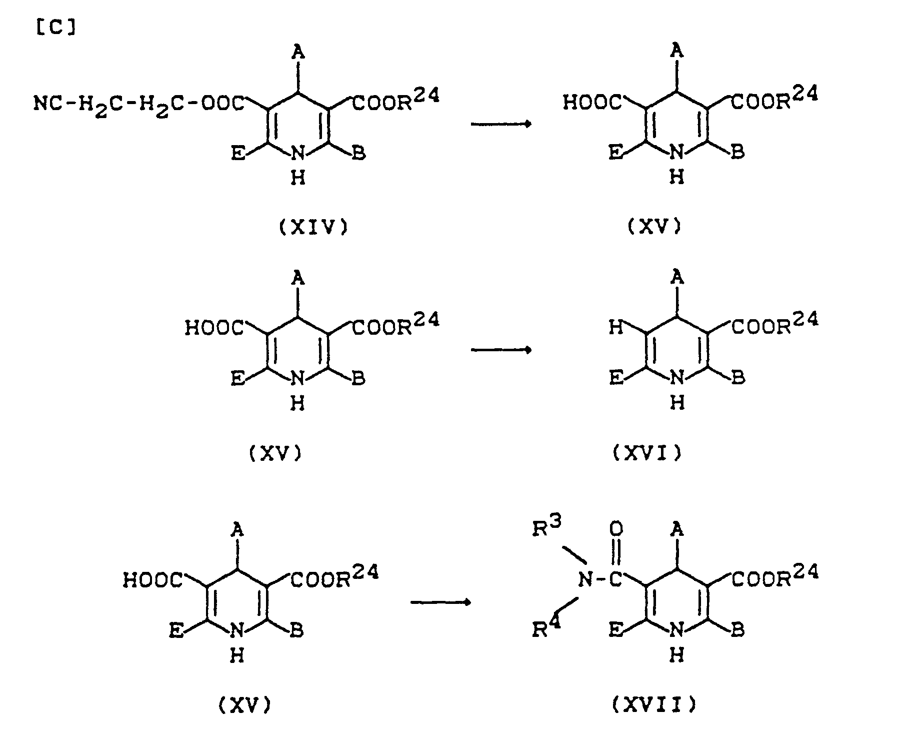

Die Dihydropyridine (XIV) können zu den Dihydropyridincarbonsäuren (XV) verseift werden, beispielsweise durch Umsetzung mit einem Alkalihydroxyd in Dimethoxyethan bei Raumtemperatur. Die Dihydropyridincarbonsäuren (XV) können beispielsweise durch Erhitzen auf 200°C in Diethylenglykol zu den Dihydropyridinen (XVI) decarboxyliert werden. Außerdem können die Dihydropyridincarbonsäuren (XV) nach bekannten Methoden zu den Dihydropyridincarbonsäureamiden (XVII) umgesetzt werden, beispielsweise durch Reaktion mit Dicyclohexylcarbodiimid.

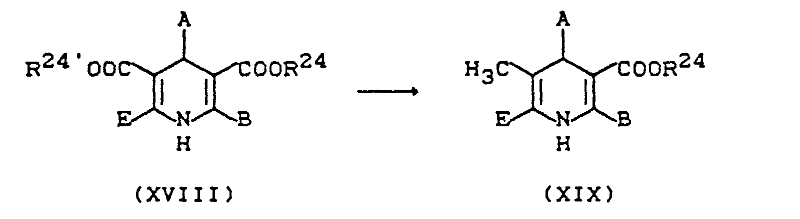

Die Dihydropyridine (XVIII) können mit üblichen Reduktionsmitteln zu den Dihydropyridinen (XIX) reduziert werden, beispielsweise durch Umsetzung Lithiumaluminiumhydrid in Tetrahydrofuran, bei Raumtemperatur oder in der Siedehitze.

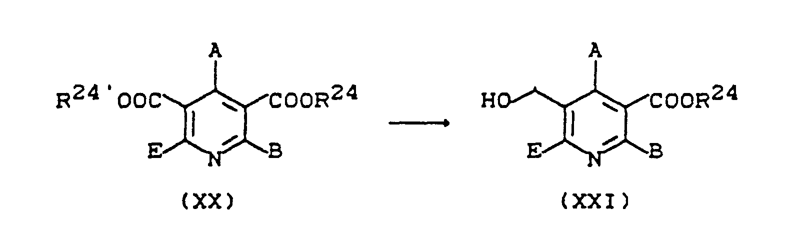

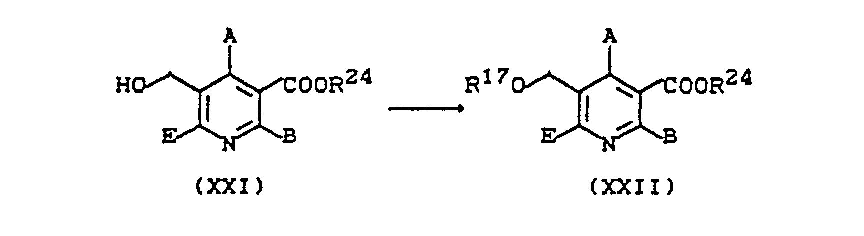

Die Pyridine (XX), die wie oben beschrieben, aus den Dihydropyridinen (XVIII) durch Oxidation hergestellt werden, können durch geeignete Reduktionsmittel, wie beispielsweise Lithiumaluminiumhydrid, Diisobutylaluminiumhydrid oder Natrium-bis-(2-methoxyethoxy)-dihydroaluminat in inerten Lösemitteln, wie beispielsweise Tetrahydrofuran, zu den Pyridinen (XXI) reduziert werden.

Die Pyridine (XXI) können nach bekannten Methoden zu den Pyridinen (XXII) umgesetzt werden, beispielsweise durch Reaktion mit einem Alkyl- oder Benzylhalogenat in Gegenwart einer Base wie beispielsweise Natriumhydrid oder beispielsweise durch Umsetzung mit einem Trialkylsilylhalogenid oder einem Säurehalogenid in Gegenwart einer Base wie Imidazol, Pyridin oder Triethylamin. Die Hydroxygruppe der Pyridine (XXI) kann nach bekannten Methoden in eine Abgangsgruppe überführt werden, z.B. durch Umsetzung mit Trifluormethansulfonsäureanhydrid, Thionylchlorid oder Methansulfonsäurechlorid in Gegenwart einer Base. Die Abgangsgruppe kann dann nach bekannten Methoden gegen Nucleophile ausgetauscht werden.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutarylCoenzym A (HGM-CoA) Reduktase und infolge dessen Inhibitoren der Cholesterolbiosynthese. Sie können deshalb zur Behandlung von Hyperlipoproteinämie, Lipoproteinämie oder Arteriosklerose eingesetzt werden. Die erfindungsgemäßen Wirkstoffe bewirken außerdem eine Senkung des Cholesteringehaltes im Blut.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Herstellungsbeispiele

### Beispiel 1

### (E/Z)-4-Carboxyethyl-5-(4-fluorphenyl)-2-methyl-pent-4-en-3-on

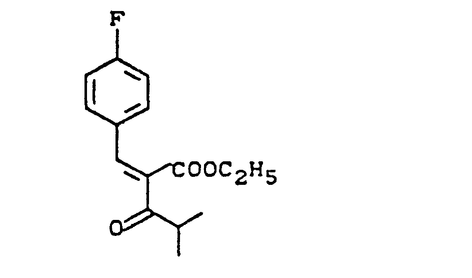

62 g (0,5 Mol) 4-Fluorbenzaldehyd und 79 g (0,5 mol) Isobutyrylessigsäureethylester werden in 300 ml Isopropanol vorgelegt und mit einem Gemisch aus 2,81 ml (28 mmol) Piperidin und 1,66 ml (29 mmol) Essigsäure in 40 ml Isopropanol versetzt. Man läßt 48 Stunden bei Raumtemperatur rühren, engt im Vakuum ein und destilliert den Rückstand im Hochvakuum.
Kp 0,5 mm: 127°C
Ausbeute: 108,7 g (82,3% der Theorie)

### Beispiel 2

### 1,4-Dihydro-2,6-diisopropyl-4-(4-fluorphenyl)-pyridin-3,5-dicarbonsäure-diethylester

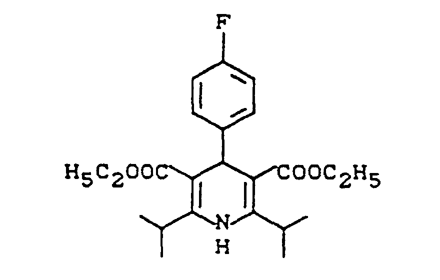

98 g (0,371 mol) der Verbindung aus Beispiel 1 werden mit 58,3 g (0,371 mol) 3-Amino-4-methyl-pent-2-en-säureethylester in 300 ml Ethanol 18 h am Rückfluß gekocht. Die Mischung wird auf Raumtemperatur abgekühlt, das Lösungsmittel im Vakuum abgedampft und die nicht umgesetzten Ausgangsmaterialien im Hochvakuum bei 130°C abdestilliert. Den zurückbleibenden Sirup verrührt man mit n-Hexan und saugt den ausgefallenen Niederschlag ab, wäscht mit n-Hexan nach und trocknet im Exsikkator. Ausbeute: 35 g (23,4% der Theorie)
¹H-NMR (CDCl₃): δ = 1,1 - 1,3 (m, 18H); 4,05 - 4,25 (m, 6H); 5,0 (s, 1H); 6,13 (s, 1H); 6,88 (m, 2H); 7,2 (m, 2H) ppm.

### Beispiel 3

### 2,6-Diisopropyl-4-(4-fluorphenyl)-pyridin-3,5-dicarbonsäure-diethylester

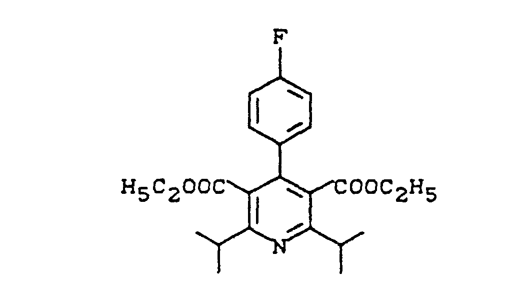

Zu einer Lösung von 6,6 g (16,4 mmol) der Verbindung aus Beispiel 2 in 200 ml Methylenchlorid p.A. gibt man 3,8 g (16,4 mmol) 2,3-Dichlor-5,6-dicyan-p-benzochinon und rührt 1 h bei Raumtemperatur. Dann wird über Kieselgur abgesaugt, die Methylenchloridphase dreimal mit je 100 ml Wasser extrahiert und über Magnesiumsulfat getrocknet. Nach Einengen im Vakuum wird der Rückstand an einer Säule (100 g Kieselgel 70-230 mesh, ø 3,5 cm, mit Essigester/Petrolether 1:9) chromatographiert.
Ausbeute: 5,8 g (87,9% der Theorie)
¹H-NMR (CDCl₃): δ = 0,98 (t, 6H); 1,41 (d, 12H); 3,1 (m, 2H); 4,11 (q, 4H); 7,04 (m, 2H); 7,25 (m, 2H) ppm.

### Beispiel 4

### 2,6-Diisopropyl-4-(4-fluorphenyl)-5-hydroxymethyl-pyridin-3-carbonsäure-ethylester

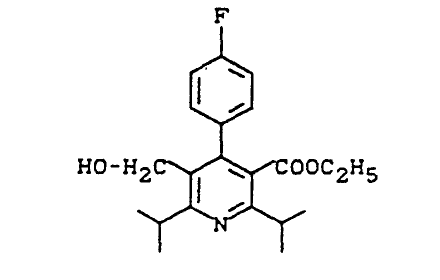

Unter Stickstoff gibt man zu einer Lösung von 9,2 g (23 mmol) der Verbindung aus Beispiel 3 in 100 ml trockenem Tetrahydrofuran bei -10°C bis -5°C 21 ml (80,5 mmol) einer 3,5 molaren Lösung von Natrium-bis-(2-methoxyethoxy)-dihydroaluminat in Toluol und rührt 5 h bei Raumtemperatur. Nach Abkühlen auf 0°C tropft man vorsichtig 100 ml Wasser zu und extrahiert dreimal mit je 100 ml Essigester. Die vereinigten organischen Phasen werden mit gesättigter Natriumchlorid-Lösung gewaschen, über Magnesiumsulfat getrocknet und im Vakuum eingedampft. Der Rückstand wird an einer Säule (200 g Kieselgel 70-230 mesh, ø 4,5 cm, mit Essigester/Petrolether 3:7) chromatographiert.
Ausbeute: 7,2 g (87,2% der Theorie)
¹H-NMR (CDCl₃): δ = 0,95 (t, 3H); 1,31 (m, 12H); 3,05 (m, 1H); 3,48 (m, 1H), 3,95 (q, 2H); 4,93 (d, 2H); 7,05 - 7,31 (m, 4H) ppm.

### Beispiel 5

### 5-(tert.Butyldimethylsilyloxymethyl)-2,6-diisopropyl-4-(4-fluorphenyl)-pyridin-3-carbonsäure-ethylester

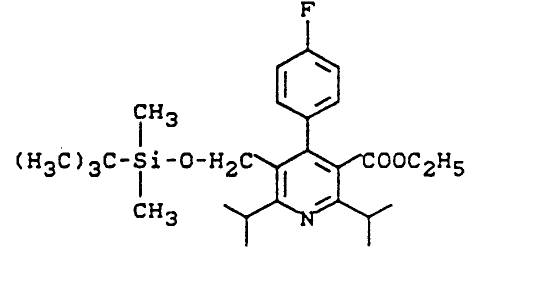

Zu einer Lösung von 4,5 g (12,5 mmol) der Verbindung aus Beispiel 4 in 50 ml Dimethylformamid gibt man bei Raumtemperatur 2,1 g (13,8 mmol) tert.Butyldimethylsilylchlorid, 1,8 g (27,5 mmol) Imidazol und 0,05 g 4-Dimethylaminopyridin. Es wird über Nacht bei Raumtemperatur gerührt, mit 200 ml Wasser versetzt und mit 1 N Salzsäure auf pH 3 eingestellt. Die Mischung wird dreimal mit je 100 ml Ether extrahiert, die vereinigten organischen Phasen einmal mit gesättigter Natriumchlorid-Lösung gewaschen, über Magnesiumsulfat getrocknet und im Vakuum eingeengt. Der Rückstand wird an einer Säule (150 g Kieselgel, 70-230 mesh, ø 4 cm, mit Essigester/Petrolether 1:9) chromatographiert.
Ausbeute: 4,2 g (73,7% der Theorie)
¹H-NMR (CDCl₃): δ = 0,0 (s, 6H); 0,9 (s, 9H); 1,02 (t, 3H); 1,35 (m, 12H); 3,1 (m, 1H); 3,47 (m, 1H); 4,03 (q, 2H); 4,4 (s, 2H); 7,05 - 7,40 (m, 4H) ppm.

### Beispiel 6

### 3-(tert.Butyldimethylsilyloxymethyl)-2,6-diisopropyl-4-(4-fluorphenyl)-5-hydroxymethyl-pyridin

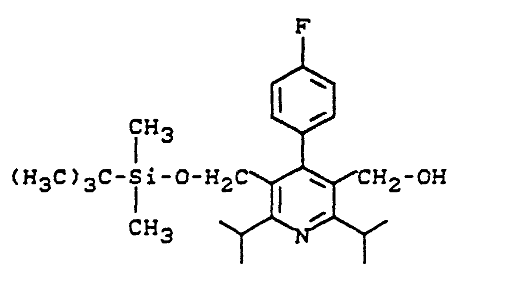

Unter Stickstoff gibt man zu einer Lösung von 4,2 g (9,2 mmol) der Verbindung aus Beispiel 5 in 100 ml trockenem Tetrahydrofuran bei 0°C 9,2 ml (32,2 mmol) einer 3,5 molaren Lösung von Natrium-bis-(2-methoxyethoxy)-dihydroaluminat in Toluol und rührt über Nacht bei Raumtemperatur. Nach Abkühlen auf 0°C tropft man vorsichtig 100 ml Wasser zu und extrahiert dreimal mit je 100 ml Essigester. Die vereinigten organischen Phasen werden einmal mit gesättigter Natriumchlorid-Lösung gewaschen, über Magnesiumsulfat getrocknet und im Vakum eingeengt. Der Rückstand wird an einer Säule (100 g Kieselgel 70-230 mesh, ø 3,5 cm, mit Essigester/Petrolether 2:8) chromatographiert.
Ausbeute: 2,4 g (60% der Theorie)
¹H-NMR (CDCl₃): δ = 0,2 (s, 6H); 1,11 (s, 9H); 1,6 (m, 12H); 3,7 (m, 2H); 4,55 (s, 2H); 4,65 (d, 2H); 7,35 - 7,55 (m, 4H) ppm.

### Beispiel 7

### 5-(tert.Butyldimethylsilyloxymethyl)-2,6-diisopropyl)-4-(4-fluorphenyl)-pyridin-3-carbaldehyd

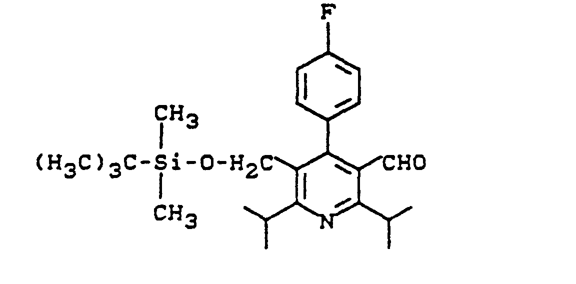

Zu einer Lösung von 2,7 g (6,2 mmol) der Verbindung aus Beispiel 6 in 50 ml Methylenchlorid gibt man 1,24g (12,4 mmol) neutrales Aluminiumoxid und 2,7 g (12,4 mmol) Pyridiniumchlorochromat und rührt 1 h bei Raumtemperatur. Man saugt über Kieselgur ab und wäscht mit 200 ml Methylenchlorid nach. Die Methylenchloridphase wird im Vakuum eingeengt und der Rückstand an einer Säule (100 g Kieselgel 70-230 mesh, ø 3,5 cm, mit Essigester/Petrolether 1:9) chromatographiert.
Ausbeute: 2 g (77% der Theorie)
¹H-NMR (CDCl₃): δ = 0,0 (s, 6H); 0,9 (s, 9H); 1,35 (m, 12H); 3,5 (m, 1H); 3,9 (m, 1H); 4,38 (s, 2H); 7,15 - 7,35 (m, 4H); 9,8 (s, 1H) ppm.

### Beispiel 8

### (E)-3-[5-tert.Butyldimethylsilyloxymethyl-2,6-diisopropyl-4-(4-fluorphenyl)-pyrid-3-yl]-prop-2-enal

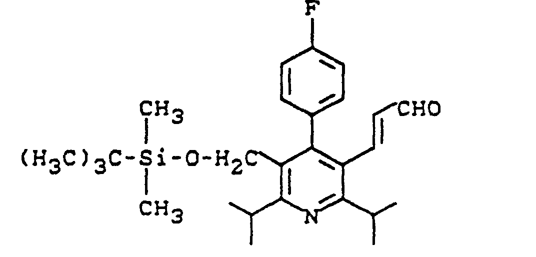

Unter Stickstoff tropft man zu einer Suspension von 180 mg (6 mmol) 80%igem Natriumhydrid in 15 ml trockenem Tetrahydrofuran bei -5°C 1,6 g (6 mmol) Diethyl-2-(cyclohexylamino)-vinylphosphonat gelöst in 30 ml trockenem Tetrahydrofuran. Nach 30 min werden bei derselben Temperatur 2 g (4,7 mmol) der Verbindung aus Beispiel 7 in 40 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert. Die vereinigten organischen Phasen werden mit gesättigter Natriumchlorid-Lösung gewaschen und über Magnesiumsulfat getrocknet. Nach Einengen im Vakuum wird der Rückstand in 70 ml Toluol aufgenommen, mit einer Lösung von 0,9 g (7 mmol) Oxalsäure-Dihydrat in 30 ml Wasser versetzt und 30 min zum Rückfluß erhitzt. Nach Abkühlen auf Raumtemperatur werden die Phasen getrennt, die organische Phase mit gesättigter Natriumchlorid-Lösung gewaschen, über Magnesiumsulfat getrocknet und im Vakuum eingeengt. Der Rückstand wird an einer Säule (100 g Kieselgel 70-230 mesh ø 3,5 cm, mit Essigester/Petrolether 1:9) chromatographiert.
Ausbeute: 2 g (95% der Theorie)
¹H-NMR (CDCl₃): δ = 0,0 (s, 6H); 0,9 (s, 9H); 1,38 (m, 12H); 3,36 (m, 1H); 3,48 (m, 1H); 4,48 (s, 2H); 6,03 (dd, 1H); 7,12-7,35 (m, 5H); 9,45 (d, 1H) ppm.

### Beispiel 9

### Methyl-(E)-7-[5-tert.butyldimethylsilyloxymethyl-2,6-diisopropyl-4-(4-fluorphenyl)-pyrid-3-yl]-5-hydroxy-3-oxo-hept-6-enoat

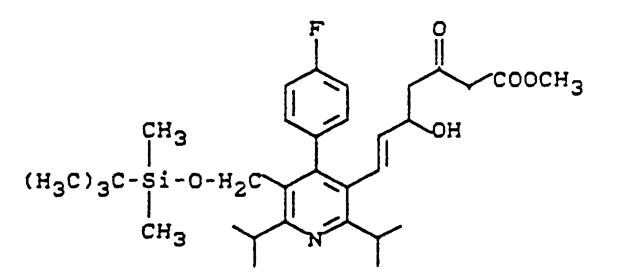

Unter Stickstoff tropft man zu einer Suspension von 330 mg (11 mmol) 80%igem Natriumhydrid in 30 ml trockenem Tetrahydrofuran bei -5°C 1,02 g (8,8 mmol) Acetessigsäuremethylester in 5 ml trockenem Tetrahydrofuran. Nach 15 min werden bei derselben Temperatur 5,5 ml (8,8 mmol) 15%iges Butyllithium in n-Hexan zugetropft und 15 min nachgerührt. Anschließend werden 2 g (4,4 mmol) der Verbindung aus Beispiel 8 gelöst in 20 ml trockenem Tetra-Zu einer Lösung von 1,9 g (3,7 mmol) der Verbindung aus Beispiel 9 in 40 ml trockenem Tetrahydrofuran gibt man bei Raumtemperatur 4,5 ml (4,5 mmol) 1 M Triethylboran-Lösung in Tetrahydrofuran, leitet während 5 min Luft durch die Lösung und kühlt auf -30°C Innentemperatur ab. Es werden 160 mg (4,5 mmol) Natriumborhydrid und langsam 3 ml Methanol zugegeben, 30 min bei -30°C gerührt und dann mit einem Gemisch von 12 ml 30%igem Wasserstoffperoxid und 25 ml Wasser versetzt. Die Temperatur läßt man dabei bis 0°C ansteigen und rührt noch 30 min nach. Die Mischung wird dreimal mit je 70 ml Essigester extrahiert, die vereinigten organischen Phasen je einmal mit gesättigter Natriumhydrogencarbonat-Lösung und gesättigter Natriumchlorid-Lösung gewaschen, über Magnesiumsulfat getrocknet und im Vakuum eingeengt. Der Rückstand wird an einer Säule (80 g Kieselgel 230-400 mesh, ø 2,5 cm, mit Essigester/Petrolether 4:6) chromatographiert. Ausbeute: 1,5 g (78,9% der Theorie)
¹H-NMR (CDCl₃): δ = 0,0 (s, 6H); 0,9 (s, 9H); 1,35 (m, 12H); 1,5 (m, 2H); 2,5 (m, 2H); 3,35 (m, 1H); 3,45 (m, 1H); 3,8 (s, 3H); 4,15 (m, 1H); 4,45 (m, 3H); 5,32 (dd, 1H); 6,38 (d, 1H); 7,05 - 7,25 (m, 4H) ppm.

### Beispiel 11

### Methyl-erythro-(E)-7-[2,6-diisopropyl-4-(4-fluorphenyl)-5-hydroxymethyl-pyrid-3-yl]-3,5-dihydroxy-hept-6-enoat

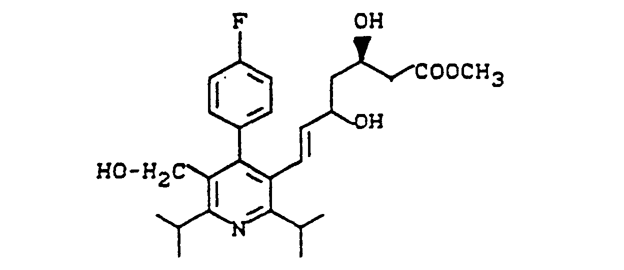

Zu 8,4 g (14,6 mmol) der Verbindung aus Beispiel 10 gelöst in 135 ml Methanol gibt man 15 ml 0,1 N Salzsäure und rührt 4 Tage bei Raumtemperatur. Die Mischung wird im Vakuum eingeengt, der Rückstand in Dichlormethan aufgenommen und mehrmals mit gesättigter Natriumhydrogencarbonat-Lösung gewaschen. Die organische Phase wird über Magnesiumsulfat getrocknet, im Vakuum eingeengt und der Rückstand an einer Säule (Kieselgel 70-230 mesh, mit Essigester/Petrolether 4:6) chromatographiert.
Ausbeute: 3,5 g (52,5% der Theorie)
¹H-NMR (CDCl₃): δ = 1,25 (m, 6H); 1,33 (d, 6H); 1,40 (m, 2H); 2,41 (m, 2H); 3,30 (m, 1H); 3,45 (m, 1H); 3,71 (s, 3H); 4,07 (m, 1H); 4,28 (m, 1H); 4,39 (d, 2H); 5,25 (dd, 1H); 6,30 (d, 1H); 7,08 (m, 4H) ppm.

### Beispiel 12

### 5-Benzyloxymethyl-2,6-diisopropyl-4-(4-fluorphenyl)-pyridin-3-carbonsäure-ethylester

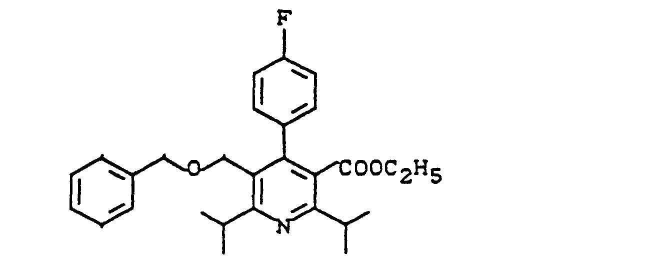

Unter Stickstoff tropft man zu einer Suspension von 414 mg (13,8 mmol) 80%igem Natriumhydrid in 20 ml Dimethylformamid bei 0°C 4,5 g (12,5 mmol) der Verbindung aus Beispiel 4 in 50 ml Dimethylformamid und rührt 30 min. bei derselben Temperatur. Anschließend werden 1,65 ml (13,8 mmol) Benzylbromid in 20 ml Dimethylformamid zugetropft und weitere 3 h bei Raumtemperatur gerührt. Die Mischung wird bei 0°C auf 300 ml Wasser gegossen und dreimal mit je 150 ml Ether extrahiert. Die vereinigten organischen Phasen werden einmal mit gesättigter Natriumchlorid-Lösung gewaschen, über Magnesiumsulfat getrocknet und im Vakuum eingeengt. Der Rückstand wird an einer Säule (100 g Kieselgel 70-230 mesh, ø 3,5 cm, mit Essigester/Petrolether 1:10) chromatographiert. Ausbeute: 2,6 g (46,4% der Theorie)
¹H-NMR (CDCl₃): δ = 0,95 (t, 3H); 1,3 (m, 12H); 3,05 (m, 1H); 3,38 (m, 1H); 3,97 (q, 2H); 4,2 (s, 2H); 4,38 (s, 2H); 7,02 (m, 2H); 7,25 (m, 7H) ppm.

### Beispiel 13

### 3-Benzyloxymethyl-2,6-diisopropyl-4-(4-fluorphenyl)-5-hydroxymethyl-pyridin

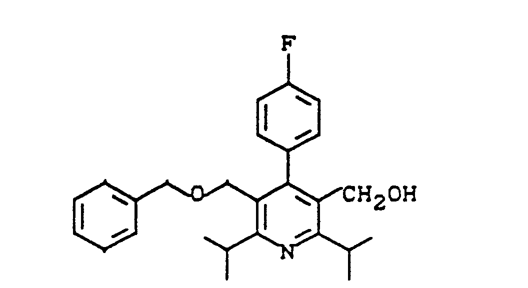

2,5 g (5,5 mmol) der Verbindung aus Beispiel 12 werden analog Beispiel 6 umgesetzt.
Ausbeute: 1,5 g (68% der Theorie)
¹H-NMR (CDCl₃): δ = 1,3 (m, 12H); 3,35 (m, 1H); 3,45 (m, 1H); 4,13 (s, 2H); 4,35 (m, 4H); 7,08 (m, 2H); 7,25 (m, 7H) ppm.

### Beispiel 14

### 5-Benzyloxymethyl-2,6-diisopropyl-4-(4-fluorphenyl)-pyridin-3-carbaldehyd

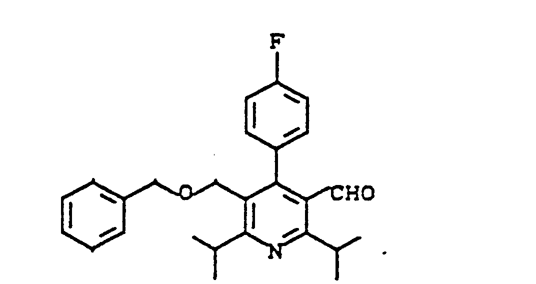

1,5 g (3,6 mmol) der Verbindung aus Beispiel 13 werden analog Beispiel 7 umgesetzt.
Ausbeute: 1,1 g (75,9% der Theorie)
¹H-NMR (CDCl₃): δ = 1,3 (m, 12H); 3,4 (m, 1H); 3,85 (m, 1H); 4,18 (s, 2H); 4,38 (s, 2H); 7,05 - 7,35 (m, 9H); 9,75 (s, 1H) ppm.

### Beispiel 15

### (E)-3-[5-Benzyloxymethyl-2,6-diisopropyl-4-(4-fluorphenyl)-pyrid-3-yl]-prop-2-enal

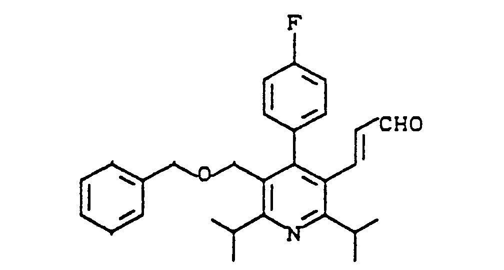

1,1 g (2,7 mmol) der Verbindung aus Beispiel 14 werden analog Beispiel 8 umgesetzt.
Ausbeute: 450 mg (38,8% der Theorie)
¹H-NMR (CDCl₃): δ = 1,35 (m, 12H); 3,35 (m, 1H); 3,42 (m, 1H); 4,21 (s, 2H); 4,41 (s, 2H); 6,0 (dd, 1H); 7,05 - 7,4 (m, 10H); 9,38 (d, 1H) ppm.

### Beispiel 16

### Methyl-(E)-7-[5-benzyloxymethyl-2,6-diisopropyl-4-(4-fluorphenyl)-pyrid-3-yl]-5-hydroxy-3-oxo-hept-6-enoat

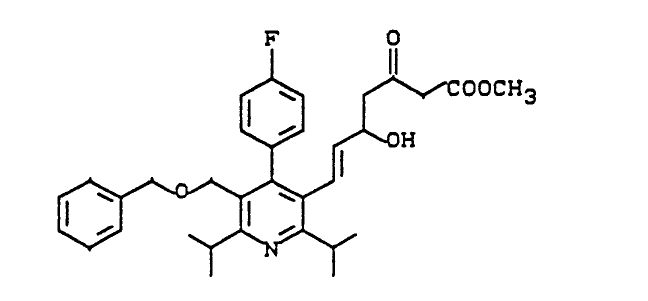

431 mg (1 mmol) der Verbindung aus Beispiel 15 werden analog Beispiel 9 umgesetzt. Ausbeute: 300 mg (54,8% der Theorie)

### Beispiel 17

### Methyl-erythro-(E)-7-[3-benzyloxymethyl-2,6-diisopropyl-4-(4-fluorphenyl)-pyrid-5-yl]-3,5-dihydroxy-hept-6-enoat

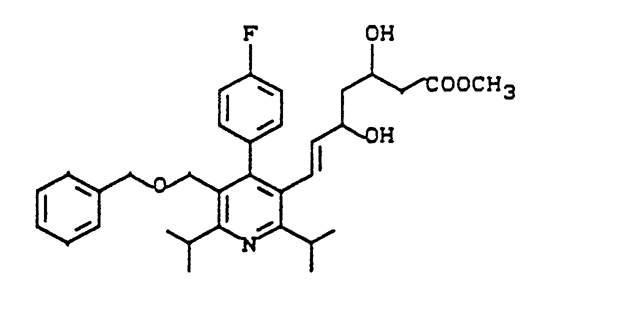

300 mg (0,55 mmol) der Verbindung aus Beispiel 16 werden analog Beispiel 10 umgesetzt.
Ausbeute: 180 mg (59,6% der Theorie)
¹H-NMR (CDCl₃): δ = 1,2 - 1,35 (m, 12H); 1,4 (m, 2H); 2,41 (m, 2H); 3,3 (m, 2H); 3,73 (s, 3H); 4,05 (m, 1H); 4,15 (s, 2H); 4,28 (m, 1H); 4,35 (s, 2H); 5,25 (dd, 1H); 6,3 (d, 1H); 6,95 - 7,35 (m, 9H) ppm.

### Beispiel 18

### 1,4-Dihydro-4-(4-fluorphenyl)-2-isopropyl-6-methylpyridin-3,5-dicarbonsäure-3-ethyl-5-methylester

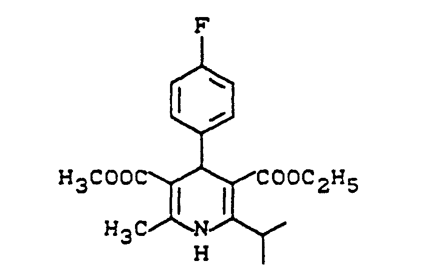

15 g (56,8 mmol) der Verbindung aus Beispiel 1 und 6,5 g (56,8 mol) 3-Aminocrotonsäuremethylester werden in 150 ml Ethanol 20 h am Rückfluß gekocht. Die Mischung wird abgekühlt, abfiltriert und im Vakuum eingeengt. Der Rückstand wird an einer Säule (250 g Kieselgel 70-230 mesh, ø 4,5 cm, mit Essigester/Petrolether 3:7) chromatographiert.
Ausbeute: 13,6 g (66,3% der Theorie)
¹H-NMR (CDCl₃): δ = 1,2 (m, 9H); 2,35 (s, 3H); 3,65 (s, 3H); 4,12 (m, 3H); 4,98 (s, 1H); 5,75 (s, 1H); 6,88 (m, 2H); 7,25 (m, 2H) ppm.

### Beispiel 19

### 4-(4-Fluorphenyl)-2-isopropyl-6-methyl-pyridin-3,5-dicarbonsäure-3-ethyl-5-methylester

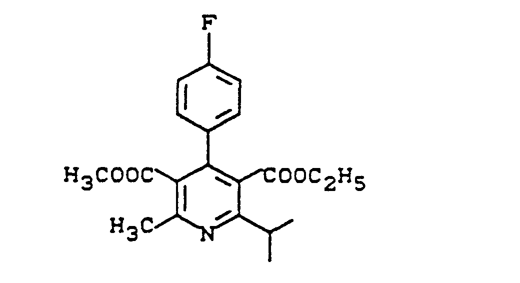

13,5 g (37,4 mmol) der Verbindung aus Beispiel 18 werden analog Beispiel 3 umgesetzt.
Ausbeute: 9,5 g (70,9% der Theorie)
¹H-NMR (CDCl₃): δ = 0,98 (t, 3H); 1,31 (d, 6H); 2,6 (s, 3H); 3,11 (m, 1H); 3,56 (s, 3H); 4,03 (q, 2H); 7,07 (m, 2H); 7,25 (m, 2H) ppm.

### Beispiel 20

### 4-(4-Fluorphenyl)-5-hydroxymethyl-2-isopropyl-6-methylpyridin-3-carbonsäure-ethylester

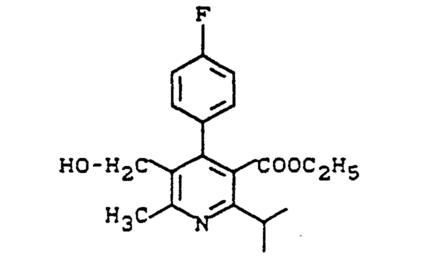

Unter Stickstoff gibt man zu einer Lösung von 9,5 g (26,5 mmol) der Verbindung aus Beispiel 19 in 200 ml absolutem Tetrahydrofuran bei 0°C 26,5 ml (92,75 mmol) einer 3,5 molaren Lösung von Natrium-bis-(2-methoxyethoxy)-dihydroaluminat in Toluol und rührt 30 min bei Raumtemperatur. Nach erneutem Abkühlen auf 0°C, tropft man vorsichtig 200 ml Wasser zu und extrahiert dreimal mit je 150 ml Essigester. Die vereinigten organischen Phasen werden einmal mit gesättigter Natriumchlorid-Lösung gewaschen, über Magnesiumsulfat getrocknet und im Vakuum eingeengt. Der Rückstand wird an einer Säule (200 g Kieselgel 70-230 mesh, ø 4,5 cm, mit Essigester/ Petrolether 2:8) chromatographiert.
Ausbeute: 4,2 g (48,2% der Theorie)
¹H-NMR (CDCl₃): δ = 0,98 (t, 3H); 1,3 (d, 6H); 2,73 (s, 3H); 3,05 (m, 1H); 3,98 (q, 2H); 4,45 (d, 2H); 7,1 (m, 2H); 7,25 (m, 2H) ppm.

### Beispiel 21

### (E/Z)-4-Carboxyethyl-5-(4-fluor-3-phenoxyphenyl)-2-methyl-pent-4-en-3-on

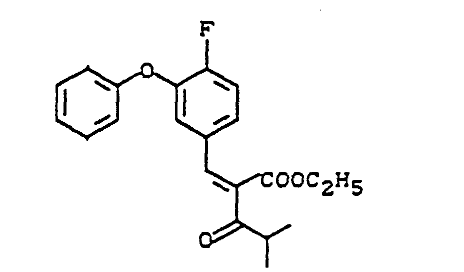

49 g (0,31 mol) Isobutyrylessigsäureethylester und 67 g (0,31 mol) 3-Phenoxy-4-fluorbenzaldehyd werden in 300 ml Isopropanol vorgelegt und mit einem Gemisch aus 1,81 ml (18 mmol) Piperidin und 1,06 ml (18,6 mmol) Essigsäure in 30 ml Isopropanol versetzt. Man rührt über Nacht bei Raumtemperatur, engt dann im Vakuum ein und trocknet im Hochvakuum. Ausbeute: 110 g (wurde ohne weitere Reinigung in Beispiel 22 eingesetzt).

### Beispiel 22

### 1,4-Dihydro-2,6-diisopropyl-4-(4-fluor-3-phenoxy-phenyl)-pyridin-3,5-dicarbonsäure-diethylester

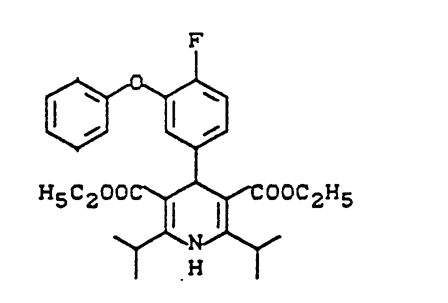

30 g (84,3 mmol) der Verbindung aus Beispiel 21 und 13,2 g (84,3 mmol) 3-Amino-4-methyl-pent-2-en-säure-ethylester werden in 150 ml Ethanol über Nacht am Rückfluß gekocht. Man kühlt die Mischung auf 0°C, saugt den ausgefallenen Niederschlag ab, wäscht mit Petrolether nach und trocknet im Exsikkator.
Ausbeute: 18,4 g (44,2% der Theorie)
¹H-NMR (CDCl₃): δ = 1,05 - 1,25 (m, 18H); 4,05 - 4,2 (m, 6H); 4,95 (s, 1h); 6,03 (s, 1H); 6,85 - 7,1 (m, 6H); 7,3 (m, 2H) ppm.

### Beispiel 23

### 2,6-Diisopropyl-4-(4-fluor-3-phenoxy-phenyl)-pyridin-3,5-dicarbonsäure-diethylester

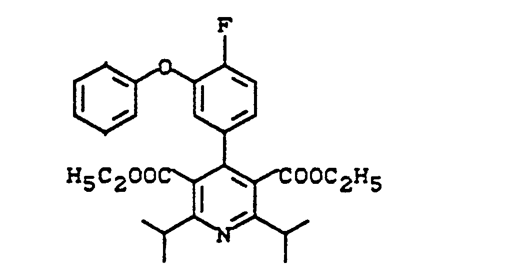

18,4 g (37,2 mmol) der Verbindung aus Beispiel 22 werden analog Beispiel 3 umgesetzt.
Ausbeute: 17,6 g (96% der Theorie)
¹H-NMR (CDCl₃): δ = 1,05 (t, 6H); 1,29 (d, 12H); 3,08 (m, 2H); 4,05 (q, 4H); 6,95 - 7,35 (m, 8H) ppm.

### Beispiel 24

### 2,6-Diisopropyl-4-(4-fluor-3-phenoxy-phenyl)-5-hydroxymethyl-pyridin-3-carbonsäure-ethylester

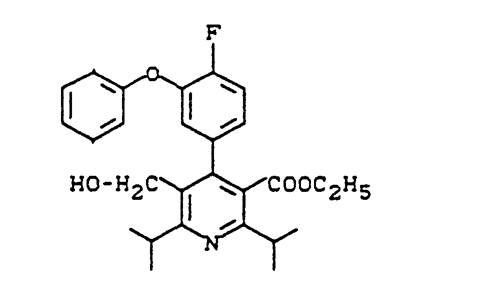

10 g (20,3 mmol) der Verbindung aus Beispiel 23 werden analog Beispiel 4 umgesetzt.
Ausbeute: 4,9 g (59,0% der Theorie)
¹H-NMR (CDCl₃): δ = 1,07 (t, 3H); 1,3 (m, 12H); 3,04 (m, 1H); 3,47 (m, 1H); 4,05 (m, 2H); 4,45 (s, 2H); 6,95 - 7,4 (m, 8H) ppm.

### Beispiel 25

### 1,4-Dihydro-2,6-dimethyl-4-(4-fluorphenyl)-pyridin-3,5-dicarbonsäuremethyl-(2-cyanethyl)-ester

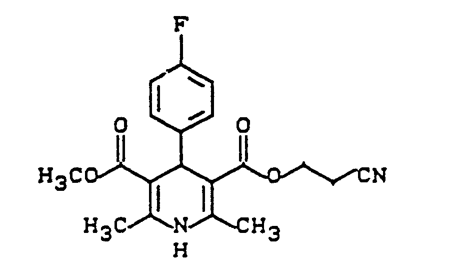

In 150 ml Ethanol werden 15,4 g (0,1 mol) 3-Aminocrotonsäure-2-cyanethylester, 12,4 g (0,1 mol) p-Fluorbenzaldehyd und 11,6 g Acetessigsäuremethylester über Nacht unter Rückfluß erhitzt. Nach dem Entfernen des Lösemittels am Rotationsverdampfer nimmt man in Essigester auf, wäscht mit Wasser, trocknet und erhält nach Entfernen des Lösemittels im Vakuum 33,8 g Rohprodukt. Rohausbeute: 94,4% der Theorie
¹H-NMR (DMSO): δ = 1,15 (tr, 3H, CH₃); 2,3 (m, 6H, CH₃); 2,75 (m, 2H, CH₂CN); 3,55 (s, 3H, OCH₃); 4,15 (m, 2H, OCH₂); 4,9 (m, 1H, p-FC₆H₄-CH); 6,9 - 7,3 (m, 4H, Aromaten -H); 8,8, 9,0 (2s, 1H, NH) ppm.

### Beispiel 26

### 1,4-Dihydro-2,6-dimethyl-4-(4-fluorphenyl)-pyridin-3,5-dicarbonsäure-methylester

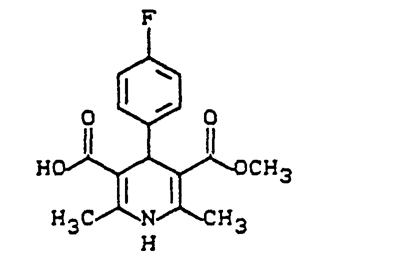

Zu einer Lösung von 12 g (0,3 mol) Natriumhydroxid in 300 ml Wasser/150 ml 1,2-Dimethoxyethan gibt man 33,8 g Rohprodukt aus Beispiel 25. Die Suspension erwärmt sich, es bildet sich eine klare Lösung. Nach Rühren bei 25°C über Nacht gibt man 100 ml Wasser hinzu, wäscht dreimal mit Dichlormethan, stellt mit verdünnter Salzsäure auf pH 1 ein und extrahiert das klebrig ausgefallene Produkt mit Dichlormethan. Nach dem Trocknen und Einengen des Lösemittels im Vakuum erhält man 25,8 g Rohprodukt. Rohausbeute: 84,5% der Theorie
¹H-NMR (DMSO): δ = 2,25 (s, 6H, CH₃); 3,55 (s, 3H, OCH₃); 4,85 (breites s, 1H, FC₆H₄-CH); 6,9 - 7,3 (m, 4H, Aromaten-H); 8,85 (breites s, 1H, NH); 1,7 (breit, 1H, COOH) ppm.

### Beispiel 27

### 1,4-Dihydro-2,6-dimethyl-4-(4-fluorphenyl)-pyridin-3-carbonsäuremethylester

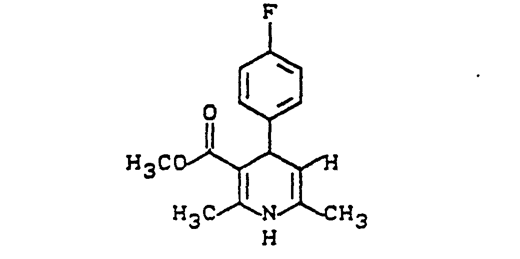

In 90 ml Bis-(2-hydroxyethyl)-ether (Diglycol) werden 12,5 g (41 mmol) Rohprodukt aus Beispiel 26 suspendiert und auf 200°C Badtemperatur erhitzt, wobei eine starke Gasentwicklung stattfindet. Nach beendeter Gasentwicklung wird die nun klare Lösung schnell abgekühlt, mit 500 ml Wasser/500 ml Ether gewaschen, die wäßrige Phase noch zweimal mit Ether gewaschen, die vereinigten Etherphasen mit Wasser, 1 N Natronlauge und Wasser gewaschen und getrocknet. Nach dem Entfernen des Lösemittels am Rotationsverdampfer erhält man 8,7 g Rohprodukt. Rohausbeute: 81,2% der Theorie

### Beispiel 28

### 2,6-Dimethyl-4-(4-fluorphenyl)-pyridin-3-carbonsäuremethylester

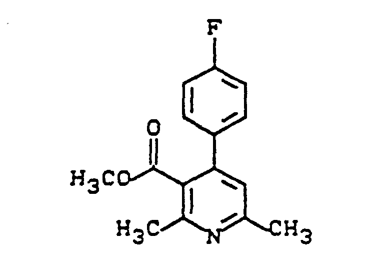

In 90 ml Eisessig werden 8,6 g (33 mmol) Rohprodukt aus Beispiel 27 und 3,3 g Chrom-VI-oxid 1 h unter Rückfluß erhitzt. Das Lösemittel wird am Rotationsverdampfer entfernt, der Rückstand mit Essigester/Petrolether 1:1 versetzt und vom Ungelöstem abgesaugt. Die Mutterlauge wird im Vakuum eingeengt und über 500 g Kieselgel mit Essigester/Petrolether 1:1 chromatographiert.
Ausbeute: 1,45 g (16,3% der Theorie)
¹H-NMR (CDCl₃): δ = 2,6 (s, 6H, CH₃); 3,65 (s, 3H, OCH₃); 7,0 (s, 1H, Pyridin-H); 7,1-7,4 (m, 4H, Aromaten-H) ppm.

### Beispiel 29

### 2,6-Dimethyl-4-(4-fluorphenyl)-3-hydroxymethyl-pyridin

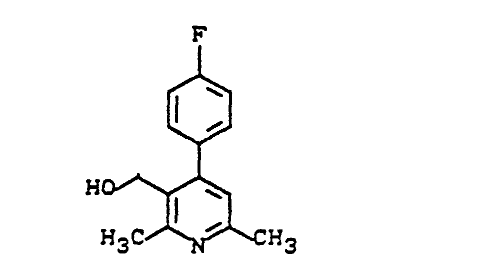

Unter Stickstoff werden bei -78°C 1,35 g (5,2 mmol) der Verbindung aus Beispiel 28 in 25 ml absolutem Tetrahydrofuran mit 5,3 ml (5,3 mmol) Diisobutylaluminiumhydrid (1 m in Toluol) versetzt und nach Aufwärmen auf 25°C mit 20%iger Kaliumhydroxydlösung hydrolysiert. Die wäßrige Phase wird mit Essigester gewaschen und die vereinigten organischen Phasen getrocknet. Nach dem Einengen im Vakuum erhält man 1,12 g Rohprodukt.
Ausbeute: 93% der Theorie
¹H-NMR (CD₃OD): δ = 2,5 (s, 3H, CH₃); 2,7 (s, 3H, CH₃); 4,5 (s, 2H, CH₂OH); 4,6 (s, OH); 7,0 (s, 1H, Pyridin-H); 7,1 - 7,6 (m, 4H, Aromaten-H) ppm.

### Beispiel 30

### 2,6-Dimethyl-4-(4-fluorphenyl)-pyridin-3-carbaldehyd

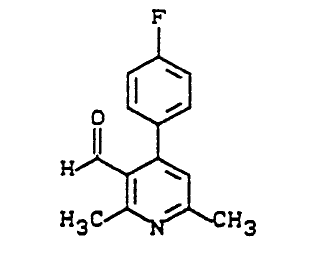

Zu 1,0 g (4,3 mmol) der Verbindung aus Beispiel 29 in 20 ml Dichlormethan gibt man portionsweise 1,5 g (7 mmol) Pyridiniumdichromat, rührt 2 h bei 25°C, chromatographiert nach Einengen über 150 g Kieselgel mit Dichlormethan/Methanol 10:1 und erhält 0,71 g Produkt.
Ausbeute: 72% der Theorie
¹H-NMR (DMSO): δ = 2,5 (s, 3H, CH₃); 2,7 (s, 3H, CH₃); 7,2 (s, 1H, Pyridin-H); 7,3 - 7,6 (m, 4H, Aromaten-H); 9,95 (s, 1H, CHO) ppm.

### Beispiel 31

### (E)-3-[2,6-Dimethyl-4-(4-fluorphenyl)-pyrid-3-yl]-prop-2-enal

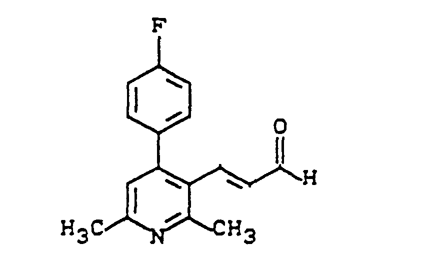

Zu 75,5 mg (3,2 mmol) Natriumhydrid in 3 ml absolutem Tetrahydrofuran gibt man unter Stickstoffatmosphäre innerhalb 15 min bei 0°C 778 mg (3,2 mmol) [2-(Cyclohexylamino)vinyl]-phosphorsäurediethylester in 3 ml Tetrahydrofuran, rührt 15 min bei 0°C und tropft eine Lösung von 0,6 g (2,6 mmol) der Verbindung aus Beispiel 30 in 3 ml Acetonitril/3 ml Dimethylformamid zu. Nach 1 h 15 min tropft man bei 0°C 0,55 ml (0,77 mmol) n-Butyllithium (1,5 m in Hexan) hinzu, rührt 15 min bei 0°C und tropft 180 mg (0,7 mmol) der Verbindung aus Beispiel 31 in 3 ml Tetrahydrofuran hinzu. Nach 1 h hydrolysiert man mit gesättigter Ammoniumchloridlösung, wäscht dreimal mit Dichlormethan, trocknet die organische Phase und erhält nach Entfernen des Lösemittels im Vakuum 0,25 g Öl. Rohausbeute: 95,5% der Theorie
¹H-NMR (CDCl₃): δ = 2,55, 2,58 (2s, 6H, CH₃); 2,6 (2H, CH₂); 3,45 (s, 2H, CH₂CO₂); 3,75 (s, 3H, OCH₃); 4,6 (m, 1H, CHOH); 5,45 (dd, 1H, CH-CHOH); 6,5 (d, 1H, CH=CH-CHOH); 6,9 (s, 1H, Pyridin-H); 7,0 - 7,4 (m, 4H, Aromaten-H) ppm.

### Beispiel 33 (Vergleichsbeispiel)

### Methyl-erythro-(E)-7-[2,6-dimethyl-4-(4-fluorphenyl)-pyridin-3-yl]-3,5-dihydroxy-hept-6-enoat

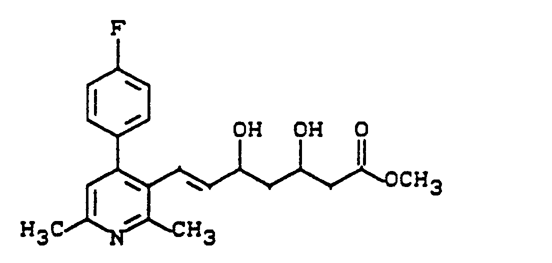

Durch eine Lösung von 0,25 g (0,67 mmmol) der Verbindung aus Beispiel 32 und 0,81 ml (0,81 mmol) Triethylboran Analog Beispiel 25 erhält man aus 2,75 g (0,1 mol) Isopropyliden-4-fluor-benzoylessigsäureethylester und 15,4 g (0,1 mol) 3-Aminocrotonsäure-2-cyanethylester 32,6 g Rohprodukt.
Rohausbeute: 93,6% der Theorie
¹H-NMR (CDCl₃): δ = 0,7 - 1,3 (m, 9H, CH₃); 2,3, 2,35 (2s, 3H, CH₃); 2,75 (m, 2H, CH₂CN); 3,9 - 4,4 (m, 5H, CHCH₃, CH₂O); 5,6 5,7, 6,1, 6,2 (4s, 1H, CH); 7,0-8,0 (m, 4H, Aromaten-H) ppm.

### Beispiel 35

### 1,4-Dihydro-2-(4-fluorphenyl)-4-isopropyl-6-methylpyridin-3,5-dicarbonsäure-3-ethylester

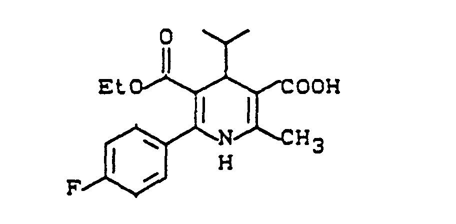

Analog Beispiel 26 erhält man aus 36 g (93,2 mmol) der Verbindung aus Beispiel 34 7,17 g Rohprodukt. Rohausbeute: 22,2% der Theorie
¹H-NMR (DMSO): = = 0,8 (m, 9H, CH₃); 1,6 (m, 1H, CHCH₃); 2,2, 2,25 (2s, 3H, CH₃); 3,8 (m, 3H, CH₂O, CH); 7,2 - 7,5 (m, 4H, Aromaten-H) ppm.

### Beispiel 36

### 1,4-Dihydro-2-(4-fluorphenyl)-4-isopropyl-6-methyl-pyridin-3-carbonsäureethylester

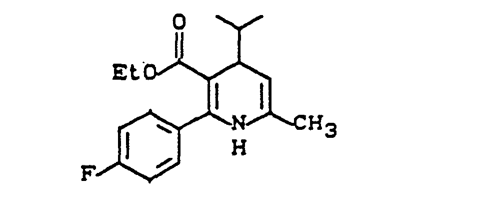

Analog Beispiel 27 erhält man aus 11,3 g (32,6 mmol) Beispiel 35 7,15 g Rohprodukt.
Rohausbeute: 7,25% der Theorie
¹H-NMR (CDCl₃): δ = 0,8 - 1,3 (m, 9H, CH₃); 2,5, 2,6 (2s, 3H, CH₃); 3,1 (m, 1H, CHCH₃); 3,8 - 4,2 (m, 2H, CH₂O); 4,55, 5,2 (br, 1H, CH); 6,8 (s, 1H, CH); 6,9-8,0 (m, 4H, Aromaten-H) ppm.

### Beispiel 37

### 2-(4-Fluorphenyl)-4-isopropyl-6-methyl-pyridin-3-carbonsäureethylester

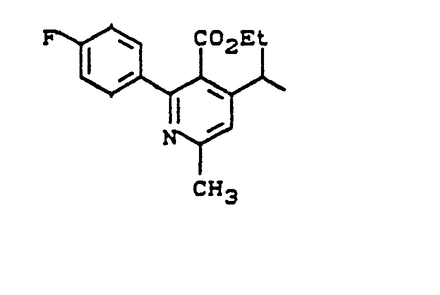

Analog Beispiel 28 erhält man aus 6,95 g (22,9 mmol) der Verbindung aus Beispiel 36 nach Chromatographie (Kieselgel, Toluol/Ethanol 95:5) 2,82 g.
Ausbeute: 41% der Theorie
¹H-NMR (CDCl₃): δ = 1,0 (tr, 3H, CH₃); 1,3 (d, 6H, CH₃CH); 2,6 (s, 3H, CH₃); 3,1 (sept, 1H, CH); 4,1 (q, 2H, CH₂O); 7,0 (s, 1H, Pyridin-H); 7,1 - 7,6 (m, 4H, Aromaten-H) ppm.

### Beispiel 38

### 2-(4-Fluorphenyl)-3-hydroxymethyl-4-isopropyl-6-methylpyridin

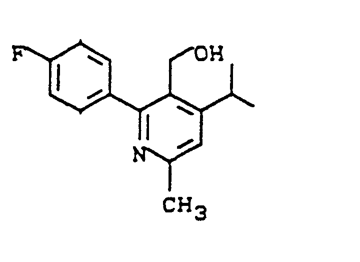

5,5 g (18,3 mmol) der Verbindung aus Beispiel 37 werden analog Beispiel 29 umgesetzt. Nach Trocknen im Exsikkator erhält man 4,24 g Rohprodukt.
Ausbeute: 89% der Theorie
¹H-NMR (CDCl₃): δ = 1,3 (d, 6H); 2,55 (s, 3H); 3,36 (m, 1H); 4,49 (s, 2H); 7,09 (m, 3H); 7,53 (m, 2H) ppm.

### Beispiel 39

### 2-(4-Fluorphenyl)-4-isopropyl-6-methyl-pyridin-3-carbaldehyd

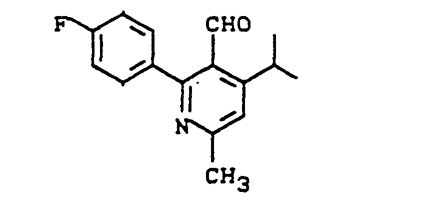

4,1 g (15,85 mmol) der Verbindung aus Beispiel 38 werden analog Beispiel 30 umgesetzt.
Ausbeute: 2,23 g (54,7% der Theorie)
¹H-NMR(CDCl₃): δ = 1,3 (d, 6H); 2,65 (s, 3H); 3,91 (m, 1H); 7,10 - 7,28 (m, 3H); 7,5 (m, 2H); 9,91 (s, 1H) ppm.

### Beispiel 40

### (E)-3-[2-(4-Fluorphenyl)-4-isopropyl-6-methyl-pyrid-3-yl]-prop-2-enal

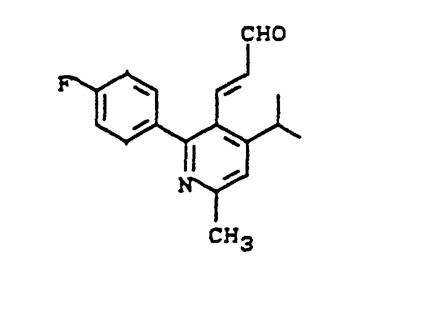

2,13 g (8,3 mmol) der Verbindung aus Beispiel 39 werden analog Beispiel 8 umgesetzt.
Ausbeute: 1,34 g Rohprodukt (57% der Theorie)
¹H-NMR (CDCl₃): δ = 12,8 (d, 6H); 2,6 (s, 3H); 3,27 (m, 1H); 6,11 (dd, 1H); 7,05 - 7,55 (m, 6H); 9,55 (d, 1H) ppm.

### Beispiel 41

### Methyl-(E)-7-[2-(4-fluorphenyl)-4-isopropyl-6-methylpyrid-3-yl]-5-hydroxy-3-oxo-hept-6-enoat

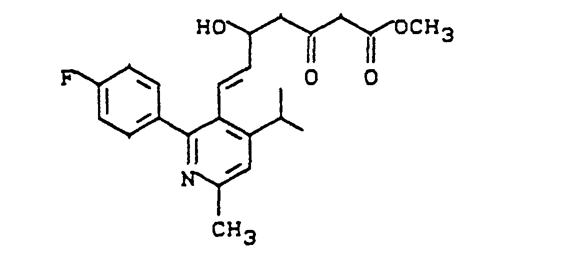

1,07 g (3,78 mmol) der Verbindung aus Beispiel 40 werden analog Beispiel 32 umgesetzt.
Ausbeute: 0,34 g Rohprodukt (22,5% der Theorie)
¹H-NMR (CDCl₃): δ = 1,25 (d, 6H); 2,5 (m, 2H); 2,57 (s, 3H); 3,2 (m, 1H); 3,42 (s, 2H); 3,75 (s, 3H); 4,45 (m, 1H); 5,3 (dd, 1H); 6,6 (d, 1H); 7,05 (m, 3H); 7,43 (m, 2H) ppm.

### Beispiel 42 (Vergleichsbeispiel)

### Methyl-erythro-(E)-7-[2-(4-fluorphenyl)-2-isopropyl-6-methyl-pyrid-3-yl]-3,5-dihydroxy-hept-6-enoat

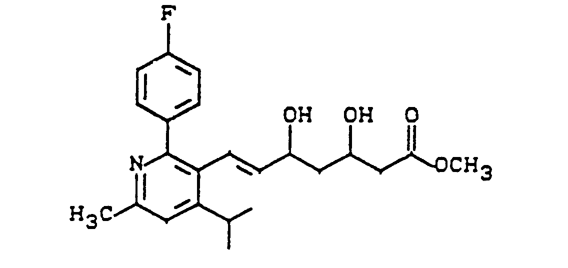

200 mg (0,5 mmol) der Verbindung aus Beispiel 41 werden analog Beispiel 33 umgesetzt.
Ausbeute: 21,5 mg (10,7% der Theorie)
¹H-NMR (CDCl₃): δ = 1,23 (d, 6H); 1,5 (m, 2H); 2,45 (m, 2H); 2,58 (s, 3H); 3,21 (m, 1H); 3,72 (s, 3H); 4,11 (m, 1H); 4,38 (m, 1H); 5,31 (dd, 1H); 6,55 (d, 1H); 7,05 (m, 3H); 7,4 (m, 2H) ppm.

### Beispiel 43

### 1,4-Dihydro-4-(4-fluorphenyl)-2-isopropyl-6-methylpyridin-3,5-dicarbonsäure-5-(2-cyanethyl)-3-ethylester

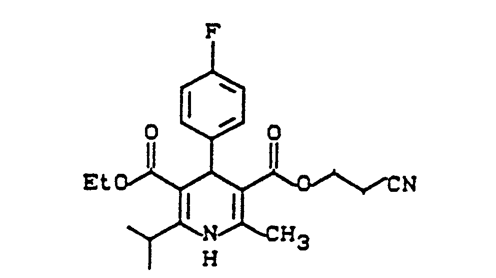

Analog Beispiel 25 erhält man aus 26,4 g (0,1 mol) 4-Fluorbenzyliden-2-butanoylessigsäureethylester und 15,4 g (0,1 mol) 3-Aminocrotonsäure-2-cyanethylester 44,6 g Rohprodukt.
Rohausbeute: 100% der Theorie
¹H-NMR (DMSO): δ = 1,15 (m, 9H, CH₃-CH₂, CH₃-CH-CH₃); 2,3 (s, 3H, CH₃); 2,45 (m, 2H, CH₂-CN); 4,0 (q, 2H, CH₂O); 4,1 (m, 1H, CHCH₃); 4,15 (m, 2H, CH₂O); 4,4 (s, 1H, p-FC₆H₄CH); 6,9 - 7,3 (m, 4H, Aromaten-H); 8,3 (s, 1H, NH) ppm.

### Beispiel 44

### 1,4-Dihydro-4-(4-fluorphenyl)-2-isopropyl-6-methylpyridin-3,5-dicarbonsäure-3-ethylester

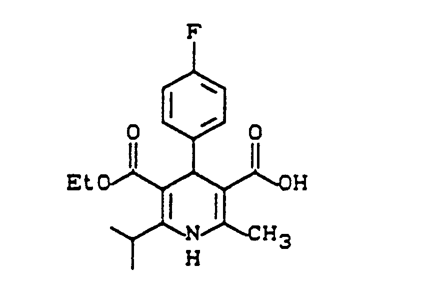

Analog Beispiel 26 erhält man aus 10,2 g (25,6 mmol) der Verbindung des Beispiels 43 8,5 g Rohprodukt. Rohausbeute: 95% der Theorie
¹H-NMR (DMSO): δ = 1,15 (m, 9H, CH₃CH₂, CH₃CHCH₃); 2,25, 2,3 (2s, 3H, CH₃); 4,0 (m, 3H, CH₂O, CH₃CH); 4,85, 6,3 (2s, 1H, FC₆H₄-CH); 6,9 - 7,3 (m, 4H, Aromaten-H); 8,1 (s, 1H, NH); 10,9 (s, 1H, COOH) ppm.

### Beispiel 45

### 1,4-Dihydro-4-(4-fluorphenyl)-2-isopropyl-6-methylpyridin-3-carbonsäureethylester

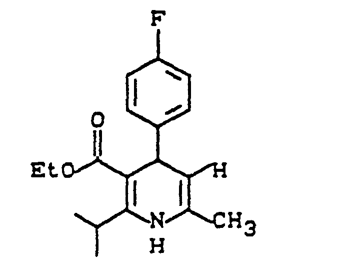

Analog Beispiel 27 erhält man aus 8,35 g (24 mmol) der Verbindung aus Beispiel 44 5,6 g Rohprodukt. Rohausbeute: 77,5% der Theorie
¹H-NMR (CDCl₃): δ = 1,2 (m, 9H, CH₃CH₂, CH₃CHCH₃); 2,6 (s, 3H, CH₃); 4,1 (m, CHCH₃, CH₂O); 4,5, 4,6 (2d, 1H, FC₆H₄CH); 5,3 (s, 1H, NH); 6,9 - 7,4 (m, >5H, Aromaten-H) ppm.

### Beispiel 46

### 4-(4-Fluorphenyl)-2-isopropyl-6-methyl-pyridin-3-carbonsäureethylester

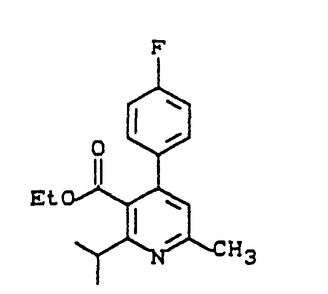

Analog Beispiel 37 erhält man aus 5,5 g (18,2 mmol) der Verbindung des Beispiels 45 nach Chromatographie über Kieselgel (Dichlormethan) 2,9 g rotes Öl.
Ausbeute: 53% der Theorie
¹H-NMR (CDCl₃): δ = 1,05 (tr, 3H, CH₃CH₂); 1,35 (d, 6H, CH₃CH); 2,6 (s, 3H, CH₃); 3,15 (sept., 1H, CH); 4,1 (q, 2H, CH₂); 6,95 (s, 1H, Pyridin-H); 7,1 - 7,4 (m, 4H, Aromaten-H) ppm.

### Beispiel 47

### 4-(4-Fluorphenyl)-3-hydroxymethyl-2-isopropyl-6-methylpyridin

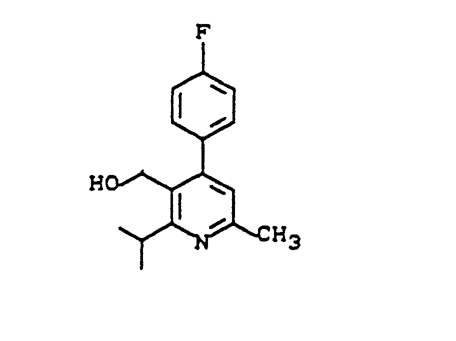

Analog Beispiel 29 erhält man aus 2,8 g (9,3 mmol) der Verbindung des Beispiels 46 2,19 g Produkt.
Ausbeute: 91% der Theorie
¹H-NMR (CDCl₃): δ = 1,3 (d, 6H, CH₃CH); 1,5 (br, 1H, OH); 2,5 (s, 3H, CH₃); 3,5 (sept., 1H, CH); 4,6 (s, 2H, CH₂); 6,9 (s, 1H, Pyridin-H); 7,1 - 7,5 (m, 4H, Aromaten-H) ppm.

### Beispiel 48

### 4-(4-Fluorphenyl)-2-isopropyl-6-methyl-pyridin-3-carbaldehyd

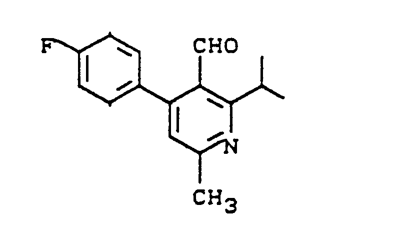

Analog Beispiel 30 erhält man aus 2,0 g (7,7 mmol) der Verbindung des Beispiels 47 0,56 g Produkt.
Ausbeute: 28,3% der Theorie
¹H-NMR (CDCl₃); δ = 1,3 (d, 6H, CH₃CH); 2,6 (s, 3H, CH₃); 3,6 (sept, 1H, CH); /,0 (s, 1H, Pyridin-H); 7,1 - 7,4 (m, 4H, Aromaten-H); 10,0 (s, 1H, CHO) ppm.

### Beispiel 49

### (E)-3-[4-(4-Fluorphenyl)-2-isopropyl-6-methyl-pyrid-3-yl]-prop-2-enal

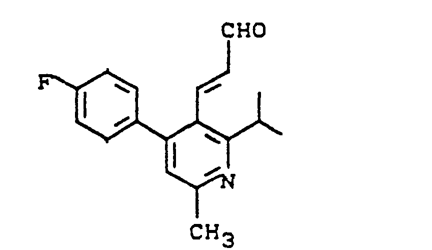

Analog Beispiel 31 erhält man aus 0,51 g (1,99 mmol) der Verbindung des Beispiels 48 0,48 g.
Ausbeute: 85,5% der Theorie
¹H-NMR (CDCl₃): δ = 1,2 (d, 6H, CH₃CH); 2,5 (s, 3H, CH₃); 3,3 (sept, 1H, CH); 6,0 (dd, 1H, CHCHO); 6,9 (s, 1H, Pyridin-H); 7,0 - 7,3 (m, 4H, Aromaten-H); 7,5 (d, 1H,CH); 9,5 (d, 1H, CHO) ppm.

### Beispiel 50

### Methyl-(E)-7-[4-(4-fluorphenyl)-2-isopropyl-6-methylpyrid-3-yl]-5-hydroxy-3-oxo-hept-6-enoat

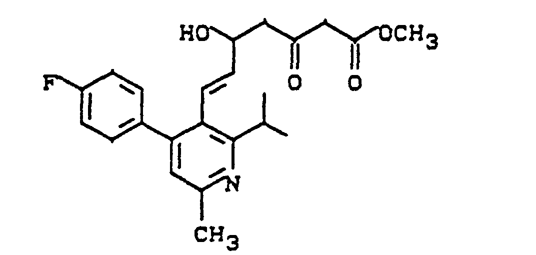

Analog Beispiel 32 erhält man aus 0,41 g (1,44 mmol) der Verbindung des Beispiels 49 0,22 g.
Ausbeute: 38,2% der Theorie
¹H-NMR (CDCl₃): δ = 1,3 (d, 6H, CH₃CH); 2,5 (s, 3H, CH₃); 3,3 (sept, 1H, CH); 3,5 (s, 2H, CH₂); 3,25 (s, 3H, OCH₃); 4,6 (m, 1H; CHOH); 5,3 (dd, 1H, CH); 6,6 (d, 1H, CH); 6,9 (s, 1H, Pyridin-H); 7,0 - 7,3 (m, 4H, Aromaten-H) ppm.

### Beispiel 51 (Vergleichsbeispiel)

### Methyl-erythro-(E)-7-[4-(4-fluorphenyl)-2-isopropyl-6-methyl-pyrid-3-yl]-3,5-dihydroxy-hept-6-enoat

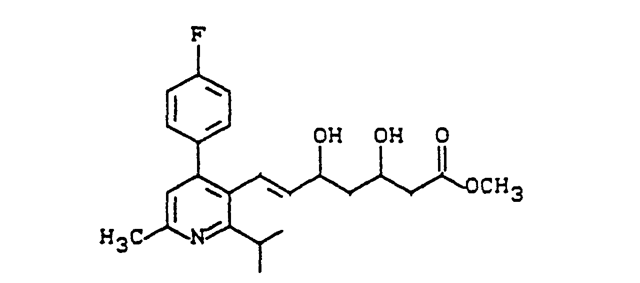

1,2 g (3,01 mmol) der Verbindung aus Beispiel 50 werden analog Beispiel 33 umgesetzt.
Ausbeute: 320 mg (26,6% der Theorie)
¹H-NMR (CDCl₃): δ = 1,28 (d, 6H); 1,40 (m, 2H); 2,45 (m, 2H); 2,55 (s, 3H); 3,35 (m, 1H); 3,72 (s, 3H); 4,15 (m, 1H); 4,39 (m, 1H); 5,30 (dd, 1H); 6,55 (d, 1H); 6,88 (s, 1H); 7,0 - 7,30 (m, 4H) ppm.

### Beispiel 52

### 1,4-Dihydro-2,6-dimethyl-4-(4-fluorphenyl)-5-phenylpyridin-3-carbonsäuremethylester

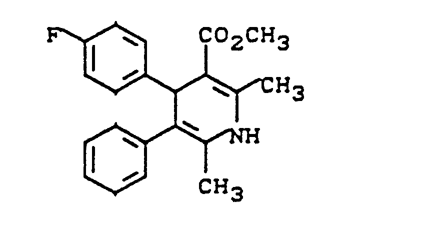

In 150 ml Ethanol werden 24,0 g (0,1 mol) 1-(4-Fluorphenyl)-2-phenyl-buten-3-on, 23 g (0,2 mol) 3-Amino-crotonsäuremethylester und 6 ml (0,1 mol) Eisessig über Nacht unter Rückfluß erhitzt, nach Zugabe von 11,5 g (0,1 mol) 3-Aminocrotonsäuremethylester und 3 ml Eisessig 18 h unter Rückfluß erhitzt und nach wiederholter Zugabe von 11,5 g (0,1 mol) 3-Aminocrotonsäuremethylester und 3 ml Eisessig nochmals 18 h unter Rückfluß erhitzt. Das Lösemittel wird im Vakuum entfernt, der Rückstand mit 80 ml Methanol versetzt, Ungelöstes abgesaugt, die methanolische Lösung in Vakuum eingeengt, aus dem Rückstand bei 73°C - 90°C/18 mbar und anschließend bei 60°C bis 70°C/0,2 mbar überschüssigen Aminocrotonester abdestilliert. Man erhält 37,5 g Rohprodukt als glasharte Schmelze.
Rohausbeute: >100% der Theorie
¹H-NMR (CDCl₃): δ = 1,85 (s, 3H, CH₃); 2,85 (s, 3H, CH₃); 3,6 (s, 3H, CH₃); 4,65, 5,4 (2br, s, 1H, CH); 6,7 - 7,4 (m, 9H, Aromaten-H) ppm.

### Beispiel 53

### 2,6-Dimethyl-4-(4-fluorphenyl)-5-phenyl-pyridin-3-carbonsäuremethylester

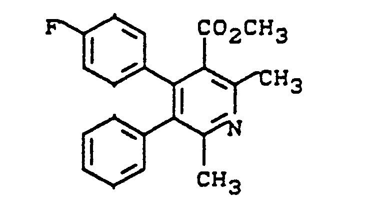

Analog Beispiel 37 erhält man aus 37,3 g (0,1 mol, roh) der Verbindung des Beispiels 52 20,4 g Feststoff. Ausbeute: 49,4% der Theorie
¹H-NMR (CDCl₃): δ = 2,45 (s, 3H,CH₃); 2,6 (s, 3H, CH₃); 3,5 (s, 3H, CH₃); 6,7 - 7,4 (m, 9H, Aromaten-H) ppm.

### Beispiel 54

### 2,6-Dimethyl-4-(4-fluorphenyl)-3-hydroxymethyl-5-phenylpyridin

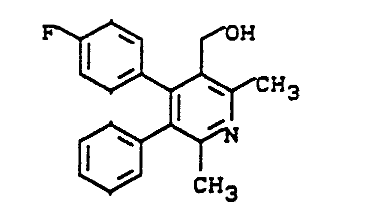

Analog Beispiel 29 erhält man aus 20,2 g (60 mmol) der Verbindung des Beispiels 53, 12,4 g Rohprodukt.
Ausbeute: 67% der Theorie)
¹H-NMR (CDCl₃): δ = 2,0 (br, s, 1H, OH); 2,3 (s, 3H, CH₃); 2,75 (s, 3H, CH₃); 4,45 (s, 2H, CH₂); 6,8 - 7,3 (m, 9H, Aromaten-H) ppm.

### Beispiel 55

### 2,6-Dimethyl-4-(4-fluorphenyl)-5-phenyl-pyridin-3-carbaldehyd

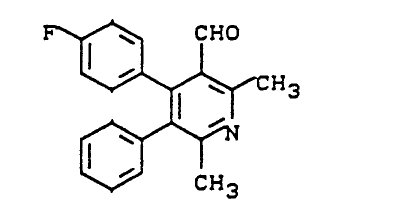

Analog Beispiel 30 erhält man aus 6,0 g (19,5 mmol) der Verbindung des Beispiels 54 nach Chromatographie über Kieselgel (Dichlormethan) 3,8 g Feststoff.
Ausbeute: 64% der Theorie
¹H-NMR (CDCl₃): δ = 2,4 (s, 3H,CH₃); 2,9 (s, 3H, CH₃); 6,8 - 7,3 (m, 9H, Aromaten-H) 9,8 (s, 1H, CHO) ppm.

### Beispiel 56

### (E)-3-[2,6-Dimethyl-4-(4-fluorphenyl)-5-phenyl-pyrid-3-yl]-prop-2-enal

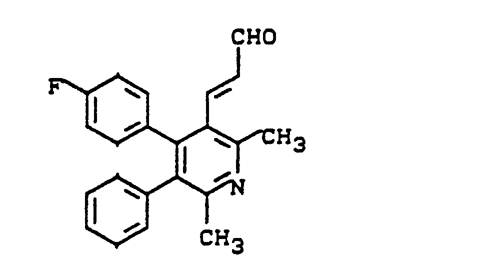

Analog Beispiel 31 erhält man aus 3,1 g (10 mmol) der Verbindung des Beispiels 55 2,0 g Rohprodukt.
Ausbeute: 60% der Theorie
¹H-NMR (CDCl₃): δ = 2,4 (s, 3H, CH₃); 2,75 (s, 3H, CH₃); 6,15 (dd, 1H, CHCHO); 6,85 (d, 1H, CH); 6,9 - 7,3 (m, 9H, Aromaten-H); . 9,4 (d, 1H, CHO) ppm.

### Beispiel 57

### Methyl-(E)-7-[2,6-dimethyl-4-(4-fluorphenyl)-5-phenylpyrid-3-yl]-5-hydroxy-3-oxo-hept-6-enoat

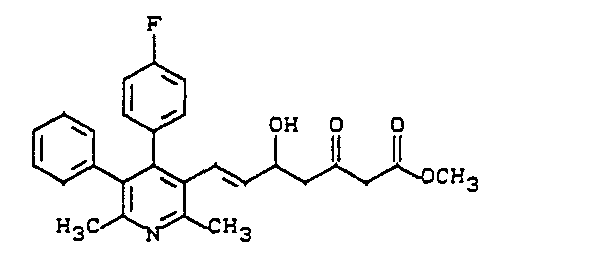

Analog Beispiel 32 erhält man aus 2,0 g (6 mmol) der Verbindung des Beispiels 56 2,4 g Rohprodukt. Rohausbeute: 89% der Theorie
¹H-NMR (CDCl₃): δ = 2,3 (s, 3H, CH₃); 2,6 (s, 3H, CH₃); 2,7 (m, 2H, CH₂); 3,45 (d, 2H, CH₂); 3,75 (2s, 3H, OCH₃); 4,5 (m, 1H, CH); 5,4 (dd, 1H, CHCHO); 6,3 (2d, 1H, CH); 6,7 - 7,3 (m, 9H, Aromaten-H) ppm.

### Beispiel 58 (Vergleichsbeispiel)

### Methyl-erythro-(E)-7-[2,6-dimethyl-4-(4-fluorphenyl)-5-phenyl-pyrid-3-yl]-3,5-dihydroxy-hept-6-enoat

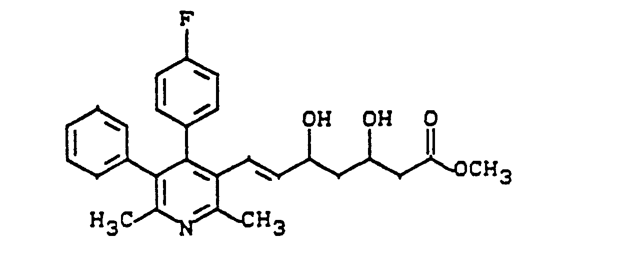

Analog Beispiel 33 erhält man aus 2,4 g (5,3 mmol) der Verbindung des Beispiels 57 nach Chromatographie über Kieselgel (Essigester) 550 mg Produkt.
Ausbeute: 23,1% der Theorie
¹H-NMR (CDCl₃): δ = 1,7 (br, s, 2H, OH); 2,3 (s, 3H, CH₃); 2,4 - 2,6 (m, 2H, CH₂); 2,65 (s, 3H, CH₃); 3,7 (s, 3H, OCH₃); 4,1 (m, 1H, CHOH); 4,45 (m, 1H, CHOH); 5,4 (dd, 1H, CHCHOH); 6,3 (d, 1H, CH); 6,7 - 7,3 (m 9H, Aromaten-H) ppm.

### Beispiel 59

### 2,6-Diisopropyl-4-(4-fluorphenyl)-5-methoxymethylpyridin-3-carbonsäureethylester

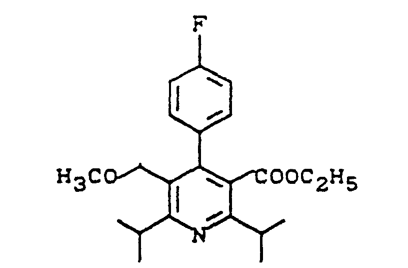

Zu 3 g (8,4 mmol) der Verbindung aus Beispiel 4 in 100 ml trockenem Tetrahydrofuran gibt man unter Stickstoffatmosphäre 0,57 ml (9,2 mmol) Methyljodid und bei -50°C 327 mg (10,9 mmol) 80%iges Natriumhydrid. Man rührt 2 Stunden nach und läßt dabei die Temperatur bis 25°C ansteigen. Die Mischung wird vorsichtig mit Wasser versetzt, mehrmals mit Ether extrahiert, die organische Phase über Magnesiumsulfat getrocknet und im Vakuum eingeengt.
Ausbeute: 2,9 g (92,7% der Theorie)
¹H-NMR (CDCl₃): δ = 0,97 (t, 3H); 1,3 (m, 12H); 3,05 (m, 1H); 3,21 (s, 3H); 3,38 (m, 1H); 3,96 (q, 2H); 4,1 (s, 2H); 7,08 (m, 2H); 7,25 (m, 2H) ppm.

### Beispiel 60

### 2,6-Diisopropyl-4-(4-fluorphenyl)-3-hydroxymethyl-5-methoxymethyl-pyridin

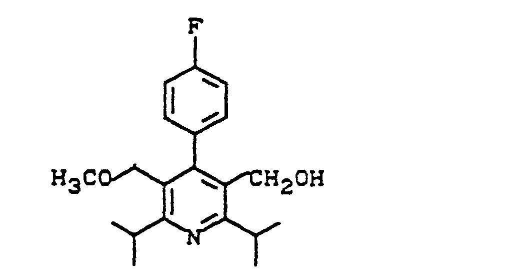

Unter Stickstoffatmosphäre werden zu 0,5 g (13,2 mmol) Lithiumaluminiumhydrid in 20 ml absolutem Tetrahydrofuran bei 60°C 2,85 g (7,6 mmol) der Verbindung aus Beispiel 59 gelöst in 30 ml absolutem Tetrahydrofuran zugetropft. Man erhitzt 1 Stunde zum Rückfluß, kühlt anschließend auf 0°C ab und tropft vorsichtig 1,5 ml Wasser und 0,3 ml 15%ige Kaliumhydroxyd-Lösung zu. Es wird vom ausgefallenen Niederschlag abgesaugt und dieser mehrmals mit Ether ausgekocht. Die vereinigten Phasen werden über Magnesiumsulfat getrocknet und im Vakuum eingeengt. Der Rückstand wird an einer Säule (Kieselgel 70-230 mesh, mit Essigester/Petrolether 1:9) chromatographiert.
Ausbeute: 1,9 g (74,8% der Theorie)
¹H-NMR (CDCl₃): δ = 1,3 (m, 12H); 3,17 (s, 3H); 3,35 (m, 1H); 3,43 (m, 1H); 4,02 (s, 2H); 4,35 (d, 2H); 7,12 (m, 2H); 7,25 (m, 2H) ppm.

### Beispiel 61

### Methyl-erythro-(E)-7-[2,6-diisopropyl-4-(4-fluorphenyl)-5-methoxymethyl-pyrid-3-yl]-3,5-dihydroxy-hept-6-enoat

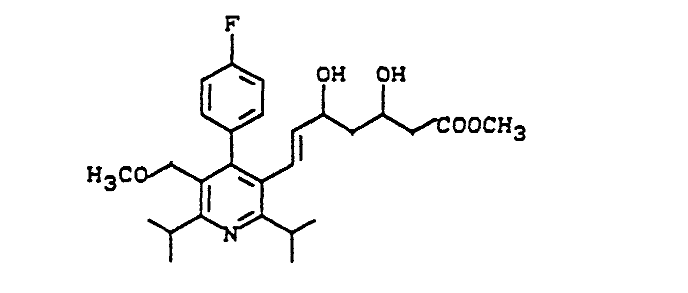

Aus der Verbindung aus Beispiel 60 wurde, in Analogie zu den Reaktionen aus den Beispielen 7, 8, 9, 10 , das Beispiel 61 hergestellt.
¹H-NMR (CDCl₃): δ = 1,23 (m, 6H); 1,32 (d, 6H); 1,40 (m, 2H); 2,43 (m, 2H); 3,18 (s, 3H); 3,32 (m, 2H); 3,73 (s, 3H); 4,05 (s, 2H); 4,08 (m, 1H); 4,29 (m, 1H); 5,23 (dd, 1H); 6,31 (d, 1H); 7,0-7,20 (m, 4H) ppm.

### Beispiel 62

### Methyl-erythro-(E)-7-[5-tert.butyldimethylsilyloxymethyl-4-(4-fluorphenyl)-2-isopropyl-6-methyl-pyrid-3-yl]-3,5-dihydroxy-hept-6-enoat

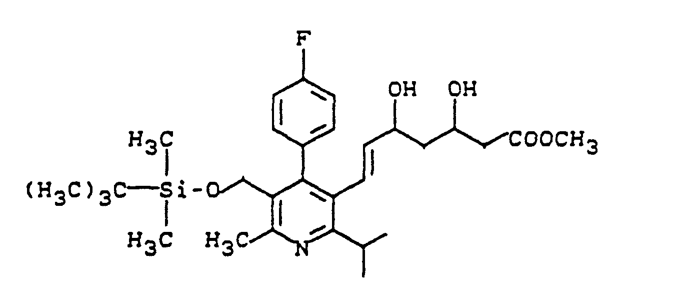

Aus der Verbindung aus Beispiel 20 wurde in Analogie zu den Reaktionen aus den Beispielen 5, 6, 7, 8, 9, 10, 11, 12 die obige Verbindung synthetisiert.
¹H-NMR (CDCl₃): δ = 0,0 (s, 6H); 0,82 (s, 9H); 1,22 (d, 6H); 1,40 (m, 2H); 2,42 (m, 2H); 2,65 (s, 3H); 3,28 (m, 1h); 3,70 (s, 3H); 4,08 (m, 1H); 4,26 (m, 1H); 4,29 (s, 2H); 5,22 (dd, 1H); 6,30 (d, 1H); 7,0 - 7,20 (m, 4H) ppm.

### Beispiel 63

### Methyl-erythro-(E)-7-C4-(4-fluorphenyl)-S-hydroxymethyl-1-isopropyl-6-methyl-pyrid-3-yl]-3,5-dihydroxy-hept-6-enoat

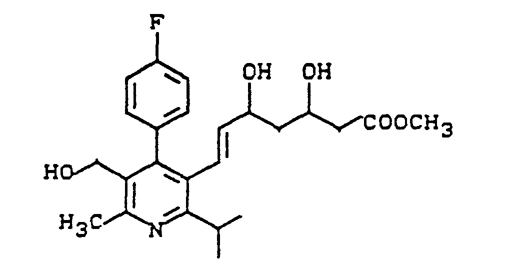

Aus der Verbindung aus Beispiel 20 wurde in Analogie zu den Reaktionen aus den Beispielen 5, 6, 7, 8, 9, 10, 11, 12 die obige Verbindung synthetisiert.
¹H-NMR (CDCl₃): δ = 1,22 (d, 6H); 1,30 - 1,60 (m, 2H); 2,43 (m, 2H); 2,69 (s, 3H); 3,32 (m, 1H); 3,72 (s, 3H); 4,07 (m, 1H); 4,30 (m, 1H); 4,41 (s, 2H); 5,25 (dd, 1H); 6,31 (d, 1H); 7,11 (m, 4H) ppm.

### Beispiel 64

### Methyl-erythro-(E)-7-[3-benzyloxymethyl-4-(4-fluorphenyl)-1-isopropyl-6-methyl-pyrid-5-yl]-3,5-dihydroxyhept-6-enoat

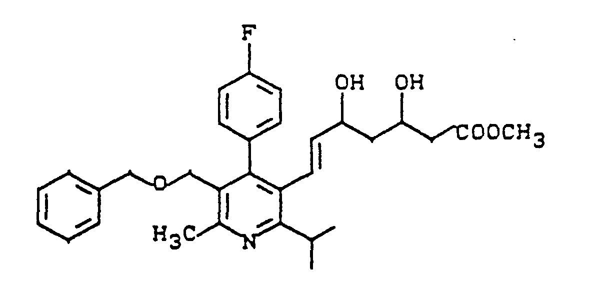

Aus der Verbindung aus Beispiel 20 wurde in Analogie zu den Reaktionen aus den Beispielen 5, 6, 7, 8, 9, 10, 11, 12 die obige Verbindung synthetisiert.
¹H-NMR (CDCl₃): δ = 1,23 (d, 6H); 1,40 (m, 2H); 2,42 (m, 2H); 2,63 (s, 3H); 3,30 (m, 1H); 3,72 (s, 3H); 4,10 (m, 1H); 4,15 (s, 2H); 4,32 (m, 1H); 4,38 (s, 2H); 5,20 (dd, 1H); 6,31 (d, 1H); 7,0-7,40 (m, 9H) ppm.

### Beispiel 65

### 3-(tert.Butyldimethylsilyloxymethyl)-2,6-diisopropyl-5-(2,2-dimethyl-butyryloxymethyl)-4-(4-fluorphenyl)-pyridin

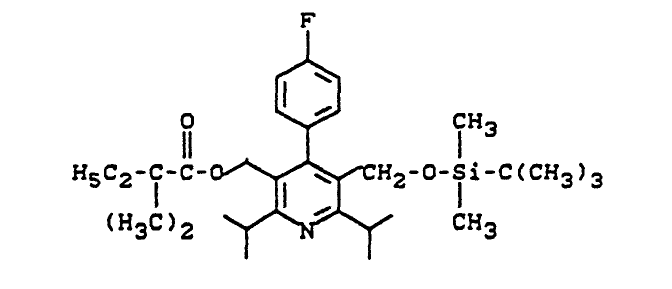

Zu 1,29 g (3 mmol) der Verbindung aus Beispiel 6 in 50 ml absolutem Tetrahydrofuran werden bei 0°C nacheinander 865 mg (3,3 mmol) Triphenylphosphin, 0,41 ml (3,3 mmol) 2,2-Dimethylbuttersäure und 0,52 ml (3,3 mmol) Azodicarbonsäurediethylester gegeben und über Nacht bei Raumtemperatur gerührt. Die Mischung wird im Vakuum eingeengt und der Rückstand an einer Säule (Kieselgel 70-230 mesh, mit Essigester/Petrolether 1:9) chromatographiert.
Ausbeute: 1,32 g (87,4% der Theorie)
¹H-NMR (CDCl₃): δ = 0,01 (s, 6H); 0,86 (t, 3H); 0,91 (s, 9H); 1,2 (s, 6H); 1,39 (m, 12H); 1,6 (q, 2H); 3,24 (m, 1H); 3,48 (m, 1H); 4,38 (s, 2H); 4,79 (s, 2H); 7,05-7,35 (m, 4H) ppm.

### Beispiel 66

### 2,6-Diisopropyl-3-(2,2-dimethyl-butyryloxymethyl)-4-(4-fluorphenyl)-5-hydroxymethyl-pyridin

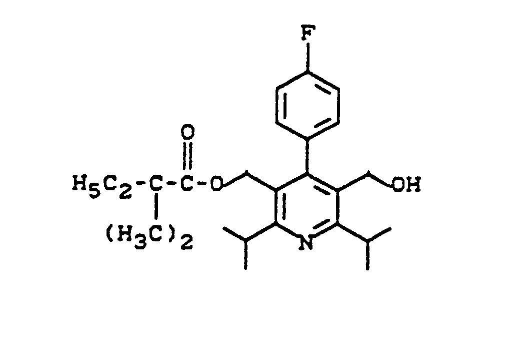

1,3 g (2,6 mmol) der Verbindung aus Beispiel 65 gelöst in 20 ml absolutem Tetrahydrofuran werden mit 2,6 ml (2,6 mmol) einer 1 molaren Tetrabutylammoniumfluorid-Lösung in Tetrahydrofuran versetzt und 1 Stunde bei Raumtemperatur gerührt. Die Mischung wird mit gesättigter Natriumhydrogencarbonat-Lösung versetzt und mehrmals mit Dichlormethan extrahiert. Die organische Phase wird über Magnesiumsulfat getrocknet, im Vakuum eingeengt und der Rückstand an einer Säule (Kieselgel 70-230 mesh, mit Laufmittel Essigester/Petrolether 1:9) chromatographiert.
Ausbeute: 1 g (95,2% der Theorie)
¹H-NMR (CDCl₃): δ = 0,71 (t, 3H); 1,02 (s, 6H); 1,21 (d, 6H); 1,25 (d, 6H); 1,43 (q, 2H); 3,09 (m, 1H); 3,38 (m, 1H), 4,3 (d, 2H); 4,61 (s, 2H); 6,95 - 7,18 (m, 4H) ppm.

### Beispiel 67

### 2,6-Diisopropyl-5-(2,2-dimethyl-butyryloxymethyl)-4-(4-fluorphenyl)-pyridin-3-carbaldehyd

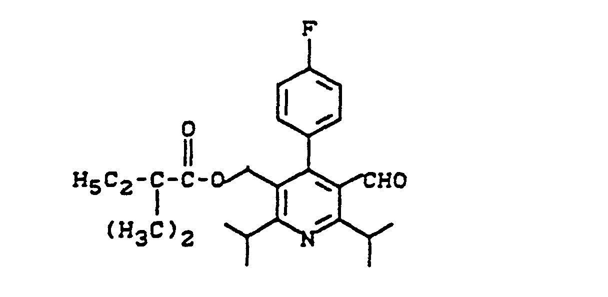

1 g (2,5 mmol) der Verbindung aus Beispiel 66 wird analog Beispiel 7 umgesetzt.
Ausbeute: 890 mg (86,4% der Theorie)
¹H-NMR (CDCl₃): δ = 0,82 (t, 3H); 1,25 (s, 6H); 1,32 (m, 12H); 1,55 (q, 2H); 3,26 (m, 1H); 3,88 (m, 1H); 4,77 (s, 2H); 7,09-7,27 (m, 4H); 9,77 (s, 1H) ppm.

### Beispiel 68

### (E)-3-[2,6-Diisopropyl-5-(2,2-dimethyl-butyryloxymethyl)-4-(4-fluorphenyl)-pyrid-3-yl]-prop-2-enal

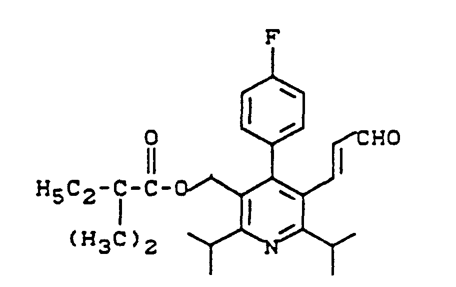

860 mg (2,1 mol) der Verbindung aus Beispiel 67 werden analog zu Beispiel 8 umgesetzt.
Ausbeute: 420 mg (45,6% der Theorie)
¹H-NMR (CDCl₃): δ = 0,82 (t, 3H); 1,14 (s, 6H); 1,31 (m, 12H); 1,53 (q, 2H); 3,22 (m, 1H); 3,33 (m, 1H); 4,75 (s, 2H); 5,99 (dd, 1H); 7,05 - 7,29 (m, 5H); 9,4 (d, 1H) ppm.

### Beispiel 69

### Methyl-(E)-7-[2,6-diisopropyl-5-(2,2-dimethyl-butynyloxymethyl)-4-(4-fluorphenyl)-pyrid-3-yl]-5-hydroxy-3-oxo-hept-6-enoat

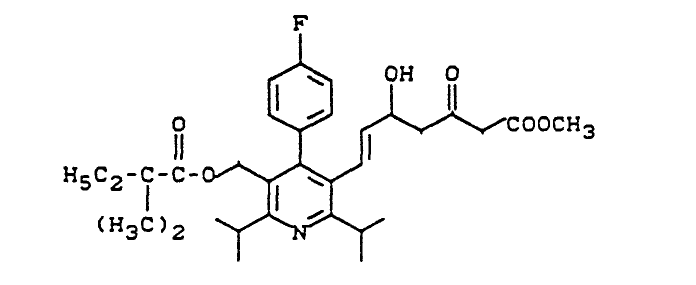

Unter Stickstoff tropft man zu einer Suspension von 54,6 mg (1,82 mmol) 80%igem Natriumhydrid in 5 ml absolutem Tetrahydrofuran bei -5°C 0,15 ml (1,4 mmol) Acetessigsäuremethylester in 2 ml absolutem Tetrahydrofuran. Nach 15 Minuten werden bei derselben Temperatur 0,89 ml (1,4 mmol) 15%iges Butyllithium in n-Hexan zugetropft und nach weiteren 15 Minuten 408 mg (1,8 mmol) trockenes Zinkbromid in 5 ml absolutem Tetrahydrofuran hinzugefügt. Man läßt noch 15 Minuten bei -5°C nachrühren, tropft 400 mg (0,91 mmol) der Verbindung aus Beispiel 68 gelöst in 10 ml trockenem Tetrahydrofuran zu und rührt über Nacht. Die Mischung wird mit gesättigter Ammoniumchlorid-Lösung versetzt und mehrmals mit Ether extrahiert. Die organische Phase wird über Magensiumsulfat getrocknet, im Vakuum eingeengt und der Rückstand an einer Säule (Kieselgel 70-230 mesh, mit Essigester/Petrolether 3:7) chromatographiert.
Ausbeute: 200 mg (38,5% der Theorie)
¹H-NMR (CDCl₃): δ = 0,8 (t, 3H); 1,12 (s, 6H); 1,27 (d, 6H); 1,32 (d, 6H); 1,53 (q, 2H); 2,45 (m, 2H); 3,18 (m, 1H); 3,27 (m, 1H); 3,43 (s, 2H); 3,74 (s, 3H), 4,50 (m, 1H); 4,73 (s, 2H); 5,28 (dd, 1H); 6,38 (d, 1H); 7,0 - 7,10 (m, 4H) ppm.

### Beispiel 70

### Methyl-erythro-(E)-7-[2,6-diisopropyl-5-(2,2-dimethylbutyryloxymethyl)-4-(4-fluorphenyl)-pyrid-3-yl]-3,5-dihydroxy-hept-6-enoat

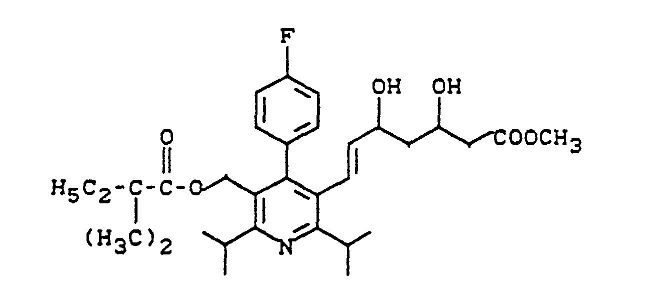

180 mg (0,32 mmol) der Verbindung aus Beispiel 69 werden analog Beispiel 10 umgesetzt.
Ausbeute: 138 mg (77,5% der Theorie)
¹H-NMR (CDCl₃): δ = 0,81 (t, 3H); 1,12 (s, 6H); 1,28 (m, 6H); 1,40 (m, 2H); 1,53 (q, 2H); 2,43 (m, 2H); 3,17 (m, 1H); 3,32 (m, 1H); 3,73 (s, 3H); 4,08 (m, 1H); 4,31 (m, 1H); 4,75 (s, 2H); 5,28 (dd, 1H); 6,32 (d, 1H); 7,0 - 7,1 (m, 4H) ppm.

### Beispiel 71

### Methyl-erythro-(E)-7-[5-benzyloxymethyl-2,6-diisopropyl-4-(4-fluorphenyl)-pyrid-3-yl]-3,5-dihydroxy-hept-6-enoat

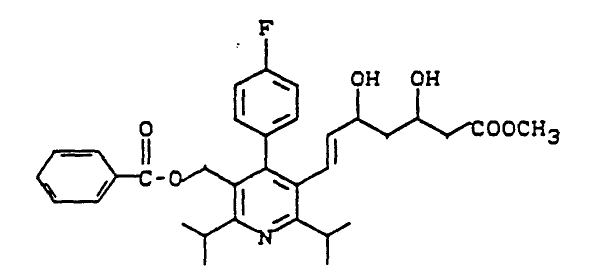

Aus der Verbindung aus Beispiel 6 wurde in Analogie zu den Reaktionen aus den Beispielen 65, 66, 67, 68, 69 und 70 die obige Verbindung synthetisiert.
¹H-NMR (CDCl₃): δ = 1,31 (m, 6H); 1,40 (m, 2H); 2,43 (m, 2H); 3,32 (m, 2H); 3,73 (s, 3H); 4,07 (m, 1H); 4,32 (m, 1H); 5,07 (s, 2H); 5,30 (dd, 1H); 6,32 (d, 1H); 6,90 - 7,20 (m, 4H); 7,42 (m, 2H); 7,55 (m, 1H); 8,02 (m, 2H) ppm.

### Beispiel 72

### Methyl-erythro-(E)-7-[3-acetoxymethyl-2,6-diisopropyl-4-(4-fluorphenyl)-pyrid-5-yl]-3,5-dihydroxy-hept-6-enoat

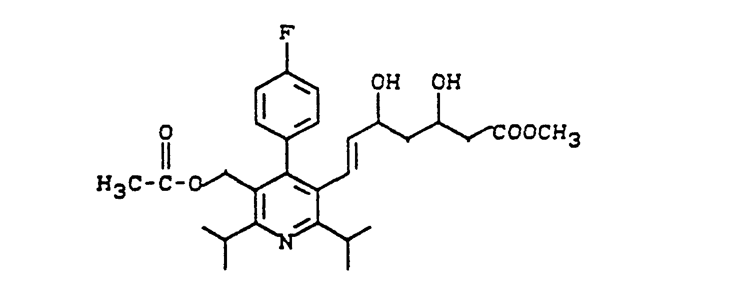

Aus der Verbindung aus Beispiel 6 wurde in Analogie zu den Reaktionen aus den Beispielen 65, 66, 67, 68, 69 und 70 die obige Verbindung synthetisiert.
¹H-NMR (CDCl₃): δ = 1,25 (m, 12H); 1,40 (m, 2H); 2,02 (s, 3H); 2,43 (m, 3H); 3,20 (m, 1H); 3,32 (m, 1H); 3,72 (s, 3H); 4,07 (m, 1H); 4,29 (m, 1H); 4,81 (s, 2H); 5,28 (dd, 1H); 6,30 (d, 1H); 7,0-7,1 (m, 4H) ppm.

### Beispiel 73

### (E/Z)-3-Carboxymethyl-4-(4-fluorphenyl)but-3-en-2-on

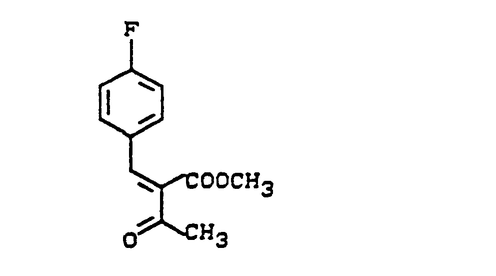

62 g (0,5 mol) 4-Fluorbenzaldehyd und 53,9 ml (0,5 mol) Acetessigsäuremethylester werden in 300 ml Isopropanol vorgelegt, mit einem Gemisch aus 2,81 ml (28 mmol) Piperidin und 1,66 ml (29 mmol) essigsäure in 40 ml Isopropanol versetzt und 48 h bei Raumtemperatur gerührt. Die Mischung wird im Vakuum eingeengt und der Rückstand im Hochvakuum destilliert. Kp 0,5 mm (66,66 Pascals): 138°C Ausbeute: 50,5 g (45,5% der Theorie)

### Beispiel 74

### 1,4-Dihydro-2,6-dimethyl-4-(4-fluorphenyl)-pyridin-3,5- dicarbonsäure-dimethylester

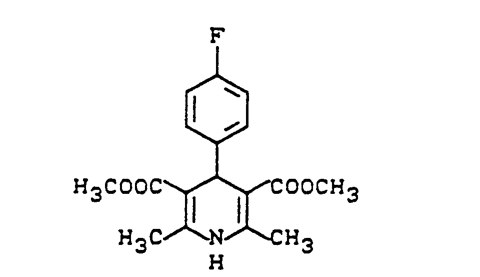

33,3 g (0,15 mol) der Verbindung aus Beispiel 73 werden mit 17,3 g (0,15 mol) 3-Aminocrotonsäuremethylester in 150 ml Ethanol 4 h unter Rückfluß gekocht. Die Mischung wird auf 0°C abgekühlt, der ausgefallene Niederschlag abgesaugt, mit wenig Petrolether nachgewaschen und im Exsikkator getrocknet.
Ausbeute: 32 g (66,8% der Theorie)
¹H-NMR (CDCl₃): δ =2,33 (s, 6H); 3,65 (s, 6H); 4,99 (s,1H); 5,77 (s, 1H); 6,89 (m, 2H); 7,22 (m, 2H) ppm.

### Beispiel 75

### 2,6-Dimethyl-4-(4-fluorphenyl)-pyridin-3,5-dicarbonsäure-dimethylester

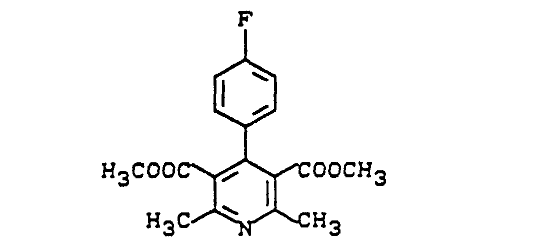

32 g (0,1 mol) der Verbindung aus Beispiel 74 werden analog Beispiel 3 umgesetzt.
Ausbeute: 27,2 g (87% der Theorie)
¹H-NMR (CDCl₃): δ = 2,59 (s, 6H); 3,56 (s, 6H); 7,08 (m, 2H); 7,25 (m, 2H) ppm.

### Beispiel 76

### 2,6-Dimethyl-4-(4-fluorphenyl)-5-hydroxy-methyl-pyridin-3-carbonsäuremethylester

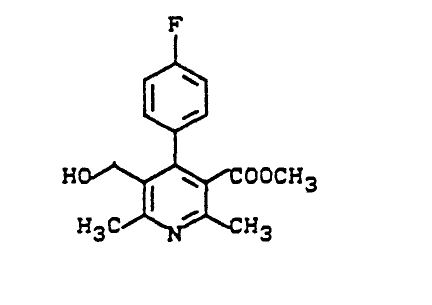

Unter Stickstoff gibt man zu einer Lösung von 14,9 g (47 mmol) der Verbindung aus Beispiel 75 in 300 ml trockenem Tetrahydrofuran bei -10°C bis -5°C 40,3 ml (141 mmol) einer 3,5 molaren Lösung von Natrium-bis-(2-methoxyethoxy)-dihydroaluminat in Toluol und rührt 30 Minuten bei Raumtemperatur. Nach Abkühlen auf 0°C tropft man vorsichtig 150 ml Wasser hinzu und extrahiert mehrmals mit Essigester. Die vereinigten organischen Phasen werden mit gesättigter Natriumchlorid-Lösung gewaschen, über Magnesiumsulfat getrocknet und im Vakuum eingeengt. Der Rückstand kristallisiert mit Ether/Petrolether. Nach Trocknen im Exsikkator erhält man 7,5 g Substanz (55,2% der Theorie)
¹H-NMR (CDCl₃): δ = 2,52 (s, 3H); 2,7 (s, 3H); 3,52 (s, 3H); 4,45 (s, 2H); 7,05 - 7,3 (m, 4H) ppm.

### Beispiel 77

### Methyl-erythro-(E)-7-[5-tert.butyldimethylsilyloxymethyl-2,6-dimethyl-4-(4-fluorphenyl)pyrid-3-yl]-3,5-dihydroxy-hept-6-enoat

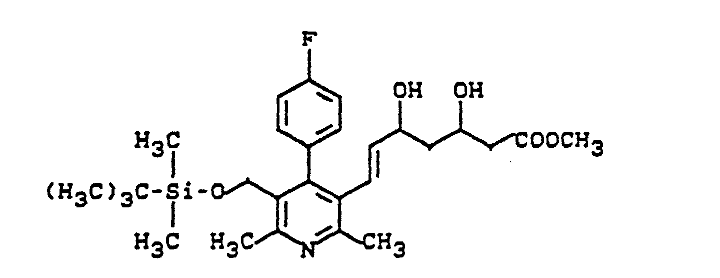

Aus der Verbindung aus Beispiel 76 wurde in Analogie zu den Reaktionen aus den Beispielen 5, 6, 7, 8, 9 und 10 das Beispiel 77 hergestellt.
¹H-NMR (CDCl₃): δ = 0,01 (s, 6H); 0,92 (s, 9H); 1,40 (m, 2H); 2,49 (m, 2H); 2,62 (s, 3H); 2,71 (s, 3H); 3,80 (s, 3H); 4,17 (m, 1H); 4,36 (s, 2H); 4,38 (m, 1H); 5,42 (dd, 1H); 6,31 (d, 1H); 7,10-7,20 (m, 4H) ppm.

### Beispiel 78

### Methyl-erythro-(E)-7-[2,6-dimethyl-4-(4-fluorphenyl)-5-hydroxymethyl-pyrid-3-yl]-3,5-dihydroxy-hept-6-enoat

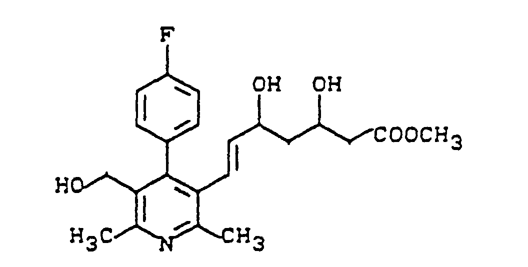

Aus der Verbindung aus Beispiel 77 wurde analog Beispiel 11 das Beispiel 78 hergestellt.

### Beispiel 79

### (E/Z)-2-Carboxyethyl-1-cyclopropyl-3-(4-fluorphenyl)-2-prop-2-en-1-on

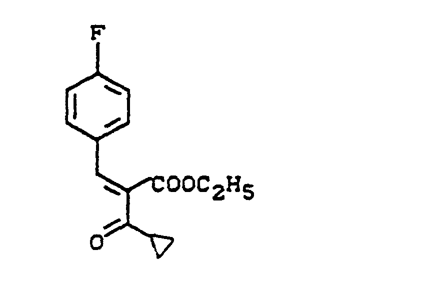

39 g (0,25 mol) Cyclopropylcarbonylessigsäureethylester und 31 g (0,25 mol) 4-Fluorbenzaldehyd werden in 150 ml trockenem Isopropanol vorgelegt und mit einem Gemisch aus 1,4 ml (14 mmol) Piperidin und 0,83 ml (14,5 mmol) Essigsäure in 20 ml Isopropanol versetzt. Man rührt 48 Stunden bie Raumtemperatur, engt im Vakuum ein und destilliert den Rückstand im Hochvakuum.
Kp 0,5 mm (66, 66 Pascals): 140°C
Ausbeute: 52,3 g (79,8% der Theorie)

### Beispiel 80

### 1,4-Dihydro-2-cyclopropyl-4-(4-fluorphenyl)-6-isopropyl-pyridin-3,5-dicarbonsäure-diethylester

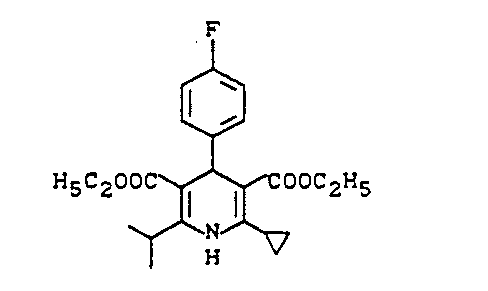

39,3 g (0,15 mol) der Verbindung aus Beispiel 79 und 23,6 g (0,15 mol) 3-Amino-4-methyl-pent-2-en-säureethylester werden in 150 ml Ethylenglykol über Nacht unter Rückfluß gekocht. Nach Abkühlen auf Raumtemperatur wird mehrmals mit Ether extrahiert, die vereinigten Etherphasen dreimal mit 10%iger Salzsäure, je einmal mit Wasser und gesättigter Natriumhydrogencarbonat-Lösung gewaschen, über Magnesiumsulfat getrocknet und im Vakuum eingeengt. Den Rückstand verrührt man mit Petrolether/ Ether, saugt ab und trocknet im Exsikkator.

Ausbeute: 22,8 g (37,8% der Theorie)
¹H-NMR (CDCl₃): δ = 0,65 (m, 2H); 1,03 (m, 2H); 1,15 (m, 13H); 2,78 (m, 1H); 4,15 (m, 4H); 5,03 (s, 1H); 5,72 (s, 1H); 6,90 (m, 2H); 7,22 (m, 2H) ppm.

### Beispiel 81

### 2-Cyclopropyl-4-(4-fluorphenyl)-6-isopropyl-pyridin-3,5-dicarbonsäure-diethylester

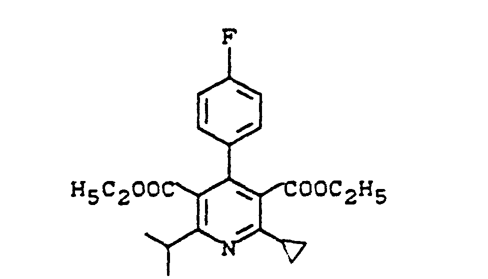

19,1 g (47 mmol) der Verbindung aus Beispiel 80 werden analog Beispiel 3 umgesetzt.
Ausbeute: 9,8 g (52,5% der Theorie)
¹H-NMR (CDCl₃): δ = 0,97 (m, 8H); 1,25 (m, 8H); 2,09 (m, 1H); 3,06 (m, 1h); 4,02 (m, 4H); 7,06 (m, 2H); 7,26 (m, 2H) ppm.

### Beispiel 82

### 6-Cyclopropyl-4-(4-fluorphenyl)-5-hydroxymethyl-2-isopropyl-pyridin-3-carbonsäure-ethylester

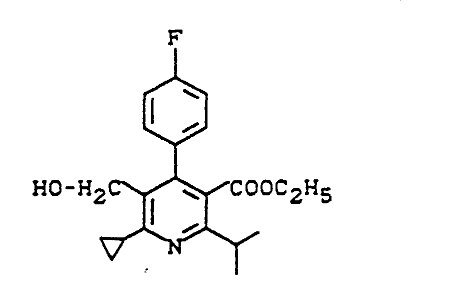

6 g (15 mmol) der Verbindung aus Beispiel 81 werden analog Beispiel 4 umgesetzt.
Ausbeute: 3,1 g (57,9% der Theorie)
¹H-NMR (CDCl₃): δ = 0,97 (t, 3H); 1,03 (m, 2H); 1,22 (m, 8H), 2,38 (m, 1H); 3,03 (m, 1H); 4,0 (q, 2H); 4,58 (s, 2H); 7,1 (m, 2H); 7,25 (m, 2H) ppm.

### Beispiel 83

### 6-Cyclopropyl-4-(4-fluorphenyl)-2-isopropyl-5-methoxymethyl-pyridin-3-carbonsäure-ethylester

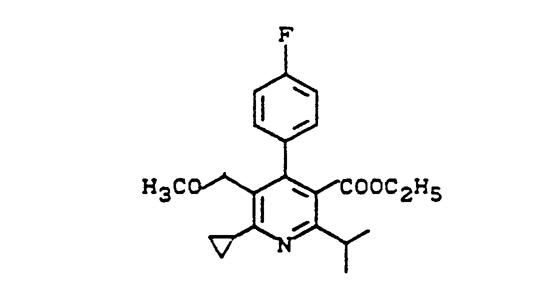

2,9 g (8 mmol) der Verbindung aus Beispiel 82 werden analog Beispiel 59 umgesetzt.
Ausbeute: 2 g (67,4% der Theorie)
¹H-NMR (CDCl₃): δ= 0,93 (t, 3H); 0,98 (m, 2H); 1,22 (d, 6H); 1,24 (m, 2H); 2,32 (m, 1H); 3,03 (m, 1H); 3,28 (s, 3H); 3,97 (q, 2H); 4,25 (s, 2H); 7,08 (m, 2H); 7,25 (m, 2H) ppm.

### Beispiel 84

### 6-Cyclopropyl-4-(4-fluorphenyl)-3-hydroxy-methyl-2-isopropyl-5-methoxymethyl-pyridin

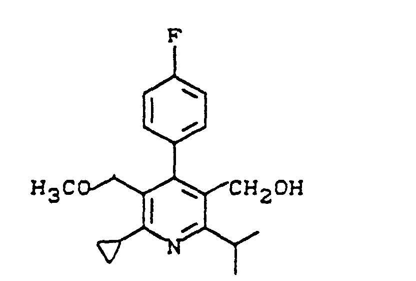

Unter Stickstoff gibt man zu einer Lösung von 1,9 g (5 mmol) der Verbindung aus Beispiel 83 in 50 ml trockenem Tetrahydrofuran 44,3 ml (15 mmol) einer 3,5 molaren Lösung von Natrium-bis-(2-methoxyethoxy)-dihydroaluminat in Toluol, rührt 2 Stunden bei Raumtemperatur und 1 Stunde unter Rückfluß. Nach Abkühlen auf 0°C tropft man vorsichtig 50 ml Wasser hinzu und extrahiert mehrmals mit Essigester. Die Essigesterphasen werden vereinigt, mit gesättigter Natriumchlorid-Lösung gewaschen, über Magnesiumsulfat getrocknet und im Vakuum eingeengt. Ausbeute: 1,6 g (97% der Theorie)
¹H-NMR (CDCl₃): δ = 0,89 (m, 2H), 1,17 (d, 6H); 1,19 (m, 2H); 2,20 (s, 1H); 3,13 (s, 3H); 3,32 (m, 1H); 4,07 (s, 2H); 4,26 (s, 2H); 7,05 (m, 2H); 7,16 (m, 2H) ppm.

### Beispiel 85

### Methyl-erythro-(E)-7-[6-cyclopropyl-2-isopropyl-4-(4-fluorphenyl)-5-methoxymethyl-pyrid-3-yl]-3,5-dihydroxyhept-6-enoat

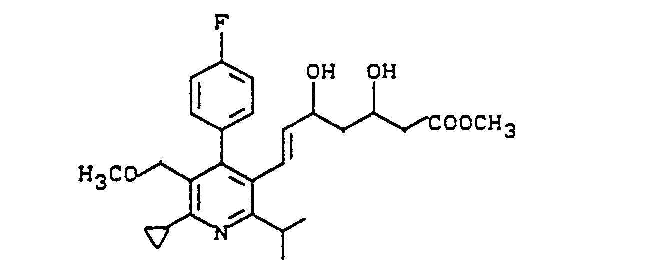

Aus der Verbindung aus Beispiel 84 wurde, in Analogie zu den Reaktionen aus den Beispielen 7, 8, 9 und 10 das Beispiel 85 hergestellt.
¹H-NMR (CDCl₃): δ= 0,95 (m, 2H); 1,17 (m, 6H); 1,22 (m, 2H); 1,40 (m, 2H); 2,25 (m, 1H); 2,44 (m, 2H); 3,22 (s, 3H); 3,23 (m, 1H); 3,73 (s, 3H); 4,07 (m, 1H); 4,18 (s, 2H); 4,28 (m, 1H); 5,22 (dd, 1H); 6,30 (d, 1H); 7,0 - 7,20 (m, 4H) ppm.

### Beispiel 86

### 5-Chlormethyl-2,6-diisopropyl-4-(4-fluorphenyl)-pyridin-3-carbonsäure-ethylester

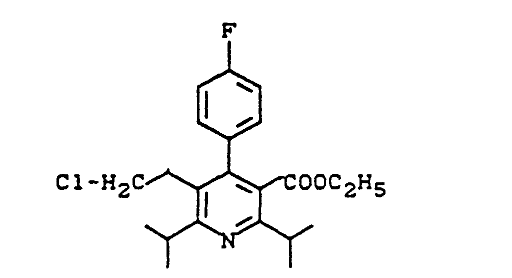

5 g (13,9 mmol) der Verbindung aus Beispiel 4 gelöst in 100 ml trockenem Tetrahydrofuran werden bei -5°C nacheinander mit 1,69 ml (20,9 mmol) Pyridin und 1,5 ml (20,9 mmol) Thionylchlorid versetzt und 15 Minuten bei derselben Temperatur gerührt. Die Mischung wird mit Essigester verdünnt, mehrmals mit gesättigter Natriumhydrogencarbonat-Lösung extrahiert, die organische Phase über Magnesiumsulfat getrocknet und im Vakuum eingeengt. Der Rückstand wird an einer Säule (Kieselgel 70 bis 230 mesh, in Petrolether/Essigester 95:5) chromatographiert.
Ausbeute: 3,2 g (65,2% der Theorie)
¹H-NMR (CDCl₃): δ = 0,98 (t, 3H); 1,30 (d, 6H); 1,35 (d, 6H); 3,05 (m, 1H); 3,45 (m, 1H); 3,98 (q, 2H); 4,38 (s, 2H); 7,13 (m, 2H); 7,31 (m, 2H) ppm.

### Beispiel 87

### 2,6-Diisopropyl-4-(4-fluorphenyl)-5-phenoxymethylpyridin-3-carbonsäure-ethylester

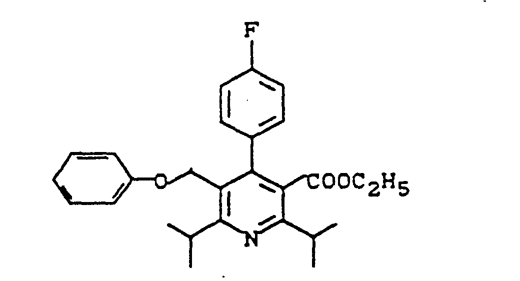

Zu einer Lösung von 2,11 g (18,2 mmol) Natriumphenolat in 50 ml absolutem Tetrahyrofuran tropft man bei 0°C 3,22 g (9,1 mmol) der Verbindung aus Beispiel 86 gelöst in 50 ml absolutem Tetrahyrofuran und kocht 4 Tage am Rückfluß. Nach Abkühlen auf Raumtemperatur wird mit 150 ml Wasser verdünnt und mehrmals mit Ether extrahiert. Die vereinigten organischen Phasen werden über Magnesiumsulfat getrocknet, im Vakuum eingeengt und der Rückstand an einer Säule (Kieselgel 70-230 mesh, mit Petrolether/Essigester 95:5) chromatographiert,
Ausbeute: 3,2 g (80,8% der Theorie)
¹H-NMR (CDCl₃): δ = 0,97 (t, 3H); 1,3 (d, 6H); 1,33 (d, 6H); 3,1 (m, 1H); 3,32 (m, 1H); 4,0 (q, 2H); 4,7 (s, 2H); 6,78 - 7,31 (m, 9H) ppm.

### Beispiel 88

### 2,6-Diisopropyl-4-(4-fluorphenyl)-5-hydroxymethyl-3-phenoxymethyl-pyridin

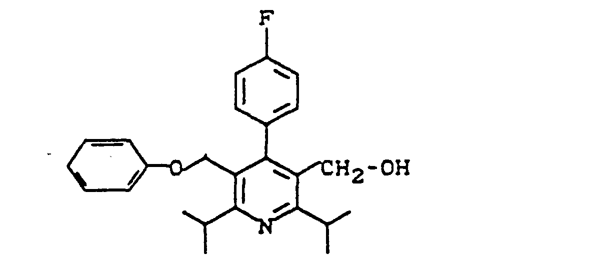

3,25 g (7,5 mmol) der Verbindung aus Beispiel 87 werden analog Beispiel 60 umgesetzt.
Ausbeute: 2,75 g (93,2% der Theorie)
¹H-NMR (CDCl₃): δ = 1,35 (m, 12H); 3,30 (m, 1H); 3,48 (m, 1H); 4,42 (d, 2H); 4,62 (s, 2H); 6,75 - 7,30 (m, 9H) ppm.

### Beispiel 89

### Methyl-erythro-(E)-7-[2,6-diisopropyl-4-(4-fluorphenyl)-5-phenoxymethyl-pyrid-3-yl]-3,5-dihydroxy-hept-6-enoat

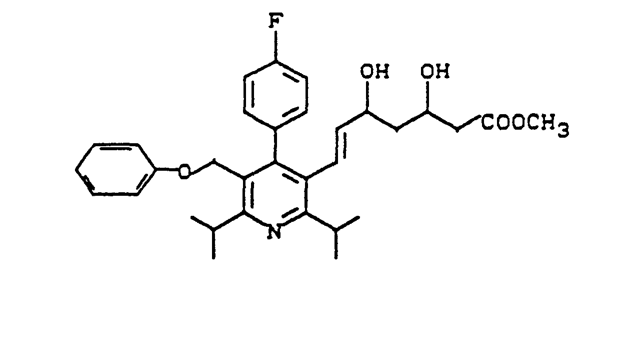

Aus der Verbindung aus Beispiel 88 wurde, in Analogie zu den Beispielen 7, 8, 9 und 10 das Beispiel 89 hergestellt.
¹H-NMR (CDCl₃): δ = 1,28 (m, 6H); 1,31 (d, 6H); 1,43 (m, 2H); 2,41 (m, 2H); 3,30 (m, 2H); 3,71 (s, 3H); 4,08 (m, 1H); 4,30 (m, 1H); 4,65 (s, 2H); 5,28 (dd, 1H); 6,35 (d, 1H); 6,75 - 7,30 (m, 9H) ppm.

### Beispiel 90

### 2,6-Diisopropyl-4-(4-fluorphenyl)-5-(tetrahydropyran-2-yl-oxymethyl)-pyridin-3-carbonsäure-ethylester

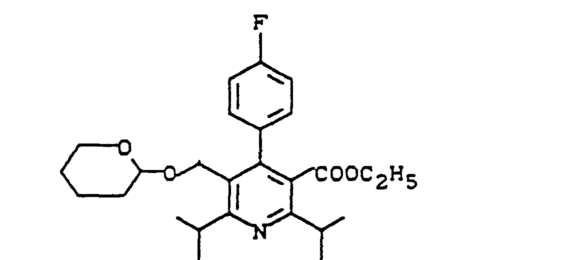

Zu einer Lösung von 5,36 g (14,9 mmol) der Verbindung aus Beispiel 4 in 100 ml trockenem Dichlormethan gibt man 1,88 g(22,4 mmol) Dihydropyran und 0,525 g (1,49 mmol) Pyridinium-p-toluol-sulfonat und kocht 48 Stunden am Rückfluß. Nach Abkühlen auf Raumtemperatur wird mit Dichlormethan verdünnt und mehrmals mit Wasser extrahiert. Die organische Phase wird über Magnesiumsulfat getrocknet, im Vakuum eingeengt und der Rückstand an einer Säule (Kieselgel 70-230 mesh, mit Dichlormethan) chromatographiert.
Ausbeute: 4,4 g (66,7% der Theorie)
¹H-NMR (CDCl₃): δ = 0,98 (t, 3H); 1,31 (m, 12H); 1,40-1,80 (m, 6H); 3,05 (m, 1H); 3,43 (m, 2H); 3,61 (m, 1H); 3,98 (q, 2H); 4,05 (d, 1H); 4,45 (m, 1H); 4,55 (d, 1H); 7,05 (m, 2H); 7,25 (m, 2H) ppm.

### Beispiel 91

### 2,6-Diisopropyl-4-(4-fluorphenyl)-3-hydroxymethyl-5-(tetrahydropyran-2-yl-oxymethyl)-pyridin

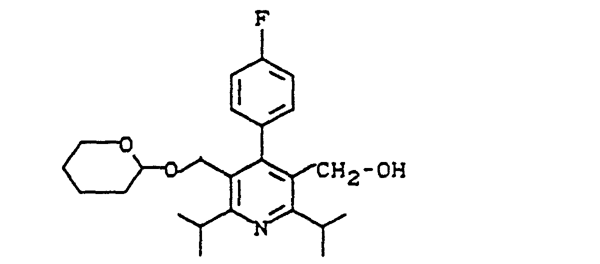

4,4 g (9,9 mmol) der Verbindung aus Beispiel 90 werden analog Beispiel 60 umgesetzt.
Ausbeute: 2,5 g (63,5% der Theorie)
¹H-NMR (CDCl₃): δ = 1,32 (m, 12H); 1,40 - 1,80 (m, 6H); 3,40 (m, 3H); 3,57 (m, 1H); 3,95 (d, 1H); 4,35 (m, 3H); 4,5 (d, 1H); 7,11 (m, 2H); 7,25 (m, 2H) ppm.

### Beispiel 92

### Methyl-erythro-(E)-7-[2,6-diisopropyl-4-(4-fluorphenyl)-5-(tetrahydropyran-2-yl-oxymethyl)-pyrid-3-yl]-3,5-dihydroxy-hept-6-enoat

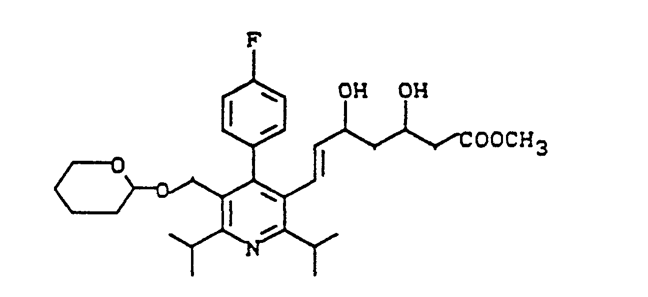

Aus der Verbindung aus Beispiel 91 wurde, in Analogie zu den Reaktionen aus den Beispielen 7,8, 9 und 10 das Beispiel 92 hergestellt.
¹H-NMR (CDCl₃): δ = 1,20 - 1,80 (m, 20H); 2,43 (m, 2H); 3,32 (m, 1H); 3,41 (m, 2H); 3,58 (m, 1H); 3,72 (s, 3H); 3,98 (d, 1H); 4,08 (m, 1H); 4,29 (m, 1H); 4,43 (m, 1H); 4,54 (d, 1H); 5,28 (dd, 1H); 6,31 (d, 1H); 7,10 (m, 4H) ppm.

### Beispiel 93

### 3-Amino-4-methyl-pent-2-en-säure-2-(trimethylsilyl)-ethylester

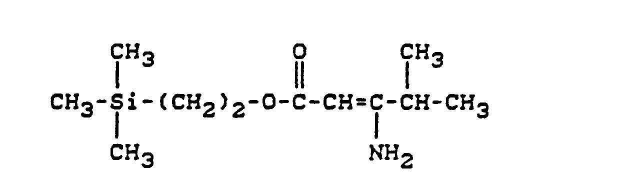

Zu 150 g (0,65 Mol) Isobutyryl-essigsäure-2-(trimethylsilyl)ethylester in 700 ml Toluol werden 3 g p-Toluolsulfonsäure gegeben und die Mischung bei Raumtemperatur mit Ammoniak-Gas gesättigt. Man läßt über Nacht stehen und kocht anschließend 8 Stunden am Rückfluß, wobei ständig Ammmoniak-Gas eingeleitet wird. Nach Abkühlen auf Raumtemperatur wird filtriert, die Toluol-Lösung mehrmals mit Wasser extrahiert, über Magnesiumsulfat getrocknet und im Vakuum eingeengt.
Ausbeute: 134 g (90% der Theorie)
¹H-NMR (CDCl₃): δ = 0,21 (s, 9H); 1,15 (m, 2H); 1,30 (m, 6H); 2,48 (m, 1H); 4,31 (m, 2H); 4,71 (s, 1H) ppm.

### Beispiel 94

### 1,4-Dihydro-2,6-diisopropyl-4-(4-fluorphenyl)-pyridin-3,5-dicarbonsäure-5-ethyl-3-(2-trimethylsilyl)-ethylester

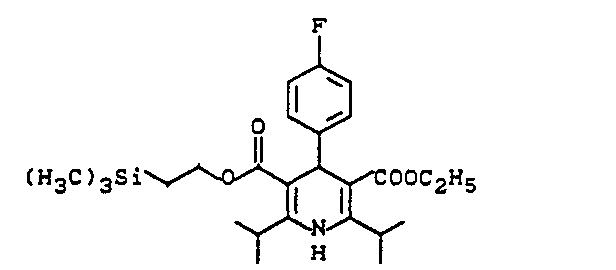

Zu einer Lösung von 45,8 g (0,2 mol) der Verbindung aus Beispiel 93 in 100 ml Ethylenglykol werden 52,7 g (0,2 mol) der Verbindung aus Beispiel 1 gegeben und über Nacht am Rückfluß gekocht. Die Mischung wird abgekühlt, mit 5 ml konz. Salzsäure versetzt und erneut 30 Minuten auf 100°C erwärmt. Nach Abkühlen auf Raumtemperatur wird im Vakuum eingeengt, der Rückstand in Essigester aufgenommen und mehrmals mit verdünnter Salzsäure extrahiert. Die organisch Phase wird anschließend je einmal mit gesättiger Natriumhydrogencarbonat- und Natriumchlorid-Lösung gewaschen, über Magnesiumsulfat getrocknet und im Vakuum eingengt. Der Rückstand wird an einer Säule (Kieselgel 70-230 mesh, mit Dichlormethan) chromatographiert.
Ausbeute: 39,2 g (41,3% der Theorie)
¹H-NMR (CDCL₃): δ = 0,01 (s, 9H); 0,95 (m, 2H); 1,18 (m, 12H); 1,22 (t, 3H); 4,10 (m, 6H); 4,97 (s, 1H); 6,10 (s, 1H); 6,85 (m, 2H); 7,18 (m, 2H) ppm.

### Beispiel 95

### 1,4-Dihydro-2,6-diisopropyl-4-(4-fluorphenyl)-pyridin-3,5-dicarbonsäure-3-ethylester

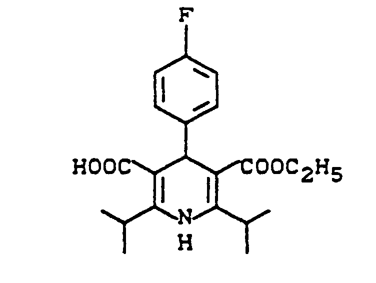

Zu einer Lösung von 38,9 g (81,8 mmol) der Verbindung aus Beipsiel 94 in 300 ml trockenem Tetrahydrofuran werden 83,3 ml (83,3 mmol) einer 1 molaren Lösung von Tetrabutylammoniumfluorid in Tetrahydrofuran gegeben und 48 Stunden bei Raumtemperatur gerührt. Nach Einengen im Vakuum wird der Rückstand in Ether aufgenommen, je dreimal mit verdünnter Natronlauge und verdünnter Schwefelsäure gewaschen, die organische Phase über Magnesiumsulfat getrocknet und erneut im Vakuum eingeengt. Ausbeute: 29 g (94,5% der Theorie)
¹H-NMR (CDCl₃): δ = 1,09 - 1,3 (m, 15H); 4,02 (q, 2H); 4,08 (m 2H); 4,18 (m, 1H); 4,89 (s, 1H); 7,03 (m, 2H); 7,12 (m, 2H) ppm.

### Beispiel 96 (Vergleichsbeispiel)

### Methyl-erythro-(E)-7-[2,6-diisopropyl-4-(4-fluorphenyl)-pyrid-3-yl]-3,5-dihydroxy-hept-6-enoat

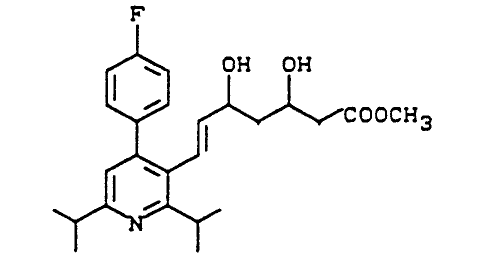

Aus der Verbindung aus Beispiel 95 wurde in Analogie zu den Reaktionen aus den Beispielen 27, 3, 29, 7, 8, 9 und 10 das Beispiel 96 hergestellt.
¹H-NMR (CDCl₃): δ = 1,28 (m, 12H); 1,50 (m, 2H); 2,47 (m, 2H); 3,05 (m, 1H); 3,35 (m, 1H); 3,72 (s, 3H); 4,13 (m, 1H); 4,38 (m, 1H); 5,31 (dd, 1H); 6,55 (d, 1H); 6,85 (s, 1H); 7,05 (m, 2H); 7,25 (m, 2H) ppm.

### Beispiel 97

### 1-(4-Fluorphenyl)-4-methyl-2-phenyl-penten-3-on

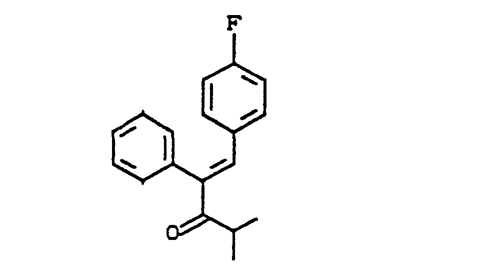

Zu 24,8 g (0,2 mol) 4-Fluorbenzaldehyd und 32,4 g (0,2 mol) Benzyl-isopropylketon in 150 ml Toluol werden 0,9 ml Piperidin gegeben und die Mischung über Nacht am Rückfluß gekocht. Nach Abkühlen auf Raumtemperatur wird mehrmals mit Wasser extrahiert, die organische Phase über Magnesiumsulfat getrocknet und im Vakuum eingeengt. Der Rückstand wird im Hochvakuum bei 0,1 mbar bis 150°C Badtemperatur andestilliert und man erhält 43,8 g Rohprodukt im Destillationsrückstand. Ausbeute: 81% der Theorie

### Beispiel 98

### 1,4-Dihydro-2,6-diisopropyl-4-(4-fluorphenyl)-5-phenylpyridin-3-carbonsäure-ethylester

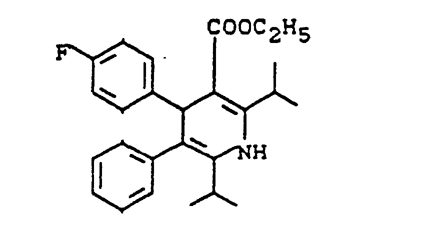

Zu 13,45 g (50 mmol) der Verbindung aus Beispiel 97 und 17,4 g (100 ml) 3-Amino-4-methyl-pent-2-en-säure-ethylester in 80 ml Ethylenglykol werden 2,86 ml (50 mmol) Eisessig gegeben und die Mischung über Nacht am Rückfluß gekocht. Nach Abkühlen auf Raumtemperatur wird im Vakuum eingeengt, der Rückstand in Dichlormethan gelöst und mehrmals mit Wasser extrahiert. Die organische Phase wird über Magnesiumsulfat getrocknet, im Vakuum eingeengt und der Rückstand in Essigester aufgenommen. Nach Extrahieren mit 10%iger Salzsäure, Wasser und gesättigter Natriumhydrogencarbonat-Lösung wird die organische Phase erneut getrocknet, eingeengt und der Rückstand an einer Säule (Kieselgel 70-230 mesh, mit Essigester/Petrolether) chromatographiert.
Ausbeute: 2,3 g (11,3% der Theorie)
¹H-NMR (CDCl₃): δ = 1,1 (m, 9H); 1,25 (m, 6H); 2,70 (m, 1H); 3,90 - 4,40 (m, 3H); 4,55 (s, 1H); 5,75 (s, 1H); 6,80 - 7,30 (m, 9H) ppm.

### Beispiel 99 (Vergleichsbeispiel)

### Methyl-erythro-(E)-7-[2,6-diisopropyl-4-(4-fluorphenyl)-5-phenyl-pyrid-3-yl]-3,5-dihydroxy-hept-6-enoat

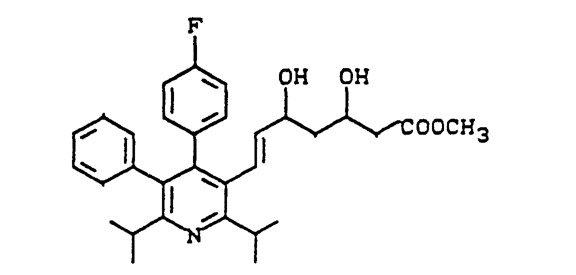

Aus der Verbindung aus Beispiel 98 wurde in Analogie zu den Reaktionen aus den Beispielen 3, 29, 7, 8, 9 und 10 das Beispiel 99 hergestellt.
¹H-NMR (CDCl₃): δ = 1,18 (d, 6H); 1,32 (m, 6H); 1,42 (m, 2H); 2,40 (m, 2H); 2,70 (d, 1H); 2,88 (m, 1H); 3,38 (m, 1H); 3,48 (d, 1H); 3,71 (s, 3H); 4,05 (m, 1H); 4,30 (m, 1H); 5,30 (dd, 1H); 6,39 (d, 1H); 6,70 - 7,20 (m, 9H) ppm.

### Beispiel 100 (Vergleichsbeispiel)

### Methyl-erythro-(E)-7-[2,6-diisopropyl-4-(4-fluorphenyl)-5-carboxyethyl-pyrid-3-yl]-3,5-dihydroxy-hept-6-enoat

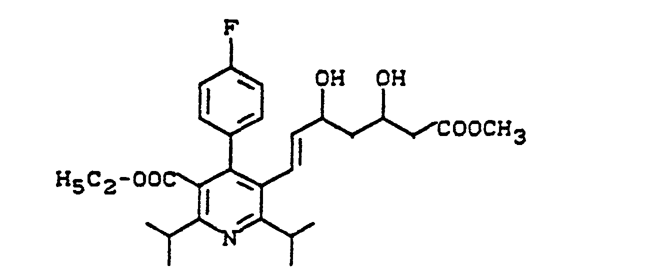

Aus der Verbindung aus Beispiel 4 wurde in Analogie zu den Reaktionen aus den Beispielen 7, 8, 69 und 10 das Beispiel 100 hergestellt.
¹H-NMR (CDCl₃): δ = 0,98 (t, 3H); 1,25 (m, 6H); 1,32 (d, 6H); 1,45 (m, 2H); 2,42 (m, 2H); 3,05 (m, 1H); 3,32 (m, 1H); 3,72 (s, ' 3H); 3,98 (q, 2H); 4,10 (m, 1h); 4,32 (m, 1H); 5,29 (dd, 1H); 6,38 (d, 1H); 7,02 (m, 2H); 7,12 (m, 2H) ppm.

### Beispiel 101

### 1,4-Dihydro-2,6-diisopropyl-4-(4-fluorphenyl)-5-morpholinocarbonyl-pyridin-3-carbonsäureethylester

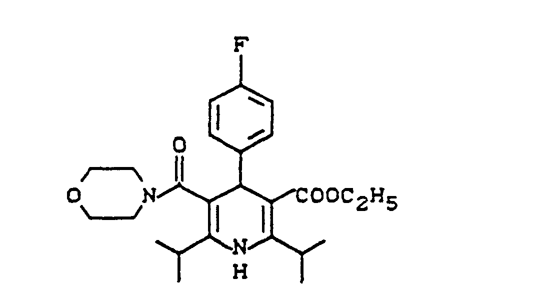

Zu 1,875 g (5 mmol) der Verbindung aus Beispiel 95 gelöst in 20 ml trockenem Tetrahydrofuran gibt man unter Stickstoffatmosphäre 1,05 g (6,5 mmol) N,N'-Carbonylimidazol und rührt 30 Minuten bei Raumtemperatur. Anschließend wird 30 Minuten am Rückfluß gekocht, mit einer Lösung von 0,87 ml (10 mmol) Morpholin in 5 ml trockenem Tetrahydrofuran versetzt und weitere 2 Stunden zum Sieden erhitzt. Nach Abkühlen auf Raumtemperatur wird im Vakuum eingeengt, der Rückstand in Dichlormethan aufgenommen und nacheinander mit 1 N Salzsäure, 1 N Natronlauge und Wasser gewaschen. Die organische Phase wird über Magnesiumsulfat getrocknet, im Vakuum eingeengt und der kristalline Rückstand im Exsikkator getrocknet. Ausbeute: 1,96 g (88% der Theorie)
¹H-NMR (CDCl₃): δ = 1,0 - 1,28 (m, 15H); 3,20 - 4,40 (komplexer Bereich, 12H); 4,70 (s, 1H); 5,50 (s, 1H); 6,90 (m, 2H); 7,20 (m, 2H) ppm.

### Beispiel 102

### 2,6-Diisopropyl-4-(4-fluorphenyl)-pyridin-3,5-dicarbonsäure-3-ethylester-5-morpholid

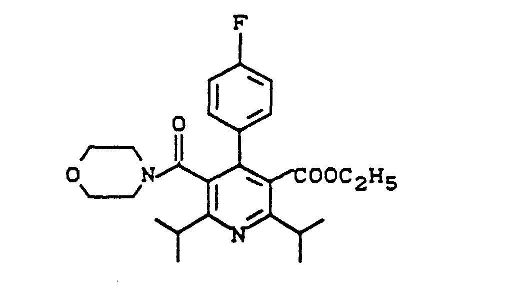

9 g (21,5 mmol) der Verbindung aus Beispiel 101 werden analog Beispiel 3 umgesetzt.
Ausbeute: 7,6 g (80% der Theorie)
¹H-NMR (CDCl₃): δ = 0,95 (t, 3H); 1,25 (m, 6H); 1,35 (m, 6H); 2,70 - 3,80 (komplexer Bereich, 10H); 4,0 (m, 2H); 7,0 - 7,50 (m, 4H) ppm.

### Beispiel 103 (Vergleichsbeispiel)

### Methyl-erythro-(E)-7-[2,6-diisopropyl-4-(4-fluorphenyl)-5-morpholinocarbonyl-pyrid-3-yl]-3,5-dihydroxy-hept-6-enoat

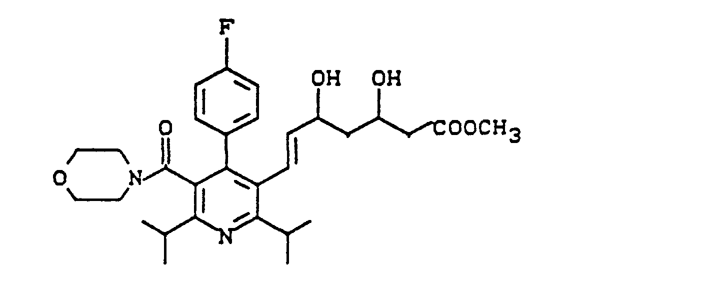

Aus der Verbindung aus Beispiel 102 wurde in Analogie zu den Reaktionen aus den Beispielen 29, 7, 8, 9 und 10 das Beispiel 103 hergestellt.
¹H-NMR (CDCl₃): δ = 1,10 - 1,50 (komplexer Bereich, 14H); 2,40 (m, 2H); 2,80 - 3,65 (komplexer Bereich, 10H); 3,75 (s, 3H); 4,10 (m, 1H); 4,35 (m, 1H); 5,25 (m, 1H); 6,45 (dd, 1H); 6,95-7,50 (m, 4H) ppm.

### Beispiel 104

### 2,6-Diisopropyl-4-(4-fluorphenyl)-3-hydroxymethyl-5-morpholinomethyl-pyridin

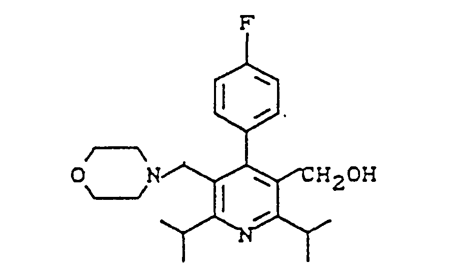

Zu 1,37 g (3,1 mmol) der Verbindung aus Beispiel 102 gelöst in 30 ml trockenem Toluol gibt man unter Stickstoffatmosphäre bei -78°C 20,6 ml (31 mmol) Diisobutylaluminiumhydrid (1 m in Toluol) und rührt 1 Stunde bei derselben Temperatur. Anschließend wird 48 Stunden bei Raumtemperatur gerührt, die Mischung unter Eiskühlung mit 20%iger Kaliumhydroxyd-Lösung hydrolysiert und mehrmals mit Toluol extrahiert. Die organische Phase wird über Magnesiumsulfat getrocknet, im Vakuum eingeengt und im Exsikkator getrocknet. Ausbeute: 1,04 g (87% der Theorie)
¹H-NMR (CDCl₃): δ = 1,28 (d, 6H); 1,32 (d, 6H); 2,15 (m, 4H); 3,18 (s, 2H); 3,45 (m, 2H); 3,52 (m, 4H); 4,32 (d, 2H); 7,05-7,20 (m, 4H) ppm.

### Beispiel 105

### Methyl-erythro-(E)-7-[2,6-diisopropyl-4-(4-fluorphenyl)-5-morpholinomethyl-pyrid-3-yl]-3,5-dihydroxy-hept-6-enoat

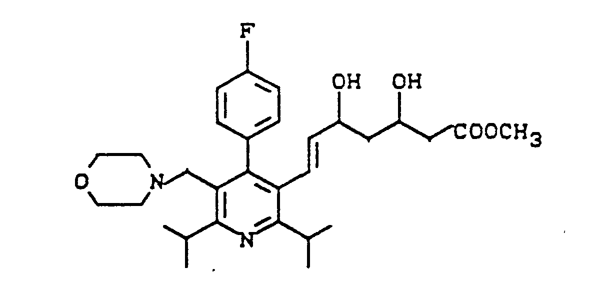

Aus der Verbindung aus Beispiel 104 wurde in Analogie zu den Reaktionen aus den Beispielen 7, 8, 9 und 10 das Beispiel 105 hergestellt.
¹H-NMR (CDCl₃): δ = 1,25 (m, 12H); 1,40 (m, 2H); 2,20 (m, 4H); 2,45 (m, 2H); 3,20 (s, 2H); 3,30 (m, 1H); 3,45 (m, 1H); 3,55 (m, 4H); 3,75 (s, 3H); 4,10 (m, 1H); 4,30 (m, 1H); 5,30 (dd, 1H); 6,25 (d, 1H); 7,0 - 7,20 (m, 4H) ppm.

### Beispiel 106

### Methyl-erythro-(E)-7-(2,6-diisopropyl)-4-(4-fluorphenyl)-5-iodomethyl-pyrid-3-yl]-3,5-dihydroxy-hept-6-enoat

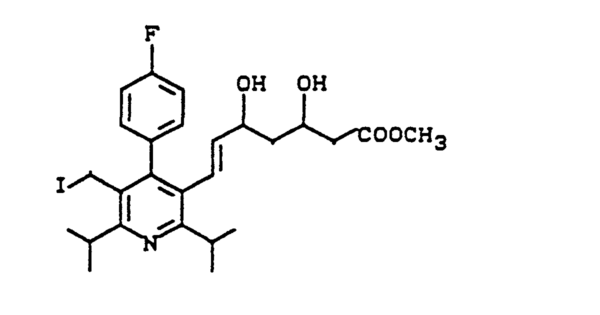

80 mg (0,1514 mmol) der Verbindung aus Beispiel 105 werden in 5 ml Methyljodid gelöst, 3 Stunden auf 30°C und über Nacht bei 60°C unter Lichtausschuß gerührt. Die Mischung wird im Vakuum eingeengt und im Exsikkator über Phosphorpentoxid getrocknet. Man erhält 120 mg Rohprodukt.
¹H-NMR (CDCl₃): δ = 1,20 - 1,70 (komplexer Bereich, 14H); 2,45 (m, 2H); 3,30 (m, 2H); 3,75 (s, 3H); 4,05 (m, 1H); 4,20 (s, 2H); 4,30 (m, 1H); 5,30 (dd, 1H); 6,25 (d, 1H); 7,0 - 7,25 (m, 4H) ppm.

### Beispiel 107

### Methyl-erythro-(E)-7-[2,6-diisopropyl-4-(4-fluorphenyl)-5-benzylthio-methyl-pyrid-3-yl]-3,5-dihydroxy-hept-6-enoat

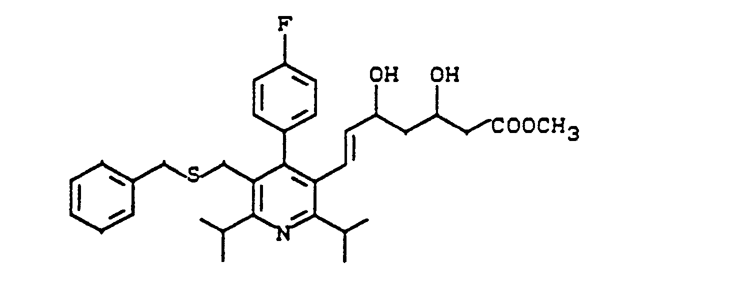

Zu 90 mg (0,158 mmol) der Verbindung aus Beispiel 106 gelöst in 2 ml trockenem Dichlormethan gibt man unter Stickstoffatmosphäre hintereinander 22,3 µl (0,19 mmol) Benzylmercaptan, 32,7 µl (0,237 mmol) Triethylamin und rührt über Nacht bei Raumtemperatur. Die Mischung wird mit Dichlormethan verdünnt und mehrmals mit Wasser extrahiert. Nach Trocknen der organischen Phase über Magnesiumsulfat und Einengen im Vakuum, wird der Rückstand an einer Säule (Kieselgel 70-230 mesh, mit Essigester/ Petrolether 1:1) chromatographiert.
Ausbeute: 20 mg (22,4% der Theorie)
¹H-NMR (CDCl₃): δ = 1,23 (m, 12H); 1,4 (m, 2H); 2,40 (m, 2H); 3,20 (m, 2H); 3,28 (s, 2H); 3,55 (s, 2H); 3,73 (s, 3H); 4,05 (m, 1H); 4,25 (m, 1H); 5,25 (dd, 1H); 6,25 (d, 1H); 6,90 - 7,28 (m, 9H) ppm.

### Beispiel 108

### 4-{[5-(3,5-dihydroxy-6-methoxycarbonylhex-1-enyl)-2,6-diisopropyl-4-(4-fluorphenyl]-pyrid-3-yl}methylmorpholinoxid

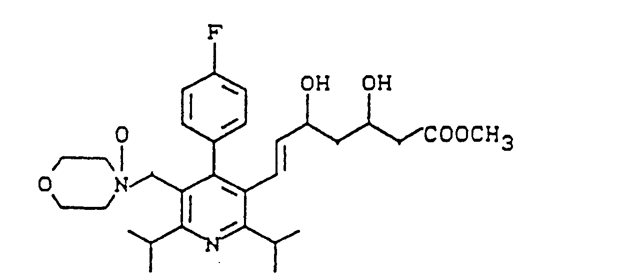

Zu 80 mg (0,1515 mmol) der Verbindung aus Beispiel 105 gelöst in 3 ml trockenem Dichlormethan gibt man 52 mg (0,303 mmol) m-Chlorperbenzoesäure und rührt 1 Stunde bei Raumtemperatur. Die Mischung wird nacheinander mit Kaliumjodid-Lösung, Natriumthiosulfat-Lösung und Natriumhydrogencarbonat-Lösung gewaschen, die organische Phase über Magnesiumsulfat getrocknet und im Vakuum eingeengt.
Ausbeute: 60 mg (73% der Theorie)
¹H-NMR (CDCl₃): δ = 1,10 - 1,55 (komplexer Bereich, 14H); 2,45 (m, 2H); 2,70 - 3,70 (komplexer Bereich, 10H); 3,75 (s, 3H); 4,0 (m, 1H); 4,10 (m, 1H); 4,30 (m, 2H); 5,25 (dd, 1H); 6,25 (d, 1H); 7,0 - 7,30 (m, 4H) ppm.

### Beispiel 109

### 2-(4-Fluorbenzoyl)-4-methyl-pent-2-en-carbonsäureethylester

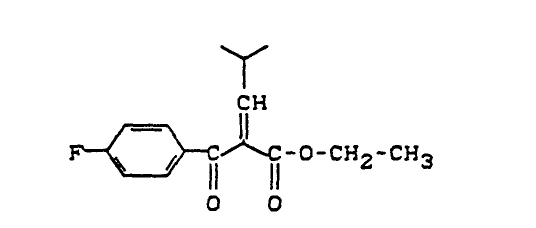

Eine Lösung von 210 g (1 mol) 4-Fluorbenzoylessigsäureethylester und 144 g (2 mol) 2-Methylpropanal wird in 100 ml Isopropanol mit 7 ml Piperidin und 5 ml Essigsäure über Nacht bei 50°C gerührt. Nach vollständiger Umsetzung wird der Ansatz bei ca. 15 Torr eingeengt und das Rohprodukt (270 g, ca. 85%ig) ohne weitere Reinigung weiter umgesetzt.

### Beispiel 110

### 3-Ethoxycarbonyl-2-(4-Fluorphenyl)-1,4-dihydro-4-isopropyl-6-methyl-5-methoxycarbonyl-pyridin

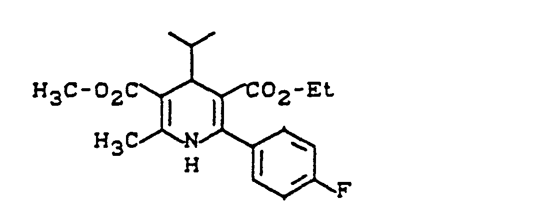

62,9 g (0,2 mol) der Verbindung aus Beispiel 109 und 21,9 g (0,19 mol) 3-Amino-crotonsäure-methylester werden in 200 ml Ethylenglykol über Nacht am Rückfluß gekocht. Man extrahiert dreimal mit Ether,wäscht die vereinigten organischen Phasen mit 2 N Salzsäure und gesättigter Kochsalzlösung, trocknet über Magnesiumsulfat und engt zur Trockene ein. Der Rückstand (65 g ) wird in zwei Portionen an je 750 g Kieselgel (230 - 400 mesh) in einer Säule (7,5 cm ø) mit Petrolether/Essigester 10:1 → 5:1 chromatographiert.
- Ausbeute:: 21,5 g (31%) gelbe Kristalle
- Schmp. :: 109°C

### Beispiel 111

### 3-Ethoxycarbonyl-2-(4-fluorphenyl)-4-isopropyl-5-methoxycarbonyl-6-methyl-pyridin

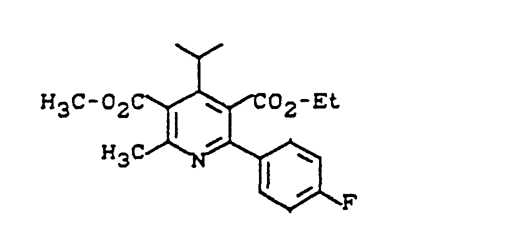

Analog Beispiel 3 wurde aus 14,9 g (14,5 mmol) der Verbindung aus Beispiel 110 das Beispiel 111 hergestellt.
Ausbeute: 15,2 g (102%) Rohprodukt, farbloses Öl, das ohne weitere Reinigung umgesetzt wird.
¹H-NMR (CDCl₃): δ = 1,02 (t, 3H, CH₂CH₃); 1,33 (d, 6H, CH(CH₃)₂); 2,55 (s, 3H, 6-CH₃); 3,15 (sept, 1H, CH(CH₃)₂); 3,95 (s, 3H, O-CH₃); 4,08 (q, 2H, CH₂-CH₃); 7,1 (m, 2H, 3'-H); 7,55 (m, 2H, 2'-H); ppm.

### Beispiel 112

### 3-Ethoxycarbonyl-2-(4-fluorphenyl)-5-hydroxymethyl-4-isopropyl-6-methyl-pyridin

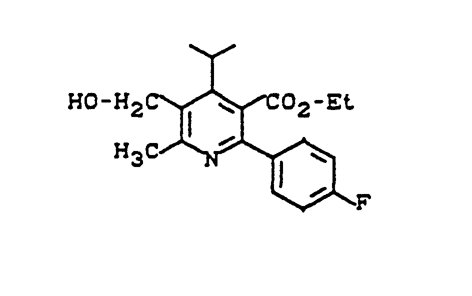

Analog Beispiel 4 wurde aus 10 g (27,8 mmol) der Verbindung aus Beispiel 111 das Beispiel 112 hergestellt. Ausbeute: 4,53 g (49% der Theorie) gelbliche Kristalle Schmp. : 113°C

### Beispiel 113

### Methyl-erythro-(E)-7-[5-tert.butyldimethyl-silyloxymethyl-2(4-fluorphenyl)-4-isopropyl-6-methyl-pyrid-3-yl]-3,5-dihydroxy-hept-6-enoat

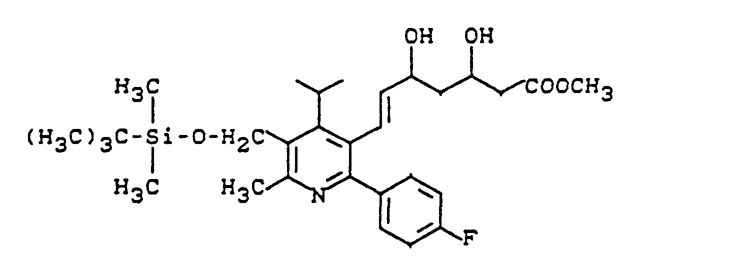

Aus der Verbindung des Beispiels 112 wurde in Analogie zu den Reaktionen aus den Beispielen 5, 6, 7, 8, 9 und 10 das Beispiel 113 hergestellt.
Man erhielt einen farblosen Schaum.
¹H-NMR (CDCl₃): δ = 0,2 (s, 6H, Si(CH₃)₂); 9,13 (s, 9H, Si-C(CH₃)₃);; 1,3 (m, 8H, CH(CH₃)₂ + CH(OH)-CH₂-CH(OH)); 2,4 (m, 2H, CH₂-COOCH₃); 2,65 (s, 3H, 6'-CH₃); 3,1 (b, 1H, OH); 3,65 (b, 1H, OH); 3,7 (s, 3H, O-CH₃); 4,1 (m, 1H, CH-OH); 4,35 (m, 1H, CH-OH); 4,8 (s, 2H, 5'-CH₂); 5,15 (dd, 1H, 6-H); 6,7 (d, 1H, 7-H); 7,0 (m, 2H, 3"-H); 7,35 (m, 2H, 2"-H) ppm.

### Beispiel 114

### Methyl-erythro-(E)-7-[2-(4-fluorphenyl)-5-hydroxymethyl-4-isopropyl-6-methyl-pyrid-3-yl]-3,5-dihydroxy-hept-6-enoat

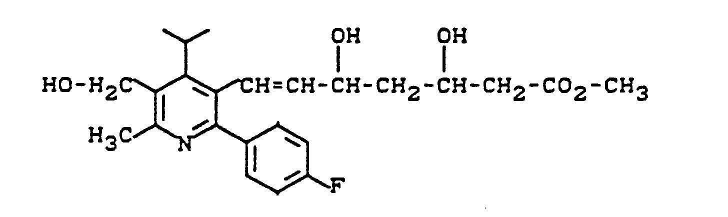

223 g (0,4 mmol) der Verbindng aus Beispiel 113 werden in 5 ml Methanol mit 0,5 ml 1 N Salzsäure 2 Tage bei Raumtemperatur gerührt. Einengen und Säulenchromatographie an 18 g Kieselgel 230-400 mesh, ø 2 cm, Chloroform/Methanol 10:1 ergeben 100 mg (57% der Theorie) farblosen Schaum.
¹H-NMR (CDCl₃): δ = 1,2 - 1,45 (m, 8H, CH(CH₃)₂ + CH(OH)-CH₂-CH(OH)); 2,4 (m, 2H, CH₂-COOCH₃); 2,7 (s, 3H, 6'-CH₃); 3,1 (b, 1H, OH); 3,6 (m, 2H, CH(CH₃)₂ + OH); 3,7 (s, 3H, O-CH₃); 4,1 (m, 1H, CHOH); 4,35 (m, 1H, CH-OH); 4,88 (s, 2H, 5'-CH₂); 5,18 (dd, 1H, 6'-H); 6,7 (d, 1H, 7-H); 7,03 (m, 2H, 3''-H); 7,38 (m, 2H, 2"-H) ppm.

### Beispiel 115

### Methyl-erythro-(E)-7-[5-benzyloxymethyl-2-(4-fluorphenyl)-4-isopropyl-6-methyl-pyrid-3-yl]-3,5-dihydroxyhept-6-enoat

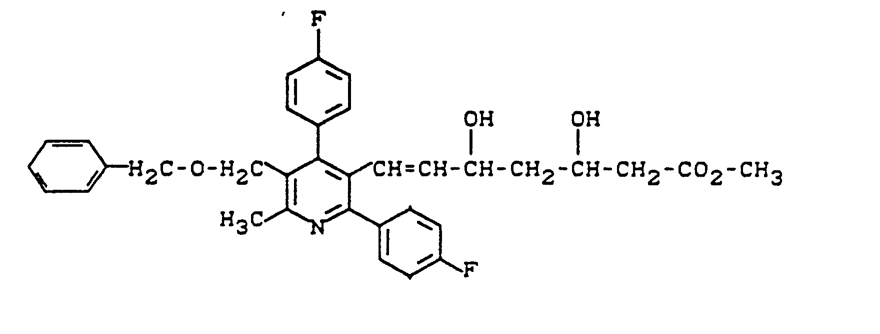

Aus der Verbindung des Beispiels 112 wurde in Analogie zu den Reaktionen aus den Beispielen 12, 6, 7, 8, 9 und 10 das Beispiel 115 hergestellt. Man erhielt einen farblosen Schaum.
¹H-NMR (CDCl₃): δ = 1,2 - 1,45 (m, 8H, CH(CH₃)₂ + CH(OH)-CH₂-CH(OH); 2,4 (m, 2H, CH₂-COOCH₃); 2,6 (s, 3H, 6'-CH₃); 3,05 (b, 1H, OH); 3,5 (m, 2H, CH(CH₃)₂ + OH); 3,72 (s, 3H, O-CH₃); 4,1 (m, 1H, CH-OH); 4,35 (m, 1H, CHOH); 4,62 (s, 2H, O-CH₂); 4,66 (s, 2H, OCH₂); 5,15 (dd, 7H, 6-H); 6,2 (d, 1H, 7-H); 7,12 (m, 2H, 3''-H); 7,3 - 7,45 (m, 7H, Aromaten-H) ppm.

### Beispiel 116

### Methyl-erythro-(E)-7-[2-(4-fluorphenyl)-4-isopropyl-5-methoxymethyl-6-methyl-pyrid-3-yl]-3,5-dihydroxy-hept-6-enoat

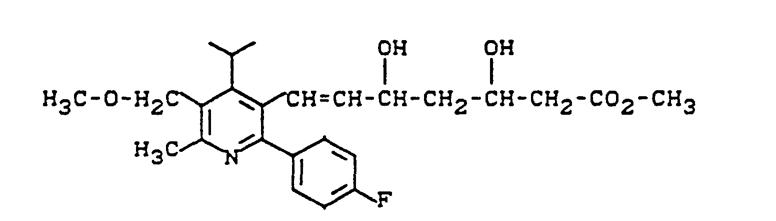

Aus der Verbindung des Beispiels 112 wurde in Analogie zu den Reaktionen aus den Beispielen 59, 6, 7, 8, 9 und 10 das Beispiel 116 hergestellt. Man erhielt einen farblosen Schaum.
¹H-NMR (CDCl₃): δ = 1,2 - 1,45 (m, 8H, CH(CH₃)₂ + CH(OH)-CH₂-CH(OH); 2,4 (m, 2H, CH₂-COOCH₃); 2,63 (s, 3H, 66'-CH₃); 3,15 (b, 1H, OH); 3,5 (m, 4H, O-CH₃ + CH(CH₃)₂); 3,62 (b, 1H, OH); 3,71 (s, 3H, COOCH₃); 4,1 (m, 1H, CH-OH); 4,35 (m, 1H, CH-OH); 4,55 (s, 2H, O-CH₂); 5,15 (dd, 1H, 6-H); 6,65 (d, 1H, 7-H); 7,0 (m, 2H, 3"-H); 7,35 (m, 2H, 2''-H) ppm.

### Beispiel 117

### 3-Benzyloxymethyl-4-(4-fluorphenyl)-6-isopropyl-5-(methyl-erythro-(E)-3,5-dihydroxy-hept-6-enoat-7-yl)-pyridin-N-oxid

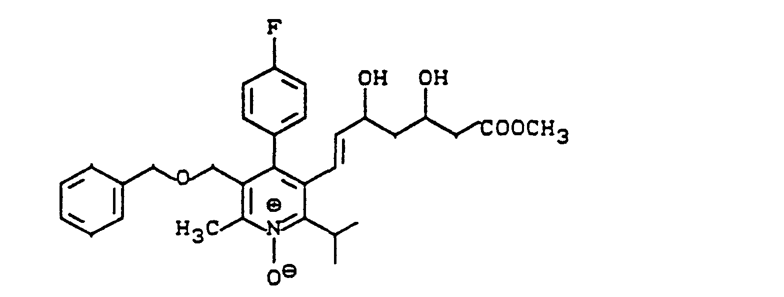

208,4 mg (0,4 mmol) der Verbindung aus Beispiel 64 gelöst in 10 ml Dichlormethan werden mit 863 mg (4 mmol) 80 %iger meta-Chlorperoxybenzoesäure versetzt und über Nacht bei Raumtemperatur gerührt. Nach Einengen im Vakuum wird der Rückstand an einer Säule (Kieselgel 70-230 mesh, mit Dichlormethan/Methanol 96:4) chromatographiert.
Ausbeute: 107 mg (50% der Theorie)
¹H-NMR (CDCl₃): δ = 1,20 - 1,60 (m, 2H); 1,45 (d, 6H); 2,40 (m, 2H); 2,58 (s, 3H); 3,62 (m, 1H); 3,72 (s, 3H); 4,08 (m, 1H); 4,12 (s, 2H); 4,30 (m, 1H); 4,38 (s, 2H); 5,23 (dd, 1H); 6,28 (d, 1H); 7,00 - 7,40 (m, 9H) ppm.

### Beispiel 118

### 3-Amino-4'-fluor-zimtsäurenitril

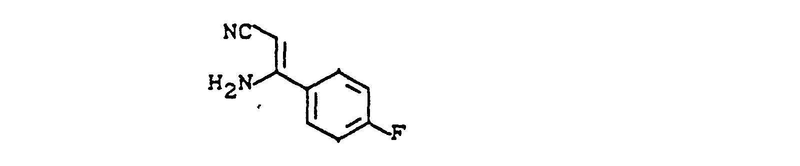

Zu einer Suspension von 30 g (1 mol) Natriumhydrid in 300 ml p.a. Tetrahydrofuran tropft man bei Raumtemperatur unter Rühren eine Lösung von 41 g (1 mol) Acetonitril, 135 g (1,1 mol) 4-Fluorbenzonitril und 7,4 g (0,1 mol) tert.-Butanol in 300 ml Tetrahydrofuran zu und erhitzt auf ca. 30°C, bis die Reaktion anspringt. Unter externem Kühlen wird der Rest bei 35 - 40°C zugetropft und anschließend 30 min unter Rückfluß gekocht. Dann werden vorsichtig 500 ml Wasser zugetropft, die wäßrige Phase zweimal mit Essigester extrahiert und die vereinigten organischen Phasen über Natriumsulfat getrocknet. Nach Abziehen des Lösemittels bleibt ein Kristallbrei, der mit Ether verrührt und abgesaugt wird (65,1 g). Das Filtrat wird zur Trockene einrotiert, mit Toluol über 300 g Kieselgel filtriert und wie oben aus Ether kristallisiert (46,3 g). Ausbeute: 111,4 g (69% der Theorie) farblose Kristalle Schmp.: 108°C

### Beispiel 119

### E/Z-4-Methoxycarbonyl-2,6-dimethyl-hept-4-en-3-on

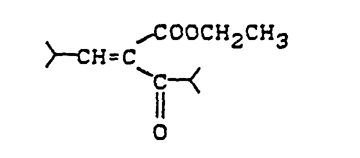

158 g (1 mol) Isobutyrylessigsäureethylester, 108 g (1,5 mol) Isobutyraldehyd, 8,75 ml Piperidin und 6,25 ml Essigsäure werden in 400 ml Isopropanol 20 h bei 50°C gerührt. Man zieht im Wasserstrahlvakuum flüchtige Komponenten ab und destilliert anschließend im Hochvakuum.
- Ausbeute:: 145 g (68 % der Theorie) farbl. Öl,
- Kp =: 60°C, 0,2 mbar

### Beispiel 120

### 3-Cyano-5-ethoxycarbonyl-2-(4-fluorphenyl)-1,4-dihydro-4,6-diisopropyl-pyridin

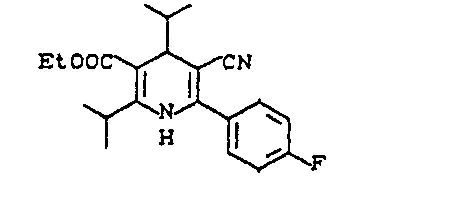

26,7 g (165 mmol) der Verbindung aus Beispiel 118 und 35 g (165 mmol) E/Z-4-Ethoxycarbonyl-2,6-dimethyl-hept-4-en-3-on aus Beispiel 130 werden 4 h bei 200°C Badtemperatur erhitzt. Dann gibt man weitere 17 g (80 mmol) der letzten Komponente zu und erhitzt über Nacht. Der Rückstand wird mit 3 l Toluol/Petrolether (1:1), 3 l Toluol und 2 l Toluol/Essigester (10:1) über 450 g Kieselgel (230-400 mesh) vorgereinigt. Aus dem eingeengten Filtrat kristallisieren aus Ether 5,7 g (9,7%) gelbliche Kristalle (Schmp.: 140°C). Das Filtrat wird nochmal an 750 g Kieselgel mit Petrolether/Essigester (10:1) chromatographiert. Man erhält zwei Zonen:
1. 7,4 g (12,5%) farblose Kristalle vom Schmp.: 141°C (aus Ether/Petrolether) und als Nebenprodukt
2. 3,5-Bis-cyano-2,6-bis-4-fluorphenyl-1,4-dihydro-4-isopropyl-pyridin [2,2 g (3,7%) gelbliche Kristalle vom Schmp.: 227 - 228°C, aus Ether/Petrolether].
Gesamtausbeute: 13,1 g (22% der Theorie)
¹H-NMR (CDCl₃): δ = 0,93 (d, 3H); 1,02 (d, 3H); 1,2 (m, 6H); 1,3 (t, 3H); 1,8 (m, 1H); 3,6 (d, 1H); 4,2 (m, 2H); 6,15 (b, 1H); 7,2 (t, 2H); 7,53 (m, 2H) ppm.

### Beispiel 121

### 3-Cyano-5-ethoxycarbonyl-2-(4-fluorphenyl)-4,6-diisopropyl-pyridin

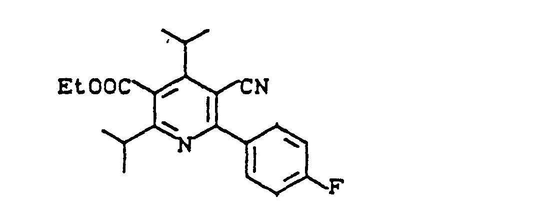

Die Darstellung erfolgt analog der Vorschrift von Beispiel 3 aus 16,0 g (45 mmol) der Verbindung aus
Beispiel 120.
- Ausbeute:: 14,5 g (91% der Theorie)
- Schmp.:: 82°C

### Beispiel 122

### 2-(4-Fluorphenyl)-3-formyl-5-hydroxymethyl-4,6-diisopropyl-pyridin

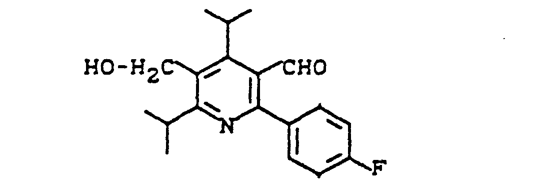

Zu einer Lösung von 14,5 g (41 mmol) der Verbindung aus Beispiel 121 in 320 ml Toluol p.a. tropft man unter Argon bei -78°C bis -75°C 110 ml (165 mmol) einer 1,5 M Lösung von Diisobutylaluminiumhydrid in Toluol, rührt 2 h bei dieser Temperatur und anschließend 1 h bei -20°C. Dann werden 350 ml Wasser und 250 ml Essigester zugetropft, mit Kieselgur abgesaugt, mit Essigester nachgewaschen und die wäßrige Phase mit 300 ml Essigester extrahiert. Die vereinigten organischen Phasen werden mit Kochsalzlösung gewaschen, über Natriumsulfat getrocknet und eingeengt. Säulenchromatographie an 500 g Kieselgel (230-400 mesh) mit Petrolether/Essigester (5:1) und Umkristallisation aus Ether/Petrolether ergibt 5,4 g (42% der Theorie) gelbliche Kristalle vom Schmp.: 147°C.

### Beispiel 123

### (E) -3-[2-(4-Fluorphenyl)-5-hydroxymethyl-4,6-diisopropyl-pyridin-3-yl]-prop-2-enal

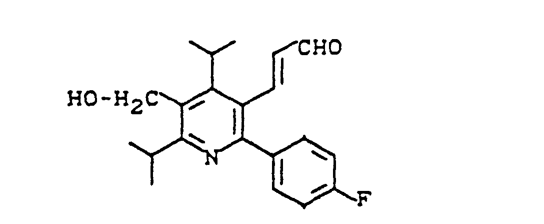

Die Darstellung erfolgt analog Beispiel 8 aus 200 mg (6,6 mmol) 80%igem Natriumhydrid, 0,86 g (3,3 mmol) Diethyl-2-(cyclohexylamino)-vinylphosphonat und 0,94 g (3 mmol) der Verbindung aus Beispiel 122.
Ausbeute: 0,46 g (45% der Theorie)
Schmp.: 210°C

### Beispiel 124

### Methyl-(E)-7-[2-(4-fluorphenyl)-5-hydroxymethyl-4,6-diisopropyl-pyridin-3-yl]-5-hydroxy-3-oxo-hept-6-enoat

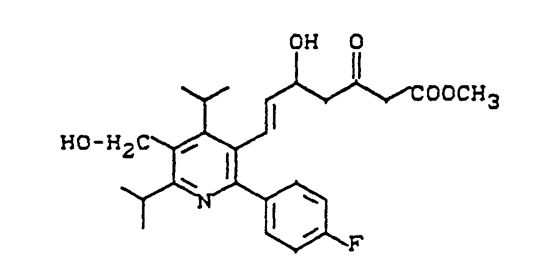

Zu einer Suspension von 0,15 g (5 mmol) 80%igem Natriumhydrid in 6,5 ml Tetrahydrofuran p.a. tropft man bei 0°C - 5°C unter Argon 0,5 ml (4,5 mmol) Acetessigsäuremethylester. Nach jeweils 15 min. werden bei 0°C zuerst innerhalb 10 min 3,65 ml (6 mmol) 15%iges Butyllithium in Hexan zugetropft, dann eine Lösung von 1,01 g (4,5 mmol) trockenem Zinkbromid in 4,5 ml Tetrahydrofuran und schließlich 0,51 g (1,5 mmol) der Verbindung aus Beispiel 123. Man rührt über Nacht bei Raumtemperatur, versetzt langsam mit gesättigter Ammoniumchlorid-Lösung, extrahiert die wäßrige Phase mit Essigester, wäscht die vereinigten organischen Phasen mit gesättigter KochsalzLösung, trocknet über Natriumsulfat und engt ein. Nach Säulenchromatographie (ø 3 cm) an 20 g Kieselgel (230-400 mesh) mit Petrolether-Essigester (1:1) erhält man 0,17 g (25% der Theorie) gelbliches Öl. R_{f} = 0,35 (Petrolether-Essigester (1:1)).

### Beispiel 125

### Methyl-erythro-(E)-7-[2-(4-fluorphenyl)-5-hydroxymethyl-4,6-diisopropyl-pyridin-3-yl]-3,5-dihydroxy-hept-6-enoat

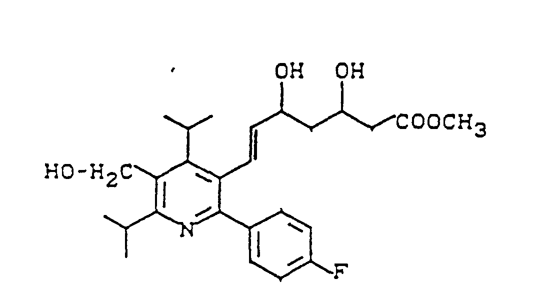

Die Darstellung erfolgt analog der Vorschrift von Beispiel 10 aus 0,15 g (0,33 mmol) der Verbindung aus
Beispiel 124.
Ausbeute: 85 mg (56% der Theorie)
¹H-NMR (CDCl₃): δ = 1,25 - 1,6 (m, 14H, CH(OH)-CH₂-CH(OH) + CH(CH₃)₂); 2,45 (m, 2H, CH₂-COOCH₃); 3,1 (b, 1H, OH); 3,45-3,7 (m, 3H, CH(CH₃)₂ + OH); 3,7 (s, 3H, OCH₃); 4,1 (m, 1H, CH(OH); 4,35 (m, 1H, CH(OH); 4,85 (s, 2H, CH₂-OH); 5,7 (dd, 1H, 6-H); 6,25 (d, 1H, 7-H); 7,05 (t, 2H, 3''-H); 7,45 (m, 2H, 2"-H) ppm.

### Beispiel 126

### 5-Ethoxycarbonyl-2-(4-fluorphenyl)-3-formyl-4,6-diisopropyl-pyridin

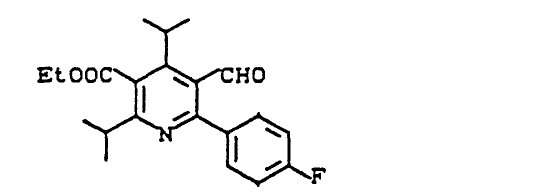

Die Darstellung erfolgt analog dem Verfahren für Beispiel 122 aus 3,5 g (10 mmol) der Verbindung aus Beispiel 121 und 35 ml 1 M Lösung von Diisobutylaluminiumhydrid in Toluol, wobei 1,5 h die Temperatur bei -75°C gehalten wird, dann auf -30°C erwärmt wird, bevor man mit Wasser versetzt. Ausbeute: 0,8 g (22% der Theorie) farblose Kristalle Schmp.: 88°C (aus Petrolether)

### Beispiel 127

### (E)-3-[5-Ethoxycarbonyl-2-(4-fluorphenyl)-4,6-diisopropyl-pyridin-3-yl]-prop-2-enal

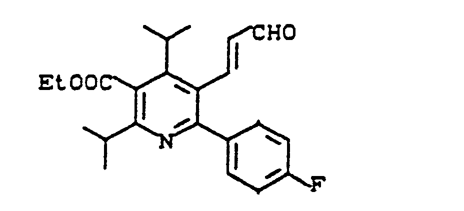

Die Darstellung erfolgt analog der Vorschrift von Beispiel 8 aus 1,25 g (3,5 mmol) der Verbindung aus Beispiel 126.
Ausbeute: 0,17 g (72% der Theorie) farbloses Öl R_{f} = 0,35 (Petrolether-Essigester (5:1)).

### Beispiel 128

### Methyl-(E)-7-[5-ethoxycarbonyl-2-(4-fluorphenyl)-4,6-diisopropyl-pyridin-3-yl]-5-hydroxy-3-oxo-hept-6-enoat

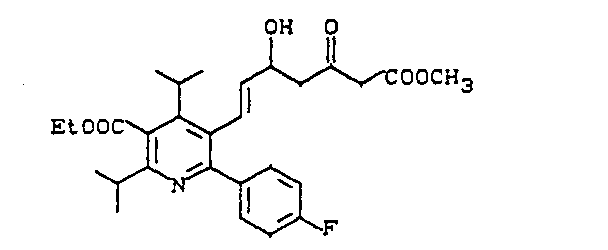

Die Darstellung erfolgt analog der Vorschrift von
Beispiel 123 aus 0,95 g (2,48 mmol) der Verbindung aus Beispiel 127.
Ausbeute: 0,83 g (67% der Theorie) farbloses Öl
R_{f} = 0,27 (Petrolether-Essigester (2:1))

### Beispiel 129 (Vergleichsbeispiel)

### Methyl-erythro-(E)-7-[5-ethoxycarbonyl-2-(4-fluorphenyl)-4,6-diisopropyl-pyridin-3-yl]-3,5-dihydroxyhept-6-enoat

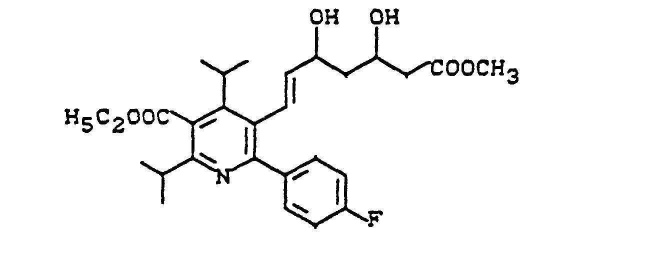

Die Darstellung erfolgt analog der Vorschrift von Beispiel 10 aus 0,89 g (1,66 mmol) der Verbindung aus
Beispiel 128.
Ausbeute: 0,65 g (78% der Theorie) farbloses Öl
¹H-NMR (CDCl₃): δ = 1,25 - 1,5 (m, 17H, CH(CH₃)₂ + CH₂-CH₃ + CH(OH)-CH₂); 2,42 (m, 2H, CH₂-COOCH₃); 2,95 (m, 1H, CH(CH₃)₂); 3,15 (b, 1H, OH); 3,2 (m 1H, CH(CH₃)₂); 3,6 (b, 1H, OH); 3,7 (s, 3H, O-CH₃); 4,1 (m, 1H, CH-OH); 4,4 (m, 3H, CH-OH + O-CH₂-CH₃); 5,2 (dd, 1H, 6-H); 6,75 (d, 1H, 7-H); 7,05 (t, 2H, 3"-H); 7,45 (m, 2H, 2''-H) ppm.

### Beispiel 130

### Methyl-erythro-(E)-7-[2-(4-fluorphenyl)-4-isopropyl-5-methoxy-methyl-6-methyl-1-oxyl-pyridin-3-yl]-3,5-dihydroxy-hept-6-enoat

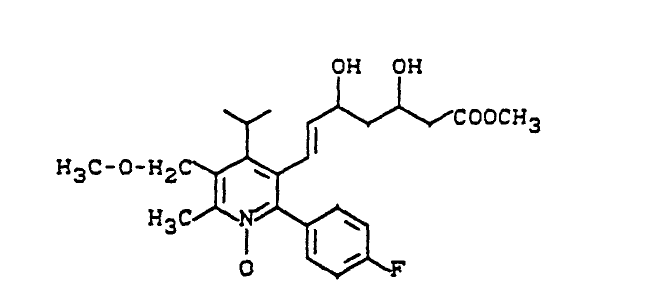

67 mg (0,15 mmol) der Verbindung aus Beispiel 116 und 54 mg (0,17 mmol) 55%ige Metachlorperbenzoesäure werden in 6 ml Dichlormethan 2 h bei Raumtemperatur gerührt. Das Lösemittel wird abgezogen, der Rückstand in 20 ml Essigester aufgenommen und mit 20 ml gesättigter Natriumhydrogencarbonatlösung gewaschen. Die wäßrige Phase wird mit 20 ml Essigester gewaschen und die vereinigten organischen Phasen über Magnesiumsulfat getrocknet. Das Rohprodukt wird an 10 g Kieselgel (230-400 mesh) in einer Säule (ø 2 cm) mit Essigester und Essigester/Methanol (10:1) chromatographiert.
Ausbeute: 57 mg (82% der Theorie) amorpher Feststoff
¹H-NMR (CDCl₃): δ = 1,1 - 1,3 (m, 2H, CH(OH)-CH₂-CH(OH)); 1,35 (d, 6H, CH(CH₃)₂); 2,38 (m, 2H, CH₂-COOCH₃); 2,6 (s, 3H, 6'-CH₃); 3,4 (b, 1H, OH); 3,5 (m, 4H, CH(CH₃)₂ + CH₂-O-CH₃); 3,28 (b, 1H, OH); 3,72 (s, 3H, COOCH₃); 4,05 (m, 1H, HO-C-H); 4,28 (m, 1H, HO-C-H); 4,55 (s, 2H, O-CH₂); 5,12 (dd, 1H, 6-H); 6,38 (d, 1H, 7-H); 7,05 - 7,25 (m, 4H, Aromaten-H) ppm.
MS: m/e = 461 (9%), M⁺.

### Beispiel 131

### 2-(4-Fluorphenyl)-3-formyl-4,6-diisopropyl-5-methoxymethyl pyridin

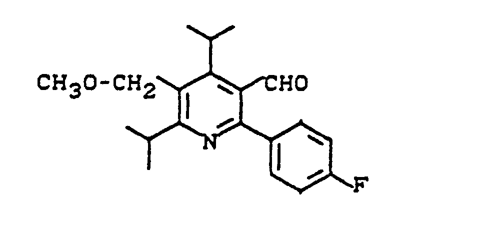

Aus 3,15 g (10 mmol) der Verbindung aus Beispiel 122 in Analogie zur Vorschrift aus Beispiel 59 erhält man 3,15 g (96 % der Theorie) farblose Kristalle vom Schmp, 77°C (aus MeOH)

### Beispiel 132

### Methyl-erythro-(E)-7-[2-(4-fluorphenyl)-4,6-diisopropyl-5-methoxymethyl-pyridin-3-yl]-3,5-dihydroxy-hept-6-enoat

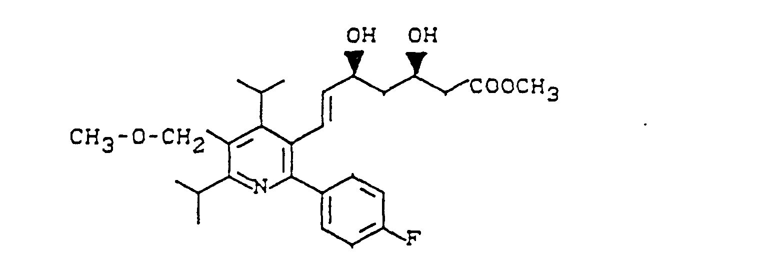

Aus der Verbindung aus Beispiel 131 erhält man gemäß den Vorschriften aus Beispiel 8, 9 und 10 farblose Kristalle vom Schmp. 92°C (aus Ether/Petrolether)
¹H-NMR (CDCl₃) : δ = 1,3 (m, 14H, CH(CH₃)₂ + 4-H); 2,4 (m, 2H, 2-H); 3,05 (b, 1H, OH); 3,3-3,57 (m, 5H, O-CH₃ + CH(CH₃)₂); 3,62 (b, 1H, OH); 3,72 (s, 3H, COOCH₃); 4,1 (m, 1H CH-OH) 4,35 (m, 1H, CH-OH); 4,55 (s, 2H, O-CH₂); 5,'5 (dd, 1H, 6-H); 6,75 (d, 1H, 7-H); 7,0 (+, 2H, 3"H); 7,45 (m, 2H, 2"-H).

### Beispiel 133

### 5-Cyano-3-ethoxycarbonyl-2-(4-fluorphenyl)-1,4-dihydro-4-isopropyl-6-methyl-pyridin

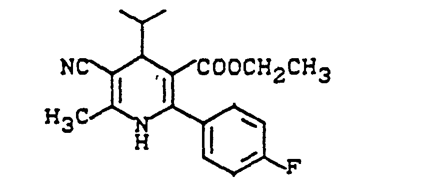

17,4 g (0,2 mol) 3-Amino-crotonsäurenitril und 56 g (0,2 mol) der Verbindung aus Beispiel 109 werden in 800 ml Ethanol über Nacht am Rückfluß gekocht. Das Lösungsmittel wird abgezogen und der Rückstand an Kieselgel mit Petrolether/Essigester 20:1 chromatographiert.
Ausbeute: 14,9 g (21 % der Theorie)
¹H-NMR ( COCl₃): δ = 1,0 (m, 9H); 1,9 (m, 1H); 2,2 (m, 3H); 3,6 (d, 1H); 3,9 (m, 2H); 5,75 (b, 1H); 7,1 (t, 2H); 7,25 (m, 2H).

### Beispiel 134

### 5-Cyano-3-ethoxycarbonyl-2-(4-fluorphenyl)-4-isopropyl-6-methyl-pyridin

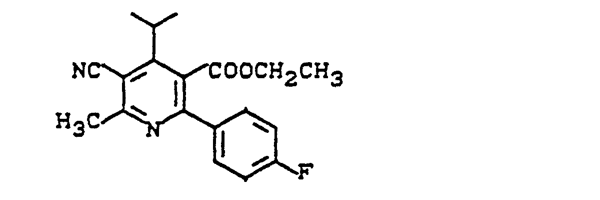

Analog Beispiel 3 aus 9,8 g (30 mmol) der Verbindung aus Beispiel 133
Ausbeute: 9,4 g (96 % der Theorie), Schmp. 76°C.

### Beispiel 135

### 5-Cyano-2-(4-fluorphenyl)-3-hydroxymethyl-4-isopropyl-5-methyl-pyridin

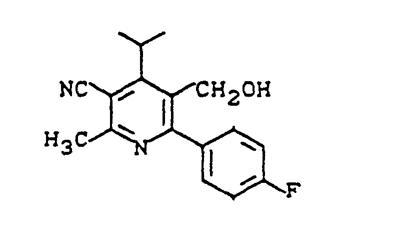

Unter Argon gibt man zu einer Lösung von 9,8 g (30 mmol) der Verbindung aus Beispiel 134 in 150 ml Toluol p.a. bei -75°C innerhalb von 1,5 h 50 ml (60 mmol) 1,2 M Diisobutylaluminiumhydrid-Lösung in Toluol zu und rührt weitere 30 min.

Bei -30°C werden vorsichtig 280 ml Wasser zugetropft, mit Kieselgur abgesaugt und mit Essigester nachgewaschen. Die wäßrige Phase wird dreimal mit Essigester extrahiert, die vereinigten organischen Phasen mit ges. Kochsalzlösung gewaschen, über Natriumsulfat getrocknet und eingeengt. Säulenchromatographie (400 g Kieselgel, 230-400 mesh, ø 6 cm, Petrolether/Essigester 10/1 → 5/1 ) ergibt 3 Zonen:
1. 3-Ethoxycarbonyl-2-(4-fluorphenyl)-5-formyl-4-isopropyl-6-methyl-pyridin 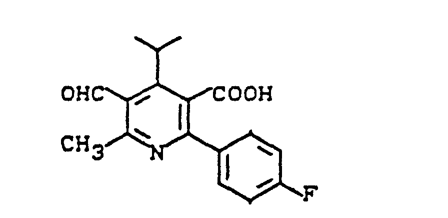 2,6 g (26 % der Theorie) vom Schmp. 53°C
2. 2,1 g (25 % der Theorie) der Titelverbindung (Beispiel 135) Schmp. 157°C aus Ether/Petrolether.
3. 2-(4-Fluorphenyl)-5-formyl-3-hydroxymethyl)-4-isopropyl-6-methyl-pyridin 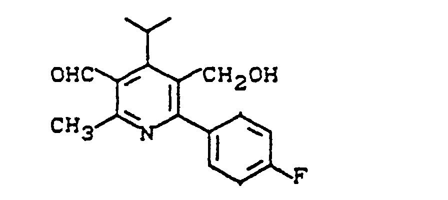 1,6 g (19 % der Theorie), Schmp. 150°C aus Ether/Petrolether.

### Beispiel 136

### 5-Cyano-2-(4-fluorphenyl)-3-formyl-4-isopropyl-6-methylpyridin.

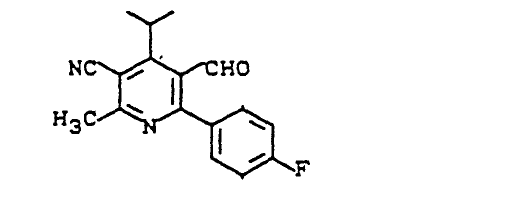

Zu 1,1 g (14 mmol) DMSO in 8 ml Methylenchlorid tropft man bei -75°C 2,2 g (10,5 mmol) Trifluoressigsäureanhydrid in 10 ml Methylenchlorid, rührt 10 min bei -70°C, tropft dann eine Lösung von 2,0 g (7 mmol) der Verbindung aus Beispiel 135 in 50 ml Methylenchlorid zu und rührt 1 h bei -70°C.

Zu der jetzt vorliegenden Suspension werden 2,1 ml (21 mmol) Triethylamin getropft und 10 min bei -65°C gerührt. Nach Erwärmen auf Raumtemperatur wird mit ges. Kochsalzlösung gewaschen, über Natriummsulfat getrocknet und eingeengt.
Rohausbeute: 2,0 g (100 % der Theorie), Schmp. 109°C
¹H-NMR (CDCl₃): δ = 1,52 (d, 6H, CH(CH₃)₂); 2,9 (s,, 3H, 6-CH₃); 4,0 (sept. 1H, CH(CH₃)₂); 7,2 (m, 2H, 3'-H); 7,5 (m, 2H, 2'H); 9,95 (s, 1H, CHO).

### Beispiel 137

### (E)-3-[5-Cyano-2-(4-fluorphenyl)-4-isopropyl-6-methylpyrid-3-yl]-prop-2-enal

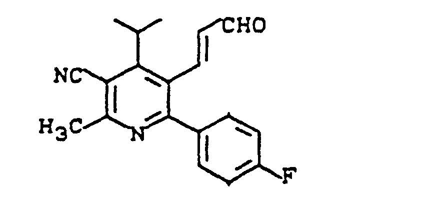

Analog Beispiel 8 aus 1,9 g der Verbindung aus Beispiel 136 Ausbeute: 0,6 g (28 % der Theorie), Schmp. 112°C (Ether-Petrolether).

### Beispiel 138

### Methyl-(E)-7-[5-cyano-2-(4-fluorphenyl)-4-isopropyl-6-methyl-pyrid-3-yl]-5-hydroxy-3-oxo-hept-6-enoat

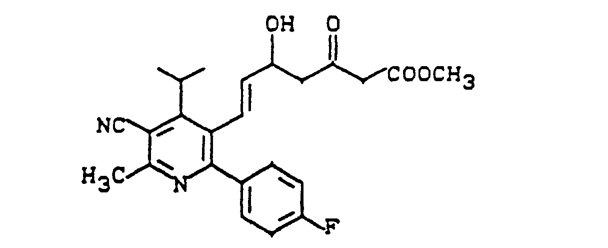

Analog Beispiel 9 aus 0,52 g (1,7 mmol) der Verbindung aus Beispiel 137 Ausbeute: 0,32 g (44 % der Theorie) gelbliches Öl.

### Beispiel 139 (Vergleichsbeispiel)

### Methyl-erythro-(E)-7-[5-Cyano-2-(4-fluorphenyl)-4-isopropyl-6-methyl-pyrid-3-yl]-3,5-dihydroxy-hept-6-enoat

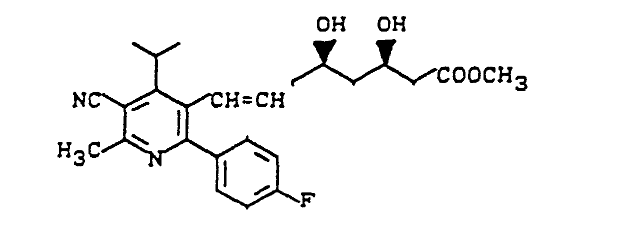

Analog Beispiel 10 aus 0,32 g der Verbindung aus Beispiel 138
Ausbeute: 0,15 g (47 % der Theorie) gelbliches Öl.
¹H-NMR (CDCl₃): δ = 1,35 (m, 2H, CH(OH)CH₂-CH(OH); 1,45 (d, 6H, CH(CH₃)₂); 2,42 (m, 2H, CH₂-COOCH₃); 2,7 (s, 3H, 6-CH₃); 3,55 (m, 2H, CH(CH₃)₂ + OH); 3,68 (b, 1H, OH); 3,72 (s, 3H, O-CH₃); 4,13 (m, 1H, CH(OH)), 4,4 (m, 1H, CH(OH)); 5,32, (dd, 1H, Olefin-H); 6,6 (d, 1H, Olefin-H); 708 (m, 2H, 3"-H); 7,45 (m, 2H, 2''-H).

### Beispiel 140

### 4-Fluorbenzoylessigsäureethylester

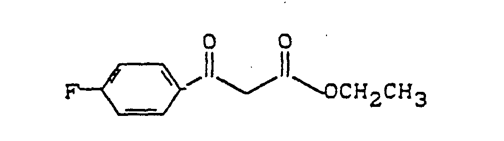

In einen Liter Diethylether p.a. werden 21,7 g (0,72 mol) Natriumhydrid (80 %ig, 20 % Mineralöl) eingewogen und anschließend 85,5 g (127 ml, 0,72 mol) Kohlensäurediethylester (VK 22-010) zugegeben. In dieser Lösung wird in der Siedehitze über einen Zeitraum von 4 Stunden eine Lösung von 100 g (0,72 mol) 4-Fluoracetophenon in 300 ml Diethylether zugetropft (kräftiger, mechanischer Rührer erforderlich; es bildet sich ein zäher Brei). Danach wird eine weitere Stunde am Rückfluß gekocht, dann auf ca. 5°C abgekühlt und bei dieser Temperatur unter N₂ zunächst eine Lösung aus 50 ml Essigsäure und 100 ml Et₂O zugetropft. Anschließend wird etwa 500 ml H₂O zugetropft und die organische Phase abgetrennt. Die Wasserphase wird nochmals mit Et₂O extrahiert (2 x 400 ml), die vereinigten etherischen Phasen mit NaHCO₃-Lösung gewaschen, über MgSO₄ getrocknet und eingeengt. Der Rückstand wird über eine kurze Vigreux-Kolonne destilliert. Ausbeute: 93g (60 %) Kp 0,4 mm (53, 33 Pascals) 99-102°C

### Beispiel 141

### 5-Cyano-3-ethoxycarbonyl-4-(4-fluorphenyl)-1,4-dihydro-2-isopropyl-6-methyl-pyridin

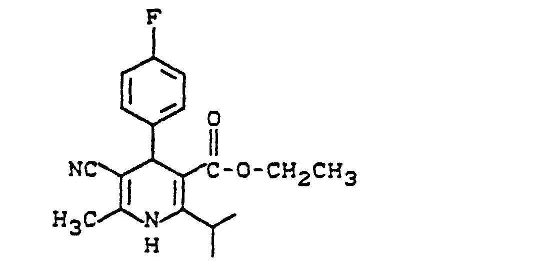

52,8 g (0,2 mol) der Verbindung aus Beispiel 1 und 16,4 g (0,2 mol) 3-Amino-crotonsäurenitril werden in 200 ml Ethylenglykol 2 h am Rückfluß gekocht. Nach dem Abkühlen hebt man das abgeschiedene Öl ab, und extrahiert noch dreimal mit Ether. Die Etherphasen werden mit Wasser gewaschen, getrocknet und eingeengt. Der Rückstand und das zuerst isolierte Öl werden aus Ether kristallisiert. Ausbeute: 31,4 g (48 %), Schmp. 168°C.

### Beispiel 142

### 5-Cyano-3-ethoxycarbonyl-4-(4-fluorphenyl)-2-isopropyl-6-methyl-pyridin

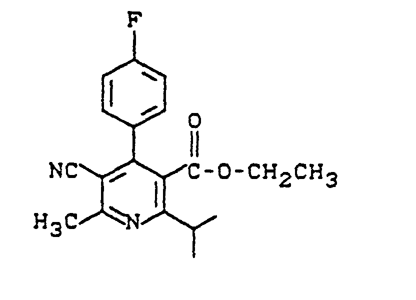

Analog Beispiel 3 werden 14,8 g (45 mmol) der Verbindung aus Beispiel 141 umgesetzt.
Ausbeute: 13,7 g (93 % der Theorie) farbl. Kristalle vom Schmp. 95°C.

### Beispiel 143

### 5-Cyano-4-(4-fluorphenyl)-3-hydroxymethyl-2-isopropyl-6-methyl-pyridin

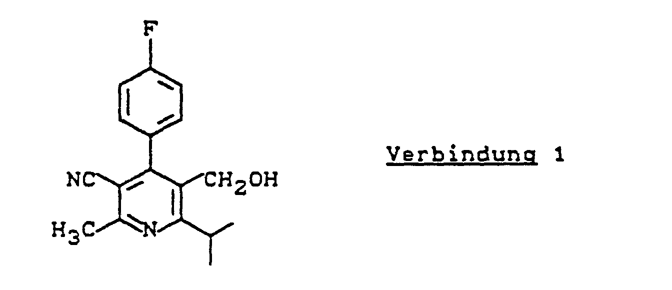

### 5-Cyano-4-(4-fluorphenyl)-5-hydroxyiminomethyl-3-hydroxymethyl-2-isopropyl-6-methyl-pyridin

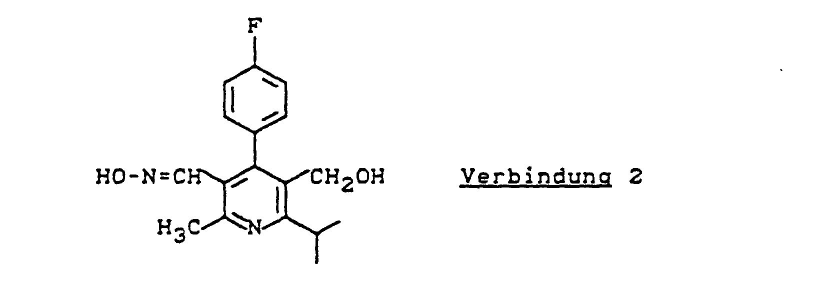

16,3 g (50 mmol) der Verbindung aus Beispiel 142 in 240 ml Toluol p.A. tropft man bei -78°C unter Argon innerhalb von 3 h 83,3 ml (0,1 mol) 1,2 M Diisobutylaluminiumhydrid Lösung in Toluol, daß die Temperatur unter -75°C bleibt.

Man rührt noch 30 min bei -75°C, läßt auf -30°C erwärmen und gibt dann vorsichtig 300 ml Wasser und 160 ml Essigester zu.

Über Kieselgur wird abgesaugt und mit Essigester nachgewaschen. Die wäßrige Phase wird dreimal mit Essigester extrahiert, die vereinigten org. Phasen mit ges. Kochsalzlösung gewaschen, über Natriumsulfat getrocknet und eingeengt. Zweimalige Säulenchromatographie über Kieselgel mit Toluol/Essigester 5:1 bzw. Petrolether/Essigester 5:1 ergibt 3,4 g einer Fraktion von R_{f}-Wert 0,2 (Petrolether/Essigester 5:1), die ein Gemisch aus 5-Cyano-4-(4-fluorphenyl)-3-hydroxymethyl-2-isopropyl-6-methyl-pyridin und 4-(4-Fluorphenyl)-5-formyl-3-hydroxy-2-isopropyl-6-methyl-pyridin darstellt.

3,16 g dieses Gemisches werden in 10 ml Methanol gelöst und zu einer Lösung von 1,22 g (17,6 mol) Hydroxylaminhydrochlorid und 1,22 g (14,9 mmol) Natriumacetat in 10 ml Wasser gegeben. Nach 3 h Rühren bei Raumtemperatur wird das Methanol abrotiert, etwas Wasser zugegeben und dreimal mit Essigester extrahiert. Die org. Phasen werden mit ges. Kochsalzlösung gewaschen, unter Natriumsulfat getrocknet und eingeengt. Den Rückstand chromatographiert man in einer Säule (100 g Kieselgel 230-400 mesh, ø 4 cm, Petrolether/Essigester 5:1). Man erhält zwei Fraktionen:
- Verbindung 1:: 0,65 g (4,9 %) farbl. Kristalle vom Schmp. 132°C
5-Cyano-4-(4-fluorphenyl)-3-hydroxymethyl-2-isopropyl-6-methyl-pyridin
¹H-NMR (CDCl₃): δ = 1,32 (d, 6H,, CH(CH₃)₂); 1,6 (b, 1H, OH); 2,7 (s, 3H, 6-CH₃); 3,5 (sept, 1H, CH(CH₃)₂); 4,5 (d, 2H, CH₂-OH); 7,15-7,35 (m, 4H, Aromaten-H).
- Verbindung 2:: 2,15 g (15,3 %) amorpher Feststoff 5-Cyano-4-(4-fluorphenyl)-5-hydroxyiminomethyl-3-hydroxymethyl-2-isopropyl-6-methyl-pyridin
¹H-NMR (CFCl₃): δ = 1,33 (d, 6H, CH(CH₃)₂); 1,4 (b, 1H, -OH); 2,7 (s, 3H,, 6-CH₃); 3,48 (m, 1H, CH(CH₃)₂); 4,4 (d, 2H, CH₂-OH); 7,15 (m, 4H, Aromaten-H); 7,77 (s, 1H, CH=N-); 8,08 (s, 1H, =N-OH).

### Beispiel 144

### 5-Cyano-4-(4-fluorphenyl)-3-formyl-2-isopropyl-6-methylpyridin

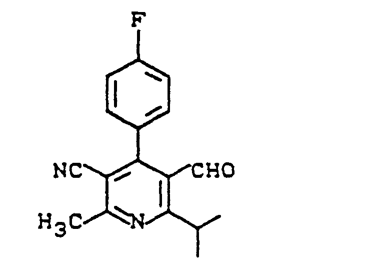

A) 0,63 g (2,2 mmol) der Verbindung 1 aus Beispiel 144 wird analog dem Verfahren aus Beispiel 136 umgesetzt. Ausbeute: 0,6 g (97 %).
B) Zu 1,1 g (14 mmol) DMSO in 8 ml Methylenchlorid p.A. tropft man bei -78°C 4,4 g (21 mmol) Trifluoressigsäureanhydrid in 15 ml Methylenchlorid und rührt 10 min bei -70°C. Dann gibt man 2,1 g (7 mmol) der Verbindung 2 aus Beispiel 143 in 50 ml Methylenchlorid zu, rührt 1 h bei -70°C, gibt jetzt 5,8 ml (42 mmol) Triethylamin zu und rührt 4 h bei Raumtemperatur. Es wird mit ges. Kochsalzlösung gewaschen, eingeengt, über 100 g Kieselgel (230-400 mesh, ø 4 cm, Petrolether/Essigester 10:1) chromatographiert und aus Essigester/Petrolether umkristallisiert. Ausbeute: 0,31 g (16 %), Schmp. 82°C
   ¹H-NMR (CDCl₃): δ= 1,32 (d, 6H, CH(CH₃)₂); 2,78 (s, 3H, 6-CH₃); 3,77 (m, 1H, CH(CH₃)₂); 7,23 (m, 2H, 3'-H); 7,36 (m, 2H, 2'-H); 9,86 (s, 1H, CHO).

### Beispiel 145

### Methyl-erythro-(E)-7-[5-cyano-4-(4-fluorphenyl)-2-isopropyl-6-methyl-pyrid-3-yl]-3,5-dihydroxy-hept-6-enoat

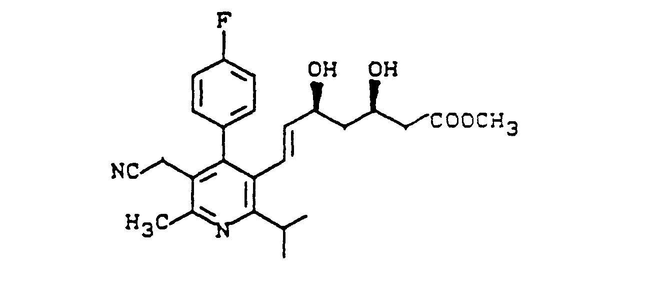

Die Umsetzung erfolgt aus der Verbindung aus Beispiel 144 in Analogie zu den Reaktionen aus Beispiel 8, 9 und 10. Farbloser Feststoff, Schmp. 124°C.
¹H-NMR (CDCl₃): δ = 1,3 (m, 8H, CH(CH₃)₂ + CH(OH)-CH₂-CH(OH)); 2,4 (m, 2H, CH₂-COOCH₃); 2,75 (s, 3H, 6-CH₃); 3,35 (m, 2H, CH(CH₃) + OH); 3,6 (b, 1H, OH); 3,7 (s, 3H O-CH₃); 4,1 (m, 1H, CH-OH); 4,35 (m, 1H, CH-OH); 5,3 (dd, 1H, 6-H); 6,4 (d, 1H, 7-H); 7,05-7,3 (m, 4H, Ar-H).

### Beispiel 146

### 5-(tert.-Butyldimethylsilyloxymethyl)-6-cyclopropyl-4-(4-fluorphenyl)-2-isopropyl-pyridin-3-carbonsäureethylester

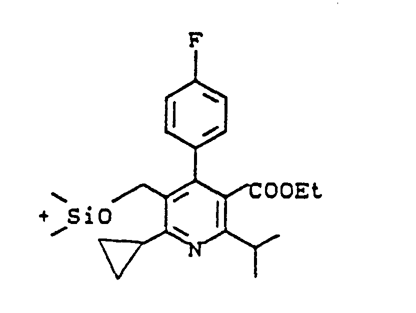

15 g (42 mmol) der Verbindung aus Beispiel 82 werden analog Beispiel 5 umgesetzt. Ausbeute: 16,9 g (85,4 % der Theorie)

### Beispiel 147

### 3-(tert.-Butyldimethylsilyloxymethyl)-2-cyclopropyl-4-(4-fluorphenyl)-5-hydroxymethyl-6-isopropyl-pyridin

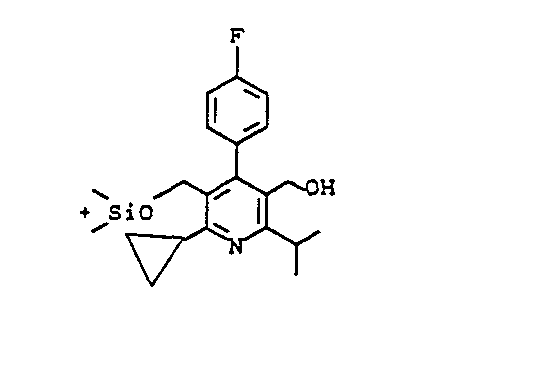

16,9 g (35,8) der Verbindung aus Beispiel 146 werden analog Beispiel 6 umgesetzt. Ausbeute: 12,3 g (80,1 % der Theorie).

### Beispiel 148

### 3-(tert.-Butyldimethylsilyloxymethyl)-2-cyclopropyl-4- (4-fluorphenyl)-6-isopropyl-5-methoxymethyl-pyridin

5,5 g (12,8 mmol) der Verbindung aus Beispiel 147 werden analog Beispiel 59 umgesetzt. Ausbeute: 5,7 g Rohprodukt.

### Beispiel 149

### 2-Cyclopropyl-4-(4-fluorphenyl)-3-hydroxymethyl-6-isopropyl-5-methoxymethyl-pyridin

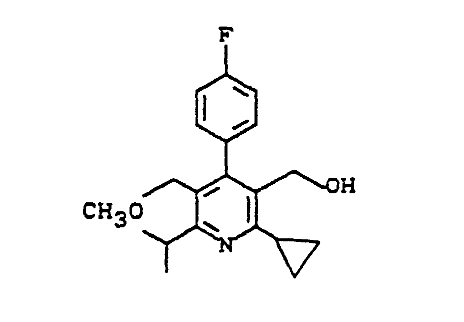

5,7 g (12,8 mmol) des Rohproduktes aus Beispiel 148 werden in absol. Tetrahydrofuran gelöst. Nach Zugabe von 12,8 ml Tetrabutylammoniumfluorid-Lösung (1 M in THF) wird über Nacht bei Raumtemperatur gerührt. Dann werden 50 ml gesättigte Natriumhydrogencarbonatlösung zugegeben und es wird mehrmals mit Methylenchlorid extrahiert. Die vereinigten organischen Phasen werden mit Natriumsulfat getrocknet, eingeengt und über Kieselgel chromatographiert (Laufmittel:Petrolether/Essigester 7:3). Ausbeute: 3,9 g (94 % der Theorie).
¹H-NMR (CDCl₃): δ= 0,0 (s, 6H); 0,87 (s, 9H); 0,8-1,0 (m, 2H); 1,22 (d, 6H); 1,10-1,30 (m, 2H); 2,31 (m, 1H); 3,12 (s, 3H); 3,30 (m, 1H); 3,98 (s, 2H); 4,47 (m, 2H); 7,0-7,3 (m, 4H) ppm.

### Beispiel 150

### Methyl-erythro-(E)-7-[2-cyclopropyl-4-(4-fluorphenyl)-6-isopropyl-5-methoxymethyl-pyrid-3-yl]-3,5-dihydroxyhept-6-enoat

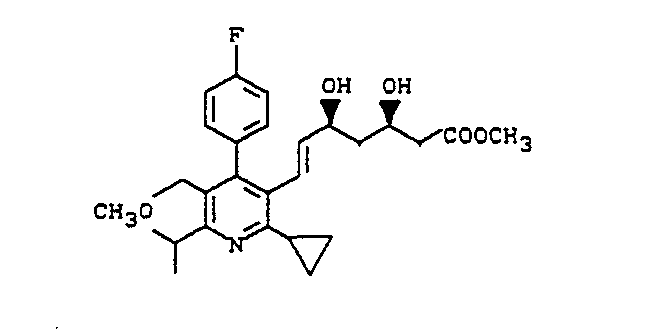

Aus der Verbindung aus Beispiel 149 wurde, in Analogie zu den Reaktionen aus den Beispielen 7, 8, 9, 10, das Beispiel 150 hergestellt.
¹H-NMR (CDCl₃): δ = 0,89 (m, 2H); 1,18 (m, 2H); 1,26 (d, 6H); 1,40 (m, 2H); 2,24 (m, 1H); 2,44 (m, 2H); 3,17 (s, 3H); 3,30 (m, 1H); 3,72 (s, 3H); 4,03 (s, 2H); 4,12 (m, 1H); 4,32 (m, 1H); 5,51 (d,d, 1H); 6,32 (d, 1H); 7,10 (m, 4H) ppm.

### Beispiel 151

### 3-Amino-3-cyclopropyl-acrylsäureethylester

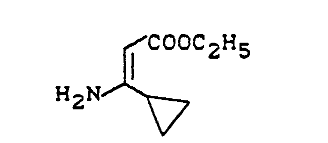

49,9 g (0,32 mol) Cyclopropylcarbonylessigsäureethylester in 200 ml trockenem Toluol werden mit 1,1 g p-Toluolsulfonsäure versetzt und bei Raumtemperatur unter Rühren mit Ammoniak-Gas gesättigt. Nach Stehenlassen über Nacht wird 8 h am Wasserabscheider unter Rückfluß gekocht, wobei kontinuierlich Ammoniak-Gas eingeleitet wird. Man läßt über Nacht abkühlen, filtriert, engt die Toluollösung im Vakuum ein und destilliert im Hochvakuum bis 65°C vom nicht umgesetzten Ausgangsmaterial ab. Die Substanz befindet sich anschließend im Rückstand. Ausbeute: 11,9 g (24 % der Theorie).

### Beispiel 152

### 1,4-Dihydro-2,6-dicyclopropyl-4-(4-fluorphenyl)-pyridin-3,5-dicarbonsäure-diethylester

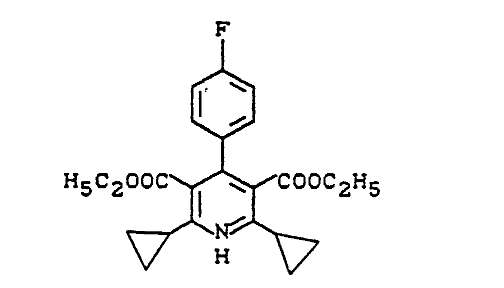

6,2 g (40 mmol) der Verbindung aus Beispiel 43 und 10,5 g (40 mmol) der Verbindung aus Beispiel 40 werden in 100 ml Ethylenglykol gelöst und über Nacht am Rückfluß gekocht. Nach Abkühlen auf Raumtemperatur wird die Mischung mehrmals mit Ether extrahiert, die organische Phase je einmal mit 10 %iger Salzsäure, gesättigter Natriumbicarbonat-Lösung und Wasser gewaschen, über Magnesiumsulfat getrocknet und im Vakuum eingeengt. Ausbeute: 10,4 g (65,1 % der Theorie).
¹H-NMR (CDCl₃): δ = 0,60 (m, 4H); 0,95 (m, 4H); 1,23 (t, 6H); 2,72 (m, 2H); 4,12 (m, 4H); 5,02 (s, 1H); 5,40 (s, 1H); 6,88 (m, 2H); 7,20 (m, 2H) ppm.

### Beispiel 153

### Methyl-erythro-(E)-7-[2,6-dicyclopropyl-4-(4-fluorphenyl)-5-methoxymethyl-pyrid-3-yl]-3,5-dihydroxy-hept-6-enoat

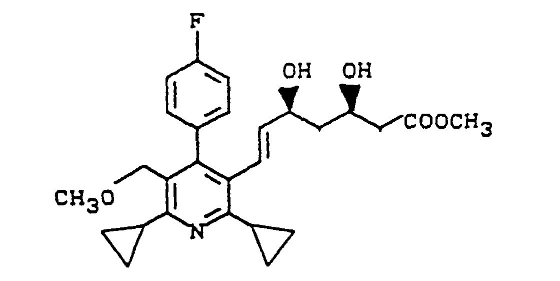

Aus der Verbindung aus Beispiel 150 wurde, in Analogie zu den Reaktionen aus den Beispielen 3, 4, 59, 60, 7, 8, 9, 10, das Beispiel 153 hergestellt.
¹H-NMR (CDCl₃): δ = 0,89 (m, 4H); 1,08 (m, 4H); 1,40 (m, 2H); 2,21 (m, 2H); 2,43 (m, 2H); 3,21 (s, 3H); 3,72 (s, 3H); 4,11 (m, 1H); 4,15 (s, 2H); 4,30 (m, 1H); 5,47 (dd, 1H); 6,30 (d, 1H); 7,10 (m, 4H) ppm.

### Beispiel 154

### 2,6-Diisopropyl-5-ethoxymethyl-4-(4-fluorphenyl)-pyridin-3-carbonsäureethylester

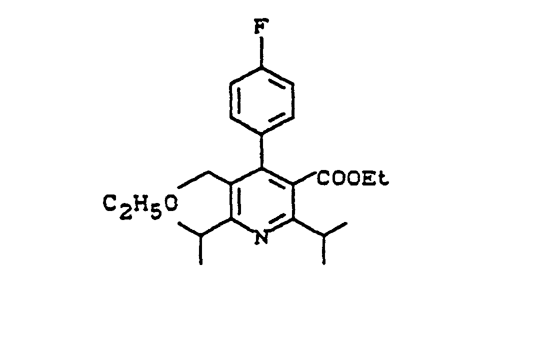

Zu einer Suspension von 2,29 g (76 mmol) Natriumhydrid (80 %ig) in 30 ml absol. Tetrahydrofuran tropft man bei Raumtemperatur 4,4 ml (76 mmol) absol. Ethanol und rührt 30 min. Dann tropft man eine Lösung von 2,7 g (7,6 mmol) der Verbindung aus Beispiel 86 in 20 ml absol. Tetrahydrofuran zu und erhitzt über Nacht unter Rückfluß. Anschließend wird der Reaktionsansatz auf Eiswasser gegossen und, nachdem der pH auf 8 eingestellt wurde, mehrmals mit Ether extrahiert. Die organische Phase wird mit Kochsalzlösung gewaschen, über Natriumsulfat getrocknet, eingeengt und über Kieselgel chromatographiert (Laufmittel Essigester/ Petrolether 5:95).
Ausbeute: 1,07 g (36,4 % der Theorie).
¹H-NMR (CDCl₃): δ = 0,95 (t, 3H); 1,13 (t, 3H); 1,31 (m, 6H); 3,05 (m, 1H); 3,32 (q, 2H); 3,41 (m, 1H); 3,97 (q, 2H); 4,18 (d, 2H); 7,08 (m, 2H); 7,27 m, 2H) ppm.

### Beispiel 155

### Methyl-erythro-(E)-7-[2,6-diisopropyl-5-ethoxymethyl-4-(4-fluorphenyl)-pyrid-3-yl]-3,5-dihydroxy-hept-6-enoat

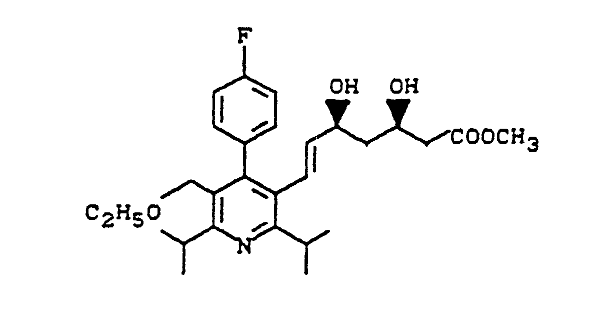

Aus der Verbindung aus Beispiel 154 wurde, in Analogie zu den Reaktionen aus den Beispielen 60, 7, 8, 9, 10, das Beispiel 155 erhalten.
¹H-NMR (CDCl₃): δ= 1,13 (t, 3H); 1,20-1,50 (m, 8H); 2,43 (m, 2H); 3,30 (m, 3H); 3,72 (s, 3H); 4,08 (m, 1H); 4,12 (s, 2H); 4,29 (m, 1H); 5,25 (dd, 1H); 6,30 (d, 1H); 7,0-7,2 (m, 4H) ppm.

Analog Beispiel 155 wurden aus Beispiel 86 folgende Verbindungen erhalten:

### Beispiel 156

Methyl-erythro-(E)-(7-[2,6-diisopropyl-4-(4-fluorphenyl)-5-propyloxymethyl-pyrid-3-yl]-3,5-dihydroxy-hept-6-enoat.

### Beispiel 157

Methyl-erythro-(E)-(7-[2,6-diisopropyl-4-(4-fluorphenyl)-5-isopropoxymethyl-pyrid-3-yl]-3,5-dihydroxy-hept-6-enoat.

### Beispiel 158

Methyl-erythro-(E)-(7-[2,6-diisopropyl-4-(4-fluorphenyl)-5-butyloxymethyl-pyrid-3-yl]-3,5-dihydroxy-hept-6-enoat.

### Beispiel 159

Methyl-erythro-(E)-(7-[2,6-diisopropyl-4-(4-fluorphenyl)-5-pentyloxymethyl-pyrid-3-yl]-3,5-dihydroxyhept-6-enoat.

### Beispiel 160

Methyl-erythro-(E)-(7-[2,6-diisopropyl-4-(4-fluorphenyl)-5-hexyloxymethyl-pyrid-3-yl]-3,5-dihydroxy-hept-6-enoat.

### Beispiel 161

### Natrium-erythro-(E)-7-[2,6-diisopropyl-5-ethoxymethyl-4-(4-fluorphenyl)-pyrid-3-yl]-3,5-dihydroxy-hept-6-enoat

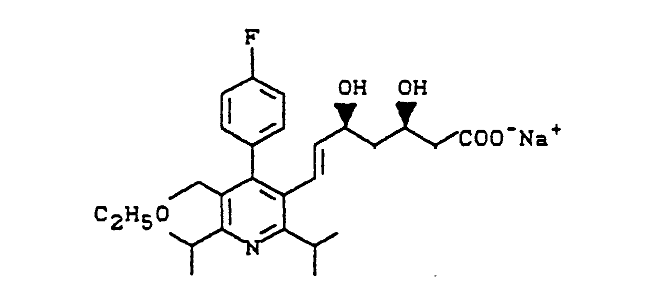

487 mg (1 mmol) der Verbindung aus Beispiel 155 werden in 10 ml Tetrahydrofuran gelöst und mit 10 ml 0,1 N Natronlauge versetzt. Nach 1 h wird das Tetrahydrofuran am Vakuum abgezogen und der wäßrige Rückstand wird gefriergetrocknet.
Ausbeute: 490 mg (99 % der Theorie).

### Beispiel 162

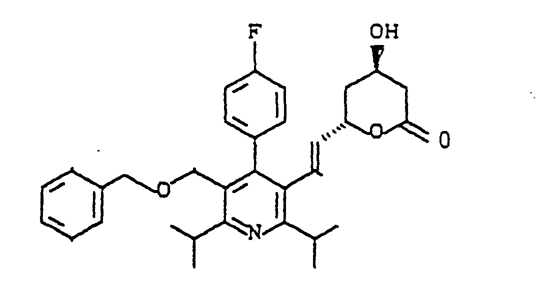

### trans-(E)-6-[2-(3-benzyloxymethyl-2,6-diisopropyl-4-(4-fluorphenyl)-pyrid-5-yl)-ethenyl]-3,4,5,6-tetrahydro-4-hydroxy-2H-pyran-2-on

5,5 g (10 mmol) der Verbindung aus Beispiel 17 werden in 100 ml Tetrahydrofuran gelöst und nach Zugabe von 100 ml 0,1 N Natronlauge wird 1 h bei Raumtemperatur gerührt. Anschließend wird mit 100 ml Wasser verdünnt, mit 1 N HCl auf pH 4,4 gestellt und mit Methylenchlorid extrahiert. Die Methylenchloridphase wird mit Natriumsulfat getrocknet und im Vakuum eingeengt. Der Rückstand wird in 100 ml absol. Toluol gelöst, mit 40 g Molekularsieb 4 Å versetzt und über Nacht unter Rückfluß erhitzt. Anschließend wird vom Molekularsieb abfiltriert, im Vakuum eingeengt und der Rückstand wird mit Petrolether kristallisiert.
Ausbeute: 4,3 g (83,2 % der Theorie)
¹H-NMR (CDCl₃): δ = 1,22 (d,6H); 1,32 (d,6H; 1,40-1,80 (m,2H); 2,45-2,70 (m,2H); 3,30 (m,2H); 4,12 (m,1H); 4,14 (s,2H); 4,45 (s,2H); 5,04 (m,1H); 5,28 (dd,1H); 6,39 (d,1H); 6,95-7,40 (m,9H)ppm.

### Beispiel 163

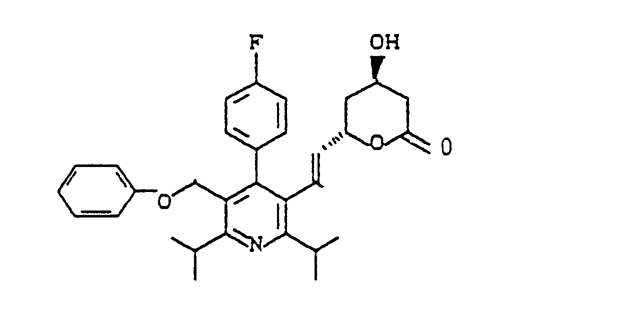

### trans-(E)-6-[2-(2,6-diisopropyl-4-(4-fluorphenyl)-5-phenoxymethyl-pyrid-3-yl)-ethenyl]-3,4,5,6-tetrahydro-4-hydroxy-2H-pyran-2-one

Analog Beispiel 162 erhält man aus 540 mg (1 mmol) der Verbindung aus Beispiel 89 450 mg (89,4 % der Theorie).
¹H-NMR (CDCl₃): δ = 1,25 (d,6H); 1,30 (d,6H); 1,40-1,70 (m,2H); 2,60 (m,2H); 3,30 (m,2H); 4,18 (m,1H); 4,65 (s,2H); 5,08 (m,1H); 5,30 (dd,1H); 6,42 (d,1H); 6,70-7,30 (m,9H)ppm.

### Beispiel 164

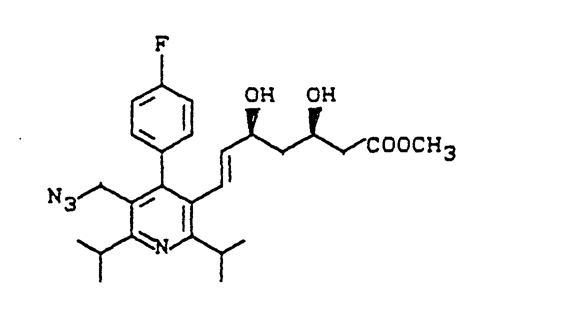

### Methyl-erythro-(E)-7-[3-azidomethyl-2,6-diisopropyl-4-(4-fluorphenyl)-pyrid-5-yl)-3,5-dihydroxy-hept-6-enoat

459 mg (1 mmol) der Verbindung aus Beispiel 11 und 320 mg (1,1 mmol) Triphenylphosphin werden in 10 ml absol. Tetrahydrofuran gelöst. Nach Zugabe von 3,2 ml einer 0,48 molaren Lösung von HN₃ in Toluol wird mit einem Eisbad gekühlt und 173 ml (1,1 mmol) Azodicarbonsäurediethylester werden zugegeben. Anschließend wird über Nacht bei Raumtemperatur gerührt und dann im Vakuum eingeengt. Der Rückstand wird über Kieselgel chromatographiert (Laufmittel: Essigester/Petrolether 1:1).
- Ausbeute:: 260 mg (53,7 % der Theorie)
¹H-NMR (CDCl₃): δ = 1,30 (m,6H); 1,39 (d,6H);1,25-1,60 (m,2H); 2,50 (m,2H); 3,37 (m,2H); 3,80 (s,3H); 4,15 (m,1H); 4,18 (s,2H); 4,36 (m,1H); 5,36 (dd,1H); 6,36 (d,1H); 7,15 (m,4H) ppm.

### Beispiel 165

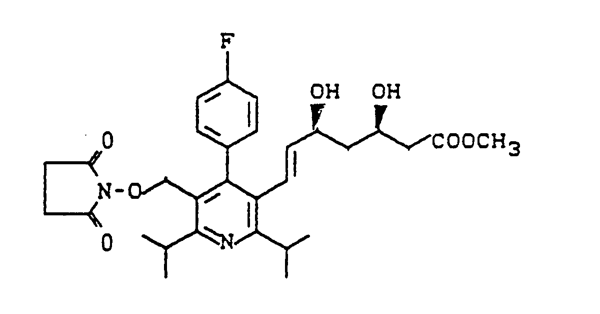

### Methyl-erythro-(E)-7-[2,6-diisopropyl-4-(4-fluorphenyl)-5-succinimidooxymethyl-pyrid-3-yl)-3,5-dihydroxy-hept-6-enoat

Die Herstellung erfolgt analog Beispiel 164 aus der Verbindung aus Beispiel 11 und N-Hydroxysuccinimid.
¹H-NMR (CDCl₃): δ = 1,28 (m,6H); 1,37 (d,6H);1,2-1,5 (m,2H); 2,43 (m,2H); 2,66 (s,4H); 3,32 (sept.,1H); 3,72 (s,3H); 3,78 (sept.,1H); 4,08 (m,1H); 4,31 (m,1H); 4,86 (s,2H); 5,28 (dd,1H); 6,33 (d,1H); 7,0-7,4 (m,4H) ppm.

### Beispiel 166

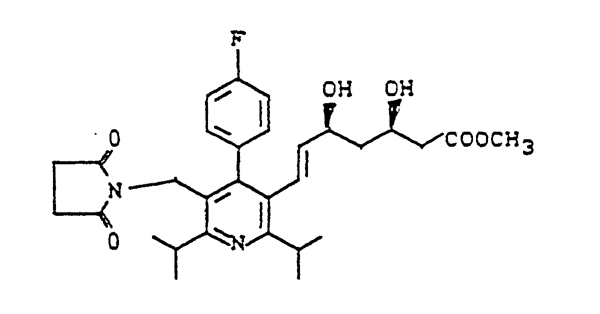

### Methyl-erythro-(E)-7-[2,6-diisopropyl-4-(4-fluorphenyl)-5-succinimidomethyl-pyrid-3-yl)-3,5-dihydroxy-hept-6-enoat

Die Herstellung erfolgt analog Beispiel 164 aus der Verbindung aus Beispiel 11 und Succinimid.
¹H-NMR (CDCl₃): δ = 1,23 (m,12H); 1,25-1,50 (m,2H); 2,43 (m,2H); 2,51 (s,4H); 3,16 (m,1H); 3,28 (m,1H); 3,73 (s,3H); 4,07 (m,1H); 4,26 (m,1H); 4,52 (s,2H); 5,25 (dd,1H); 6,20 (d,1H); 7,0-7,2 (m,4H) ppm.

### Beispiel 167

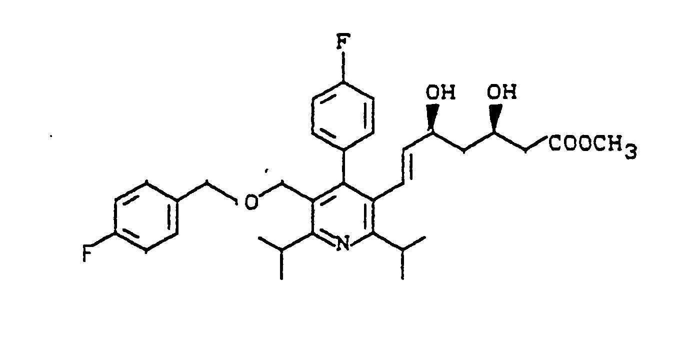

### Methyl-erythro-(E)-7-[2,6-diisopropyl-3-(4-fluorbenzyloxymethyl)-4-(4-fluorphenyl)-pyrid-5-yl)-3,5-dihydroxyhept-6-enoat

Aus der Verbindung des Beispiels 4 und 4-Fluorbenzylbromid wird analog der Beispiele 12-17 das Beispiel 166 erhalten.
¹H-NMR (CDCl₃): δ = 1,25 (m,6H); 1,32 (d,6H); 1,2-1,5 (m,2H); 2,42 (m,2H); 3,30 (m,2H); 3,72 (s,3H); 4,07 (m,1H); 4,13 (s,2H); 4,28 (m,1H); 4,30 (s,2H); 5,22 (dd,1H); 6,30 (d,1H); 6,90-7,30 (m,8H) ppm.

### Beispiel 168

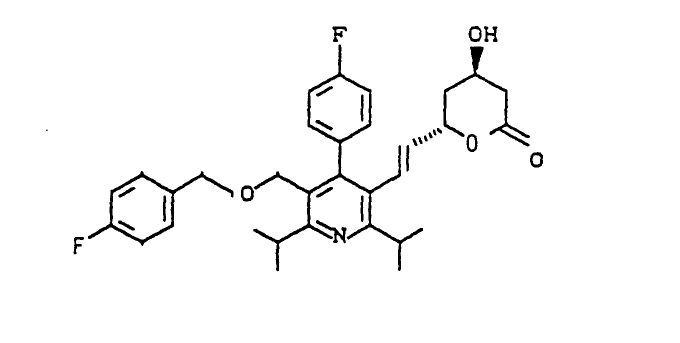

### trans-(E)-6-[2-(2,6-diisopropyl-3-(4-fluorbenzyloxy-methyl)-4-(4-fluorphenyl)-pyrid-5-yl)-ethenyl]-3,4,5,6-tetrahydro-4-hydroxy-2H-pyran-2-on

Die Herstellung erfolgt analog Beispiel 164 aus der Verbindung von Beispiel 167.
¹H-NMR (CDCl₃): δ = 1,23 (d,6H); 1,32 (d,6H);1,40-1,80 (m,2H); 2,40 (m,2H); 3,30 (m,2H); 4,13 (s,2H); 4,16 (m,1H); 4,30 (s,2H); 5,05 (m,1H); 5,28 (dd,1H); 6,37 (d,1H); 6,9-7,3 (m,8H) ppm.

### Beispiel 169

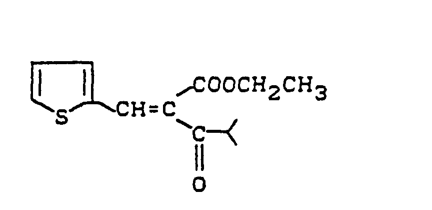

### (E/Z)-2-Ethoxycarbonyl-4-methyl-1-(thiophen-2-yl)penten-3-on

Analog der Vorschrift für Beispiel 1 wird die Titelverbindung aus Isobutyrylessigsäurethylester und Thiophen-2-carbaldehyd hergestellt.
- Ausbeute:: 86 % gelbes Öl, Kp 145°C (1,5 mbar)

### Beispiel 170

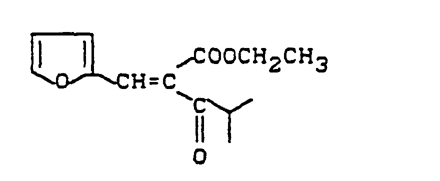

### (E/Z)-2-Ethoxycarbonyl-1-(furan-2-yl)-4-methyl-penten-3-on

Analog der Vorschrift für Beispiel 1 wird die Titelverbindung aus Furan-2-carbaldehyd und Isobutyrylessigsäureethylester hergestellt.
- Ausbeute:: 93 % gelbes Öl, Kp.: 130°C (0,5 mbar)

### Beispiel 171

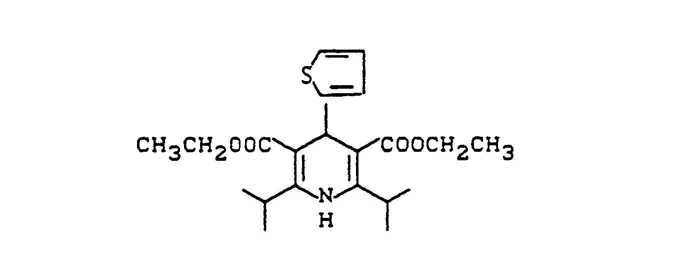

### 2,6-Diisopropyl-4-(thiopen-2-yl)-1,4-dihydropyridin-3,5-bis(carbonsäureethylester)

70 g (0,28 mol) der Verbindung aus Beispiel 169 und 44 g (0.28 mol) Ethyl-3-amino-4-methyl-pent-2-enoat wurden 24 h auf 160°C erhitzt. Man nimmt in Essigester auf, wäscht dreimal mit 6 N Salzsäure, zweimal mit Wasser und gesättiger Natriumhydrogencarbonatlösung und trocknet die org. Phase über Natriumsulfat. Man engt im Vakuum ein und chromatographiert den Rückstand an 400 g Kieselgel 230-400 mesh mit Petrolether/Dichlormethan 2:1.
- Ausbeute:: 50 g (46 %) farbl. Kristalle vom Schmp. 72°C (aus n-Hexan)

### Beispiel 172

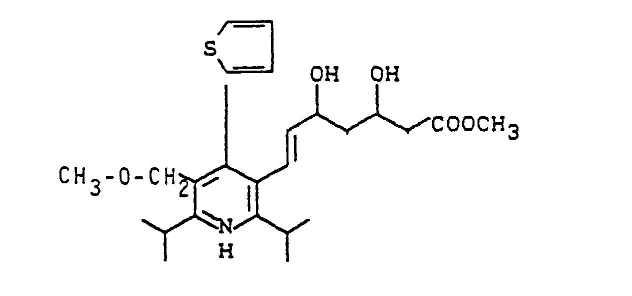

### Methyl-erythro-(E)-7-[2,6-diisopropyl-5-methoxymethyl-4(thiophen-2-yl)-pyridin-3-yl]-3,5-dihydroxy-hept-6-enoat

Ausgehend von den Verbindungen aus Beispiel 171 wurde analog zu den Vorschriften für die Beispiele 3,4,59,6, 7,8,9 und 10 die Titelverbindung hergestellt.
Farblose Kristalle vom Schmp. 94°C

### Beispiel 173

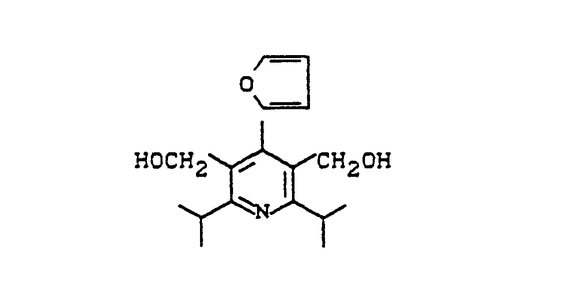

### 4-(Furan-2-yl)-3,5-bis(hydroxyethyl)-2,6-diisopropylpyridin

Ausgehend von der Verbindung aus Beispiel 170 und Ethyl-3-amino-4-methyl-pent-2-enoat wurde in Analogie zu den Verfahren aus Beispiel 171,3 und 122 die Titelverbindung hergestellt. Farbl. Kristalle vom Schmp. 212°C.

### Beispiel 174

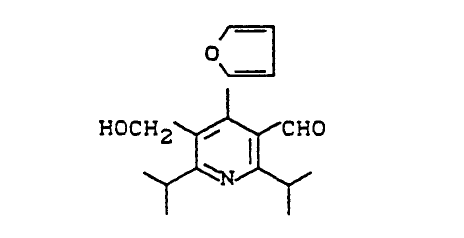

### 3-Formyl-4-(furan-2-yl)-5-hydroxymethyl-2,6-diisopropyl-pyridin

Die Synthese erfolgt aus 32 g (0.11 mol) der Verbindung aus Beispiel 173 und 28.6 g (0.13 mol) Pyridiumchlorochromat analog zur Vorschrift von Beispiel 7.
- Ausbeute:: 14,6 g (46 %)farbl. Kristalle vom Schmp. 113°C

### Beispiel 175

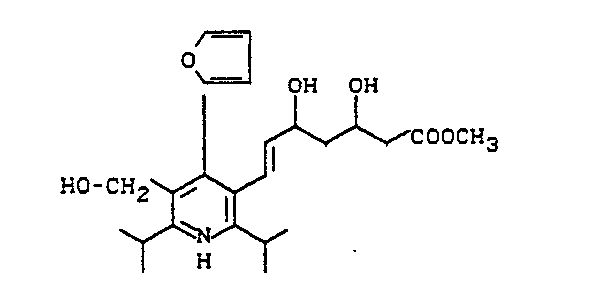

### Methyl-erythro-(E)-7-[4-(furan-2-yl)-5-hydroxymethyl-2,6-diisopropyl-pyridin-3-yl]-3,5-dihydroxy-hept-6-enoat

Analog den Verfahren aus den Beispielen 123, 124 und 125 wurde ausgehend von der Verbindung aus Beispiel 174 die Titelverbindung hergestellt.
Farbl. Kristalle vom Schmp. 86°C

### Beispiel 176

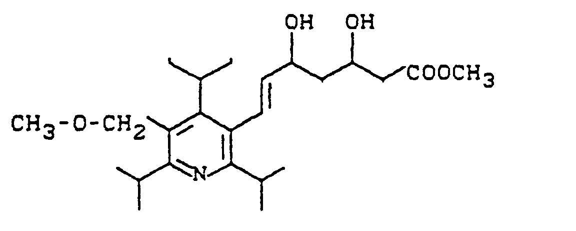

### Methyl-erythro-(E)-7-[2,4,6-triisopropyl-5-methoxymethyl -pyridin-3-yl]-3,5-dihydroxy-hept-6-enoat

Ausgehend von den Verbindungen aus Beispiel 119 und Ethyl-3-amino-4-methyl-pent-2-enoat wurde in Analyse zu den Verfahren aus den Beispielen 171, 3, 4, 59, 6, 7, 8, 9 und 10 die Titelverbindung synthetisiert.
Farbl. Öl
¹H-NMR(CDCl₃): δ = 1,1-1,35 (m,18H, isopropyl-H) 1,65-1,85 (m,2H, 4-H) 2,55 (d,2H,2-H), 3,2-3,45 (m,7H, isopropyl-H, OH, CH₃-O-CH₂) 3,7 (m,4H, OH, COOCH₃) 4,35 (m,1H, HO-CH) 4,45(s,2H,CH₃-O-CH₂) 4,62 (m,1H, HO-CH) 5,55 (dd,1H,6-H) 6,73 (d, 1H, 7-H)

### Anwendungsbeispiel

### Beispiel 177

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m KₓH_{y} Phosphat, 0,03 m Ethylendiamintetraessigsäure, 0,002 m Dithiothreit (SPE)-Puffer pH 7,2, homogenisiert. Anschließend wurde 15 Minuten bei 15 000* g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase, 35 µMol KₓH_{y}-Phosphat pH 7,2, 20 µl Enzympräparation und 56 nMol 3-Hydroxy-3-methyl-glutaryl Coenzym A (Glutaryl-3-¹⁴C) 100 000 dpm.

Nach Inkubation von 60 Minuten bei 37°C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. IC₅₀-Werte wurden durch Auftrag der prozentualen Hemmung gegen die Konzentration der Verbindung im Test durch Intrapolation bestimmt. Zur Bestimmung der relativen inhibitorischen Potenz wurde der IC₅₀-Wert der Referenzsubstanz Mevinolin als 100 gesetzt und mit dem simultan bestimmten IC₅₀-Wert der Testverbindung verglichen.

| Beispiel Nr. | relative Aktivität (Mevinolin = 1) |
|---|---|
| 17 | 20 |
| 70 | 30 |
| 72 | 17 |
| 85 | 20 |
| 89 | 10 |
| 99 | 20 |
| 100 | 30 |
| 105 | 4 |
| 107 | 3 |
| 139 | 20 |

relative in vitro Aktivitäten, Mevinolin = 1

### 2. Anwendungsbeispiel

### Beispiel 178

Die subchronische Wirkung der erfindungsgemäßen Verbindungen auf die Blutcholesterinwerte von Hunden wurde in mehrwöchigen Fütterungsexperimenten geprüft. Dazu wurde die zu untersuchende Substanz über einen mehrwöchigen Zeitraum einmal täglich in einer Kapsel gesunden Beagle-Hunden zusammen mit dem Futter p.o. gegeben. Dem Futter war außerdem während der gesamten Versuchsperiode, d.h. vor, während und nach der Applikationsperiode der zu untersuchenden Substanzen Colestyramin (4 g / 100 g Futter) als Gallensäuresequestrant beigemischt. Zweimal wöchentlich wurde den Hunden venöses Blut abgenommen und das Serumcholesterin mit einem handelsüblichen Testkit enzymatisch bestimmt. Die Serumcholesterinwerte während der Applikationsperiode wurden mit den Serumcholesterinwerten vor der Applikationsperiode (Kontrollen) verglichen.

So ergab sich z.B. für die erfindungsgemäße Verbindung Beispiel Nr. 17 nach 2-wöchiger Applikation von täglich 8 mg/kg p.o. eine Senkung des Serumcholesterins um 66%.

## Patentansprüche

1. Substituierte Pyridine der Formel 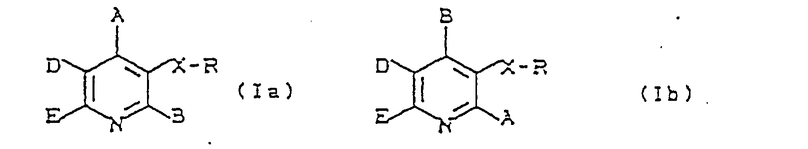 in welcher
A
- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- für Phenyl oder Naphthyl steht, das bis zu 4-fach gleich oder verschieden substituiert sein kann durch C₁-C₆-Alkyl, das gegebenenfalls durch Hydroxy oder C₁-C₆-Alkoxy substituiert sein kann, C₁-C₆-Alkoxy, C₁-C₆- Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom oder Cyano,
- für Methyl, Ethyl, Propyl, Isopropyl, Butyl oder tert. Butyl steht,
B
- für Cyclopropyl, Cyclopentyl oder Cyclohexyl steht oder
- für C₁-C₆-Alkyl steht, das substituiert sein kann durch Fluor, Chlor, Brom, Cyano, C₁-C₆-Alkoxy, C₁-C₆-Alkylthio, C₁-C₆- Alkylsulfonyl, Trifluormethyl, Trifluormethoxy, Trifluormethylsulfonyl, C₁-C₆ - Alkoxycarbonyl, Benzoyl, C₁-C₆-Alkylcarbonyl, durch eine Gruppe der Formel -NR¹R²,
worin
R¹ und R² gleich oder verschieden sind und C₁-C₆-Alkyl, Phenyl, Benzyl, Acetyl, Benzoyl, Phenylsulfonyl oder C₁-C₆-Alkylsulfonyl bedeuten, oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können,
D,E
- gleich oder verschieden sind und
- für Cyclopropyl, Cyclopentyl oder Cyclohexyl stehen, oder
- für geradkettiges oder verzweigtes C₁-C₆-Alkyl stehen, das im Fall des Substituenten D substituiert ist und im Fall des Substituenten E substituiert sein kann
durch Azido, Fluor, Chlor, Brom, Cyano, Hydroxy, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR¹R²,
worin
R¹ und R² die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, C₁-C₆- Alkyl, C₁-C₆-Alkoxy, Trifluormethyl, oder Trifluormethoxy substituiert sein können,
- für Thienyl, Furyl, Thiazolyl, Tetrazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, das bis zu 2-fach gleich oder verschieden substituiert sein kann durch Fluor, Chlor, Brom, C₁-C₆-Alkyl, C₁-C₆- Alkoxy, Phenyl, Phenoxy, Trifluormethyl, Trifluormethoxy oder C₁-C₆-Alkoxycarbonyl, oder
- für Naphthyl stehen, das bis zu 4-fach gleich oder verschieden substituiert sein kann durch C₁-C₆-Alkyl, das gegebenenfalls durch Hydroxy oder C₁-C₆-Alkoxy substituiert sein kann, C₁-C₆-Alkoxy, C₁-C₆-Alkyl-thio, C₁-C₆- Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Bron, Cyano, Trifluormethyl, Trifluormethoxy, Trifluormethylthio, C₁-C₆-Alkoxycarbonyl oder durch eine Gruppe der Formel -NR¹R²,
wobei
R¹ und R² die oben angegebene Bedeutung haben,
oder
- für eine Gruppe der Formel -CR¹¹R¹²-Y stehen,
worin
R¹¹ und R¹² gleich oder verschieden sein können und
- für Wasserstoff, oder
- für C₁-C₆- Alkyl stehen, das gegebenenfalls durch Hydroxy, Fluor, Chlor, C₁-C₆-
Alkoxy oder C₁-C₆-Alkoxycarbonyl substituiert sein kann, oder
- für Cyclopropyl, Cyclopentyl oder Cyclohexyl stehen, oder
R¹¹ und R¹² gemeinsam einen gesättigten oder ungesättigten carbocyclischen oder heterocyclischen Ring mit bis zu 6 Kohlenstoffatomen bilden,
Y - eine Gruppe der Formel -NR¹³R¹⁴, -COR¹⁵, -S-R¹⁶, SO-R¹⁶, -SO₂R¹⁶, -OR¹⁷ oder -N₃ bedeutet,
wobei
R¹³ und R¹⁴ gleich oder verschieden sind und
- für Wasserstoff, C₁-C₆-Alkyl, Phenyl oder Benzyl stehen, wobei die genannten Reste durch Fluor, Chlor, Brom, C₁-C₆-Alkyl, C₁-C₆-Alkoxy oder Trifluormethyl substituiert sein können, oder
- für eine Gruppe der Formel -COR¹⁵, -SO₂R¹⁶ stehen,
oder R¹³ und R¹⁴ gemeinsam eine Alkylenkette bilden, die durch O, N, S, N-C₁-C₆-
Alkyl, N-Benzyl, N-Phenyl, N-Carbamoyl oder N-C₁-C₆-Alkoxycarbonyl unterbrochen sein kann,
R¹⁵
- eine Gruppe -NR¹⁸R¹⁹ bedeutet, oder
- C₁-C₆-Alkyl oder C₁-C₆-Alkoxy bedeutet, oder
- gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzathiazolyl, Benzoxazolyl, Thiazolyl, Oxazolyl, Isoxazolyl oder Isothiazolyl bedeutet,
R¹⁶
- Cyclopropyl, Cyclopentyl, Cyclohexyl, oder
- gegebenenfalls durch Cyano, Fluor, Chlor, Brom, Trifluormethyl oder C₁-C₆-Alkoxycarbonyl substituiertes geradkettiges oder verzweigtes C₁-C₆-Alkyl bedeutet, oder
- gegebenenfalls ein- oder mehrfach gleich oder verschieden durch C₁-C₆-
Alkyl, C₁-C₆-Alkoxy, Fluor, Chlor, Brom, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
- Trimethylsilyl oder Dimethylethylsilyl bedeutet, oder
- eine Gruppe -NR⁹R¹⁰ bedeutet,
wobei
R⁹ und R¹⁰ die oben angegebene Bedeutung haben,
R¹⁷
- für Wasserstoff, oder
- für Cyclopropyl, Cyclopentyl oder Cyclohexyl steht, oder
- für C₁-C₆-Alkyl steht, das substituiert sein kann durch Fluor, Chlor, Brom, Cyano, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-
Alkoxycarbonyl, Benzoyl, C₁-C₆-
Alkylcarbonyl, durch eine Gruppe der Formel -NR¹R²,
worin
R¹ und R² die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten HeLeroaryl- und Arylreste bis zu 2-fach gleich oder verschieden durch Fluor, Chlor, Brom, C₁-C₆-
Alkyl, C₁-C₆-Alkoxy, Trifluormethyl oder Trifluormethoxy substituiert sein kann,
- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, C₁-C₆-Alkyl, C₁-C₆-Alkoxy, Fhenyl, Phenoxy, Trifluormethyl, Trifluormethoxy oder C₁-C₆-Alkoxycarbonyl, oder
- für Benzyl, Phenyl oder Naphthyl steht, die bis zu 4-fach gleich oder verschieden substituiert sein können durch C₁-C₆-Alkyl, C₁-C₆-Alkoxy, C₁-C₆-Alkylthio, C₁-C₆- Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR¹R²,
wobei R¹ und R² die oben angegebene Bedeutung haben,
oder
- für 2,5-Dioxo-tetrahydropyrryl,
- für Tetrahydropyranyl, oder
- für Dimethyl-tert.butylsilyl, Tripropylsilyl oder Tributylsilyl steht, oder
- eine Gruppe der Formel COR¹⁶ bedeutet,
wobei
R¹⁶ die oben angegebene Bedeutung hat,
und
R¹⁸ und R¹⁹ gleich oder verschieden sind und
- Wasserstoff bedeuten, oder
- gegebenenfalls durch Cyano, Fluor, Chlor oder Brom substituiertes C₁-C₆-
Alkyl bedeuten, oder
- gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeuten,
oder
D, E gemeinsam einen Ring der Formel 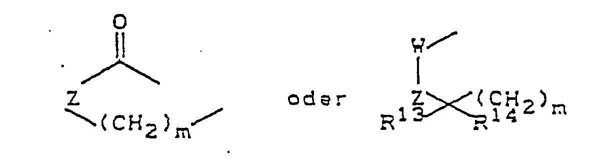 bilden,
worin
W - für eine Gruppe der Formel C=O oder für CH-OH steht,
m - für eine Zahl 1 oder 2 steht,
Z - für O, CH₂ oder NHR²⁰ steht,
R¹³ und R¹⁴ die oben angegebene Bedeutung haben,
und
R²⁰ - für Wasserstoff, C₁-C₆-Alkyl, Phenyl, Benzyl, Carbamoyl oder C₁-C₆-Alkoxy-carbonyl steht,
X - für eine Gruppe der Formel -CH=CH- sieht,
R - für eine Gruppe der Formel 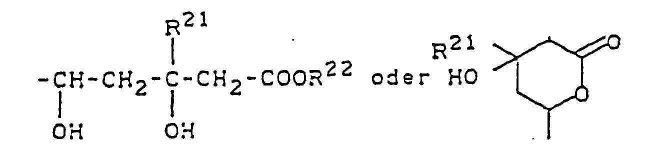
steht,
worin
R²¹ - Wasserstoff oder C₁-C₆-Alkyl bedeutet,
und
R²² - - Wasserstoff, C₁-C₆-Alkyl, Phenyl oder Benzyl bedeutet, oder
-ein Kation bedeutet,
sowie deren Oxidationsprodukte,

2. Substituierte Pyridine nach Anspruch 1,
worin
A
- für Thienyl, Furyl, Pyridyl, Pyrimidyl, Chinolyl oder Isochinolyl steht, das durch Fluor, Chlor, Methyl, Methoxy oder Trifluormethyl substituiert sein kann, oder
- für Phenyl steht, das bis zu 3-fach gleich oder verschieden substituiert sein kann durch Methyl, Hydroxymethyl, Ethyl, Propyl, Isopropyl, Hydroxyethyl, Hydroxypropyl, Butyl, Isobutyl, Methoxymethyl, Ethoxymethyl, Propoxymethyl, tert.-Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
- für Methyl, Ethyl, Propyl, Isopropyl, Butyl oder tert. Butyl steht,
B
- für Cyclopropyl, Cyclopentyl oder Cyclohexyl steht, oder
- für Methyl, Ethyl, Propyl, Isopropyl, Butyl, sec.-Butyl oder tert.Butyl steht, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
D,E
- gleich oder verschieden sind und - für Cyclopropyl, Cyclopentyl oder Cyclohexyl stehen, oder
- für Methyl, Ethyl, Propyl, Isopropyl, Butyl, Isobutyl, tert.Butyl, Pentyl, Isopentyl, Hexyl oder Isohexyl stehen, die im Fall des Substituenten D substituiert sind und im Fall des Substituenten E substituiert sein können
durch Azido, Fluor, Chlor, Brom, Iod, Cyano, Hydroxy, Methoxy, Ethoxy, Propoxy, Isopropoxy, Butoxy, Isobutoxy, tert.Butoxy, Hethylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR¹R²,
wobei
R¹ und R² gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten, oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
- für Thienyl, Furyl, Pyridyl, Pyrimidyl, Pyrazinyl, Tetrazolyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl stehen, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.-Butoxy, Fhenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder - für eine Gruppe der Formel -CR¹¹R¹²-Y stehen,
worin
R¹¹ und R¹² gleich oder verschieden sind und
- für Wasserstoff, oder
- für Methyl, Ethyl, Propyl, Isopropyl stehen, das gegebenenfalls durch Hydroxy, Fluor, Chlor, Methoxy, Ethoxy, Methoxycarbonyl, Ethoxycarbonyl substituiert sein kann, oder
- für Cyclopropyl, Cyclopentyl oder Cyclohexyl stehen,
oder R¹¹ und R¹² gemeinsam für Cyclopropyl, Cyclopentyl oder Cyclohexyl stehen,
und
Y - eine Gruppe der Formel -NR¹³R¹⁴, -COR¹⁵, -SR¹⁶, -SO-R¹⁶, -SO₂R¹⁶, -OR¹⁷ oder -N₃ bedeutet,
wobei
R¹³ und R¹⁴ gleich oder verschieden sind, und
- für Wasserstoff, Methyl, Ethyl, Propyl, Isopropyl, Butyl, Isobutyl, oder
- für gegebenenfalls durch Fluor, Chlor, Methyl oder Methoxy substituiertes Phenyl, oder
- für eine Gruppe -COR¹⁵ oder -SO₂R¹⁶ stehen, oder
R¹³ und R¹⁴ gemeinsam mit dem Stickstoffatom einen Ring der Reihe Piperidin, Piperazin, Morpholin, Morpholin-N-oxid, N-Niederalkylpiperazin, Benzylpiperazin oder Phenylpiperazin bilden,
R¹⁵
- Wasserstoff bedeutet, oder
- eine Gruppe -NR¹⁸R¹⁹ bedeutet, oder
- Methyl, Ethyl, Propyl, Isopropyl, Methoxy, Ethoxy, Propoxy, Isopropoxy, oder
- gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,
R¹⁶
- gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl, Isobutyl oder Isopentyl bedeutet, oder
- gegebenenfalls ein- oder mehrfach gleich oder verschieden durch Methyl, Ethyl, Propyl, Isopropyl, Methoxy, Fluor oder Chlor substituiertes Benzyl, Phenyl, Naphthyl, Thienyl, Furyl,. Pyridyl, Pyrimidyl, Chinolyl oder Isochinolyl bedeuteL, oder
- Trimethylsilyl oder Dimethylethylsilyl bedeutet,
- eine Gruppe -NR⁹R¹⁰ bedeutet,
wobei
R⁹ und R¹⁰ die oben angegebene Bedeutung haben,
R¹⁷
- für Wasserstoff, Cyclopropyl, Cyclopentyl oder Cyclohexyl steht, oder
- für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR¹R²,
wobei
R¹ und R² gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten HeLeroarylund Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein kann, oder
- für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.-Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.-Butoxycarbonyl substituiert sein können, oder - für Benzyl oder Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe -NR¹R² substituiert sein
kann,
wobei
R¹ und R² die oben angegebene Bedeutung haben,
oder - für 2,5-Dioxo-tetrahydropyrryl, - für Tetrahydropyranyl steht, oder - für Dimethyl-tert.butylsilyl oder Trimethylsilyl steht, oder
- eine Gruppe -COR¹⁶ bedeutet,
wobei
R¹⁶ die oben angegebene Bedeutung hat,
und
R¹⁸ und R¹⁹ gleich oder verschieden sind und
- Wasserstoff bedeuten, oder
- gegebenenfalls durch Fluor oder Chlor substituiertes Methyl, Ethyl, Propyl, Isopropyl, Butyl, Isobutyl, Pentyl, Isopentyl, Hexyl oder Isohexyl bedeuten, oder
- Phenyl bedeuten, das durch Fluor, Chlor, Methyl oder Methoxy substituiert sein kann,
oder
D und E gemeinsam einen Ring der Formel 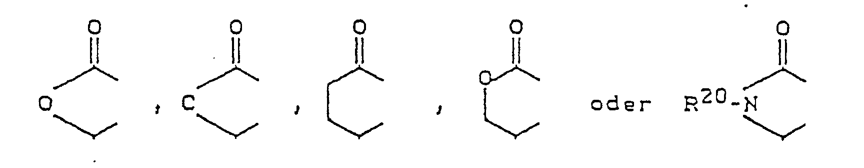 bilden,
worin
R²⁰ - für WassersLoff, Methyl, Ethyl, Propyl, Isopropyl, Carbamoyl, Methoxycarbonyl oder Ethoxycarbonyl steht,
X - für eine Gruppe der Formel -CH=CH- steht,
R - für eine Gruppe der Formel 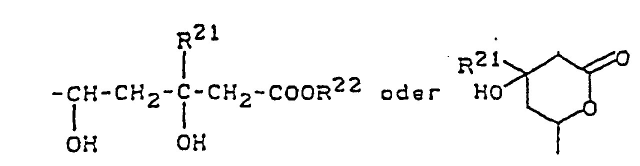 steht, worin
R²¹ - Wasserstoff, Methyl, Ethyl, Propyl, Isopropyl, Butyl, Isobutyl oder tert.Butyl bedeutet,
und
R²² - Wasserstoff, Methyl, Ethyl, Propyl, Isopropyl, Butyl, Isobutyl, tert.Butyl oder Benzyl bedeutet, oder ein Natrium-, Kalium-, Calcium -, oder Magnesium- oder Ammoniumion bedeutet,
sowie deren Oxidationsprodukte.

3. Verbindungen der allgemeinen Formeln (Ia) 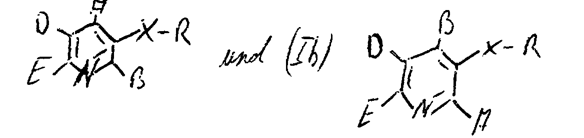 in welchen
A
- für Thienyl oder Furyl steht,
- für Phenyl steht, das bis zu 2-fach gleich oder verschieden durch Methyl, Hydroxymethyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Trifluormethyl substituiert sein kann,
- für Methyl, Ethyl, Propyl oder Isopropyl steht,
B
- für Cyclopropyl, Methyl, Ethyl, Propyl, Isopropyl, Butyl, Isobutyl oder tert.Butyl steht, das durch Fluor, Chlor, Methoxy, Phenyl oder Phenoxy substituiert sein kann,
D,E gleich oder verschieden sind und
- für Cyclopropyl, Cyclopentyl oder Cyclohexyl stehen, oder
- für Methyl, Ethyl, Propyl, Isopropyl, Butyl, Isobutyl, tert.Butyl, Pentyl, Isopentyl, Hexyl oder Isohexyl stehen, das im Fall des Substituenten D Substituiert ist und im Fall des Substituenten E substituiert sein kann durch Azido, Fluor, Chlor, Iod, Methoxy, Ethoxy, Propoxy, Isopropoxy, Methylthio, Ethylthio, Methylsulfonyl, Ethylsulfonyl, Ethoxycarbonyl, Propoxycarbonyl, Isopropoxycarbonyl oder durch eine Gruppe der Formel NR¹R²,
wobei
R¹ und R² gleich oder verschieden sind und
- für Wasserstoff, Methyl, Ethyl, Propyl, Isopropyl, Phenyl oder Benzyl stehen,
oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy, oder
- für gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Tetrazolyl, Benzthiazolyl oder Benzimidazolyl stehen, oder
- für eine Gruppe der Formel -CR¹¹R¹²-Y stehen
worin
R¹¹ und R¹² Wasserstoff, Methyl oder Ethyl bedeuten,
und
Y - eine Gruppe der Formel -NR¹³R¹⁴, -COR¹⁵, -S-R¹⁶, -SO-R¹⁶, -SO₂R¹⁶ oder -OR¹⁷ bedeutet,
wobei
R¹³ und R¹⁴ gleich oder verschieden sind, und
- für Wasserstoff, Methyl, Ethyl, Propyl, oder
- für eine Gruppe -COR¹⁵ oder -SO₂R¹⁶ stehen
oder R¹³ und R¹⁴ gemeinsam mit dem Stickstoffatom einen Ring der Reihe Morpholin oder Morpholin-N-oxid bilden,
und
R¹⁵
- Wasserstoff oder Methyl bedeutet, oder
- eine Gruppe -NR¹⁸R¹⁹ bedeutet, oder
- Methyl, Ethyl, Propyl, Methoxy oder Ethoxy bedeutet,
R¹⁶
- Trifluormethyl, Methyl, Ethyl, Propyl, Isopropyl, Butyl, Isobutyl, Isopentyl oder Benzyl bedeutet, oder
- gegebenenfalls durch ein- oder mehrere Methyl oder Chlor substituiertes Phenyl, oder Naphthyl bedeutet,
- Trimethylsilyl oder Dimethylethylsilyl, oder
- eine Gruppe -NR⁹R¹⁰ bedeutet,
wobei
R⁹ und R¹⁰ die oben angegebene Bedeutung haben,
R¹⁷
- für Wasserstoff, Cyclopropyl, Cyclopentyl oder Cyclohexyl steht, oder
- für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Methoxy, Ethoxy, Propoxy, Isopropoxy, Methylthio, Ethylthio, Methylsulfonyl, Ethylsulfonyl, Ethoxycarbonyl, Propoxycarbonyl, Isopropoxycarbonyl oder durch eine Gruppe der Formel NR¹R²,
wobei
R¹ und R² gleich oder verschieden sind und
- für Wasserstoff, Methyl, Ethyl, Propyl, Isopropyl, Phenyl oder Benzyl stehen,
oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy, oder
- für gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Benzthiazolyl oder Benzimidazolyl steht, oder
- für Benzyl oder Phenyl steht, das bis zu 2-fach gleich oder verschieden durch Methyl, Ethyl, Propyl, Isopropyl, Butyl, Isobutyl, tert.Butoxy, Methoxy, Ethoxy, Propoxy, Isopropoxy, Methylthio, Ethylthio, Methylsulfonyl, Ethylsulfonyl, Phenyl, Phenoxy, Phenylsulfonyl, Benzyloxy, Fluor, Chlor, Brom, Cyano, Trifluormethyl, Trifluormethoxy, Methoxycarbonyl, Ethoxycarbonyl oder eine Gruppe der Formel -NR¹R² substituiert sein kann,
wobei
R¹ und R² die oben angegebene Bedeutung haben,
- für 2,5-Dioxo-tetrahydropyrryl oder
- für Tetrahydropyranyl, oder
- für Dimethyl-tert.butylsilyl oder Trimethylsilyl steht, oder
- eine Gruppe -COR¹⁶ bedeutet,
wobei
R¹⁶ die oben angegebene Bedeutung hat,
und
R¹⁸ und R¹⁹ gleich oder verschieden sind und
- Wasserstoff bedeuten, oder
- Methyl, Ethyl, Propyl, Isopropyl, Butyl oder Isobutyl bedeuten, oder
- Phenyl bedeuten,
oder
D und E gemeinsam einen Ring der Formel 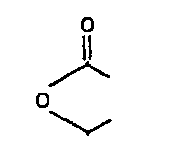 bilden,
X - für eine Gruppe der Formel 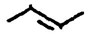 (E-konfiguriert) steht , und
R - für eine Gruppe der Formel 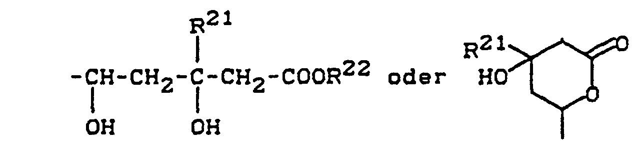
steht,
worin
R²¹ - Wasserstoff bedeutet
und
R²2 - Wasserstoff, Methyl oder Ethyl bedeutet, oder ein Natrium- oder Kaliumion bedeutet
und deren Oxidationsprodukte.

4. Methyl-erythro-(E)-7-[2,6-diisopropyl-4-(4-fluorphenyl)-5-methoxymethyl-pyrid-3-yl]-3,5-dihydroxy-hept-6-enoat
der Formel

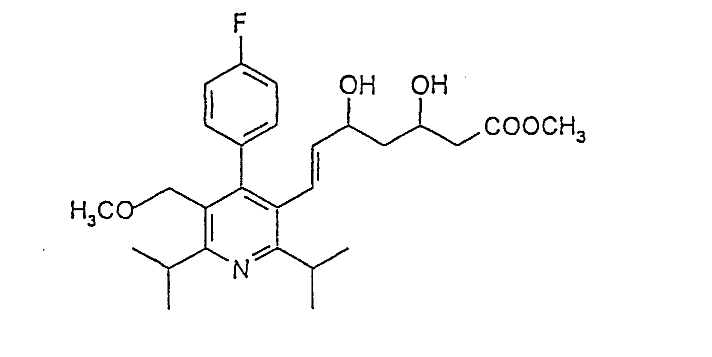

5. Salze der Verbindungen der allgemeinen Formel (le) bzw. (Ie)' 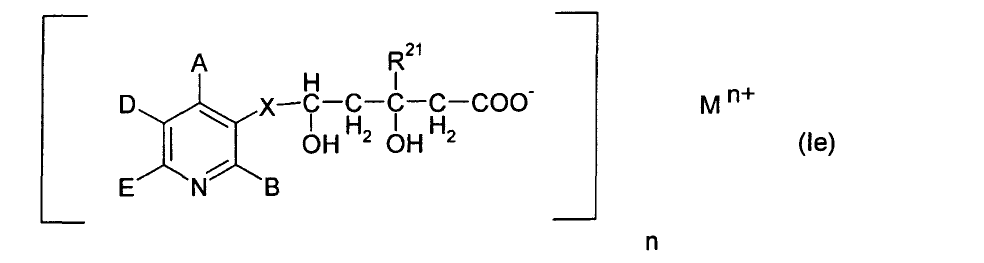 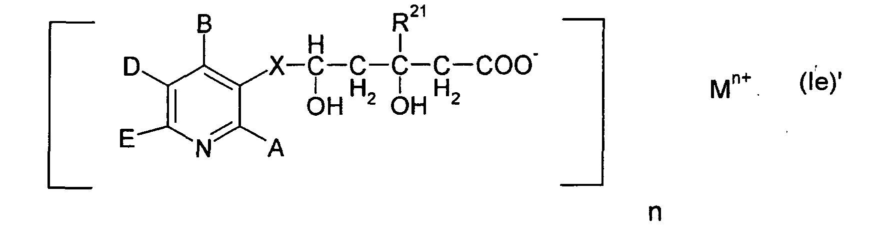 in welcher
A, B, D, E, X und R²¹ die in den Verbindungen der vorangehenden Ansprüchen 1 bis 5 angegebenen Bedeutungen haben und
Mⁿ⁺ für ein Kation steht, wobei n die Wertigkeit angibt.

6. Salze gemäß Anspruch 6 wobei Mⁿ⁺ für das Natrium- oder Kaliumion steht.

7. Carbonsäuren der allgemeinen Formel (Ic) bzw. (Ic)' 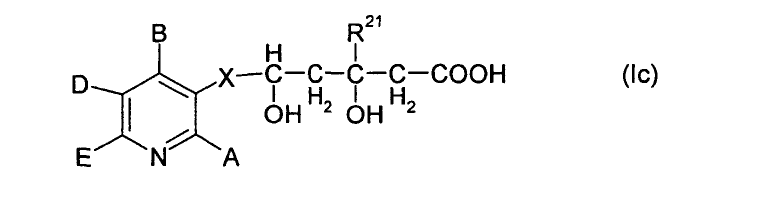 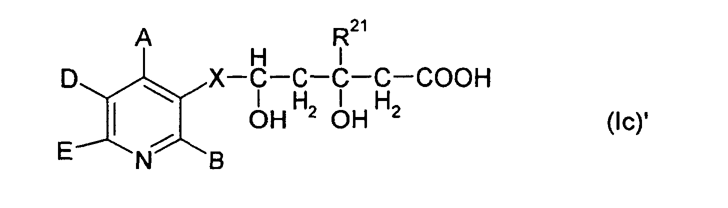 in welcher
A, B, D, E, X, und R²¹ die in den Verbindungen der vorangehenden Ansprüchen 1 bis 5 angegebenen Bedeutungen haben.

8. Ketone der Formel (VIII a) bzw. (VIII b) 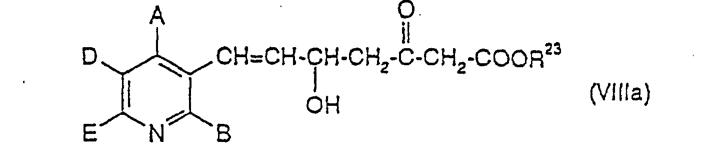 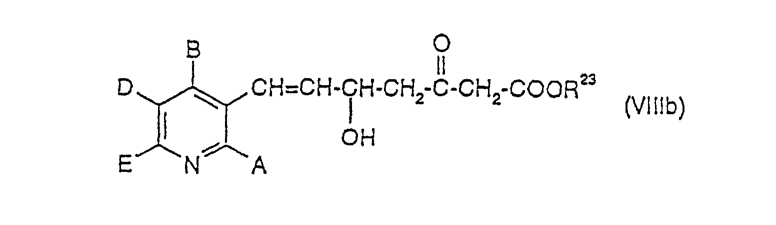 in welchen
A, B, D und E die in den vorangehenden Anspruchen angegeben Bedeutungen haben und
R²³ für Alkyl steht.

9. Verfahren zur Herstellung von Ketonen der Formel (VIIIa) bzw (VIIIb) die in Anspruch 8 definiert sind,
das **dadurch gekennzeichnet ist, daß** man
Aldehyde der allgemeinen Formel (IX) 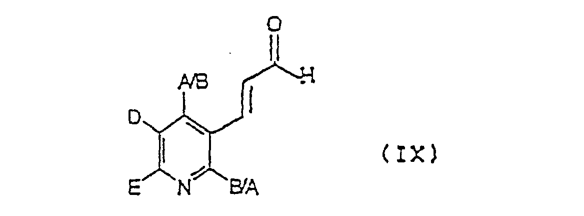 in welcher
A, B, D und E die in den vorangehenden Ansprüchen angegebenen Bedeutungen haben,
in inerten Lösemitteln mit Acetessigester der allgemeinen Formel (X) 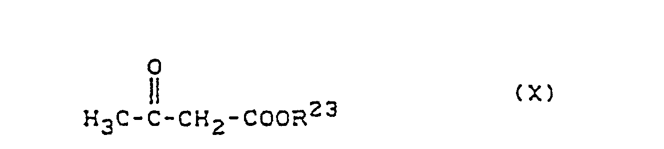 in welcher
R²³ die in Anspruch 8 angegebene Bedeutung hat,
in Anwesenheit von Basen umsetzt.

10. Verfahren nach Anspruch 9, **dadurch gekennzeichnet, daß** man die Umsetzung im Temperaturbereich von -80°C bis +50°C durchführt.

11. Verfahren zur Herstellung von substituierten Pyridinen der Formel 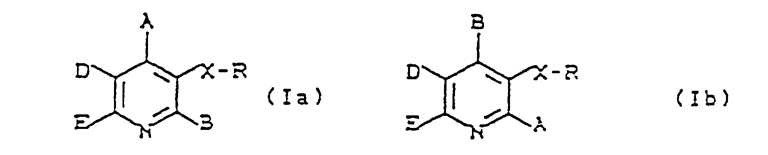 in welcher
A
- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- für Phenyl oder Naphthyl steht, das bis zu 4-fach gleich oder verschieden substituiert sein kann durch C₁-C₆-Alkyl, das gegebenenfalls durch Kydroxy oder C₁-C₆-Alkoxy substituiert sein kann, C₁-C₆-Alkoxy, C₁-C₆- Alkylthio, C₁-C₆-Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom oder Cyano,
- für Methyl, Ethyl, Propyl, Isopropyl, Butyl oder tert. Butyl steht,
B
- für Cyclopropyl, Cyclopentyl oder Cyclohexyl steht oder
- für C₁-C₆-Alkyl steht, das substituiert sein kann durch Fluor, Chlor, Brom, Cyano, C₁-C₆-Alkoxy, C₁-C₆-Alkylthio, C₁-C₆- Alkylsulfonyl, Trifluormethyl, Trifluormethoxy, Trifluormethylsulfonyl, C₁-C₆- Alkoxycarbonyl, Benzoyl, C₁-C₆-Alkylcarbonyl, durch eine Gruppe der Formel -NR¹R²,
worin
R¹ und R² gleich oder verschieden sind und C₁-C₆-Alkyl, Phenyl, Benzyl, Acetyl, Benzoyl. Phenylsulfonyl oder C₁-C₆-Alkylsulfonyl bedeuten, oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Banzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl, oder Trifluormethoxy substituiert sein können, D,E - gleich oder verschieden sind und
- für Cyclopropyl, Cyclopentyl oder Cyclohexyl stehen, oder
- für geradkettiges oder verzweigtes C₁-C₆-Alkyl stehen, das im Fall des Substituenten D substituiert ist und im Fall des Substituenten E substituiert sein kann
durch Azido, Fluor, Chlor, Brom, Cyano, Hydroxy, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR¹R²,
worin
R¹ und R² die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, C₁-C₆- Alkyl, C₁-C₆-Alkoxy, Trifluormethyl, oder Trifluormethoxy substituiert sein können,
- für Thienyl, Furyl, Thiazolyl, Tetrazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, das bis zu 2-fach gleich oder verschieden substituiert sein kann durch Fluor, Chlor, Brom, C₁-C₆-Alkyl, C₁-C₆- Alkoxy, Phenyl, Phenoxy, Trifluormethyl, Trifluormethoxy oder C₁-C₆-Alkoxycarbonyl, oder
- für Naphthyl steht, das bis zu 4-fach gleich oder verschieden substituiert sein kann durch C₁-C₆-Alkyl, das gegebenenfalls durch Hydroxy oder C₁-C₆-Alkoxy substituiert sein kann, C₁-C₆-Alkoxy, C₁-C₆-Alkylthio, C₁-C₆- Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR¹R²,
wobei
R¹ und R² die oben angegebene Bedeutung haben,
oder
- für eine Gruppe der Formel -CR¹¹R¹²-Y stehen,
worin
R¹¹ und R¹² gleich oder verschieden sein können und
- für Wasserstoff, oder
- für C₁-C₆- Alkyl stehen, das gegebenenfalls durch Hydroxy, Fluor, Chlor, C₁-C₆-Alkoxy oder C₁-C₆-Alkoxycarbonyl substituiert sein kann, oder
- für Cyclopropyl, Cyclopentyl oder Cyclohexyl stehen, oder
R¹¹ und R¹² gemeinsam einen gesättigten oder ungesättigten carbocyclischen oder heterocyclischen Ring mit bis zu 6 Kohlenstoffatomen bilden,
Y - eine Gruppe der Formel -NR¹³R¹⁴, -COR¹⁵, -S-R¹⁶, SO-R¹⁶, -SO₂R¹⁶, -OR¹⁷ oder -N₃ bedeutet,
wobei
R¹³ und R¹⁴ gleich oder verschieden sind und
- für Wasserstoff, C₁-C₆-Alkyl, Phenyl oder Benzyl stehen, wobei die genannten Reste durch Fluor, Chlor, Brom, C₁-C₆-Alkyl, C₁-C₆-Alkoxy oder Trifluormethyl substituiert sein können, oder
- für eine Gruppe der Formel -COR¹⁵, -SO₂R¹⁶ stehen,
oder R¹³ und R¹⁴ gemeinsam eine Alkylenkette bilden, die durch 0, N, S, N-C₁-C₆- Alkyl, N-Benzyl, N-Phenyl, N-Carbamoyl oder N-C₁-C₆-Alkoxycarbonyl unterbrochen sein kann,
R¹⁵
- eine Gruppe -NR¹⁸R¹⁹ bedeutet, oder
- C₁-C₆-Alkyl oder C₁-C₆-Alkoxy bedeutet, oder
- gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
R¹⁶ - Cyclopropyl, Cyclopentyl, Cyclohexyl, oder
- gegebenenfalls durch Cyano, Fluor, Chlor, Bron, Trifluormethyl oder C₁-C₆-Alkoxycarbonyl substituiertes geradkettiges oder verzweigtes C₁-C₆-Alkyl bedeutet, oder
- gegebenenfalls ein- oder mehrfach gleich oder verschieden durch C₁-C₆-Alkyl, C₁-C₆-Alkoxy, Fluor, Chlor, Brom, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
- Trimethylsilyl oder Dimethylethylsilyl bedeutet, oder
- eine Gruppe -NR⁹R¹⁰ bedeutet,
wobei
R⁹ und R¹⁰ die oben angegebene Bedeutung haben,
R¹⁷
- für Wasserstoff, oder
- für Cyclopropyl, Cyclopentyl oder Cyclohexyl steht, oder
- für C₁-C₆-Alkyl steht, das substituiert sein kann durch Fluor, Chlor, Brom, Cyano, C₁-C₆-Alkoxy, C₁-C₆-Alkylthio, C₁-C₆-Alkylsulfonyl, Trifluormethyl, Trifluormethoxy, Trifluormethylsulfonyl, C₁-C₆-Alkoxycarbonyl, Benzoyl, C₁-C₆-Alkylcarbonyl, durch eine Gruppe der Formel -NR¹R²,
worin
R¹ und R² die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, C₁-C₆-Alkyl, C₁-C₆-Alkoxy, Trifluormethyl oder Trifluormethoxy substituiert sein kann,
- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert, sein kann durch Fluor, Chlor, Brom, C₁-C₆-Alkyl, C₁-C₆-Alkoxy, Phenyl, Phenoxy, Trifluormethyl, Trifluormethoxy oder C₁-C₆-Alkoxycarbonyl, oder
- für Benzyl, Phenyl oder Naphthyl steht, die bis zu 4-fach gleich oder verschieden substituiert sein können durch C₁-C₆-Alkyl, C₁-C₆-Alkoxy, C₁-C₆-Alkylthio, C₁-C₆- Alkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, C₁-C₆-Alkoxycarbonyl oder durch eine Gruppe der Formel -NR¹R²,
wobei
R¹ und R² die oben angegebene Bedeutung haben,
oder
- für 2,5-Dioxo-tetrahydropyrryl,
- für Tetrahydropyranyl, oder
- für Dimethyl-tert.butylsilyl, Tripropylsilyl oder Tributylsilyl steht, oder
- eine Gruppe der Formel COR¹⁶ bedeutet,
wobei
R¹⁶ die oben angegebene Bedeutung hat,
und
R¹⁸ und R¹⁹ gleich oder verschieden sind und
- Wasserstoff bedeuten, oder
- gegebenenfalls durch Cyano, Fluor, Chlor oder Bron substituiertes C₁-C₆-Alkyl bedeuten, oder
- gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeuten,
oder
D, E gemeinsam einen Ring der Formel 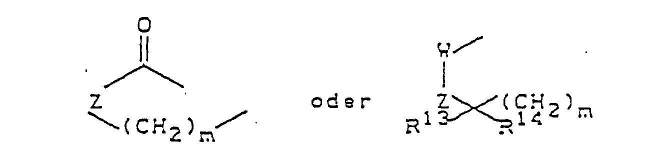 bilden,
worin
W - für eine Gruppe der Formel C=O oder für CH-OH steht,
m - für eine Zahl 1 oder 2 steht,
Z - für O, CH₂ oder NHR²⁰ steht,
R¹³ und R¹⁴ die oben angegebene Bedeutung haben, und
R²⁰ - für Wasserstoff, C₁-C₆-Alkyl, Phenyl, Benzyl, Carbamoyl oder C₁-C₆-Alkoxy-carbonyl steht,
X - für eine Gruppe der Formel -CH=CH- steht,
R - für eine Gruppe der Formel 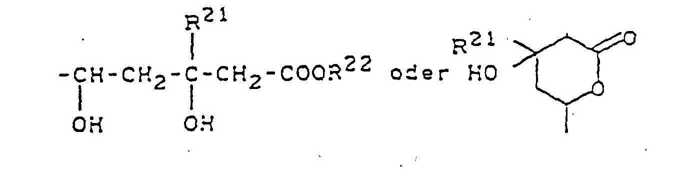
steht,
worin
R²¹ - Wasserstoff oder C₁-C₆-Alkyl bedeutet,
und
R²² - Wasserstoff C₁-C₆-Alkyl, Phenyl oder Benzyl bedeutet, oder - ein Kation bedeutet,
sowie von deren Oxidationsprodukten,
**dadurch gekennzeichnet, daß** man
Ketone der allgemeinen Formel (VIIIa, b) 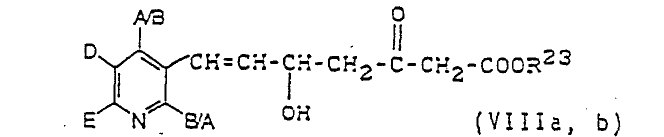 in welcher
A, B, D, E die angegebene Bedeutung
haben und
R²³ für C₁-C₆-Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,

12. Verfahren gemäß Anspruch 11 zur Herstellung von substituierten Pyridinen der Formel (Ia) bzw. (Ib)
worin
λ
- für Thienyl, Furyl, Pyridyl, Pyrimidyl, Chinolyl oder Isochinolyl steht, das durch Fluor, Chlor, Methyl, Methoxy oder Trifluormethyl substituiert sein kann, oder
- für Phenyl steht, das bis zu 3-fach gleich oder verschieden substituiert sein kann durch Methyl, Hydroxymethyl, Ethyl, Propyl, Isopropyl, Hydroxyethyl, Hydroxypropyl, Butyl, Isobutyl, Methoxymethyl, Ethoxymethyl, Propoxymethyl, tert.-Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
- für Methyl, Ethyl, Propyl, Isopropyl, Butyl oder tert. Butyl steht,
B
- für Cyclopropyl, Cyclopentyl oder Cyclohexyl steht, oder
- für Methyl, Ethyl, Propyl, Isopropyl, Butyl, sec.-Butyl oder tert.Butyl steht, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
D,E
- gleich oder verschieden sind und
- für Cyclopropyl, Cyclopentyl oder Cyclohexyl stehen, oder
- für Methyl, Ethyl, Propyl, Isopropyl, Butyl, Isobutyl, tert.Butyl, Pentyl, Isopentyl, Hexyl oder Isohexyl stehen, die im Fall des Substituente D substituiert sind und im Fall des Substituenten E substituiert sein können.
durch Azido, Fluor, Chlor, Brom, Iod, Cyano, Hydroxy, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycanbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR¹R²,
wobei
R¹ und R² gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten, oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, 3enzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
- für Thienyl, Furyl, Pyridyl, Pyrimidyl, Pyrazinyl, Tetrazolyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl stehen, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.-Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- für eine Gruppe der Formel -CR¹¹R¹²-Y stehen,
worin
R¹¹ und R¹² gleich oder verschieden sind und
- für Wasserstoff, oder
- für Methyl, Ethyl, Propyl, Isopropyl stehen, das gagebenenfalls durch Hydroxy, Fluor, Chlor, Methoxy, Ethoxy, Methoxycarbonyl, Ethoxycarbonyl substituiert sein kann, oder
- für Cyclopropyl, Cyclopentyl oder Cyclohexyl stehen,
oder R¹¹ und R¹² gemeinsam für Cyclopropyl, Cyclopentyl oder Cyclohexyl stehen,
und
Y - eine Gruppe der Formel -MR¹³R¹⁴, -COR¹⁵, -SR¹⁶, -SO-R¹⁶, -SO₂R¹⁶, -OR¹⁷ oder -N₃ bedeutet,
wobei
R¹³ und R¹⁴ gleich oder verschieden sind, und
- für Wasserstoff, Methyl, Ethyl, Propyl, Isopropyl, Butyl, Isobutyl, oder
- für gegebenenfalls .durch Fluor, Chlor, Methyl oder Methoxy subsituiertes Phenyl, oder
- für eine Gruppe -COR¹⁵ oder -SO₂R¹⁶ stehen, oder
R¹³ und R¹⁴ gemeinsam mit dem Stickstoffatom einen Ring der Reihe Piperidin, Piperazin, Morpholin, Morpholin-N-oxid, N-Niederalkylpiperazin, Benzylpiperazin oder Phenylpiperazin bilden,
R¹⁵
- Wasserstoff bedeutet, oder
- eine Gruppe -NR¹⁸R¹⁹ bedeutet, oder
- Methyl, Ethyl, Propyl, Isopropyl, Methoxy, Ethoxy, Propoxy, Isopropoxy, oder
- gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,
R¹⁶
- gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl, Isobutyl oder Isopentyl bedeutet, oder
- gegebenenfalls ein- oder mehrfach gleich oder verschieden durch Methyl, Ethyl, Propyl, Isopropyl, Methoxy, Fluor oder Chlor substituiertes Benzyl, Phenyl, Naphthyl, Thienyl, Furyl, Pyridyl, Pyrimidyl, Chinolyl oder Isochinolyl bedeutet, oder
- Trimethylsilyl oder Dimethylethylsilyl bedeutet,
- eine Gruppe -NR⁹R¹⁰ bedeutet,
wobei
R⁹ und R¹⁰ die oben angegebene Bedeutung haben,
R¹⁷
- für Wasserstoff, Cyclopropyl, Cyclopentyl oder Cyclohexyl steht, oder
- für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe -NR¹R²,
wobei
R¹ und R² gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroarylund Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein kann, oder
- für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, IsobuLyl, tert.Butyl, Methoxy, Ethoxy, Propoxy, Isopropoxy, Butoxy, Isobutoxy, tert.-Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.-Butoxycarbonyl substituiert sein können, oder
- für Benzyl oder Phenyl sLeht, das bis zu 3-fach gleich oder verschieden durch Methyl, ELhyl, Propyl, Isopropyl, Butyl, Isobutyl, tert.Butyl, Pentyl, Isopentyl, Hexyl, isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe -NR¹R² substituiert sein kann,
wobei
R¹ und R² die oben angegebene Bedeutung haben,
oder
- für 2,5-Dioxo-tetrahydropyrryl,
- für Tetrahydropyranyl steht, oder
- für Dimethyl-tert.butylsilyl oder Trimethylsilyl steht, oder
- eine Gruppe -COR¹⁶ bedeutet,
wobei
R¹⁶ die oben angegebene Bedeutung hat,
und
R¹⁸ und R¹⁹ gleich oder verschieden sind und
- Wasserstoff bedeuten, oder
- gegebenenfalls durch Fluor oder Chlor substituiertes Methyl, Ethyl, Propyl, Tsopropyl, Butyl, Isobutyl, Pentyl, Isopentyl, Hexyl oder Isohexyl bedeuten, oder.
- Phenyl bedeuten, das durch Fluor, Chlor, Methyl oder Methoxy substituiert sein kann,
oder
D und E gemeinsam einen Ring der Formel 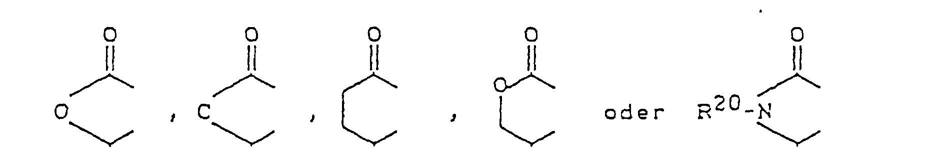 bilden,
worin
R²⁰ - für Wasserstoff, Methyl, Ethyl, Propyl, Isopropyl, Carbamoyl, Methoxy-carbonyl oder Ethoxycarbonyl steht,
X - für eine Gruppe der Formel -CH=CH- steht,
R - für eine Gruppe der Formel 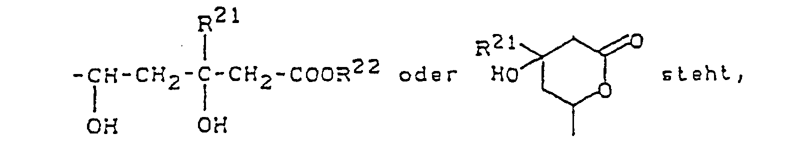 worin
R²¹ - Wasserstoff, Methyl, Ethyl, Propyl, Isopropyl, Butyl, Isobutyl oder tert.Butyl bedeutet,
und
R²² - Wasserstoff, Methyl, Ethyl, Propyl, Isopropyl, Butyl, Isobutyl, tert.Butyl oder Benzyl bedeutet, oder ein Natrium-, Kalium-, Calcium -, oder Magnesium- oder Ammoniumion bedeutet,
sowie von deren Oxidationsprodukten.

13. Verfahren gemäß Anspruch 11 zur Herstellung von Methylerythro-(E)-7-[2,6-diisopropyl-4-(4-fluorphenyl)-5-methoxy-methyl-pyrid-3-yl]-3,5-dihydroxy-hept-6-enoat der Formel

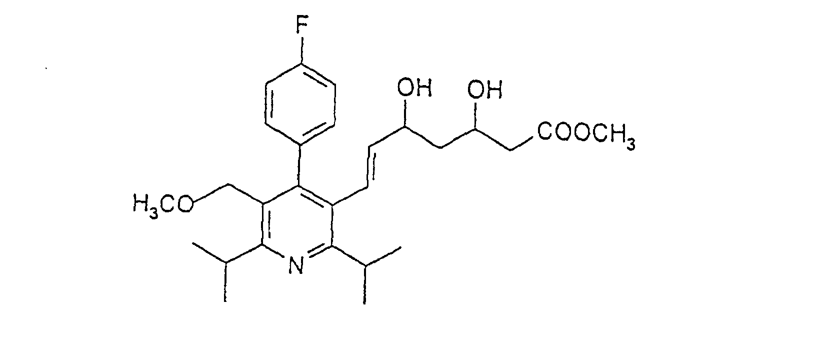

14. Verfahren gemäß einem der Ansprüche 11 bis 13, **dadurch gekennzeichnet, daß** man die Reduktion im Temperaturbereich von -80°C bis +30° C durchführt.

15. Verfahren zur Herstellung der Salze der Verbindungen der allgemeinen Formel (le) bzw. (Ie)' 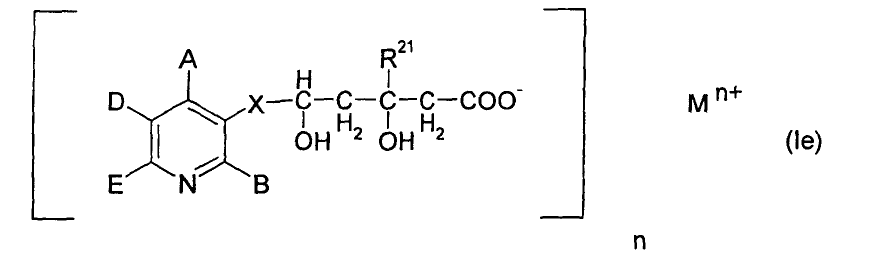 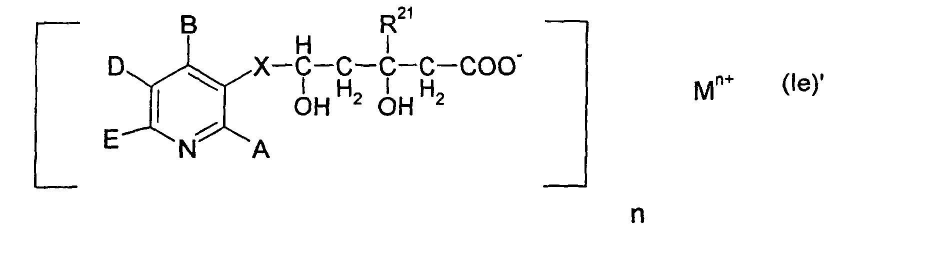 in welcher
A, B, D, E, X und R²¹ die in den Verbindungen der vorangehenden Ansprüchen 11 bis 13 angegebenen Bedeutungen haben und
Mⁿ⁺ für ein Kation steht, wobei n die Wertigkeit angibt,
**dadurch gekennzeichnet, dass** man die entsprechenden Ester oder Lactone in inerten Lösemitteln mit üblichen Basen behandelt.

16. Verfahren gemäß Anspruch 15 wobei als Base Natriumhydroxid oder Kaliumhydroxid eingesetzt werden.

17. Verfahren zur Herstellung von Carbonsäuren der allgemeinen Formel (Ic) bzw. (Ic)' 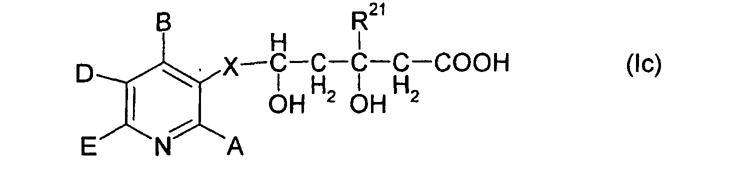 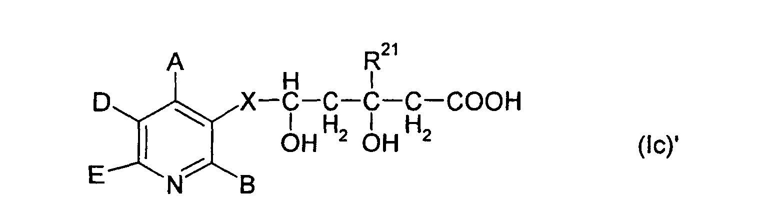 in welcher
A, B, D, E, X, und R²¹ die in den Verbindungen der vorangehenden Ansprüchen 11 bis 13 angegebenen Bedeutungen haben,
**dadurch gekennzeichnet, dass** man die entsprechenden Ester oder Lactone im ersten Schritt in inerten Lösemitteln mit üblichen Basen behandelt und die entstehenden Salze anschließend in einem zweiten Schritt durch Behandeln mit Säuren in die freien Säuren überführt.

## Claims

1. Substituted pyridines of the formula 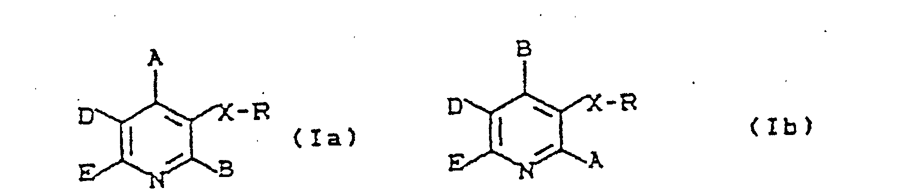 in which
A
- stands for thienyl, furyl, thiazolyl, iso-thiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzox- azolyl or benzimidazolyl, each of which can be monosubstituted or disubstituted by identical or different fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- stands for phenyl or naphthyl, each of which can be monosubstituted to tetrasubstituted by identical or different C₁-C₆-alkyl which can be optionally substituted by hydroxyl or C₁-C₆-alkoxy, C₁-C-₆-alkoxy, C₁-C-₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, or cyano,
- stands for methyl, ethyl, propyl, isopropyl, butyl or tert-butyl,
B
- stands for cyclopropyl, cyclopentyl or cyclohexyl, or
- stands for C₁-C₆-alkyl which can be substituted by fluorine, chlorine, bromine, cyano, C₁-C₆-alkoxy, C₁-C₆-alkylthio, C₁-C₆-alkylsulphonyl, trifluoromethyl, trifluoromethoxy, trifluoromethylsulphonyl, C₁-C₆- alkoxycarbonyl, benzoyl, C₁-C₆-alkylcarbonyl, or by a group of the formula -NR¹R²
wherein
R¹ and R² are identical or different and are C₁-C₆-alkyl, phenyl, benzyl, acetyl, benzoyl, phenylsulphonyl or C₁-C₆-alkylsulphonyl, or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the heteroaryl and aryl radicals mentioned can be monosubstituted or disubstituted by identical or different fluorine, chlorine, bromine, C₁-C₆-alkyl, C₁-C₆-alkoxy, trifluoromethyl or trifluoromethoxy,
D and E
- are identical or different and
- stand for cyclopropyl, cyclopentyl or cyclohexyl, or
- stand for straight-chain or branched C₁-C₆-alkyl which is substituted in the case of substituent D and can be substituted in the case of substituent E, by azido, fluorine, chlorine, bromine, cyano, hydroxyl, C₁-C₆-alkoxy, C₁-C₆-alkylthio, C₁-C₆-alkylsulphonyl, trifluoromethyl, trifluoromethoxy, trifluoromethylsulphonyl, C₁-C₆-alkoxycarbonyl, benzoyl, C₁-C₆-alkylcarbonyl, or by a group of the formula -NR¹R²
wherein R¹ and R² have the abovementioned meaning,
or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the heteroaryl and aryl radicals mentioned can be monosubstituted or disubstituted by identical or different fluorine, chlorine, bromine, C₁-C₆-alkyl, C₁-C₆-alkoxy, trifluoromethyl or trifluoromethoxy,
- stand for thienyl, furyl, thiazolyl, tetrazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl, each of which can be monosubstituted or disubstituted by identical or different fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- stand for naphthyl which can be monosubstituted to tetrasubstituted by identical or different C₁-C₆-alkyl which can be optionally substituted by hydroxyl or C₁-C₆-alkoxy, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR¹R²,
where
R¹ and R² have the abovementioned meaning,
or
- stand for a group of the formula -CR¹¹R¹²-Y,
wherein
R¹¹ and R¹² can be identical or different and
- stand for hydrogen or
- stand for C₁-C₆-alkyl which can be optionally substituted by hydroxyl, fluorine, chlorine, C₁-C₆-alkoxy or C₁-C₆-alkoxycarbonyl, or
- stand for cyclopropyl, cyclopentyl or cyclohexyl, or
R¹¹ and R¹² together form a saturated or unsaturated carboxylic or heterocyclic ring having up to 6 carbon atoms,
Y- denotes a group of the formula -NR¹³R¹⁴, -COR¹⁵, -S-R¹⁶, SO-R¹⁶, - SO₂R¹⁶, -OR¹⁷ or -N₃,
where
R¹³ and R¹⁴ are identical or different and
- stand for hydrogen, C₁-C₆-alkyl, phenyl or benzyl, where the radicals mentioned can be substituted by fluorine, chlorine, bromine, C₁-C₆-alkyl, C₁-C₆-alkoxy or trifluoromethyl, or
- stand for a group of the formula -COR¹⁵, -SO₂R¹⁶ or R¹³ and R¹⁴ together form an alkylene chain which can be interrupted by O, N, S, N-C₁-C₆-alkyl, N-benzyl, N-phenyl, N-carbamoyl or N-C₁-C₆-alkoxycarbonyl,
R¹⁵
- denotes a group -NR¹⁸R¹⁹, or
- denotes C₁-C₆-alkyl or C₁-C₆-alkoxy, or
- denotes phenyl, benzyl, benzyloxy, thienyl, furyl, pyridyl, pyrimidyl, quinolyl, isoquinolyl, benzothiazolyl, benzoxazolyl, thiazolyl, oxazolyl, isoxazolyl or isothiazolyl which are optionally substituted by C₁-C₆-alkyl, C₁-C₆-alkoxy, fluorine, chlorine, bromine, trifluoromethyl, dimethylamino or diethylamino,
R¹⁶
- denotes cyclopropyl, cyclopentyl, cyclohexyl, or
- denotes straight-chain or branched C₁-C₆-alkyl which is optionally substituted by cyano, fluorine, chlorine, bromine, trifluoromethyl or C₁-C₆-alkoxycarbonyl, or
- denotes phenyl, naphthyl, benzyl, thienyl, furyl, pyrimidyl, pyridyl, quinolyl, isoquinolyl, benzothiazolyl, benzoxazolyl, thiazolyl, oxazolyl, isoxazolyl or isothiazolyl which are optionally monosubstituted or polysubstituted by identical or different C₁-C₆-alkyl, C₁-C₆-alkoxy, fluorine, chlorine, bromine, trifluoromethyl, dimethylamino or diethylamino, or
- denotes trimethylsilyl or dimethylethylsilyl, or
- denotes a group -NR⁹R¹⁰
where
R⁹ and R¹⁰ have the abovementioned meaning,
R¹⁷
- stands for hydrogen, or
- stands for cyclopropyl, cyclopentyl or cyclohexyl, or
- stands for C₁-C₆-alkyl which can be substituted by fluorine, chlorine, bromine, cyano, C₁-C₆-alkoxy, C₁-C₆-alkylthio, C₁-C₆-alkylsulphonyl, trifluoromethyl, trifluoromethoxy, trifluoromethylsulphonyl, C₁-C₆-alkoxycarbonyl, benzoyl, C₁-C₆-alkylcarbonyl, or by a group of the formula -NR¹ R²,
wherein
R¹ and R² have the abovementioned meaning,
or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the heteroaryl and aryl radicals mentioned can be monosubstituted or disubstituted by identical or different fluorine, chlorine, bromine, C₁-C₆-alkyl, C₁-C₆-alkoxy, trifluoromethyl or trifluoromethoxy,
- stands for thienyl, furyl, thiazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl, each of which can be monosubstituted or disubstituted by identical or different fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- stands for benzyl, phenyl or naphthyl, each of which can be monosubstituted to tetrasubstituted by identical or different C₁-C₆-alkyl, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR¹ R²,
where
R¹ and R² have the abovementioned meaning,
or
- stand for 2,5-dioxotetrahydropyrryl,
- stand for tetrahydropyranyl, or
- stand for dimethyl-tert-butylsilyl, tripropylsilyl or tributylsilyl, or
- denotes a group of the formula COR¹⁶,
where
R¹⁶ has the abovementioned meaning,
and
R¹⁸ and R¹⁹ are identical or different
and
- denote hydrogen, or
- denote C₁-C₆-alkyl which is optionally substituted by cyano, fluorine, chlorine or bromine, or
- denote phenyl, benzyl, thienyl, furyl, pyridyl, pyrimidyl, quinolyl, isoquinolyl, benzothiazolyl, benzoxazolyl, thiazolyl, oxazolyl, isoxazolyl or isothiazolyl which are optionally substituted by C₁-C₆-alkyl, C₁-C₆-alkoxy, fluorine, chlorine, bromine, trifluoromethyl, dimethylamino or diethylamino,
or
D and E together form a ring of the formula 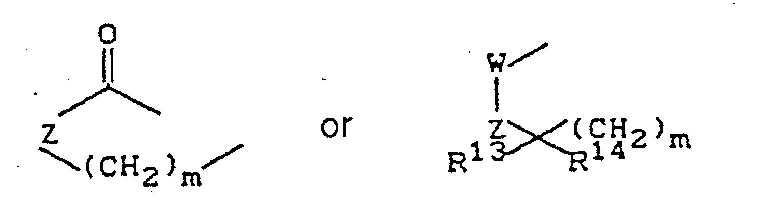 wherein
W - stands for a group of the formula C=O or for CH-OH,
m - stands for a number 1 or 2,
Z - stands for O, CH₂ or NHR²⁰,
R¹³ and R¹⁴ have the abovementioned meaning,
and
R²⁰ - stands for hydrogen, C₁-C₆-alkyl, phenyl, benzyl, carbamoyl or C₁-C₆-alkoxycarbonyl,
X - stands for a group of the formula -CH=CH-,
R - stands for a group of the formula 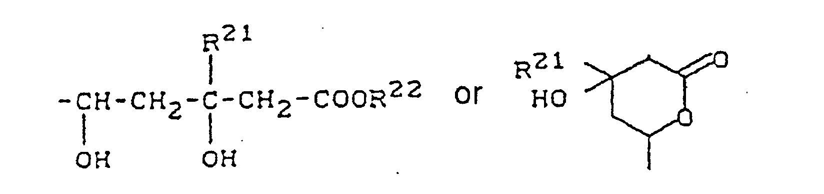 wherein
R²¹ - denotes hydrogen or C₁-C₆-alkyl,
and
R²²
- denotes hydrogen, C₁-C₆-alkyl, phenyl or benzyl, or
- denotes a cation,
and their oxidation products.

2. Substituted pyridines according to Claim 1,
in which
A
- stands for thienyl, furyl, pyridyl, pyrimidyl, quinolyl or isoquinolyl, each of which can be substituted by fluorine, chlorine, methyl, methoxy or trifluoromethyl, or
- stands for phenyl which can be monosubstituted, disubstituted or trisubstituted by identical or different methyl, hydroxymethyl, ethyl, propyl, isopropyl, hydroxyethyl, hydroxypropyl, butyl, isobutyl, methoxymethyl, ethoxymethyl, propoxymethyl, tert-butyl, methoxy, ethoxy, propoxy, isopropoxy, butoxy, isobutoxy, tert-butoxy, methylthio, ethylthio, propylthio, isopropylthio, methylsulphonyl, ethylsulphonyl, propylsulphonyl, isopropylsulphonyl, phenyl, phenoxy, benzyl, benzyloxy, fluorine, chlorine, bromine, cyano, trifluoromethyl, trifluoromethoxy, methoxycarbonyl, ethoxycarbonyl, propoxycarbonyl, isopropoxycarbonyl, butoxycarbonyl, isobutoxycarbonyl or tert-butoxycarbonyl,
- stands for methyl, ethyl, propyl, isopropyl, butyl or tert-butyl,
B
- stands for cyclopropyl, cyclopentyl or cyclohexyl, or
- stands for methyl, ethyl, propyl, isopropyl, butyl, sec-butyl or tert-butyl, each of which can be substituted by fluorine, chlorine, bromine, cyano, methoxy, ethoxy, propoxy, isopropoxy, butoxy, sec-butoxy, tert-butoxy, methylthio, ethylthio, propylthio, isopropylthio, methylsulphonyl, ethylsulphonyl, propylsulphonyl, isopropylsulphonyl, trifluoromethyl, trifluoromethoxy, methoxycarbonyl, ethoxycarbonyl, butoxycarbonyl, isobutoxycarbonyl, tert-butoxycarbonyl, benzoyl, acetyl, pyridyl, pyrimidyl, thienyl, furyl, phenyl, phenoxy, phenylthio, phenylsulphonyl, benzyloxy, benzylthio or benzylsulphonyl,
D and E
- are identical or different and
- stand for cyclopropyl, cyclopentyl or cyclohexyl, or
- stand for methyl, ethyl, propyl, isopropyl, butyl, isobutyl, tert-butyl, pentyl, isopentyl, hexyl or isohexyl, each of which is substituted in the case of substituent D and can be substituted in the case of substituent E by azido, fluorine, chlorine, bromine, iodine, cyano, hydroxyl, methoxy, ethoxy, propoxy, isopropoxy, butoxy, isobutoxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tert-butylsulphonyl, trifluoromethyl, trifluoromethoxy, methoxycarbonyl, ethoxycarbonyl, propoxycarbonyl, isopropoxycarbonyl, butoxycarbonyl, isobutoxycarbonyl, tert-butoxycarbonyl, benzoyl, acetyl, ethylcarbonyl, or by a group -NR¹R²,
where
R¹ and R² are identical or different and denote methyl, ethyl, propyl, isopropyl, butyl, isobutyl, tert-butyl, phenyl, benzyl, acetyl, methylsulphonyl, ethylsulphonyl, propylsulphonyl, isopropylsulphonyl or phenylsulphonyl, or by pyridyl, pyrimidyl, pyrazinyl, pyridazinyl, quinolyl, isoquinolyl, thienyl, furyl, phenyl, phenoxy, phenylthio, phenylsulphonyl, benzyloxy, benzylthio or benzylsulphonyl, where the heteroaryl and aryl radicals mentioned can be substituted by fluorine, chlorine, methyl, ethyl, propyl, isopropyl, isobutyl, tert-butyl, methoxy, ethoxy, propoxy, isopropoxy, butoxy, isobutoxy, tert-butoxy, trifluoromethyl or trifluoromethoxy, or
- stand for thienyl, furyl, pyridyl, pyrimidyl, pyrazinyl, tetrazolyl, pyridazinyl, oxazolyl, isooxazolyl, imidazolyl, pyrazolyl, thiazolyl, isothiazolyl, quinolyl, isoquinolyl, benzoxazolyl, benzimidazolyl or benzthiazolyl, where the radicals mentioned can be substituted by fluorine, chlorine, methyl, ethyl, propyl, isopropyl, butyl, isobutyl, tert-butyl, methoxy, ethoxy, propoxy, isopropoxy, butoxy, isobutoxy, tert-butoxy, phenyl, phenoxy, trifluoromethyl, trifluoromethoxy, methoxycarbonyl; ethoxycarbonyl, isopropoxycarbonyl, propoxycarbonyl, butoxycarbonyl, isobutoxycarbonyl or tert-butoxycarbonyl, or
- for a group of the formula -CR¹¹R¹²-Y,
wherein
R¹¹ and R¹² are identical or different and
- stand for hydrogen or
- stand for methyl, ethyl, propyl, isopropyl, each of which can be optionally substituted by hydroxyl, fluorine, chlorine, methoxy, ethoxy, methoxycarbonyl, ethoxycarbonyl,
- stand for cyclopropyl, cyclopentyl or cyclohexyl,
or R¹¹ and R¹² together stand for cyclopropyl, cyclopentyl or cyclohexyl,
and
Y - denotes a group of the formula -NR¹³R¹⁴, -COR¹⁵, -SR¹⁶, -SO-R¹⁶, -SO₂R¹⁶, -OR¹⁷ or -N₃,
where
R¹³ and R¹⁴ are identical or different, and
- stand for hydrogen, methyl, ethyl, propyl, isopropyl, butyl, isobutyl, or
- stand for phenyl which is optionally substituted by fluorine, chlorine, methyl or methoxy, or
- stand for a group -COR¹⁵ or -SO₂R¹⁶, or
R¹³ and R¹⁴ together with the nitrogen atom form a ring from the series comprising piperidine, piperazine, morpholine, morpholine-N-oxide, N-lower alkylpiperazine, benzylpiperazine and phenylpiperazine,
R¹⁵
- denotes hydrogen, or
- denotes a group -NR¹⁸R¹⁹, or
- denotes methyl, ethyl, propyl, isopropyl, methoxy, ethoxy, propoxy, isopropoxy, or
- denotes phenyl, benzyl, benzyloxy, thienyl, furyl, pyridyl, pyrimidyl, quinolyl or isoquinolyl which are optionally substituted by methyl, methoxy, fluorine or chlorine,
R¹⁶
- denotes methyl, ethyl, propyl, isopropyl, butyl, isobutyl or isopentyl which are optionally substituted by fluorine, chlorine, methoxycarbonyl, ethoxycarbonyl, propoxycarbonyl, isopropoxycarbonyl, butoxycarbonyl or isobutoxycarbonyl, or
- denotes benzyl, phenyl, naphthyl, thienyl, furyl, pyridyl, pyrimidyl, quinolyl or isoquinolyl which are optionally monosubstituted or polysubstituted by identical or different methyl, ethyl, propyl, isopropyl, methoxy, fluorine or chlorine, or
- denotes trimethylsilyl or dimethylethylsilyl,
- denotes a group -NR⁹R¹⁰
where
R⁹ and R¹⁰ have the abovementioned meaning,
R¹⁷
- stands for hydrogen, cyclopropyl, cyclopentyl or cyclohexyl, or
- stands for methyl, ethyl, propyl, isopropyl, butyl, isobutyl, tert-butyl, pentyl, isopentyl, hexyl or isohexyl, each of which can be substituted by fluorine, chlorine, bromine, cyano, methoxy, ethoxy, propoxy, isopropoxy, butoxy, isobutoxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tertbutylsulphonyl, trifluoromethyl, trifluoromethoxy, methoxycarbonyl, ethoxycarbonyl, propoxycarbonyl, isopropoxycarbonyl, butoxycarbonyl, isobutoxycarbonyl, tert-butoxycarbonyl, benzoyl, acetyl, ethylcarbonyl, or by a group -NR¹ R²,
where
R¹ and R² are identical or different and denote methyl, ethyl, propyl, isopropyl, butyl, isobutyl, tert-butyl, phenyl, benzyl, acetyl, methylsulphonyl, ethylsulphonyl, propylsulphonyl, isopropylsulphonyl or phenylsulphonyl,
or by pyridyl, pyrimidyl, pyrazinyl, pyridazinyl, quinolyl, isoquinolyl, thienyl, furyl, phenyl, phenoxy, phenylthio, phenylsulphonyl, benzyloxy, benzylthio or benzylsulphonyl, where the heteroaryl and aryl radicals mentioned can be substituted by fluorine, chlorine, methyl, ethyl, propyl, isopropyl, isobutyl, tert-butyl, methoxy, ethoxy, propoxy, isopropoxy, butoxy, isobutoxy, tert-butoxy, trifluoromethyl or trifluoromethoxy, or
- stands for thienyl, furyl, pyridyl, pyrimidyl, pyrazinyl, pyridazinyl, oxazolyl, isooxazolyl, imidazolyl, pyrazolyl, thiazolyl, isothiazolyl, quinolyl, isoquinolyl, benzoxazolyl, benzimidazolyl or benzthiazolyl, where the radicals mentioned can be substituted by fluorine, chlorine, methyl, ethyl, propyl, isopropyl, butyl, isobutyl, tert-butyl, methoxy, ethoxy, propoxy, isopropoxy, butoxy, isobutoxy, tertbutoxy, phenyl, phenoxy, trifluoromethyl, trifluoromethoxy, methoxycarbonyl, ethoxycarbonyl, isopropoxycarbonyl, propoxycarbonyl, butoxycarbonyl, isobutoxycarbonyl or tert-butoxycarbonyl, or
- stands for benzyl or phenyl, each of which can be monosubstituted, disubstituted or trisubstituted by identical or different methyl, ethyl, propyl, isopropyl, butyl, isobutyl, tert-butyl, pentyl, isopentyl, hexyl, isohexyl, methoxy, ethoxy, propoxy, isopropoxy, butoxy, isobutoxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tert-butylsulphonyl, phenyl, phenoxy, phenylthio, phenylsulphonyl, benzyl, benzyloxy, benzylthio, benzylsulphonyl, fluorine, chlorine, bromine, cyano, trifluoromethyl, trifluoromethoxy, trifluoromethylthio, methoxycarbonyl, ethoxycarbonyl, propoxycarbonyl, isopropoxycarbonyl, butoxycarbonyl, isobutoxycarbonyl, tert-butoxycarbonyl or by a group -NR¹R²,
where
R¹ and R² have the abovementioned meaning, or
- stands for 2,5-dioxotetrahydropyrryl,
- stands for tetrahydropyranyl, or
- stands for dimethyl-tert-butylsilyl or trimethylsilyl, or
- denotes a group -COR¹⁶,
where
R¹⁶ has the abovementioned meaning,
and
R¹⁸ and R¹⁹ are identical or different and
- denote hydrogen, or
- denote methyl, ethyl, propyl, isopropyl, butyl, isobutyl, pentyl, isopentyl, hexyl or isohexyl which are optionally substituted by fluorine or chlorine, or
- denote phenyl which can optionally be substituted by fluorine, chlorine, methyl or methoxy,
or
D and E together form a ring of the formula 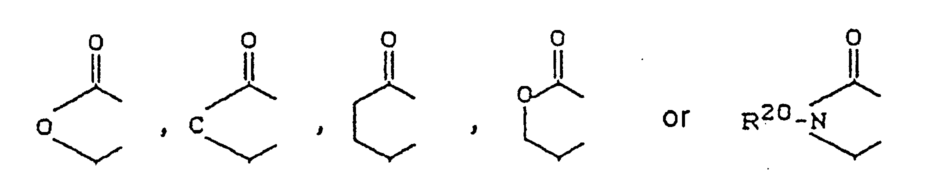 wherein
R²⁰ - stands for hydrogen, methyl, ethyl, propyl, isopropyl, carbamoyl, methoxycarbonyl or ethoxycarbonyl,
X - stands for a group of the formula -CH=CH-,
R - stands for a group of the formula 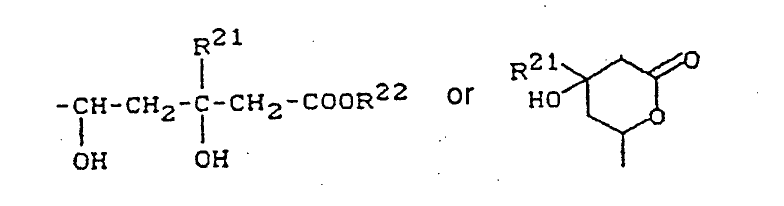
wherein
R²¹ - denotes hydrogen, methyl, ethyl, propyl, isopropyl, butyl, isobutyl or tert-butyl, and
R²² - denotes hydrogen, methyl, ethyl, propyl, isopropyl, butyl, isobutyl, tert-butyl or benzyl, or a sodium, potassium, calcium, magnesium or ammonium ion,
and their oxidation products.

3. Compounds of the general formulae 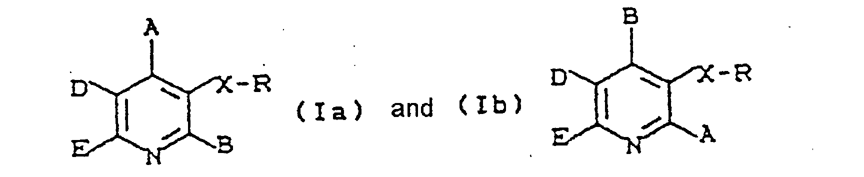 in which
A
- stands for thienyl or furyl,
- stands for phenyl which can be monosubstituted or disubstituted by identical or different methyl, hydroxymethyl, ethyl, propyl, isopropyl, butyl, isobutyl, tert-butyl, methoxy, ethoxy, propoxy, isopropoxy, phenoxy, benzyloxy, fluorine, chlorine hydroxymethyl, trifluoromethyl,
- stands for methyl, ethyl, propyl or isopropyl,
B
- stands for cyclopropyl, methyl, ethyl, propyl, isopropyl, butyl, isobutyl or tert-butyl, each of which can be substituted by fluorine, chlorine, methoxy, phenyl or phenoxy,
D and E are identical or different and
- stand for cyclopropyl, cyclopentyl or cyclohexyl, or
- stand for methyl, ethyl, propyl, isopropyl, butyl, isobutyl, tert-butyl, pentyl, isopentyl, hexyl or isohexyl, each of which is substituted in the case of substituent D and can be substituted in the case of substituent E by azido, fluorine, chlorine, iodine, methoxy, ethoxy, propoxy, isopropoxy, methylthio, ethylthio, methylsulphonyl, ethylsulphonyl, ethoxycarbonyl, propoxycarbonyl, isopropoxycarbonyl or by a group of the formula NR¹R²,
where
R¹ and R² are identical or different and
- stand for hydrogen, methyl, ethyl, propyl, isopropyl, phenyl or benzyl,
or by pyridyl, pyrimidyl, quinolyl, thienyl, furyl, phenyl, phenoxy, phenylsulphonyl or benzyloxy which are optionally substituted by fluorine, chlorine, methyl, methoxy, trifluoromethyl or trifluoromethoxy, or
- stand for thienyl, furyl, pyridyl, pyrimidyl, quinolyl, isoquinolyl, benzoxazolyl, tetrazolyl, benzthiazolyl or benzimidazolyl which are optionally substituted by fluorine, chlorine, methyl, ethyl, propyl, isopropyl, methoxy, phenyl, methoxycarbonyl or ethoxycarbonyl, or
- stand for a group of the formula -CR¹¹R¹²-Y,
wherein
R¹¹ and R¹²- denote hydrogen, methyl or ethyl
and
Y - denotes a group of the formula -NR¹³R¹⁴, -COR¹⁵, -S-R¹⁶, -SO-R¹⁶, -SO₂R¹⁶ or -OR¹⁷,
where
R¹³ and R¹⁴ are identical or different, and
- stand for hydrogen, methyl, ethyl, propyl, or
- stand for a group -COR¹⁵ or -SO₂R¹⁶
or R¹³ and R¹⁴ together with the nitrogen atom form a ring from the series comprising morpholine and morpholine-N-oxide,
and
R¹⁵
- denotes hydrogen or methyl, or
- denotes a group -NR¹⁸R¹⁸, or
- denotes methyl, ethyl, propyl, methoxy or ethoxy,
R¹⁶
- denotes trifluoromethyl, methyl, ethyl, propyl, isopropyl, butyl, isobutyl, isopentyl or benzyl, or
- denotes phenyl or naphthyl which is optionally substituted by one or more methyl or chlorine,
- denotes trimethylsilyl or dimethlethylsilyl, or
- denotes a group -NR⁹R¹⁰,
where
R⁹ and R¹⁰ have the abovementioned meaning,
R¹⁷
- stands for hydrogen, cyclopropyl, cyclopentyl or cyclohexyl, or
- stands for methyl, ethyl, propyl, isopropyl, butyl, isobutyl, tert-butyl, pentyl, isopentyl, hexyl or isohexyl, each of which can be substituted by fluorine, chlorine, methoxy, ethoxy, propoxy, isopropoxy, methylthio, ethylthio, methylsulphonyl, ethylsulphonyl, ethoxycarbonyl, propoxycarbonyl, isopropoxycarbonyl or by a group of the formula NR¹R²,
where
R¹ and R² are identical or different and
- stand for hydrogen, methyl, ethyl, propyl, isopropyl, phenyl or benzyl,
or by pyridyl, pyrimidyl, quinolyl, thienyl, furyl, phenyl, phenoxy, phenylsulphonyl or benzyloxy, which are optionally substituted by fluorine, chlorine, methyl, methoxy, trifluoromethyl or trifluoromethoxy, or
- stand for thienyl, furyl, pyridyl, pyrimidyl, quinolyl, isoquinolyl, enzoxazolyl, benzthiazolyl or benzimidazolyl which are optionally substituted by fluorine, chlorine, methyl, ethyl, propyl, isopropyl, methoxy, phenyl, methoxycarbonyl or ethoxycarbonyl, or
- stands for benzyl or phenyl, each of which can be mono-substituted or disubstituted by identical or different methyl, ethyl, propyl, isopropyl, butyl, isobutyl, tert-butoxy, methoxy, ethoxy, propoxy, isopropoxy, methylthio, ethylthio, methylsulphonyl, ethylsulphonyl, phenyl, phenoxy, phenylsulphonyl, benzyloxy, fluorine, chlorine, bromine, cyano, trifluoromethyl, trifluoromethoxy, methoxycarbonyl, ethoxycarbonyl or a group of the formula -NR¹R²,
where
R¹ and R² have the abovementioned meaning, or
- stand for 2,5-dioxotetrahydropyrryl,
- stands for tetrahydropyranyl, or
- stands for dimethyl-tert-butylsilyl or trimethylsilyl, or
- denotes a group -COR¹⁶
where
R¹⁶ has the abovementioned meaning,
and
R¹⁸ and ¹⁹ are identical or different and
- denote hydrogen, or
- denote methyl, ethyl, propyl, isopropyl, butyl or isobutyl, or
- denote phenyl,
or
D and E together form a ring of the formula 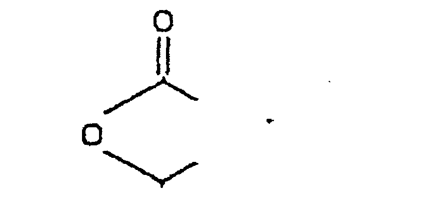 X - stands for a group of the formula 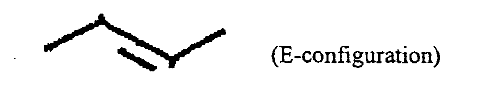 and
R - stands for a group of the formula 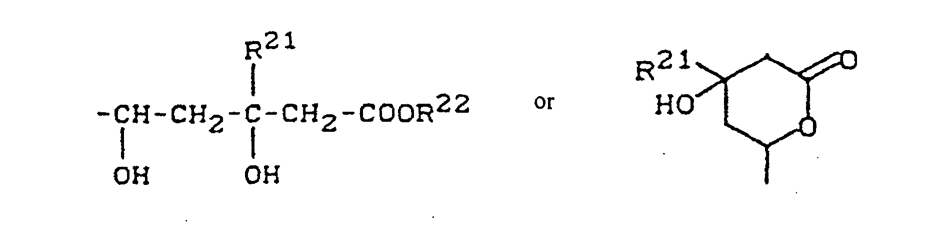 wherein
R²¹ - denotes hydrogen
and
R²² - denotes hydrogen, methyl or ethyl, or a sodium or potassium ion,
and their oxidation products.

4. Methyl-erythro-(E)-7-[2,6-diisopropyl-4-(4-fluorophenyl)-5-methoxymethylpyrid-3-yl]-3,5-dihydroxyhept-6-enoate of the formula

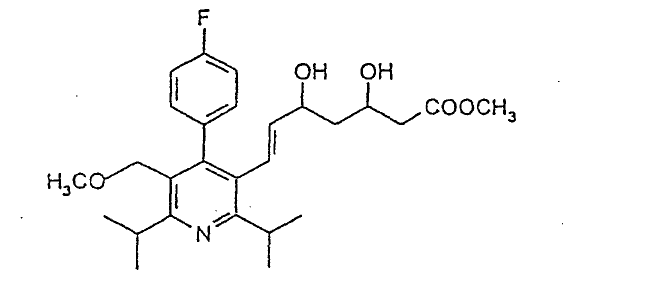

5. Salts of the compounds of the general formula (Ie) or (Ie)' 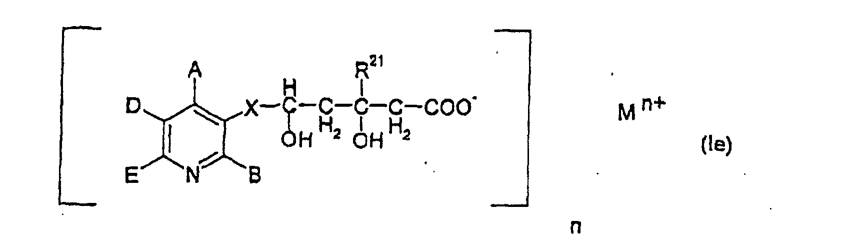 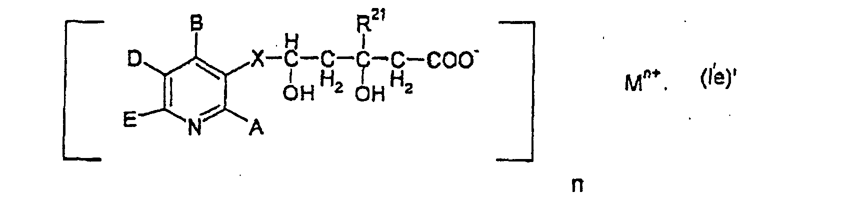 in which
A, B, D, E, X and R²¹ have the meanings given in the compounds of the preceding Claims 1 to 5, and
Mⁿ⁺ stands for a cation, n specifying the valency.

6. Salts according to Claim 5 where Mⁿ⁺ stands for a sodium or potassium ion.

7. Carboxylic acids of the general formula (Ic) or (Ic)' 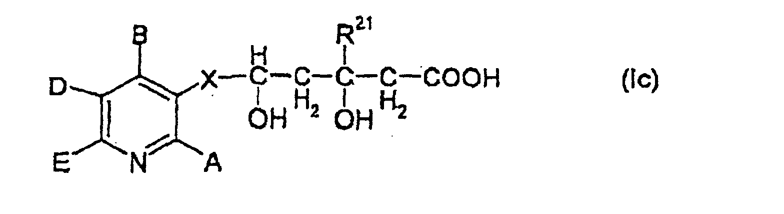 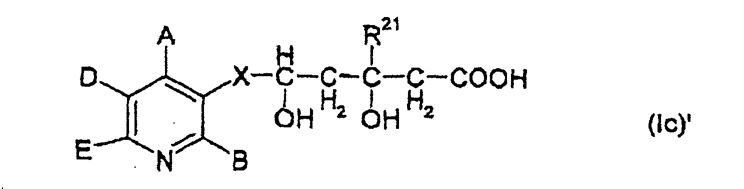 in which
A, B, D, E, X and R²¹ have the meanings given in the compounds of the preceding Claims 1 to 4.

8. Ketones of the formula (VIIIa) or (VIIIb) 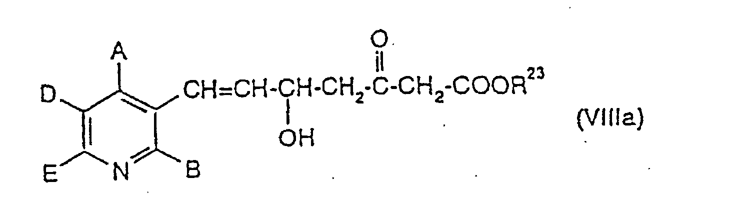 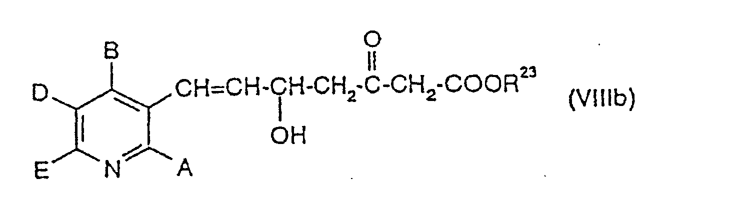 in which
A, B, D and E have the meanings given in the preceding claims, and
R²³ stands for ethyl.

9. Process for preparing ketones of the formula (VIIIa) or (VIIIb) which are as defined in Claim 8, which process is **characterized in that** aldehydes of the general formula (IX) 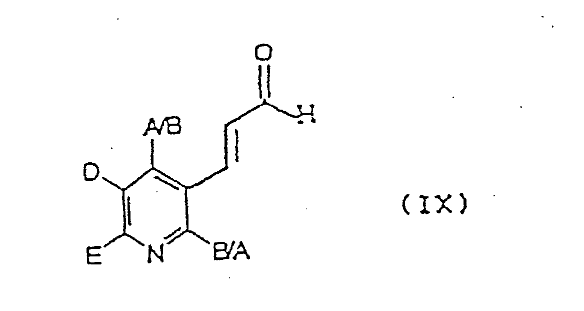 in which
A, B, D and E have the meanings given in the preceding claims, are reacted
in inert solvents with acetoacetates of the general formula (X) 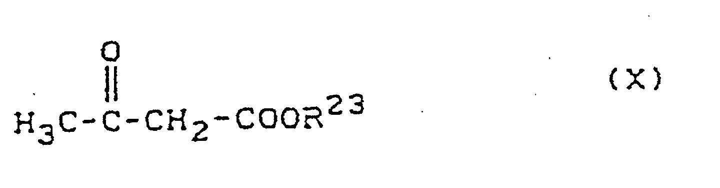 in which
R²³ has the meaning given in Claim 9,
in the presence of bases.

10. Process according to Claim 9, **characterized in that** the reaction is carried out in a temperature range from -80°C to +50°C.

11. Process for preparing substituted pyridines of the formula 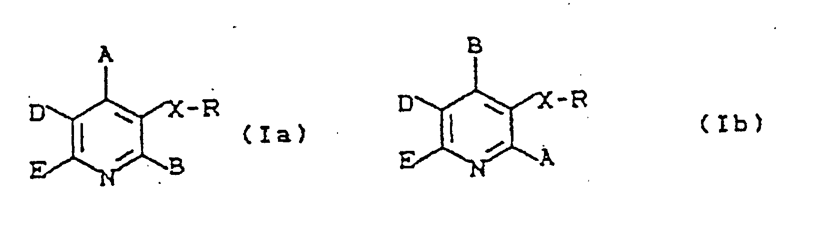 in which
A
- stands for thienyl, furyl, thiazolyl, iso-thiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl, each of which can be monosubstituted or disubstituted by identical or different fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- stands for phenyl or naphthyl, each of which can be monosubstituted to tetrasubstituted by identical or different C₁-C₆-alkyl which can be optionally substituted by hydroxyl or C₁-C₆-alkoxy, C₁-C-₆-alkoxy, C₁-C-₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, or cyano,
- stands for methyl, ethyl, propyl, isopropyl, butyl or tert-butyl,
B
- stands for cyclopropyl, cyclopentyl or cyclohexyl, or
- stands for C₁-C₆-alkyl which can be substituted by fluorine, chlorine, bromine, cyano, C₁-C₆-alkoxy, C₁-C₆-alkylthio, C₁-C₆-alkylsulphonyl, trifluoromethyl, trifluoromethoxy, trifluoromethylsulphonyl, C₁-C₆-alkoxycarbonyl, benzoyl, C₁-C₆-alkylcarbonyl, or by a group of the formula -NR¹R²
wherein
R¹ and R² are identical or different and are C₁-C₆-alkyl, phenyl, benzyl, acetyl, benzoyl, phenylsulphonyl or C₁-C₆-alkylsulphonyl, or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the heteroaryl and aryl radicals mentioned can be monosubstituted or disubstituted by identical or different fluorine, chlorine, bromine, C₁-C₆-alkyl, C₁-C₆-alkoxy, trifluoromethyl or trifluoromethoxy,
D and E
- are identical or different and
- stand for cyclopropyl, cyclopentyl or cyclohexyl, or
- stand for straight-chain or branched C₁-C₆-alkyl which is substituted in the case of substituent D and can be substituted in the case of substituent E, by azido, fluorine, chlorine, bromine, cyano, hydroxyl, C₁-C₆-alkoxy, C₁-C₆-alkylthio, C₁-C₆-alkylsulphonyl, trifluoromethyl, trifluoromethoxy, trifluoromethylsulphonyl, C₁-C₆-alkoxycarbonyl, benzoyl, C₁-C₆-alkylcarbonyl, or by a group of the formula -NR¹R²
wherein
R¹ and R² have the abovementioned meaning, or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the heteroaryl and aryl radicals mentioned can be monosubstituted or disubstituted by identical or different fluorine, chlorine, bromine, C₁-C₆-alkyl, C₁-C₆-alkoxy, trifluoromethyl or trifluoromethoxy,
- stand for thienyl, furyl, thiazolyl, tetrazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl, each of which can be monosubstituted or disubstituted by identical or different fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- stand for naphthyl which can be monosubstituted to tetrasubstituted by identical or different C₁-C₆-alkyl which can be optionally substituted by hydroxyl or C₁-C₆-alkoxy, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR¹R²,
where
R¹ and R² have the abovementioned meaning,
or
- stand for a group of the formula -CR¹¹R¹²-Y,
wherein
R¹¹ and R¹² can be identical or different and
- stand for hydrogen or
- stand for C₁-C₆-alkyl which can be optionally substituted by hydroxyl, fluorine, chlorine, C₁-C₆-alkoxy or C₁-C₆-alkoxycarbonyl, or
- stand for cyclopropyl, cyclopentyl or cyclohexyl, or
R¹¹ and R¹² together form a saturated or unsaturated carboxylic or heterocyclic ring having up to 6 carbon atoms,
Y- denotes a group of the formula -NR¹³R¹⁴, -COR¹⁵, -S-R¹⁶, SO-R¹⁶, - SO₂R¹⁶, -OR¹⁷ or -N₃,
where
R¹³ and R¹⁴ are identical or different and
- stand for hydrogen, C₁-C₆-alkyl, phenyl or benzyl, where the radicals mentioned can be substituted by fluorine, chlorine, bromine, C₁-C₆-alkyl, C₁-C₆-alkoxy or trifluoromethyl, or
- stand for a group of the formula -COR¹⁵, -SO₂R¹⁶ or R¹³ and R¹⁴ together form an alkylene chain which can be interrupted by O, N, S, N-C₁-C₆-alkyl, N-benzyl, N-phenyl, N-carbamoyl or N-C₁-C₆-alkoxycarbonyl,
R¹⁵
- denotes a group -NR¹⁸R¹⁹, or
- denotes C₁-C₆-alkyl or C₁-C₆-alkoxy, or
- denotes phenyl, benzyl, benzyloxy, thienyl, furyl, pyridyl, pyrimidyl, quinolyl, isoquinolyl, benzothiazolyl, benzoxazolyl, thiazolyl, oxazolyl, isoxazolyl or isothiazolyl which are optionally substituted by C₁-C₆-alkyl, C₁-C₆-alkoxy, fluorine, chlorine, bromine, trifluoromethyl, dimethylamino or diethylamino,
R¹⁶
- denotes cyclopropyl, cyclopentyl, cyclohexyl, or - denotes straight-chain or branched C₁-C₆-alkyl which is optionally substituted by cyano, fluorine, chlorine, bromine, trifluoromethyl or C₁-C₆-alkoxycarbonyl, or
- denotes phenyl, naphthyl, benzyl, thienyl, furyl, pyrimidyl, pyridyl, quinolyl, isoquinolyl, benzothiazolyl, benzoxazolyl, thiazolyl, oxazolyl, isoxazolyl or isothiazolyl which are optionally monosubstituted or polysubstituted by identical or different C₁-C₆-alkyl, C₁-C₆-alkoxy, fluorine, chlorine, bromine, trifluoromethyl, dimethylamino or diethylamino, or
- denotes trimethylsilyl or dimethylethylsilyl, or
- denotes a group -NR⁹R¹⁰
where
R⁹ and R¹⁰ have the abovementioned meaning,
R¹⁷ - stands for hydrogen, or
- stands for cyclopropyl, cyclopentyl or cyclohexyl, or
- stands for C₁-C₆-alkyl which can be substituted by fluorine, chlorine, bromine, cyano, C₁-C₆-alkoxy, C₁-C₆-alkylthio, C₁-C₆-alkylsulphonyl, trifluoromethyl, trifluoromethoxy, trifluoromethylsulphonyl, C₁-C₆-alkoxycarbonyl, benzoyl, C₁-C₆-alkylcarbonyl, or by a group of the formula -NR¹ R²,
wherein
R¹ and R² have the abovementioned meaning,
or by pyridyl, pyrimidyl, pyrazinyl, pyridazinyl, quinolyl, isoquinolyl, pyrrolyl, indolyl, thienyl, furyl, imidazolyl, oxazolyl, thiazolyl, phenyl, phenoxy, phenylthio, phenylsulphonyl, benzyloxy, benzylthio, benzylsulphonyl, phenylethoxy, phenylethylthio or phenylethylsulphonyl, where the heteroaryl and aryl radicals mentioned can be monosubstituted or disubstituted by identical or different fluorine, chlorine, bromine, C₁-C₆-alkyl, C₁-C₆-alkoxy, trifluoromethyl or trifluoromethoxy,
- stands for thienyl, furyl, thiazolyl, isothiazolyl, oxazolyl, isoxazolyl, pyridyl, pyrimidyl, pyrazinyl, pyridazinyl, indolyl, isoindolyl, quinolyl, isoquinolyl, phthalazinyl, quinoxalinyl, quinazolinyl, cinnolinyl, benzothiazolyl, benzoxazolyl or benzimidazolyl, each of which can be monosubstituted or disubstituted by identical or different fluorine, chlorine, bromine, C₁-C₆-alkyl, C₁-C₆-alkoxy, phenyl, phenoxy, trifluoromethyl, trifluoromethoxy or C₁-C₆-alkoxycarbonyl, or
- stands for benzyl, phenyl or naphthyl, each of which can be monosubstituted to tetrasubstituted by identical or different C₁-C₆-alkyl, C₁-C₆-alkoxy, C₁-C₆-alkylthio, C₁-C₆-alkylsulphonyl, phenyl, phenyloxy, phenylthio, phenylsulphonyl, benzyl, benzyloxy, benzylthio, benzylsulphonyl, phenethyl, phenylethoxy, phenylethylthio, phenylethylsulphonyl, fluorine, chlorine, bromine, cyano, trifluoromethyl, trifluoromethoxy, trifluoromethylthio, C₁-C₆-alkoxycarbonyl or by a group of the formula -NR¹ R²,
where
R¹ and R² have the abovementioned meaning, or
- stand for 2,5-dioxotetrahydropyrryl,
- stand for tetrahydropyranyl, or
- stand for dimethyl-tert-butylsilyl, tripropylsilyl or tributylsilyl, or
- denotes a group of the formula COR¹⁶,
where
R¹⁶ has the abovementioned meaning,
and
R¹⁸ and R¹⁹ are identical or different and
- denote hydrogen, or
- denote C₁-C₆-alkyl which is optionally substituted by cyano, fluorine, chlorine or bromine, or
- denote phenyl, benzyl, thienyl, furyl, pyridyl, pyrimidyl, quinolyl, isoquinolyl, benzothiazolyl, benzoxazolyl, thiazolyl, oxazolyl, isoxazolyl or isothiazolyl which are optionally substituted by C₁-C₆-alkyl, C₁-C₆-alkoxy, fluorine, chlorine, bromine, trifluoromethyl, dimethylamino or diethylamino,
or
D and E together form a ring of the formula 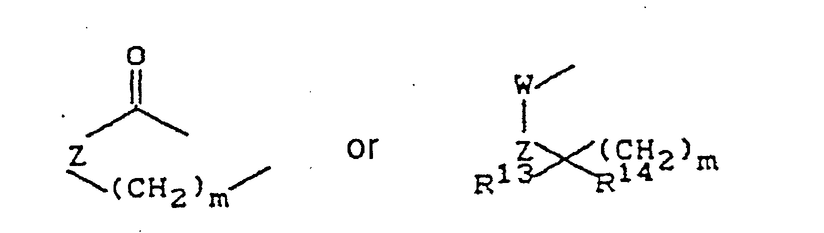 wherein
W - stands for a group of the formula C=O or for CH-OH,
m - stands for a number 1 or 2,
Z - stands for O, CH₂ or NHR²⁰,
R¹³ and R¹⁴ have the abovementioned meaning;
and
R²⁰ - stands for hydrogen, C₁-C₆-alkyl, phenyl, benzyl, carbamoyl or C₁-C₆-alkoxycarbonyl,
X - stands for a group of the formula -CH=CH-,
R - stands for a group of the formula 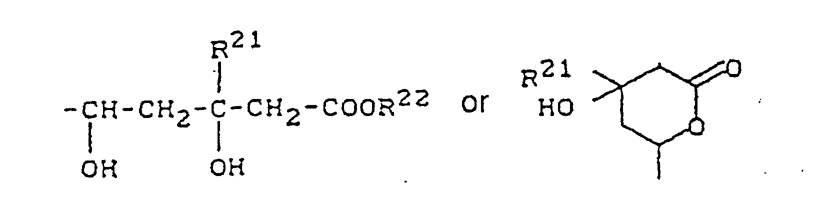 wherein
R²¹ - denotes hydrogen or C₁-C₆-alkyl,
and
R²²
- denotes hydrogen, C₁-C₆-alkyl, phenyl or benzyl, or
- denotes a cation,
and their oxidation products,
**characterized in that**
ketones of the general formula (VIIIa,b) 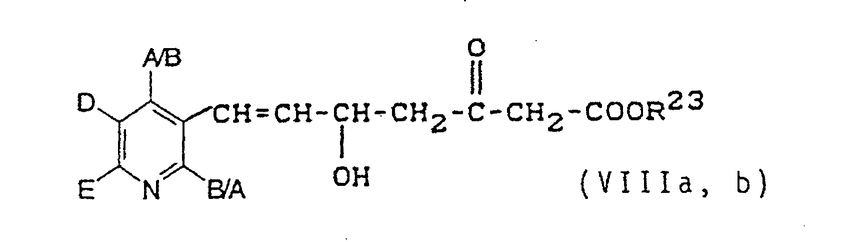 in which
A, B, D, E have the given meaning, and
R²³ stands for C₁-C₆-alkyl
are reduced,
in the case of the preparation of the acids, the esters are hydrolysed,
in the case of the preparation of the lactones, the carboxylic acids are cyclized,
in the case of the preparation of the salts, either the esters or the lactones are hydrolysed.

12. Process according to Claim 11 for preparing substituted pyridines of the formula (Ia) or (Ib)
in which
A
- stands for thienyl, furyl, pyridyl, pyrimidyl, quinolyl or isoquinolyl, each of which can be substituted by fluorine, chlorine, methyl, methoxy or trifluoromethyl, or
- stands for phenyl which can be monosubstituted, disubstituted or trisubstituted by identical or different methyl, hydroxymethyl, ethyl, propyl, isopropyl, hydroxyethyl, hydroxypropyl, butyl, isobutyl, methoxymethyl, ethoxymethyl, propoxymethyl, tert-butyl, methoxy, ethoxy, propoxy, isopropoxy, butoxy, isobutoxy, tert-butoxy, methylthio, ethylthio, propylthio, isopropylthio, methylsulphonyl, ethylsulphonyl, propylsulphonyl, isopropylsulphonyl, phenyl, phenoxy, benzyl, benzyloxy, fluorine, chlorine, bromine, cyano, trifluoromethyl, trifluoromethoxy, methoxycarbonyl, ethoxycarbonyl, propoxycarbonyl, isopropoxycarbonyl, butoxycarbonyl, isobutoxycarbonyl or tert-butoxycarbonyl,
- stands for methyl, ethyl, propyl, isopropyl, butyl or tert-butyl,
B
- stands for cyclopropyl, cyclopentyl or cyclohexyl, or
- stands for methyl, ethyl, propyl, isopropyl, butyl, sec-butyl or tert-butyl, each of which can be substituted by fluorine, chlorine.bromine, cyano, methoxy, ethoxy, propoxy, isopropoxy, butoxy, sec-butoxy, tert-butoxy, methylthio, ethylthio, propylthio, isopropylthio, methylsulphonyl, ethylsulphonyl, propylsulphonyl, isopropylsulphonyl, trifluoromethyl, trifluoromethoxy, methoxycarbonyl, ethoxycarbonyl, butoxycarbonyl, isobutoxycarbonyl, tert-butoxycarbonyl, benzoyl, acetyl, pyridyl, pyrimidyl, thienyl, furyl, phenyl, phenoxy, phenylthio, phenylsulphonyl, benzyloxy, benzylthio or benzylsulphonyl,
D and E
- are identical or different and
- stand for cyclopropyl, cyclopentyl or cyclohexyl, or
- stand for methyl, ethyl, propyl, isopropyl, butyl, isobutyl, tert-butyl, pentyl, isopentyl, hexyl or isohexyl, each of which is substituted in the case of substituent D and can be substituted in the case of substituent E by azido, fluorine, chlorine, bromine, iodine, cyano, hydroxyl, methoxy, ethoxy, propoxy, isopropoxy, butoxy, isobutoxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tert-butylsulphonyl, trifluoromethyl, trifluoromethoxy, methoxycarbonyl, ethoxycarbonyl, propoxycarbonyl, isopropoxycarbonyl, butoxycarbonyl, isobutoxycarbonyl, tert-butoxycarbonyl, benzoyl, acetyl, ethylcarbonyl, or by a group -NR¹R²,
where
R¹ and R² are identical or different and denote methyl, ethyl, propyl, isopropyl, butyl, isobutyl, tert-butyl, phenyl, benzyl, acetyl, methylsulphonyl, ethylsulphonyl, propylsulphonyl, isopropylsulphonyl or phenylsulphonyl,
or by pyridyl, pyrimidyl, pyrazinyl, pyridazinyl, quinolyl, isoquinolyl, thienyl, furyl, phenyl, phenoxy, phenylthio, phenylsulphonyl, benzyloxy, benzylthio or benzylsulphonyl, where the heteroaryl and aryl radicals mentioned can be substituted by fluorine, chlorine, methyl, ethyl, propyl, isopropyl, isobutyl, tert-butyl, methoxy, ethoxy, propoxy, isopropoxy, butoxy, isobutoxy, tert-butoxy, trifluoromethyl or trifluoromethoxy, or
- stand for thienyl, furyl, pyridyl, pyrimidyl, pyrazinyl, tetrazolyl, pyridazinyl, oxazolyl, isooxazolyl, imidazolyl, pyrazolyl, thiazolyl, isothiazolyl, quinolyl, isoquinolyl, benzoxazolyl, benzimidazolyl or benzthiazolyl, where the radicals mentioned can be substituted by fluorine, chlorine, methyl, ethyl, propyl, isopropyl, butyl, isobutyl, tert-butyl, methoxy, ethoxy, propoxy, isopropoxy, butoxy, isobutoxy, tert-butoxy, phenyl, phenoxy, trifluoromethyl, trifluoromethoxy, methoxycarbonyl; ethoxycarbonyl, isopropoxycarbonyl, propoxycarbonyl, butoxycarbonyl, isobutoxycarbonyl or tert-butoxycarbonyl, or
- for a group of the formula -CR¹¹R¹²-Y,
wherein
R¹¹ and R¹² are identical or different and
- stand for hydrogen or
- stand for methyl, ethyl, propyl, isopropyl, each of which can be optionally substituted by hydroxyl, fluorine, chlorine, methoxy, ethoxy, methoxycarbonyl, ethoxycarbonyl,
- stand for cyclopropyl, cyclopentyl or cyclohexyl,
or R¹¹ and R¹² together stand for cyclopropyl, cyclopentyl or cyclohexyl,
and
Y - denotes a group of the formula -NR¹³R¹⁴, -COR¹⁵, -SR¹⁶, -SO-R¹⁶, -SO₂R¹⁶,-OR¹⁷ or -N₃,
where
R¹³ and R¹⁴ are identical or different, and
- stand for hydrogen, methyl, ethyl, propyl, isopropyl, butyl, isobutyl, or
- stand for phenyl which is optionally substituted by fluorine, chlorine, methyl or methoxy, or
- stand for a group -COR¹⁵ or -SO₂R¹⁶, or
R¹³ and R¹⁴ together with the nitrogen atom form a ring from the series comprising piperidine, piperazine, morpholine, morpholineN-oxide, N-lower alkylpiperazine, benzylpiperazine and phenylpiperazine,
R¹⁵
- denotes hydrogen, or
- denotes a group -NR¹⁸R¹⁹, or
- denotes methyl, ethyl, propyl, isopropyl, methoxy, ethoxy, propoxy, isopropoxy, or
- denotes phenyl, benzyl, benzyloxy, thienyl, furyl, pyridyl, pyrimidyl, quinolyl or isoquinolyl which are optionally substituted by methyl, methoxy, fluorine or chlorine,
R¹⁶
- denotes methyl, ethyl, propyl, isopropyl, butyl, isobutyl or isopentyl which are optionally substituted by fluorine, chlorine, methoxycarbonyl, ethoxycarbonyl, propoxycarbonyl, isopropoxycarbonyl, butoxycarbonyl or isobutoxycarbonyl, or
- denotes benzyl, phenyl, naphthyl, thienyl, furyl, pyridyl, pyrimidyl, quinolyl or isoquinolyl which are optionally monosubstituted or polysubstituted by identical or different methyl, ethyl, propyl, isopropyl, methoxy, fluorine or chlorine, or
- denotes trimethylsilyl or dimethylethylsilyl,
- denotes a group -NR⁹R¹⁰
where
R⁹ and R¹⁰ have the abovementioned meaning,
R¹⁷
- stands for hydrogen, cyclopropyl, cyclopentyl or cyclohexyl, or
- stands for methyl, ethyl, propyl, isopropyl, butyl, isobutyl, tert-butyl, pentyl, isopentyl, hexyl or isohexyl, each of which can be substituted by fluorine, chlorine, bromine, cyano, methoxy, ethoxy, propoxy, isopropoxy, butoxy, isobut-oxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tert-butylsulphonyl, trifluoromethyl, trifluoromethoxy, methoxycarbonyl, ethoxycarbonyl, propoxycarbonyl, isopropoxycarbonyl, butoxycarbonyl, isobutoxycarbonyl, tert-butoxycarbonyl, benzoyl, acetyl, ethylcarbonyl, or by a group -NR¹R²,
where
R¹ and R² are identical or different and denote methyl, ethyl, propyl, isopropyl, butyl, isobutyl, tert-butyl, phenyl, benzyl, acetyl, methylsulphonyl, ethylsulphonyl, propylsulphonyl, isopropylsulphonyl or phenylsulphonyl,
or by pyridyl, pyrimidyl, pyrazinyl, pyridazinyl, quinolyl, isoquinolyl, thienyl, furyl, phenyl, phenoxy, phenylthio, phenylsulphonyl, benzyloxy, benzylthio or benzylsulphonyl, where the heteroaryl and aryl radicals mentioned can be substituted by fluorine, chlorine, methyl, ethyl, propyl, isopropyl, isobutyl, tert-butyl, methoxy, ethoxy, propoxy, isopropoxy, butoxy, isobutoxy, tert-butoxy, trifluoromethyl or trifluoromethoxy, or
- stands for thienyl, furyl, pyridyl, pyrimidyl, pyrazinyl, pyridazinyl, oxazolyl, isooxazolyl, imidazolyl, pyrazolyl, thiazolyl, isothiazolyl, quinolyl, isoquinolyl, benzoxazolyl, benzimidazolyl or benzthiazolyl, where the radicals mentioned can be substituted by fluorine, chlorine, methyl, ethyl, propyl, isopropyl, butyl, isobutyl, tert-butyl, methoxy, ethoxy, propoxy, isopropoxy, butoxy, isobutoxy, tert-butoxy, phenyl, phenoxy, trifluoromethyl, trifluoromethoxy, methoxycarbonyl, ethoxycarbonyl, isopropoxycarbonyl, propoxycarbonyl, butoxycarbonyl, isobutoxycarbonyl or tert-butoxycarbonyl, or
- stands for benzyl or phenyl, each of which can be monosubstituted, disubstituted or trisubstituted by identical or different methyl, ethyl, propyl, isopropyl, butyl, isobutyl, tert-butyl, pentyl, isopentyl, hexyl, isohexyl, methoxy, ethoxy, propoxy, isopropoxy, butoxy, isobutoxy, tert-butoxy, methylthio, ethylthio, propylthio, isopropylthio, butylthio, isobutylthio, tert-butylthio, methylsulphonyl, ethylsulphonyl, propylsulphonyl, isopropylsulphonyl, butylsulphonyl, isobutylsulphonyl, tert-butylsulphonyl, phenyl, phenoxy, phenylthio, phenylsulphonyl, benzyl, benzyloxy, benzylthio, benzylsulphonyl, fluorine, chlorine, bromine, cyano, trifluoromethyl, trifluoromethoxy, trifluoromethylthio, methoxycarbonyl, ethoxycarbonyl, propoxycarbonyl, isopropoxycarbonyl, butoxycarbonyl, isobutoxycarbonyl, tert-butoxycarbonyl or by a group -NR¹R²,
where
R¹ and R² have the abovementioned meaning, or
- stands for 2,5-dioxotetrahydropyrryl,
- stands for tetrahydropyranyl, or
- stands for dimethyl-tert-butylsilyl or trimethylsilyl, or
- denotes a group -COR¹⁶,
where
R¹⁶ has the abovementioned meaning,
and
R¹⁸ and R¹⁹ are identical or different and
- denote hydrogen, or
- denote methyl, ethyl, propyl, isopropyl, butyl, isobutyl, pentyl, isopentyl, hexyl or isohexyl which are optionally substituted by fluorine or chlorine, or
- denote phenyl which can optionally be substituted by fluorine, chlorine, methyl or methoxy,
or
D and E together form a ring of the formula 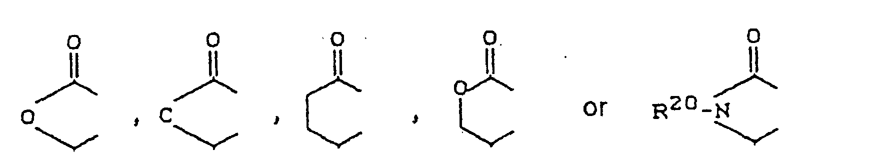 wherein
R²⁰ - stands for hydrogen, methyl, ethyl, propyl, isopropyl, carbamoyl, methoxycarbonyl or ethoxycarbonyl,
X - stands for a group of the formula -CH=CH-,
R - stands for a group of the formula 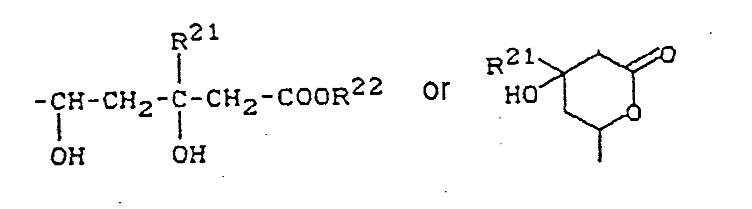
wherein
R²¹ - denotes hydrogen, methyl, ethyl, propyl, isopropyl, butyl, isobutyl or tert-butyl, and
R²² - denotes hydrogen, methyl, ethyl, propyl, isopropyl, butyl, isobutyl, tert-butyl or benzyl, or a sodium, potassium, calcium, magnesium or ammonium ion,
and their oxidation products.

13. Process according to Claim 11 for preparing methyl-erythro-(E)-7-[2,6-diisopropyl-4-(4-fluorophenyl)-5-methoxymethylpyrid-3-yl]-3,5-dihydroxyhept-6-enoate of the formula

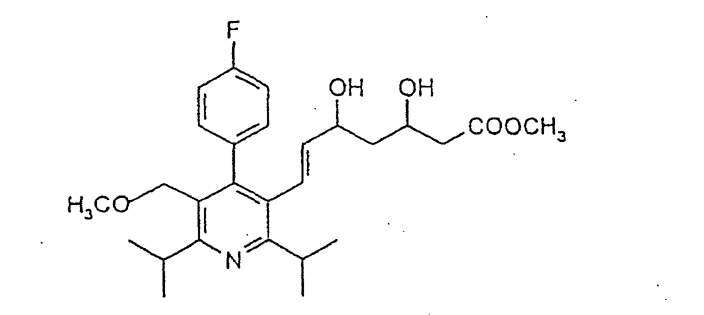

14. Process according to one of Claims 11 to 13, **characterized in that** the reduction is carried out in a temperature range from -80°C to +30°C.

15. Process for preparing the salts of compounds of the general formula (Ie) or (Ie)' 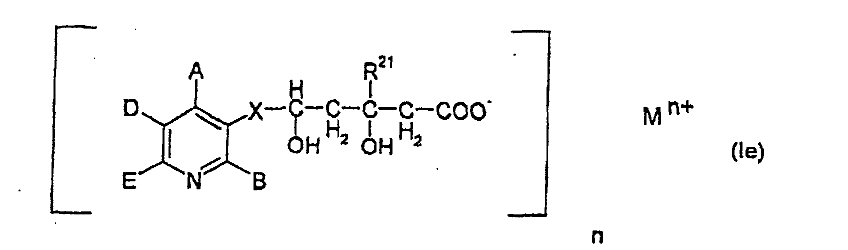 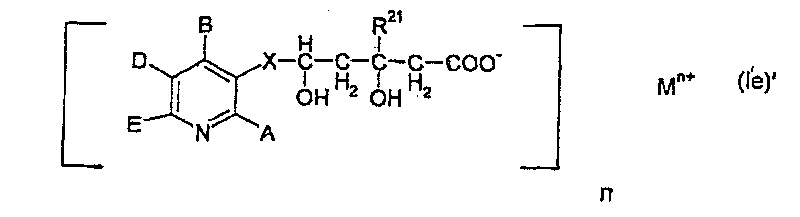 in which
A, B, D, E, X and R²¹ have the meanings given in the compounds of the preceding Claims 11 to 14 and
Mⁿ⁺ stands for a cation, n specifying the valency,
**characterized in that** the corresponding esters or lactones are heated in inert solvents with customary bases.

16. Process according to Claim 15 using sodium hydroxide or potassium hydroxide as base.

17. Process for preparing carboxylic acids of the general formula (Ic) or (Ic)' 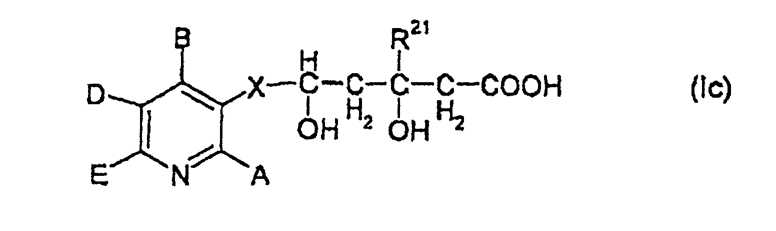 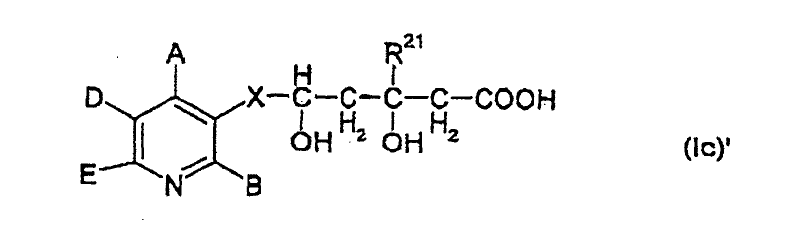 in which
A, B, D, E, X and R²¹ have the meanings given in the compounds of the preceding Claims 12 to 15,
**characterized in that** the corresponding esters and lactones are treated in a first step in inert solvents with customary bases and the salts which are formed are subsequently converted in a second step into the free acids by treating with acids.

## Revendications

1. Pyridines substituées de formule 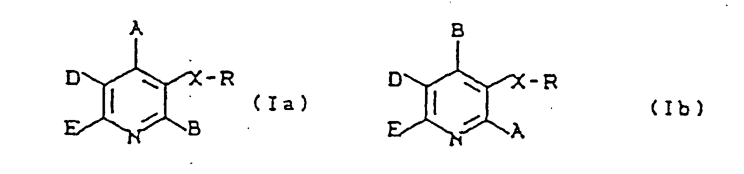 dans laquelle
A
- représente un reste thiényle, furyle, thiazolyle, isothiazolyle, oxazolyle, isoxazolyle, pyridyle, pyrimidyle, pyrazinyle, pyridazinyle, indolyle, isoindolyle, quinolyle, isoquinolyle, phtalazinyle, quinoxalinyle, quinazolinyle, cinnolinyle, benzothiazolyle, benzoxazolyle ou benzimidazolyle, qui peut être substitué jusqu'à deux fois identiques ou différentes par du fluor, du chlore, du brome, un radical alkyle en C₁ à C₆, alkoxy en C₁ à C₆, phényle, phénoxy, trifluorométhyle, trifluorométhoxy ou (alkoxy en C₁ à C₆)-carbonyle, ou bien
- un reste phényle ou naphtyle qui peut être substitué jusqu'à 4 fois identiques ou différentes par un radical alkyle en C₁ à C₆ qui peut porter éventuellement un substituant hydroxy ou alkoxy en C₁ à C₆, par un radical alkoxy en C₁ à C₆, alkylthio en C₁ à C₆, alkylsulfonyle en C₁ à C₆, phényle, phényloxy, phénylthio, phénylsulfonyle, benzyle, benzyloxy, benzylthio, benzylsulfonyle, phénéthyle, phényléthoxy, phényléthylthio, phényléthylsulfonyle, fluor, chlore, brome ou cyano,
- un reste méthyle, éthyle, propyle, isopropyle, butyle
ou tertio-butyle,
B
- représente un reste cyclopropyle, cyclopentyle ou cyclohexyle, ou bien
- un reste alkyle en C₁ à C₆ qui peut être substitué par du fluor, du chlore, du brome, un radical cyano, alkoxy en C₁ à C₆, alkylthio en C₁ à C₆, alkyl-sulfonyle en C₁ à C₆, trifluorométhyle, trifluorométhoxy, trifluorométhylsulfonyle, (alkoxy en C₁ à C₆)-carbonyle, benzoyle, (alkyle en C₁ à C₆)-carbonyle, par un groupe de formule -NR¹R²,
dans laquelle
R¹ et R² sont identiques ou différents et représentent un radical alkyle en C₁ à C₆, phényle, benzyle, acétyle, benzoyle, phénylsulfonyle ou alkylsulfonyle en C₁ à C₆,
ou par un reste pyridyle, pyrimidyle, pyrazinyle, pyridazinyle, quinolyle, isoquinolyle, pyrrolyle, indolyle, thiényle, furyle, imidazolyle, oxazolyle, thiazolyle, phényle, phénoxy, phénylthio, phénylsulfonyle, benzyloxy, benzylthio, benzyl-sulfonyle, phényléthoxy, phényléthylthio ou phényléthylsulfonyle, les restes hétéroaryle et aryle mentionnés pouvant être substitués jusqu'à 2 fois identiques ou différentes par du fluor, du chlore, du brome, un radical alkyle en C₁ à C₆, alkoxy en C₁ à C₆, trifluorométhyle ou trifluorométhoxy,
D,E
- sont identiques ou différents et représentent
- un reste cyclopropyle, cyclopentyle ou cyclohexyle,
ou bien
- un reste alkyle en C₁ à C₆ linéaire ou ramifié qui est substitué dans le cas du substituant D et qui peut être substitué dans le cas du substituant E par un radical azido, du fluor, du chlore, du brome, un radical cyano, hydroxy, alkoxy en C₁ à C₆, alkylthio en C₁ à C₆, alkylsulfonyle en C₁ à C₆, trifluorométhyle, trifluorométhoxy, trifluorométhylsulfonyle, (alkoxy en C₁ à C₆)-carbonyle, benzoyle, (alkyle en C₁ à C₆)-carbonyle, par un groupe de formule -NR¹R²,
dans laquelle
R¹ et R² ont la définition indiquée ci-dessus,
ou par un reste pyridyle, pyrimidyle, pyrazinyle, pyridazinyle, quinolyle, isoquinolyle, pyrrolyle, indolyle, thiényle, furyle, imidazolyle, oxazolyle, thiazolyle, phényle, phénoxy, phénylthio, phénylsulfonyle, benzyloxy, benzylthio, benzyl-sulfonyle, phényléthoxy, phényléthylthio ou phényléthylsulfonyle, les restes hétéroaryle et aryle mentionnés pouvant être substitués jusqu'à 2 fois identiques ou différentes par du fluor, du chlore, du brome, un radical alkyle en C₁ à C₆, alkoxy en C₁ à C₆, trifluorométhyle ou trifluorométhoxy,
- un reste thiényle, furyle, thiazolyle, tétrazolyle, isothiazolyle, oxazolyle, isoxazolyle, pyridyle, pyrimidyle, pyrazinyle, pyridazinyle, indolyle, isoindolyle, quinolyle, isoquinolyle, phtalazinyle, quinoxalinyle, quinazolinyle, cinnolinyle, benzothiazolyle, benzoxazolyle ou benzimidazolyle, qui peut être substitué jusqu'à 2 fois identiques ou différentes par du fluor, du chlore, du brome, un radical alkyle en C₁ à C₆, alkoxy en C₁ à C₆, phényle, phénoxy, trifluorométhyle, trifluorométhoxy ou (alkoxy en C₁ à C₆)-carbonyle, ou bien
- un reste naphtyle qui peut être substitué jusqu'à 4 fois identiques ou différentes par un radical alkyle en C₁ à C₆ qui peut porter éventuellement un substituant hydroxy ou alkoxy en C₁ à C₆, par un radical alkoxy en C₁ à C₆, alkylthio en C₁ à C₆, alkylsulfonyle en C₁ à C₆, phényle, phényloxy, phénylthio, phényl-sulfonyle, benzyle, benzyloxy, benzylthio, benzyl-sulfonyle, phénéthyle, phényléthoxy, phényléthylthio, phényléthylsulfonyle, fluoro, chloro, bromo, cyano, trifluorométhyle, trifluorométhoxy, trifluorométhyl-thio, (alkoxy en C₁ à C₆)-carbonyle ou par un groupe de formule -NR¹R²,
dans laquelle
R¹ et R² ont la définition indiquée ci-dessus,
ou bien
- un groupe de formule -CR¹¹R¹²-Y
dans laquelle
R¹¹ et R¹² peuvent être identiques ou différents et représentent
- l'hydrogène, ou bien
- un reste alkyle en C₁ à C₆ qui peut porter éventuellement un substituant hydroxy, fluoro, chloro, alkoxy en C₁ à C₆ ou (alkoxy en C₁ à C₆)-carbonyle, ou bien
- un reste cyclopropyle, cyclopentyle ou cyclohexyle, ou bien
R¹¹ et R¹² forment conjointement un noyau carbocyclique ou hétérocyclique saturé ou non saturé ayant jusqu'à 6 atomes de carbone,
Y - représente un groupe de formule -NR¹³R¹⁴, -COR¹⁵, -S-R¹⁶, SO-R¹⁶, -SO₂R¹⁶, -OR¹⁷ ou -N₃,
dans lequel
R¹³ et R¹⁴ sont identiques ou différents et représentent
- l'hydrogène, un reste alkyle en C₁ à C₆, phényle ou benzyle, les restes mentionnés pouvant être substitués par du fluor, du chlore, du brome, un radical alkyle en C₁ à C₆, alkoxy en C₁ à C₆ ou trifluorométhyle, ou bien
- un groupe de formule -COR¹⁵, -SO₂R¹⁶,
ou bien R¹³ et R¹⁴ forment conjointement une chaîne alkylénique qui peut être interrompue par O, N, S, un groupe N-alkyle en C₁ à C₆, N-benzyle, N-phényle, N-carbamoyle ou N-(alkoxy en C₁ à C₆)-carbonyle,
R¹⁵ représente
- un groupe -NR¹⁸R¹⁹, ou bien
- un reste alkyle en C₁ à C₆ ou alkoxy en C₁ à C₆, ou bien
- un reste phényle, benzyle, benzyloxy, thiényle, furyle, pyridyle, pyrimidyle, quinolyle, isoquinolyle, benzothiazolyle, benzoxazolyle, thiazolyle, oxazolyle, isoxazolyle ou isothiazolyle éventuellement substitué par un radical alkyle en C₁ à C₆, alkoxy en C₁ à C₆, fluoro, chloro, bromo, trifluorométhyle, diméthylamino ou diéthylamino,
R¹⁶ désigne
- un reste cyclopropyle, cyclopentyle, cyclohexyle, ou
- un reste alkyle en C₁ à C₆ linéaire ou ramifié éventuellement substitué par un radical cyano, fluoro, chloro, bromo, trifluorométhyle, ou (alkoxy en C₁ à C₆)-carbonyle, ou bien
- un reste phényle, naphtyle, benzyle, thiényle, furyle, pyrimidyle, pyridyle, quinolyle, isoquinolyle, benzothiazolyle, benzoxazolyle, thiazolyle, oxazolyle,
- isoxazolyle, ou isothiazolyle portant éventuellement un ou plusieurs substituants, identiques ou différents, alkyle en C₁ à C₆, alkoxy en C₁ à C₆, fluoro, chloro, bromo, trifluorométhyle, diméthylamino ou diéthylamino, ou bien
- le reste triméthylsilyle ou diméthyl-éthylsilyle,
- un groupe -NR⁹R¹⁰,
dans lequel
R⁹ et R¹⁰ ont la définition indiquée ci-dessus,
R¹⁷ représente
- l'hydrogène ou
- un reste cyclopropyle, cyclopentyle ou cyclohexyle, ou
- un reste alkyle en C₁ à C₆ qui peut être substitué par du fluor, du chlore, du brome, un radical cyano, alkoxy en C₁ à C₆, alkylthio en C₁ à C₆, alkylsulfonyle en C₁ à C₆, trifluorométhyle, trifluorométhoxy, trifluorométhylsulfonyle, (alkoxy en C₁ à C₆)-carbonyle, benzoyle, (alkyle en C₁ à C₆)-carbonyle, par un groupe de formule -NR¹R²,
dans laquelle
R¹ et R² ont la définition indiquée ci-dessus,
ou par un reste pyridyle, pyrimidyle, pyrazinyle, pyridazinyle, quinolyle, isoquinolyle, pyrrolyle, indolyle, thiényle, furyle, imidazolyle, oxazolyle, thiazolyle, phényle, phénoxy, phénylthio, phényl-sulfonyle, benzyloxy, benzylthio, benzyl-sulfonyle, phényléthoxy, phényléthylthio ou phényléthylsulfonyle, les restes hétéroaryle et aryle mentionnés pouvant porter jusqu'à 2 substituants, identiques ou différents, fluoro, chloro, bromo, alkyle en C₁ à C₆, alkoxy en C₁ à C₆, trifluorométhyle ou trifluorométhoxy,
- un reste thiényle, furyle, thiazolyle, isothiazolyle, oxazolyle, isoxazolyle, pyridyle, pyrimidyle, pyrazinyle, pyridazinyle, indolyle, isoindolyle, quinolyle, isoquinolyle, phtalazinyle, quinoxalinyle, quinazolinyle, cinnolinyle, benzothiazolyle, benzoxazolyle ou benzimidazolyle, qui peut être substitué jusqu'à 2 fois identiques ou différentes par du fluor, du chlore, du brome, un radical alkyle en C₁ à C₆, alkoxy en C₁ à C₆, phényle, phénoxy, trifluorométhyle, trifluorométhoxy ou (alkoxy en C₁ à C₆)-carbonyle, ou bien
- un reste benzyle, un reste phényle ou un reste naphtyle, qui peuvent être substitués jusqu'à 4 fois identiques ou différentes par un radical alkyle en C₁ à C₆, alkoxy en C₁ à C₆, alkylthio en C₁ à C₆, alkylsulfonyle en C₁ à C₆, phényle, phényloxy, phénylthio, phénylsulfonyle, benzyle, benzyloxy, benzylthio, benzylsulfonyle, phénéthyle, phényléthoxy, phényléthylthio, phényléthylsulfonyle, fluoro, chloro, bromo, cyano, trifluorométhyle, trifluorométhoxy, trifluorométhylthio, (alkoxy en C₁ à C₆)-carbonyle ou par un groupe de formule -NR¹R²,
dans laquelle
R¹ et R² ont la définition indiquée ci-dessus,
ou bien
- le reste 2,5-dioxo-tétrahydropyrryle,
- le reste tétrahydropyrannyle, ou bien
- le reste diméthyl-tertio-butylsilyle, tripropylsilyle ou tributylsilyle, ou bien
- un groupe de formule COR¹⁶,
dans laquelle
R¹⁶ a la définition indiquée ci-dessus,
et
R¹⁸ et R¹⁹ sont identiques ou différents et représentent
- l'hydrogène, ou bien
- un reste alkyle en C₁ à C₆ éventuellement substitué par un radical cyano, fluoro, chloro ou bromo, ou bien
- un reste phényle, benzyle, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en C₁ à C₆, alkoxy en C₁ à C₆, fluoro, chloro, bromo, trifluorométhyle, diméthylamino ou diéthylamino,
ou bien
D, E forment ensemble un noyau de formule 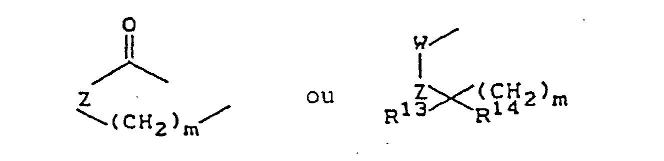 dans laquelle
W - est un groupe de formule C=O ou CH-OH,
m - est le nombre 1 ou 2,
Z - représente O, CH₂ ou NHR²⁰,
R¹³ et R¹⁴ ont la définition indiquée ci-dessus, et
R²⁰- représente l'hydrogène, un reste alkyle en C₁ à C₆, phényle, benzyle, carbamoyle ou (alkoxy en C₁ à C₆)-carbonyle,
X - désigne un groupe de formule -CH=CH-,
R - est un groupe de formule 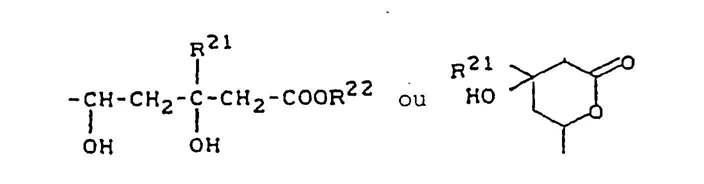 dans laquelle
R²¹ - représente l'hydrogène ou un reste alkyle en C₁ à C₆, et
R²²
- représente l'hydrogène, un reste alkyle en C₁ à C₆, phényle ou benzyle, ou bien
- un cation,
ainsi que leurs produits d'oxydation.

2. Pyridines substituées suivant la revendication 1,
dans lesquelles
A
- représente un reste thiényle, furyle, pyridyle, pyrimidyle, quinolyle ou isoquinolyle qui peut être substitué par un radical fluoro, chloro, méthyle, méthoxy ou trifluorométhyle, ou bien
- un reste phényle qui peut porter jusqu'à 3 substituants, identiques ou différents, méthyle, hydroxyméthyle, éthyle, propyle, isopropyle, hydroxyéthyle, hydroxypropyle, butyle, isobutyle, méthoxyméthyle, éthoxyméthyle, propoxyméthyle, tertio-butyle, méthoxy, éthoxy, propoxy, isopropoxy, butoxy, isobutoxy, tertio-butoxy, méthylthio, éthylthio, propylthio, isopropylthio, méthylsulfonyle, éthylsulfonyle, propylsulfonyle, isopropylsulfonyle, phényle, phénoxy, benzyle, benzyloxy, fluoro, chloro, bromo, cyano, trifluorométhyle, trifluorométhoxy, méthoxycarbonyle, éthoxycarbonyle, propoxycarbonyle, isopropoxycarbonyle, butoxycarbonyle, isobutoxycarbonyle ou tertio-butoxycarbonyle,
- un reste méthyle, éthyle, propyle, isopropyle, butyle
ou tertio-butyle,
B - représente
- un reste cyclopropyle, cyclopentyle ou cyclohexyle, ou bien
- un reste méthyle, éthyle, propyle, isopropyle, butyle, sec.-butyle ou tertio-butyle qui peut être substitué par du fluor, du chlore, du brome, un radical cyano, méthoxy, éthoxy, propoxy, isopropoxy, butoxy, sec.-butoxy, tertio-butoxy, méthylthio, éthylthio, propylthio, isopropylthio, méthyl-sulfonyle, éthylsulfonyle, propylsulfonyle, isopropylsulfonyle, trifluorométhyle, trifluorométhoxy, méthoxycarbonyle, éthoxycarbonyle, butoxycarbonyle, isobutoxycarbonyle, tertio-butoxycarbonyle, benzoyle, acétyle, pyridyle, pyrimidyle, thiényle, furyle, phényle, phénoxy, phénylthio, phénylsulfonyle, benzyloxy, benzylthio ou benzyl-sulfonyle,
D,E
- sont identiques ou différents et représentent
- un reste cyclopropyle, cyclopentyle ou cyclohexyle, ou bien
- un reste méthyle, éthyle, propyle, isopropyle, butyle, isobutyle, tertio-butyle, pentyle, isopentyle, hexyle ou isohexyle, ces restes étant substitués dans le cas du substituant D et pouvant être substitués dans le cas du substituant E, par
un radical azido, fluoro, chloro, bromo, iodo, cyano, hydroxy, méthoxy, éthoxy, propoxy, isopropoxy, butoxy, isobutoxy, tertio-butoxy, méthylthio, éthylthio, propylthio, isopropylthio, butylthio, isobutylthio, tertio-butylthio, méthylsulfonyle, éthylsulfonyle, propylsulfonyle, isopropylsulfonyle, butylsulfonyle, isobutylsulfonyle, tertio-butylsulfonyle, trifluorométhyle, trifluorométhoxy, méthoxycarbonyle, éthoxycarbonyle, propoxycarbonyle, isopropoxycarbonyle, butoxycarbonyle, isobutoxycarbonyle, tertio-butoxycarbonyle, benzoyle, acétyle, éthylcarbonyle ou par un groupe -NR¹R²,
dans lequel
R¹ et R² sont identiques ou différents et représentent un reste méthyle, éthyle, propyle, isopropyle, butyle, isobutyle, tertio-butyle, phényle, benzyle, acétyle, méthylsulfonyle, éthylsulfonyle, propylsulfonyle, isopropylsulfonyle ou phénylsulfonyle,
ou par un reste pyridyle, pyrimidyle, pyrazinyle, pyridazinyle, quinolyle, isoquinolyle, thiényle, furyle, phényle, phénoxy, phénylthio, phénylsulfonyle, benzyloxy, benzylthio ou benzylsulfonyle, les restes hétéroaryle et aryle mentionnés pouvant être substitués par du fluor, du chlore, un radical méthyle, éthyle, propyle, isopropyle, isobutyle, tertio-butyle, méthoxy, éthoxy, propoxy, isopropoxy, butoxy, isobutoxy, tertio-butoxy, trifluorométhyle ou trifluorométhoxy, ou bien
- un reste thiényle, furyle, pyridyle, pyrimidyle, pyrazinyle, tétrazolyle, pyridazinyle, oxazolyle, isooxazolyle, imidazolyle, pyrazolyle, thiazolyle, isothiazolyle, quinolyle, isoquinolyle, benzoxazolyle, benzimidazolyle ou benzothiazolyle, les restes mentionnés pouvant être substitués par un radical fluoro, chloro, méthyle, éthyle, propyle, isopropyle, butyle, isobutyle, tertio-butyle, méthoxy, éthoxy, propoxy, isopropoxy, butoxy, isobutoxy, tertio-butoxy, phényle, phénoxy, trifluorométhyle, trifluorométhoxy, méthoxycarbonyle, éthoxycarbonyle, isopropoxycarbonyle, propoxycarbonyle, butoxycarbonyle, isobutoxycarbonyle ou tertio-butoxycarbonyle, ou bien
- un groupe de formule -CR¹¹R¹²-Y
dans laquelle
R¹¹ et R¹² sont identiques ou différents et représentent
- l'hydrogène, ou
- un reste méthyle, éthyle, propyle, isopropyle, qui peut être substitué le cas échéant par un radical hydroxy, fluoro, chloro, méthoxy, éthoxy, méthoxycarbonyle, éthoxycarbonyle, ou bien
- un reste cyclopropyle, cyclopentyle ou cyclohexyle,
ou bien R¹¹ et R¹² forment ensemble un reste cyclopropyle, cyclopentyle ou cyclohexyle, et
Y - désigne un groupe de formule -NR¹³R¹⁴, -COR¹⁵, -SR¹⁶, -SO-R¹⁶, -SO₂-R¹⁶, -OR¹⁷ ou -N₃,
dans lequel
R¹³ et R¹⁴ sont identiques ou différents et représentent
- l'hydrogène, un reste méthyle, éthyle, propyle, isopropyle, butyle, isobutyle, ou bien
- un reste phényle portant éventuellement un substituant fluoro, chloro, méthyle ou méthoxy, ou bien
- un groupe -COR¹⁵ ou -SO₂R¹⁶, ou bien R¹³ et R¹⁴ forment conjointement avec l'atome d'azote un noyau de la série pipéridine, pipérazine, morpholine, morpholine-N-oxyde, N-(alkyle inférieur)-pipérazine, benzylpipérazine
ou phénylpipérazine,
R¹⁵ représente
- l'hydrogène, ou bien
- un groupe -NR¹⁸R¹⁹, ou bien
- un reste méthyle, éthyle, propyle, isopropyle, méthoxy, éthoxy, propoxy, isopropoxy, ou bien
- un reste phényle, benzyle, benzyloxy, thiényle, furyle, pyridyle, pyrimidyle, quinolyle ou isoquinolyle portant éventuellement un substituant méthyle, méthoxy, fluoro ou chloro,
R¹⁶ représente
- un reste méthyle, éthyle, propyle, isopropyle, butyle, isobutyle, ou isopentyle éventuellement substitué par du fluor, du chlore, un radical méthoxycarbonyle, éthoxycarbonyle, propoxycarbonyle, isopropoxycarbonyle, butoxycarbonyle ou isobutoxycarbonyle, ou bien
- un reste benzyle, phényle, naphtyle, thiényle, furyle, pyridyle, pyrimidyle, quinolyle ou isoquinolyle portant éventuellement un ou plusieurs substituants, identiques ou différents, méthyle, éthyle, propyle, isopropyle, méthoxy, fluoro ou chloro, ou bien
- le reste triméthylsilyle ou diméthyl-éthylsilyle,
- un groupe -NR⁹R¹⁰,
dans lequel
R⁹ et R¹⁰ ont la définition indiquée ci-dessus, R¹⁷ représente
- l'hydrogène, un reste cyclopropyle, cyclopentyle ou cyclohexyle, ou bien
- un reste méthyle, éthyle, propyle, isopropyle, butyle, isobutyle, tertio-butyle, pentyle, isopentyle, hexyle ou isohexyle, qui peut être substitué par du fluor, du chlore, du brome, un radical cyano, méthoxy, éthoxy, propoxy, isopropoxy, butoxy, isobutoxy, tertio-butoxy, méthylthio, éthylthio, propylthio, isopropylthio, butylthio, isobutylthio, tertio-butylthio, méthyl-sulfonyle, éthylsulfonyle, propylsulfonyle, isopropylsulfonyle, butylsulfonyle, isobutylsulfonyle, tertio-butylsulfonyle, trifluorométhyle, trifluorométhoxy, méthoxy-carbonyle, éthoxycarbonyle, propoxycarbonyle, isopropoxycarbonyle, butoxycarbonyle, isobutoxycarbonyle, tertio-butoxycarbonyle, benzoyle, acétyle, éthylcarbonyle, ou par un groupe -NR¹R²,
dans lequel
R¹ et R² sont identiques ou différents et représentent un reste méthyle, éthyle, propyle, isopropyle, butyle, isobutyle, tertio-butyle, phényle, benzyle, acétyle, méthylsulfonyle, éthylsulfonyle, propylsulfonyle, isopropylsulfonyle ou phényl-sulfonyle,
ou par un reste pyridyle, pyrimidyle, pyrazinyle, pyridazinyle, quinolyle, isoquinolyle, thiényle, furyle, phényle, phénoxy, phénylthio, phénylsulfonyle, benzyloxy, benzylthio ou benzylsulfonyle, les restes hétéroaryle et aryle mentionnés pouvant être substitués par du fluor, du chlore, un radical méthyle, éthyle, propyle, isopropyle, isobutyle, tertio-butyle, méthoxy, éthoxy, propoxy, isopropoxy, butoxy, isobutoxy, tertio-butoxy, trifluorométhyle ou trifluorométhoxy, ou bien
- un reste thiényle, furyle, pyridyle, pyrimidyle, pyrazinyle, pyridazinyle, oxazolyle, isooxazolyle, imidazolyle, pyrazolyle, thiazolyle, isothiazolyle, quinolyle, isoquinolyle, benzoxazolyle, benzimidazolyle ou benzothiazolyle, les restes mentionnés pouvant être substitués par du fluor, du chlore, un radical méthyle, éthyle, propyle, isopropyle, butyle, isobutyle, tertio-butyle, méthoxy, éthoxy, propoxy, isopropoxy, butoxy, isobutoxy, tertio-butoxy, phényle, phénoxy, trifluorométhyle, trifluorométhoxy, méthoxycarbonyle, éthoxycarbonyle, isopropoxycarbonyle, propoxycarbonyle, butoxycarbonyle, isobutoxycarbonyle ou tertio-butoxycarbonyle, ou bien
- un reste benzyle ou phényle qui peut être substitué jusqu'à 3 fois identiques ou différentes par un radical méthyle, éthyle, propyle, isopropyle, butyle, isobutyle, tertio-butyle, pentyle, isopentyle, hexyle, isohexyle, méthoxy, éthoxy, propoxy, isopropoxy, butoxy, isobutoxy, tertio-butoxy, méthylthio, éthylthio, propylthio, isopropylthio, butylthio, isobutylthio, tertio-butylthio, méthylsulfonyle, éthylsulfonyle, propylsulfonyle, isopropyl-sulfonyle, butylsulfonyle, isobutylsulfonyle, tertio-butylsulfonyle, phényle, phénoxy, phénylthio, phényl-sulfonyle, benzyle, benzyloxy, benzylthio, benzylsulfonyle, fluor, chlore, brome, cyano, trifluorométhyle, trifluorométhoxy, trifluorométhylthio, méthoxycarbonyle, éthoxycarbonyle, propoxycarbonyle, isopropoxycarbonyle, butoxycarbonyle, isobutoxycarbonyle, tertio-butoxycarbonyle, ou par un groupe -NR¹R²,
dans lequel
R¹ et R² ont la définition indiquée ci-dessus,
ou représentent
- le reste 2,5-dioxo-tétrahydropyrryle,
- le reste tétrahydropyrannyle, ou bien
- le reste diméthyl-tertio-butylsilyle ou triméthylsilyle, ou bien
un groupe -COR¹⁶,
dans lequel
R¹⁶ a la définition indiquée ci-dessus,
et
R¹⁸ et R¹⁹ sont identiques ou différents et représentent
- l'hydrogène, ou bien
- un reste méthyle, éthyle, propyle, isopropyle, butyle, isobutyle, pentyle, isopentyle, hexyle ou isohexyle éventuellement substitué par du fluor ou du chlore, ou bien
- un reste phényle qui peut être substitué par un radical fluoro, chloro, méthyle ou méthoxy,
ou bien
D et E forment ensemble un noyau de formule 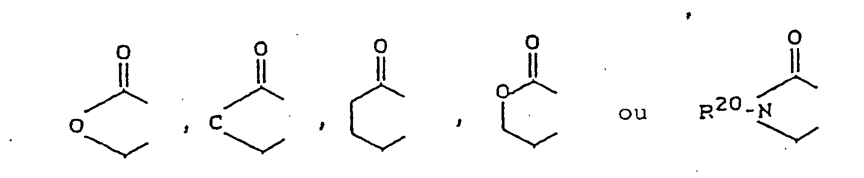 dans laquelle
R²⁰ représente l'hydrogène, un reste méthyle, éthyle, propyle, isopropyle, carbamoyle, méthoxycarbonyle ou éthoxycarbonyle,
X - est un groupe de formule -CH=CH-,
R - est un groupe de formule 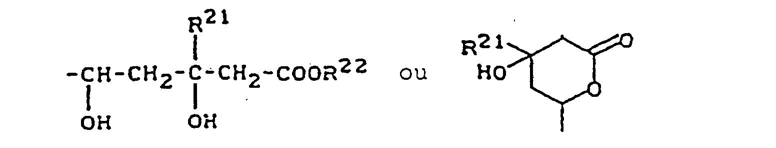 dans laquelle
R²¹ représente l'hydrogène, un reste méthyle, éthyle, propyle, isopropyle, butyle, isobutyle ou tertio-butyle, et
R²² représente l'hydrogène, un reste méthyle, éthyle, propyle, isopropyle, butyle, isobutyle, tertio-butyle ou benzyle, ou un ion sodium, potassium, calcium, magnésium ou ammonium,
ainsi que leurs produits d'oxydation.

3. Composés de formules générales (Ia) 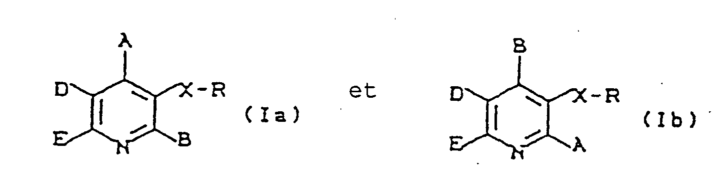 dans lesquelles
A
- désigne un reste thiényle ou furyle,
- un reste phényle qui peut être substitué jusqu'à 2 fois identiques ou différentes par un radical méthyle, hydroxyméthyle, éthyle, propyle, isopropyle, butyle, isobutyle, tertio-butyle, méthoxy, éthoxy, propoxy, isopropoxy, phénoxy, benzyloxy, fluoro, chloro, trifluorométhyle,
- un reste méthyle, éthyle, propyle ou isopropyle,
B
- est un reste cyclopropyle, méthyle, éthyle, propyle, isopropyle, butyle, isobutyle ou tertio-butyle, qui peut porter un substituant fluoro, chloro, méthoxy, phényle ou phénoxy,
D,E sont identiques ou différents et représentent
- un reste cyclopropyle, cyclopentyle ou cyclohexyle, ou bien
- un reste méthyle, éthyle, propyle, isopropyle, butyle, isobutyle, tertio-butyle, pentyle, isopentyle, hexyle ou isohexyle qui est substitué dans le cas du substituant D ou qui peut être substitué dans le cas du substituant E, par un radical azido, fluoro, chloro, iodo, méthoxy, éthoxy, propoxy, isopropoxy, méthylthio, éthylthio, méthylsulfonyle, éthylsulfonyle, éthoxycarbonyle, propoxycarbonyle, isopropoxycarbonyle ou par un groupe de formule NR¹R²,
dans laquelle
R¹ et R² sont identiques ou différents et représentent,
- l'hydrogène, un reste méthyle, éthyle, propyle, isopropyle, phényle ou benzyle,
ou par un reste pyridyle, pyrimidyle, quinolyle, thiényle, furyle, phényle, phénoxy, phénylsulfonyle ou benzyloxy éventuellement substitué par un radical fluoro, chloro, méthyle, méthoxy, trifluorométhyle ou trifluorométhoxy, ou bien
- un reste thiényle, furyle, pyridyle, pyrimidyle, quinolyle, isoquinolyle, benzoxazolyle, tétrazolyle, benzothiazolyle ou benzimidazolyle éventuellement substitué par un radical fluoro, chloro, méthyle, éthyle, propyle, isopropyle, méthoxy, phényle, méthoxycarbonyle, éthoxycarbonyle,
ou bien
- un groupe de formule -CR¹¹R¹²-Y
dans laquelle
R¹¹ et R¹² représentent l'hydrogène, un reste méthyle
ou éthyle, et
Y - est un groupe de formule -NR¹³R¹⁴, -COR¹⁵, -S-R¹⁶, -SO-R¹⁶, -SO₂R¹⁶ ou -OR¹⁷,
dans laquelle
R¹³ et R¹⁴ sont identiques ou différents et représentent
- l'hydrogène, un reste méthyle, éthyle, propyle,
ou bien
- un groupe COR¹⁵ ou -SO₂R¹⁶,
ou bien R¹³ et R¹⁴ forment conjointement avec l'azote un noyau de la série morpholine ou morpholine-N-oxyde, et
R¹⁵ représente
- l'hydrogène ou un reste méthyle, ou bien.
- un groupe -NR¹⁸R¹⁹, ou bien
- un reste méthyle, éthyle, propyle, méthoxy ou éthoxy,
R¹⁶ représente
- un reste trifluorométhyle, méthyle, éthyle, propyle, isopropyle, butyle, isobutyle, isopentyle ou benzyle, ou bien
- un reste phényle ou naphtyle portant éventuellement un ou plusieurs substituants méthyle ou chloro,
- un reste triméthylsilyle ou diméthyléthyl-silyle, ou bien
- un groupe -NR⁹R¹⁰,
dans lequel
R⁹ et R¹⁰ ont la définition indiquée ci-dessus, R¹⁷ représente
- l'hydrogène, un reste cyclopropyle, cyclopentyle ou cyclohexyle, ou bien
- un reste méthyle, éthyle, propyle, isopropyle, butyle, isobutyle, tertio-butyle, pentyle, isopentyle, hexyle ou isohexyle, qui peut être substitué par un radical fluoro, chloro, méthoxy, éthoxy, propoxy, isopropoxy, méthylthio, éthylthio, méthylsulfonyle, éthylsulfonyle, éthoxycarbonyle, propoxycarbonyle, isopropoxycarbonyle, ou par un groupe -NR¹R²,
dans lequel
R¹ et R² sont identiques ou différents et représentent
- l'hydrogène, un reste méthyle, éthyle, propyle, isopropyle, phényle ou benzyle,
ou bien un reste pyridyle, pyrimidyle, quinolyle, thiényle, furyle, phényle, phénoxy, phénylsulfonyle ou benzyloxy portant éventuellement un substituant fluoro, chloro, méthyle, méthoxy, trifluorométhyle ou trifluorométhoxy, ou bien
- un reste thiényle, furyle, pyridyle, pyrimidyle, quinolyle, isoquinolyle, benzoxazolyle, benzothiazolyle ou benzimidazolyle portant éventuellement un substituant fluoro, chloro, méthyle, éthyle, propyle, isopropyle, méthoxy, phényle, méthoxycarbonyle, éthoxycarbonyle, ou bien
- un reste benzyle ou phényle qui peut être substitué jusqu'à 2 fois identiques ou différentes par un radical méthyle, éthyle, propyle, isopropyle, butyle, isobutyle, tertio-butoxy, méthoxy, éthoxy, propoxy, isopropoxy, méthylthio, éthylthio, méthyl-sulfonyle, éthylsulfonyle, phényle, phénoxy, phénylsulfonyle, benzyloxy, fluoro, chloro, bromo, cyano, trifluorométhyle, trifluorométhoxy, méthoxycarbonyle, éthoxycarbonyle ou par un groupe de formule -NR¹R²,
dans laquelle
R¹ et R² ont la définition indiquée ci-dessus,
- le reste 2,5-dioxo-tétrahydropyrryle, ou bien
- le reste tétrahydropyrannyle, ou bien
- le reste diméthyl-tertio-butylsilyle ou triméthylsilyle, ou bien
- un groupe -COR¹⁶,
dans lequel
R¹⁶ a la définition indiquée ci-dessus,
et
R¹⁸ et R¹⁹ sont identiques ou différents et représentent
- l'hydrogène, ou bien
- un reste méthyle, éthyle, propyle, isopropyle, butyle ou isobutyle, ou bien
- le reste phényle,
ou bien
D, E forment ensemble un noyau de formule 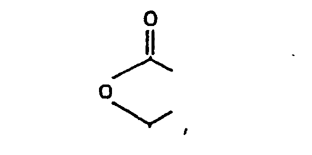 X - désigne un groupe de formule 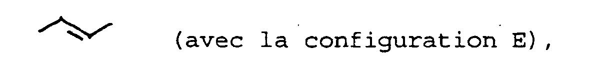 et
R - désigne un groupe de formule 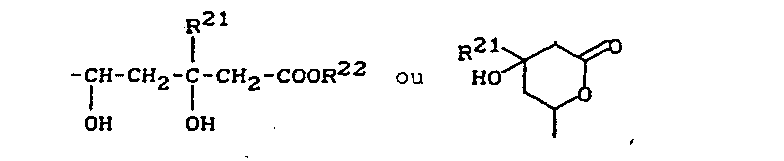 dans laquelle
R²¹ représente l'hydrogène
et
R²² représente l'hydrogène, un groupe méthyle ou éthyle, ou un ion sodium ou potassium,
et leurs produits d'oxydation.

4. L'érythro-(E)-7-[2,6-diisopropyl-4-(4-fluorophényl)-5-méthoxyméthyl-pyrid-3-yl]-3,5-dihydroxy-hept-6-énoate de méthyle de formule

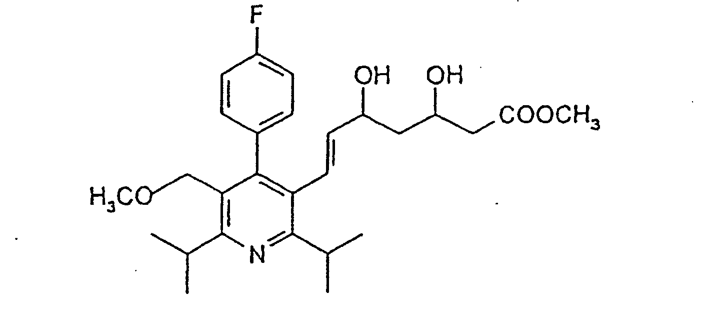

5. Sels des composés de formule générale (Ie) ou (Ie)' 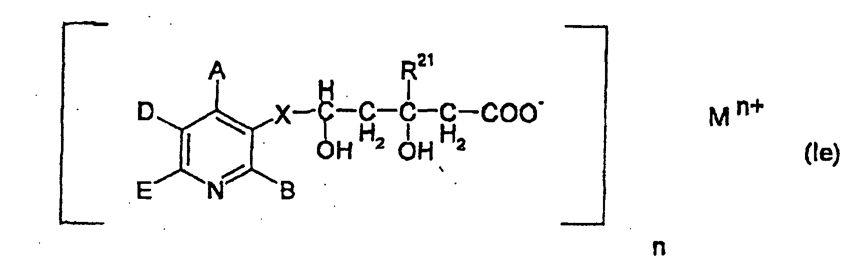 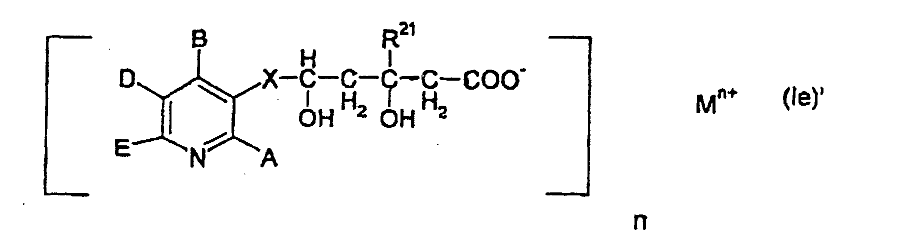 dans laquelle
A, B, D, E, X et R²¹ ont les définitions indiquées pour les composés des revendications 1 à 5 précédentes et Mⁿ⁺ représente un cation, n indiquant la valence.

6. Sels suivant la revendication 5, dans lesquels Mⁿ⁺ désigne l'ion sodium ou potassium.

7. Acides carboxyliques de formule générale (Ic) ou (Ic)' 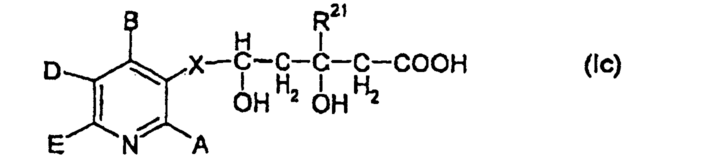 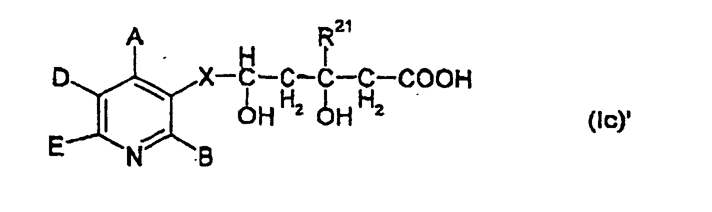 dans laquelle
A, B, D, E, X et R²¹ ont les définitions indiquées pour les composés des revendications 1 à 4 précédentes.

8. Cétones de formule (VIIIa) ou (VIIIb) 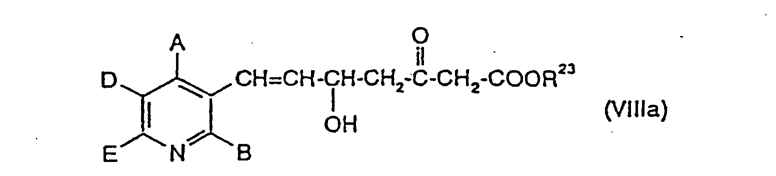 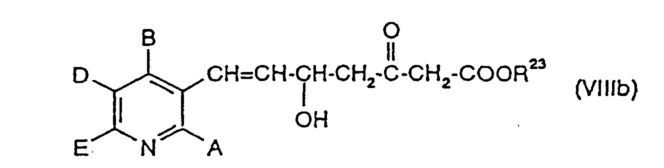 dans laquelle
A, B, D et E ont les définitions indiquées dans les revendications précédentes et
R²³ est un reste alkyle.

9. Procédé de production de cétones de formules (VIIIa) et (VIIIb) telles que définies dans la revendication 8,
qui est **caractérisée en ce qu'**on fait réagir des aldéhydes de formule générale (IX) 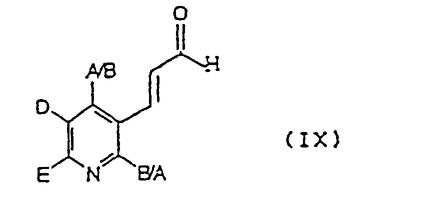 dans laquelle
A, B, D et E ont les définitions indiquées dans les revendications précédentes,
dans des solvants inertes, avec un ester d'acide acétylacétique de formule générale (X) 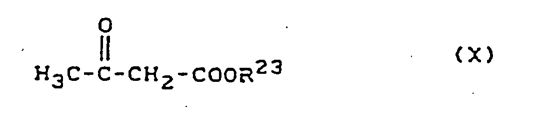 dans laquelle
R²³ a la définition indiquée dans la revendication 9, en présence de bases.

10. Procédé suivant la revendication 9, **caractérisé en ce qu'**on conduit la réaction dans la plage de températures de -80 °C à +50 °C.

11. Procédé de production de pyridines substituées de formule 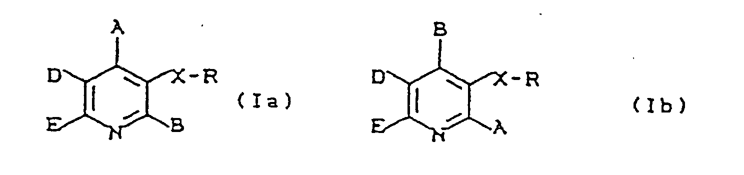 formule dans laquelle
A
- représente un reste thiényle, furyle, thiazolyle, isothiazolyle, oxazolyle, isoxazolyle, pyridyle, pyrimidyle, pyrazinyle, pyridazinyle, indolyle, isoindolyle, quinolyle, isoquinolyle, phtalazinyle, quinoxalinyle, quinazolinyle, cinnolinyle, benzothiazolyle, benzoxazolyle ou benzimidazolyle, qui peut être substitué jusqu'à 2 fois identiques ou différentes par du fluor, du chlore, du brome, un radical alkyle en C₁ à C₆, alkoxy en C₁ à C₆, phényle, phénoxy, trifluorométhyle, trifluorométhoxy ou (alkoxy en C₁ à C₆)-carbonyle, ou bien
- un reste phényle ou naphtyle qui peut être substitué jusqu'à 4 fois, identiques ou différentes, par un radical alkyle en C₁ à C₆ qui peut porter éventuellement un substituant hydroxy ou alkoxy en C₁ à C₆, par un radical alkoxy en C₁ à C₆, alkylthio en C₁ à C₆, alkylsulfonyle en C₁ à C₆, phényle, phényloxy, phénylthio, phénylsulfonyle, benzyle, benzyloxy, benzylthio, benzylsulfonyle, phénéthyle, phényléthoxy, phényléthylthio, phényléthylsulfonyle, fluor, chlore, brome ou cyano,
- un reste méthyle, éthyle, propyle, isopropyle, butyle
ou tertio-butyle,
B représente
- un reste cyclopropyle, cyclopentyle ou cyclohexyle,
ou bien
- un reste alkyle en C₁ à C₆ qui peut être substitué par du fluor, du chlore, du brome, un radical cyano, alkoxy en C₁ à C₆, alkylthio en C₁ à C₆, alkylsulfonyle en C₁ à C₆, trifluorométhyle, trifluorométhoxy, trifluorométhylsulfonyle, (alkoxy en C₁ à C₆)-carbonyle, benzoyle, (alkyle en C₁ à C₆)-carbonyle, par un groupe de formule -NR₁R₂,
dans laquelle
R¹ et R² sont identiques ou différents et représentent un radical alkyle en C₁ à C₆, phényle, benzyle, acétyle, benzoyle, phénylsulfonyle ou alkylsulfonyle en C₁ à C₆,
ou par un reste pyridyle, pyrimidyle, pyrazinyle, pyridazinyle, quinolyle, isoquinolyle, pyrrolyle, indolyle, thiényle, furyle, imidazolyle, oxazolyle, thiazolyle, phényle, phénoxy, phénylthio, phénylsulfonyle, benzyloxy, benzylthio, benzylsulfonyle, phényléthoxy, phényléthylthio ou phényléthylsulfonyle, les restes hétéroaryle et aryle mentionnés pouvant être substitués jusqu'à 2 fois identiques ou différentes par du fluor, du chlore, du brome, un radical alkyle en C₁ à C₆, alkoxy en C₁ à C₆, trifluorométhyle ou trifluorométhoxy,
D,E sont identiques ou différents et représentent
- un reste cyclopropyle, cyclopentyle ou cyclohexyle,
ou bien
- un reste alkyle en C₁ à C₆ linéaire ou ramifié qui est substitué dans le cas du substituant D et qui peut être substitué dans le cas du substituant E
par un radical azido, du fluor, du chlore, du brome, un radical cyano, hydroxy, alkoxy en C₁ à C₆, alkylthio en C₁ à C₆, alkylsulfonyle en C₁ à C₆, trifluorométhyle, trifluorométhoxy, trifluorométhylsulfonyle, (alkoxy en C₁ à C₆)-carbonyle, benzoyle, (alkyle en C₁ à C₆)-carbonyle, par un groupe de formule -NR¹R²,
dans laquelle
R¹ et R² ont la définition indiquée ci-dessus,
ou par un reste pyridyle, pyrimidyle, pyrazinyle, pyridazinyle, quinolyle, isoquinolyle, pyrrolyle, indolyle, thiényle, furyle, imidazolyle, oxazolyle, thiazolyle, phényle, phénoxy, phénylthio, phénylsulfonyle, benzyloxy, benzylthio, benzyl-sulfonyle, phényléthoxy, phényléthylthio ou phényléthylsulfonyle, les restes hétéroaryle et aryle mentionnés pouvant être substitués jusqu'à 2 fois identiques ou différentes par du fluor, du chlore, du brome, un radical alkyle en C₁ à C₆, alkoxy en C₁ à C₆, trifluorométhyle ou trifluorométhoxy,
- un reste thiényle, furyle, thiazolyle, tétrazolyle, isothiazolyle, oxazolyle, isoxazolyle, pyridyle, pyrimidyle, pyrazinyle, pyridazinyle, indolyle, isoindolyle, quinolyle, isoquinolyle, phtalazinyle, quinoxalinyle, quinazolinyle, cinnolinyle, benzothiazolyle, benzoxazolyle ou benzimidazolyle, qui peut être substitué jusqu'à 2 fois identiques ou différentes par du fluor, du chlore, du brome, un radical alkyle en C₁ à C₆, alkoxy en C₁ à C₆, phényle, phénoxy, trifluorométhyle, trifluorométhoxy ou (alkoxy en C₁ à C₆)-carbonyle, ou bien
- un reste naphtyle qui peut être substitué jusqu'à 4 fois, identiques ou différentes, par un radical alkyle en C₁ à C₆ qui peut porter un substituant hydroxy ou alkoxy en C₁ à C₆, par un radical alkoxy en C₁ à C₆, alkylthio en C₁ à C₆, alkylsulfonyle en C₁ à C₆, phényle, phényloxy, phénylthio, phényl-sulfonyle, benzyle, benzyloxy, benzylthio, benzyl-sulfonyle, phénéthyle, phényléthoxy, phényléthylthio, phényléthylsulfonyle, fluoro, chloro, bromo, cyano, trifluorométhyle, trifluorométhoxy, trifluorométhyl-thio, (alkoxy en C₁ à C₆)-carbonyle ou par un groupe de formule -NR¹R²,
dans laquelle
R¹ et R² ont la définition indiquée ci-dessus,
ou bien
- un groupe de formule -CR¹¹R¹²-Y
dans laquelle
R¹¹ et R¹² peuvent être identiques ou différents et représentent
- l'hydrogène, ou bien
- un reste alkyle en C₁ à C₆ qui peut porter éventuellement un substituant hydroxy, fluoro, chloro, alkoxy en C₁ à C₆ ou (alkoxy en C₁ à C₆)-carbonyle, ou bien
- un reste cyclopropyle, cyclopentyle ou cyclohexyle, ou bien R¹¹ et R¹² forment conjointement un noyau carbocyclique ou hétérocyclique saturé ou non saturé ayant jusqu'à 6 atomes de carbone,
Y - représente un groupe de formule -NR¹³R¹⁴, -COR¹⁵, -S-R¹⁶, SO-R¹⁶, -SO₂R¹⁶, -OR¹⁷ ou -N₃,
dans lequel
R¹³ et R¹⁴ sont identiques ou différents et représentent
- l'hydrogène, un reste alkyle en C₁ à C₆, phényle ou benzyle, les restes mentionnés pouvant être substitués par du fluor, du chlore, du brome, un radical alkyle en C₁ à C₆, alkoxy en C₁ à C₆ ou trifluorométhyle,
ou bien
- un groupe de formule -COR¹⁵, -SO₂R¹⁶, ou bien R¹³ et R¹⁴ forment conjointement une chaîne alkylénique qui peut être interrompue par O, N, S, un groupe N-alkyle en C₁ à C₆, N-benzyle, N-phényle, N-carbamoyle ou N-(alkoxy en C₁ à C₆)-carbonyle,
R¹⁵ représente
- un groupe -NR¹⁸R¹⁹,
ou bien
- un reste alkyle en C₁ à C₆ ou alkoxy en C₁ à C₆,
ou bien
- un reste phényle, benzyle, benzyloxy, thiényle, furyle, pyridyle, pyrimidyle, quinolyle, isoquinolyle, benzothiazolyle, benzoxazolyle, thiazolyle, oxazolyle, isoxazolyle ou isothiazolyle éventuellement substitué par un radical alkyle en C₁ à C₆, alkoxy en C₁ à C₆, fluoro, chloro, bromo, trifluorométhyle, diméthylamino ou diéthylamino,
R¹⁶ désigne
- un reste cyclopropyle, cyclopentyle, cyclohexyle, ou
- un reste alkyle en C₁ à C₆ linéaire ou ramifié éventuellement substitué par un radical cyano, fluoro, chloro, bromo, trifluorométhyle, ou (alkoxy en C₁ à C₆)-carbonyle, ou bien
- un reste phényle, naphtyle, benzyle, thiényle, furyle, pyrimidyle, pyridyle, quinolyle, isoquinolyle, benzothiazolyle, benzoxazolyle, thiazolyle, oxazolyle, isoxazolyle, ou isothiazolyle portant éventuellement un ou plusieurs substituants, identiques ou différents, alkyle en C₁ à C₆, alkoxy en C₁ à C₆, fluoro, chloro, bromo, trifluorométhyle, diméthylamino ou diéthylamino, ou bien
- un groupe -NR⁹R¹⁰,
dans lequel
R⁹ et R¹⁰ ont la définition indiquée ci-dessus, R¹⁷ représente
- l'hydrogène, ou
- un reste cyclopropyle, cyclopentyle ou cyclohexyle, ou
- un reste alkyle en C₁ à C₆ qui peut être substitué par du fluor, du chlore, du brome, un radical cyano, alkoxy en C₁ à C₆, alkylthio en C₁ à C₆, alkylsulfonyle en C₁ à C₆, trifluorométhyle, trifluorométhoxy, trifluorométhylsulfonyle, (alkoxy en C₁ à C₆)-carbonyle, benzoyle, (alkyle en C₁ à C₆)-carbonyle, par un groupe de formule -NR¹R²,
dans laquelle
R¹ et R² ont la définition indiquée ci-dessus,
ou par un reste pyridyle, pyrimidyle, pyrazinyle, pyridazinyle, quinolyle, isoquinolyle, pyrrolyle, indolyle, thiényle, furyle, imidazolyle, oxazolyle, thiazolyle, phényle, phénoxy, phénylthio, phénylsulfonyle, benzyloxy, bénzylthio, benzylsulfonyle, phényléthoxy, phényléthylthio ou phényléthylsulfonyle, les restes hétéroaryle et aryle mentionnés pouvant porter jusqu'à 2 substituants, identiques ou différents, fluoro, chloro, bromo, alkyle en C₁ à C₆, alkoxy en C₁ à C₆, trifluorométhyle ou trifluorométhoxy,
- un reste thiényle, furyle, thiazolyle, isothiazolyle, oxazolyle, isoxazolyle, pyridyle, pyrimidyle, pyrazinyle, pyridazinyle, indolyle, isoindolyle, quinolyle, isoquinolyle, phtalazinyle, quinoxalinyle, quinazolinyle, cinnolinyle, benzothiazolyle, benzoxazolyle ou benzimidazolyle, qui peut être substitué jusqu'à deux fois identiques ou différentes par du fluor, du chlore, du brome, un radical alkyle en C₁ à C₆, alkoxy en C₁ à C₆, phényle, phénoxy, trifluorométhyle, trifluorométhoxy ou (alkoxy en C₁ à C₆)-carbonyle, ou bien
- un reste benzyle, un reste phényle ou un reste naphtyle, qui peuvent être substitués jusqu'à 4 fois identiques ou différentes par un radical alkyle en C₁ à C₆, alkoxy en C₁ à C₆, alkylthio en C₁ à C₆, alkylsulfonyle en C₁ à C₆, phényle, phényloxy, phénylthio, phénylsulfonyle, benzyle, benzyloxy, benzylthio, benzyl-sulfonyle, phénéthyle, phényléthoxy, phényléthylthio, phényléthylsulfonyle, fluoro, chloro, bromo, cyano, trifluorométhyle, trifluorométhoxy, trifluorométhylthio, (alkoxy en C₁ à C₆)-carbonyle ou par un groupe de formule -NR¹R²,
dans laquelle
R¹ et R² ont la définition indiquée ci-dessus,
ou bien
- le reste 2,5-dioxo-tétrahydropyrryle,
- le reste tétrahydropyrannyle, ou bien
- le reste diméthyl-tertio-butylsilyle, tripropylsilyle ou tributylsilyle, ou bien
- un groupe de formule COR¹⁶,
dans laquelle
R¹⁶ a la définition indiquée ci-dessus,
et
R¹⁸ et R¹⁹ sont identiques ou différents et représentent
- l'hydrogène, ou bien
- un reste alkyle en C₁ à C₆ éventuellement substitué par un radical cyano, fluoro, chloro
ou bromo, ou bien
- un reste phényle, benzyle, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en C₁ à C₆, alkoxy en C₁ à C₆, fluoro, chloro, bromo, trifluorométhyle, diméthylamino ou diéthylamino,
ou bien
D, E forment ensemble un noyau de formule 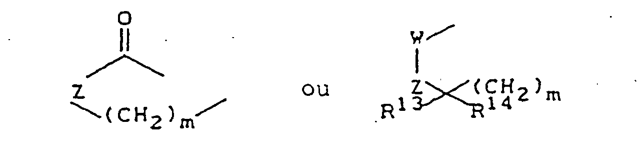 dans laquelle
W - est un groupe de formule C=O ou CH-OH,
m - est le nombre 1 ou 2,
Z - représente O, CH₂ ou NHR²⁰,
R¹³ et R¹⁴ ont la définition indiquée ci-dessus, et
R²⁰ représente
- l'hydrogène, un reste alkyle en C₁ à C₆, phényle, benzyle, carbamoyle ou (alkoxy en C₁ à C₆)-carbonyle,
X désigne un groupe de formule -CH=CH-,
R est un groupe de formule 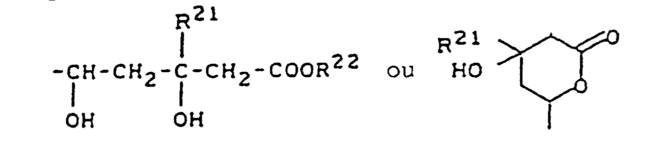 dans laquelle
R²¹ représente l'hydrogène ou un reste alkyle en C₁ à C₆,
et
R²² représente
- l'hydrogène, un reste alkyle en C₁ à C₆, phényle ou benzyle, ou bien
- un cation,
ainsi que de leurs produits d'oxydation,
**caractérisé en ce qu'**on réduit des cétones de formule générale (VIIIa, b) 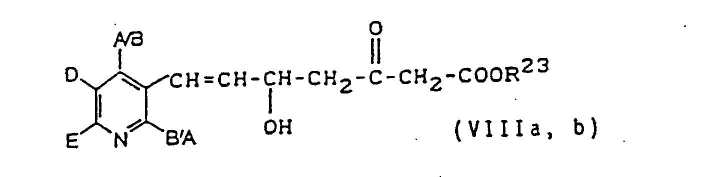 dans laquelle
A, B, D, E ont la définition indiquée et
R²³ est un reste alkyle en C₁ à C₆,
dans le cas de la production des acides, on saponifie les esters,
dans le cas de la production des lactones, on cyclise les acides carboxyliques,
dans le cas de la production des sels, on saponifie les esters ou les lactones.

12. Procédé suivant la revendication 11 pour la production de pyridines substituées de formule (Ia) ou (Ib)
dans laquelle
A
- représente un reste thiényle, furyle, pyridyle, pyrimidyle, quinolyle ou isoquinolyle qui peut être substitué par un radical fluoro, chloro, méthyle, méthoxy ou trifluorométhyle, ou bien
- un reste phényle qui. peut être substitué jusqu'à 3 fois identiques ou différentes par un radical méthyle, hydroxyméthyle, éthyle, propyle, isopropyle, hydroxyéthyle, hydroxypropyle, butyle, isobutyle, méthoxyméthyle, éthoxyméthyle, propoxyméthyle, tertio-butyle, méthoxy, éthoxy, propoxy, isopropoxy, butoxy, isobutoxy, tertio-butoxy, méthylthio, éthylthio, propylthio, isopropylthio, méthyl-sulfonyle, éthylsulfonyle, propylsulfonyle, isopropylsulfonyle, phényle, phénoxy, benzyle, benzyloxy, fluoro, chloro, bromo, cyano, trifluorométhyle, trifluorométhoxy, méthoxycarbonyle, éthoxycarbonyle, propoxycarbonyle, isopropoxycarbonyle, butoxycarbonyle, isobutoxycarbonyle ou tertio-butoxycarbonyle,
- un reste méthyle, éthyle, propyle, isopropyle, butyle
ou tertio-butyle,
B représente
- un reste cyclopropyle, cyclopentyle ou cyclohexyle, ou bien
- un reste méthyle, éthyle, propyle, isopropyle, butyle, sec.-butyle ou tertio-butyle qui peut être substitué par un radical fluoro, chloro, bromo, cyano, méthoxy, éthoxy, propoxy, isopropoxy, butoxy, sec.-butoxy, tertio-butoxy, méthylthio, éthylthio, propylthio, isopropylthio, méthylsulfonyle, éthylsulfonyle, propylsulfonyle, isopropylsulfonyle, trifluorométhyle, trifluorométhoxy, méthoxycarbonyle, éthoxycarbonyle, butoxycarbonyle, isobutoxycarbonyle, tertio-butoxycarbonyle, benzoyle, acétyle, pyridyle, pyrimidyle, thiényle, furyle, phényle, phénoxy, phénylthio, phénylsulfonyle, benzyloxy, benzylthio ou benzylsulfonyle,
D,E sont identiques ou différents et représentent
- un reste cyclopropyle, cyclopentyle ou cyclohexyle, ou bien
- un reste méthyle, éthyle, propyle, isopropyle, butyle, isobutyle, tertio-butyle, pentyle, isopentyle, hexyle ou isohexyle, ces restes étant substitués dans le cas du substituant D et pouvant être substitués dans le cas du substituant E, par un radical azido, fluoro, chloro, bromo, iodo, cyano, hydroxy, méthoxy, éthoxy, propoxy, isopropoxy, butoxy, isobutoxy, tertio-butoxy, méthylthio, éthylthio, propylthio, isopropylthio, butylthio, isobutylthio, tertio-butylthio, méthylsulfonyle, éthylsulfonyle, propylsulfonyle, isopropylsulfonyle, butylsulfonyle, isobutylsulfonyle, tertio-butylsulfonyle, trifluorométhyle, trifluorométhoxy, méthoxycarbonyle, éthoxycarbonyle, propoxycarbonyle, isopropoxycarbonyle, butoxycarbonyle, isobutoxycarbonyle, tertio-butoxycarbonyle, benzoyle, acétyle, éthylcarbonyle, ou par un groupe -NR¹R²,
dans lequel
R¹ et R² sont identiques ou différents et représentent un reste méthyle, éthyle, propyle, isopropyle, butyle, isobutyle, tertio-butyle, phényle, benzyle, acétyle, méthylsulfonyle, éthylsulfonyle, propylsulfonyle, isopropylsulfonyle ou phénylsulfonyle,
ou par un reste pyridyle, pyrimidyle, pyrazinyle, pyridazinyle, quinolyle, isoquinolyle, thiényle, furyle, phényle, phénoxy, phénylthio, phényl-sulfonyle, benzyloxy, benzylthio ou benzylsulfonyle, les restes hétéroaryle et aryle mentionnés pouvant être substitués par un radical fluoro, chloro, méthyle, éthyle, propyle, isopropyle, isobutyle, tertio-butyle, méthoxy, éthoxy, propoxy, isopropoxy, butoxy, isobutoxy, tertio-butoxy, trifluorométhyle ou trifluorométhoxy, ou bien
- un reste thiényle, furyle, pyridyle, pyrimidyle, pyrazinyle, tétrazolyle, pyridazinyle, oxazolyle, isooxazolyle, imidazolyle, pyrazolyle, thiazolyle, isothiazolyle, quinolyle, isoquinolyle, benzoxazolyle, benzimidazolyle ou benzothiazolyle, les restes mentionnés pouvant être substitués par du fluor, du chlore, un radical méthyle, éthyle, propyle, isopropyle, butyle, isobutyle, tertio-butyle, méthoxy, éthoxy, propoxy, isopropoxy, butoxy, isobutoxy, tertio-butoxy, phényle, phénoxy, trifluorométhyle, trifluorométhoxy, méthoxycarbonyle, éthoxycarbonyle, isopropoxycarbonyle, propoxycarbonyle, butoxycarbonyle, isobutoxycarbonyle ou tertio-butoxycarbonyle, ou bien
- un groupe de formule -CR¹¹R¹²-Y
dans laquelle
R¹¹ et R¹² sont identiques ou différents et représentent
- l'hydrogène, ou
- un reste méthyle, éthyle, propyle, isopropyle, qui peut être substitué éventuellement par un radical hydroxy, fluoro, chloro, méthoxy, éthoxy, méthoxy-carbonyle, éthoxycarbonyle, ou bien
- un reste cyclopropyle, cyclopentyle ou cyclohexyle,
ou bien R¹¹ et R¹² forment ensemble un reste cyclopropyle, cyclopentyle ou cyclohexyle, et
Y désigne un groupe de formule -NR¹³R¹⁴, -COR¹⁵, -SR¹⁶, -SO-R¹⁶, -SO₂-R¹⁶, -OR¹⁷ ou -N₃,
dans laquelle
R¹³ et R¹⁴ sont identiques ou différents et représentent
- l'hydrogène, un reste méthyle, éthyle, propyle, isopropyle, butyle, isobutyle, ou bien
- un reste phényle éventuellement substitué par un radical fluoro, chloro, méthyle ou méthoxy,
ou bien
- un groupe -COR¹⁵ ou -SO₂R¹⁶, ou bien R¹³ et R¹⁴ forment conjointement avec l'atome d'azote un noyau de la série pipéridine, pipérazine, morpholine, morpholine-N-oxyde, N-(alkyle inférieur)-pipérazine, benzylpipérazine
ou phénylpipérazine,
R¹⁵ représente
- l'hydrogène, ou
- un groupe -NR¹⁸R¹⁹, ou bien
- un reste méthyle, éthyle, propyle, isopropyle, méthoxy, éthoxy, propoxy, isopropoxy, ou bien
- un reste phényle, benzyle, benzyloxy, thiényle, furyle, pyridyle, pyrimidyle, quinolyle ou isoquinolyle portant éventuellement un substituant méthyle, méthoxy, fluoro ou chloro,
R¹⁶ désigne
- un reste méthyle, éthyle, propyle, isopropyle, butyle, isobutyle ou isopentyle éventuellement substitué par un radical fluoro, chloro, méthoxycarbonyle, éthoxycarbonyle, propoxycarbonyle, isopropoxycarbonyle, butoxycarbonyle ou isobutoxycarbonyle, ou bien
- un reste benzyle, phényle, naphtyle, thiényle, furyle, pyridyle, pyrimidyle, quinolyle ou isoquinolyle éventuellement substitué une ou plusieurs fois identiques ou différentes par un radical méthyle, éthyle, propyle, isopropyle, méthoxy, fluoro ou chloro, ou bien
- un reste triméthylsilyle ou diméthyléthyl-silyle,
- un groupe -NR⁹R¹⁰,
dans lequel
R⁹ et R¹⁰ ont la définition indiquée ci-dessus,
R¹⁷ désigne
- l'hydrogène, un reste cyclopropyle, cyclopentyle ou cyclohexyle, ou bien
- un reste méthyle, éthyle, propyle, isopropyle, butyle, isobutyle, tertio-butyle, pentyle, isopentyle, hexyle ou isohexyle, qui peut être substitué par un radical fluoro, chloro, bromo, cyano, méthoxy, éthoxy, propoxy, isopropoxy, butoxy, isobutoxy, tertio-butoxy, méthylthio, éthylthio, propylthio, isopropylthio, butylthio, isobutylthio, tertio-butylthio, méthylsulfonyle, éthylsulfonyle, propylsulfonyle, isopropylsulfonyle, butylsulfonyle, isobutylsulfonyle, tertio-butylsulfonyle, trifluorométhyle, trifluorométhoxy, méthoxycarbonyle, éthoxycarbonyle, propoxycarbonyle, isopropoxycarbonyle, butoxycarbonyle, isobutoxycarbonyle, tertio-butoxycarbonyle, benzoyle, acétyle, éthylcarbonyle, ou par un groupe -NR¹R²,
dans lequel
R¹ et R² sont identiques ou différents et représentent un radical méthyle, éthyle, propyle, isopropyle, butyle, isobutyle, tertio-butyle, phényle, benzyle, acétyle, méthylsulfonyle, éthylsulfonyle, propylsulfonyle, isopropylsulfonyle ou phénylsulfonyle,
ou par un reste pyridyle, pyrimidyle, pyrazinyle, pyridazinyle, quinolyle, isoquinolyle, thiényle, furyle, phényle, phénoxy, phénylthio, phénylsulfonyle, benzyloxy, benzylthio ou benzylsulfonyle, les restes hétéroaryle et aryle mentionnés pouvant être substitués par un radical fluoro, chloro, méthyle, éthyle, propyle, isopropyle, isobutyle, tertio-butyle, méthoxy, éthoxy, propoxy, isopropoxy, butoxy, isobutoxy, tertio-butoxy, trifluorométhyle ou trifluorométhoxy,
ou bien
- un reste thiényle, furyle, pyridyle, pyrimidyle, pyrazinyle, pyridazinyle, oxazolyle, isooxazolyle, imidazolyle, pyrazolyle, thiazolyle, isothiazolyle, quinolyle, isoquinolyle, benzoxazolyle, benzimidazolyle ou benzothiazolyle, les restes mentionnés pouvant être substitués par un radical fluoro, chloro, méthyle, éthyle, propyle, isopropyle, butyle, isobutyle, tertio-butyle, méthoxy, éthoxy, propoxy, isopropoxy, butoxy, isobutoxy, tertio-butoxy, phényle, phénoxy, trifluorométhyle, trifluorométhoxy, méthoxycarbonyle, éthoxycarbonyle, isopropoxycarbonyle, propoxycarbonyle, butoxycarbonyle, isobutoxycarbonyle ou tertio-butoxycarbonyle,
ou bien
- un reste benzyle ou phényle qui peut être substitué jusqu'à 3 fois identiques ou différentes par un radical méthyle, éthyle, propyle, isopropyle, butyle, isobutyle, tertio-butyle, pentyle, isopentyle, hexyle, isohexyle, méthoxy, éthoxy, propoxy, isopropoxy, butoxy, isobutoxy, tertio-butoxy, méthylthio, éthylthio, propylthio, isopropylthio, butylthio, isobutylthio, tertio-butylthio, méthyl-sulfonyle, éthylsulfonyle, propylsulfonyle, isopropylsulfonyle, butylsulfonyle, isobutylsulfonyle, tertio-butylsulfonyle, phényle, phénoxy, phénylthio, phénylsulfonyle, benzyle, benzyloxy, benzylthio, benzylsulfonyle, fluoro, chloro, bromo, cyano, trifluorométhyle, trifluorométhoxy, trifluorométhylthio, méthoxy-carbonyle, éthoxycarbonyle, propoxycarbonyle, isopropoxycarbonyle, butoxycarbonyle, isobutoxycarbonyle, tertio-butoxycarbonyle, ou par un groupe -NR¹R²,
dans lequel
R¹ et R² ont la définition indiquée ci-dessus, ou bien
- le reste 2,5-dioxo-tétrahydropyrryle,
- le reste tétrahydropyrannyle, ou bien
- le reste diméthyl-tertio-butylsilyle ou triméthylsilyle, ou bien
- un groupe -COR¹⁶,
dans lequel
R¹⁶ a la définition indiquée ci-dessus,
et
R¹⁸ et R¹⁹ sont identiques ou différents et représentent
- l'hydrogène, ou bien
- un reste méthyle, éthyle, propyle, isopropyle, butyle, isobutyle, pentyle, isopentyle, hexyle
ou isohexyle éventuellement substitué par du fluor ou du chlore, ou bien
- un reste phényle qui peut être substitué par-un radical fluoro, chloro, méthyle ou méthoxy,
ou bien
D et E forment ensemble un noyau de formule 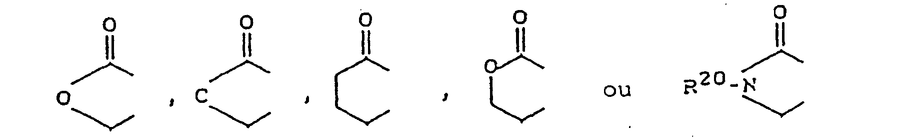 dans laquelle
R²⁰ représente l'hydrogène, un reste méthyle, éthyle, propyle, isopropyle, carbamoyle, méthoxycarbonyle ou éthoxycarbonyle,
X - est un groupe de formule -CH=CH-,
R - est un groupe de formule 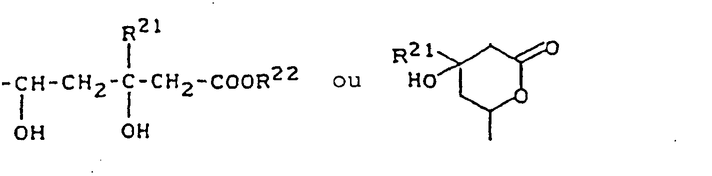 dans laquelle
R²¹ désigne l'hydrogène, un reste méthyle, éthyle, propyle, isopropyle, butyle, isobutyle ou tertio-butyle,
et
R²² est l'hydrogène, un reste méthyle, éthyle, propyle, isopropyle, butyle, isobutyle, tertio-butyle ou benzyle, ou un ion sodium, potassium, calcium ou magnésium ou un ion ammonium,
ainsi que de leurs produits d'oxydation.

13. Procédé suivant la revendication 11 pour la production de l'érythro-(E)-7-[2,6-diisopropyl-4-(4-fluorophényl)-5-méthoxy-méthyl-pyrid-3-yl]-3,5-dihydroxy-hept-6-énoate de méthyle de formule

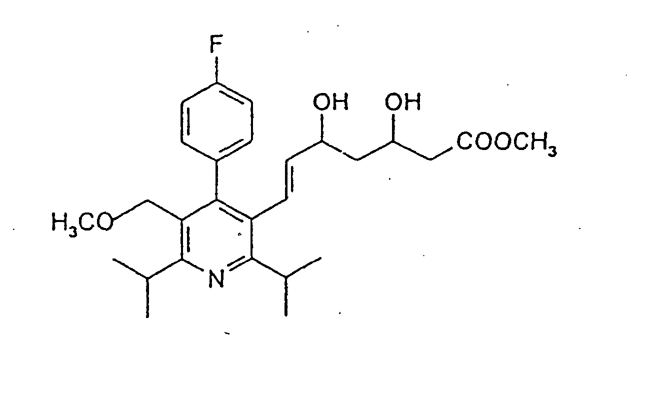

14. Procédé suivant l'une des revendications 11 à 13, **caractérisé en ce qu'**on conduit la réduction dans la plage de températures de -80 °C à 30 °C.

15. Procédé de production des sels des composés de formule générale (Ie) ou (le)' 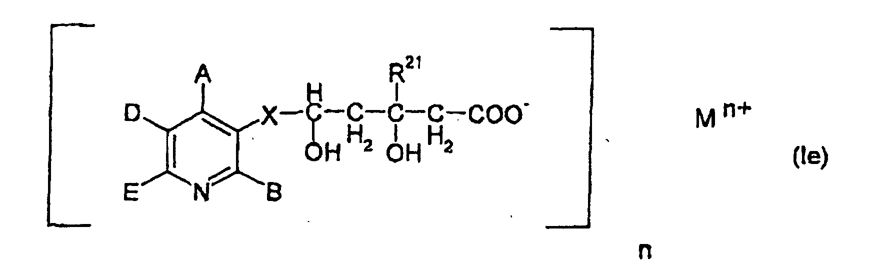 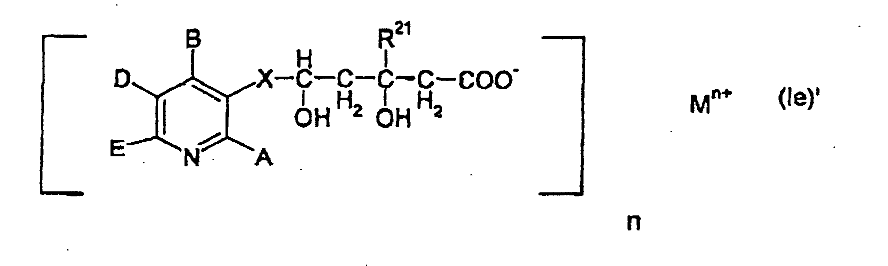 formule dans laquelle
A, B, D, E, X et R²¹ ont les définitions indiquées pour les
composés des revendications 11 à 14 précédentes et
Mⁿ⁺ désigne un cation, n indiquant la valence,
**caractérisé en ce qu'**on traite avec des bases usuelles les esters ou lactones correspondants dans des solvants inertes.

16. Procédé suivant la revendication 15, dans lequel on utilise comme base l'hydroxyde de sodium ou l'hydroxyde de potassium.

17. Procédé de production d'acides carboxyliques de formule générale (Ic) ou (Ic)' 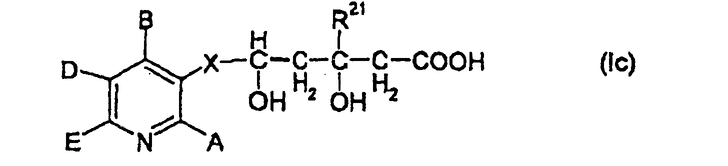 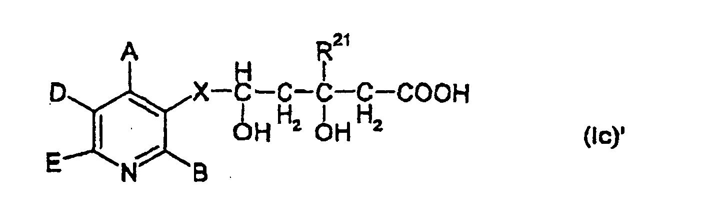 formule dans laquelle
A, B, D, E, X et R²¹ ont les définitions indiquées pour les composés des revendications 12 à 15 précédentes, **caractérisé en ce que**, dans une première étape, on traite avec des bases usuelles les esters ou lactones correspondants dans des solvants inertes puis, dans une seconde étape, on transforme les sels formés en acides libres, par traitement avec des acides.
